# EUROPEAN PATENT APPLICATION

(11) **EP 3 858 332 A1**
(43) Date of publication of application: **04.08.2021**
(21) Application number: 20155012.6
(22) Date of filing: 31.01.2020
(51) Int. Cl.: A61K 9/127, A61K 47/69

(54) **BOTTOM-UP ASSEMBLY OF SYNTHETIC EXTRACELLULAR VESICLES**

(71) Applicant: Max-Planck-Gesellschaft zur Förderung der Wissenschaften e.V., 80539 München (DE)
(72) Inventor: STAUFER, Oskar, 68239 Mannheim (DE); PLAZMAN, Yilia, 70195 Stuttgart (DE); SPATZ, Joachim P., 70569 Stuttgart (DE)
(74) Representative: Arth, Hans-Lothar

(57) **Abstract**

The present invention relates to a method for producing synthetic extracellular vesicles comprising a lipid bilayer including at least two lipids, optionally one or more extracellular vesicle associated proteins, and optionally one or more nucleic acid molecules. The inventive synthetic extracellular vesicles are formed by emulsification using a mechanic emulsifier in the form of polymer shell stabilized synthetic extracellular vesicles. The inventive method allows producing synthetic extracellular vesicles miming the composition and function of natural extracellular vesicles. Therefore, synthetic extracellular vesicles with specific protein and nucleic acids compositions are also disclosed herein, as well as their therapeutic uses.

## Description

The present invention relates to a method for producing synthetic extracellular vesicles comprising a lipid bilayer including at least two lipids, optionally one or more extracellular vesicle associated proteins, and optionally one or more nucleic acid molecules. The inventive synthetic extracellular vesicles are formed by emulsification using a mechanic emulsifier in the form of polymer shell stabilized synthetic extracellular vesicles. The inventive method allows producing synthetic extracellular vesicles miming the composition and function of natural extracellular vesicles. Therefore, synthetic extracellular vesicles with specific protein and nucleic acids composition are also disclosed herein, as well as their therapeutic uses.

### Background of the invention

Extracellular vesicles are membrane-contained small vesicles, secreted by all types of pro- and eukaryotic cells, and play a crucial role in intercellular signaling under both physiological and pathological conditions.

In physiological conditions, extracellular vesicles are important mediators for cell-to-cell and inter-tissue communication, thus playing a role in regulating homeostasis as well as other conditions. In pathological conditions, the information transferred by extracellular vesicles, mainly cancer cell extracellular vesicles, may have detrimental effects. Indeed, extracellular vesicles have been demonstrated to contribute to various pathologies such as tumorigenesis and metastasis, inflammation, and immune system activation.

As a result of the above-mentioned functions, extracellular vesicles may serve as novel tools for various therapeutic and diagnostic applications, such as anti-tumour therapy, pathogen vaccination, immune-modulatory and regenerative therapies and drug delivery. Indeed, extracellular vesicles can be used for the target-specific delivery of nucleic acids, proteins or small molecules into the intracellular environment, where they also act on a genetic level.

The three main categories of extracellular vesicles are apoptotic bodies, shedding microvesicles and exosomes. Microvesicles and exosomes are smaller compared to apoptotic bodies. Additionally they differ from apoptotic bodies in their content, since they rarely contain DNA.

The main function of microvesicles and exosomes is the intercellular transfer of lipids, RNA, and cytosolic proteins, thereby affecting cell metabolism and functions, including, but not limited to migration, cell proliferation and differentiation.

A detailed and precise characterization of the intercellular signalling mechanisms mediated by extracellular vesicles is essential to develop extracellular vesicle-based therapeutic applications. However, the methods of the current art to isolate and purify extracellular vesicles are very complex, long and error prone providing extracellular vesicle preparations with low yield and purity and high variability between different batches, which hamper a correct understanding of extracellular vesicle biology and of their interactions with the environment. Moreover, the exosome preparations oft contain also microvesicles.

Therefore synthetic and cleaner vesicle formulations are not only highly sought for therapeutic and clinical applications but also to study fundamental aspects of extracellular vesicle biology, signalling, as well as the role of their individual components.

Currently, synthetic exosomes are prepared by two types of methodologies: top-down or bottom-up (Garcia-Manrique P. et al., 2017. Fully Artificial Exosomes: Towards New Theranostic Biomaterials. Trends in Biotechnology). In top-down methodologies, the production of artificial exosomes begins with cultured and eventually engineered cells that are then processed to produce membrane fragments to be used to form the vesicles. Even if these methods enable synthesis of exosomes similarly to their natural counterpart, they still have some drawbacks. Indeed cargo loading is not tightly controlled due to the passive encapsulation of the surrounding medium during membrane fragment self-assembly. Moreover, the final purification steps are identical to those used for exosome isolation, which are time-consuming and characterized by low purity and yield.

The international patent application WO 2019/027847 describes the synthesis of bispecific nanoparticle vesicles that are able to redirect immune effector cells towards cancer cells for killing. However, the nanoparticle vesicles are prepared by transducing a population of cells comprising vesicles, such as exosomes, with polynucleotides coding the polypeptides of interest, and thus isolating the transduced vesicles or exosomes. The exosomes released into the culture media are purified using traditional approaches as differential centrifugation, density-gradient- or cushion-based ultracentifugation, precipitation with commercial kits, and affinity and size exclusion chromatography. Thus, the extracellular vesicles or exosomes described in WO 2019/027847 do not possess a membrane bilayer with a defined lipid composition. Moreover, the vesicle preparation can still contain impurities due to the isolation procedure.

Bottom-up methodologies to prepare synthetic exosomes involves the preparation of a synthetic bilayer that is then functionalized with selected proteins to mimic desired exosome functions. However, most of these methodologies are characterized by low encapsulation efficiency and high costs, as the methods are adapted from conventional liposome production routes. Moreover, methodologies to synthetize exosomes containing a specific composition of both exosome proteins and nucleic acids such as miRNAs are still missing.

Therefore there is still an urgent need for efficient procedures to produce fully synthetic exosomes, or extracellular vesicles with a high defined composition, low variability between different batches, high purity and efficiency.

It is the objective of the present invention to provide synthetic extracellular vesicles assembled with a highly controlled composition and produced surrounded by a stabilizing polymer shell, which can be used for therapeutic applications.

The objective of the present invention is solved by the teaching of the independent claims. Further advantageous features, aspects and details of the invention are evident from the dependent claims, the description, the figures, and the examples of the present application.

### Brief description of the invention

The present invention provides a method for high-throughput bottom-up assembly of fully synthetic extracellular vesicles with analogous functionalities to naturally occurring cell-derived extracellular vesicles. The production method is based on charge-mediated assembly of predefined functionalized lipid vesicles and encapsulation of miRNAs within the polymer shell stabilized lipid vesicles. "Charge mediated" assembly refers to the process in which the negative charge of the vesicles and the negative charge on the periphery of the polimer shell stabilized vesicles are complexed by the cations, such as for example Mg²⁺ cations. Following their release into an aqueous environment, the respective protein-functionalized synthetic extracellular vesicles can interact with target cells thus influencing their functions, such as metabolism, proliferation, or growth.
The synthetic extracellular vesicles obtained by the highly controlled droplet - stabilized assembly provide a robust platform for therapeutic applications and moreover allow getting new insights into fundamental functioning-principles of extracellular vesicles.

In comparison with the prior art methods, a first advantage of the invention is to provide extracellular vesicles with high stability and high controlled composition due to the assembly in stabilizing polymer shell surrounded vesicles. The composition of the extracellular vesicles can be adjusted in term of lipid type and charge, lipid ratio, protein to lipid ratio, nucleic acid content.

The assembly in polymer shell stabilized vesicles allows also encapsulation of nucleic acids at high efficiency, which is very hard to obtain with the current methods.

Moreover, the inventive method allows adjusting the vesicle dimensions by regulating the emulsification speed. The method also allows obtaining extracellular vesicles preparations with high purity and reproducibility between different batches.

Moreover the inventive method allows design and assembly of fully synthetic extracellular vesicles by a polymer shell-stabilized approach that hold a therapeutic potential as their laboriously isolated natural analogues, so that they can be used in a multitude of clinical settings.

Moreover the inventive method for bottom-up assembly of extracellular vesicles, allows controlling the quantity of each individual extracellular vesicle components, which is an essential aspect for therapeutic applications, and also to decipher their roles on disease related states, representing an essential advantage in comparison with natural exosomes.

Non-limiting examples of the extracellular vesicle types are from the group of vesicles that include an exosome, a microvesicle, an apoptotic vesicle, and a liposome.

In particular, the present invention is directed to a method for producing synthetic extracellular vesicles comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for conjugation to an extracellular vesicle associated protein;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
wherein the amphiphilic copolymer forms a polymer shell stabilizing the synthetic extracellular vesicle, and
wherein the the synthetic extracellular vesicles have a diameter comprised between 70 nm and 5000 nm.

According to an aspect of the present invention, the method further comprises after step d) the following steps:
d') removing the polymer shell from the polymer shell-stabilized synthetic extracellular vesicles obtained in step d);
e) purifying the synthetic extracellular vesicles by centrifugation.

According to a particular aspect of the present invention, the step d') comprises removing the polymer shell from the polymer shell-stabilized synthetic extracellular vesicles obtained in step d) by adding a destabilizing agent.

According to a more particular aspect of the present invention, the water phase of step a) comprises at least one lipid coupled to a functional ligand selected from biotin, N-hydroxysuccinimide ester, N-Hydroxysulfosuccinimide, nitrilotriacetic acid-nickel, amine, pyridyl disulfide , pyridyldithiopropionate, carboxylic acid, maleimide, aromatic maleimid, dithiopyridinyl, N-benzylguanine, cyanuric chloride, thiol and
wherein the method optionally comprises after step e) the following step:
f) coupling the synthetic extracellular vesicles with at least one macromolecule comprising at least one moiety reacting with one of said functional ligands, wherein the macromolecule is selected from the group comprising an extracellular vesicle associated protein, or a fragment thereof, a carbohydrate, a nucleic acid, a polypeptide, a cell receptor, an imaging probe.

According to a still more particular aspect of the present invention, the water phase of step a) comprises one or more nucleic acid molecules selected from the group comprising miRNA molecules miR-17, miR-18a, miR-19a, miR-19b-1, miR-20a, miR-92a; miR-21, miR-30d-5p, miR-33b, miR-124, miR-125, miR-126, miR-130, miR-132, miR-133b, miR-140-5p, miR-191, miR-222, miR-451, miR-494, miR-575, miR-630, miR-638, miR-1202, miR-1207-5p, miR-1225-5p, miR-1268, miR-6087, miR-92a-3p-e, miR-K12-3, let-7a.

Further miRNA molecules suitable for the method and the synthetic extracellular vesicles disclosed herein are listed in Table 3.

According to a still more particular aspect of the present invention, the extracellular vesicle associated protein, or a fragment thereof, is selected from the group comprising:
a transmembrane protein selected from the group comprising tetraspanin proteins CD9, CD37, CD47, CD53, CD63, CD81, CD82, CD151, Tspan8, heterotrimeric G protein subunit alpha, integrin α - chains and β-chains, transferrin receptor 1, transferrin receptor 2, lysosome associated membrane proteins, heparan sulfate proteoglycans, syndecans, extracellular matrix metalloproteinase inducer, A Disintegrin And Metalloproteinase Domain 10, CD3, CD11c, CD14, CD29, CD31, CD41, CD42a, CD44, CD45, CD50 or intercellular adhesion molecule 1, CD55, CD59, CD73, CD80, CD86, CD90, sonic hedgehog, major histocompatibility complex I, major histocompatibility complex II, epidermal growth factor receptor 2, epithelial cell adhesion molecule, glycophorin A, Acetylcholinesterase S and E, amyloid beta precursor protein, multidrug resistance-associated protein 1, stem cells antigen-1, or a fragment thereof;
a cytosolic protein selected from the group comprising the protein complexes endosomal sorting complexes required for transport I, II and III, tumour susceptibility gene 101, charged multivesicular body protein, Apoptosis-Linked Gene 2-Interacting Protein X, vacuolar protein sorting 4 homolog A 4A and 4B, arrestin domain-containing protein, flotillin-1, flotillin-2; caveolins, EH-domain containing 1 - 4, ras homolog family member A, annexins, heat shock proteins, ADP-ribosylation factor 6, syntenin, microtubule-associated protein Tau, or a fragment thereof;
a functional protein selected from the group comprising cytokines, growth factors, interleukins, milk fat globule-EGF factor 8 protein , adhesion proteins, extracellular matrix proteins, nicotinamide phosphoribosyltransferase, signal transduction proteins, Wnta and Wntb, Fas, Fas Ligand, RANK, RANK Ligand, indolamin-2,3-dioxygenase, cytotoxic T-lymphocyte-associated protein 4 - immunoglobulin fusion protein, tumor necrosis factor-related apoptosis-inducing ligand, or a fragment thereof;
a protein associated to intracellular compartments selected from the group comprising histone proteins, lamin A/C, inner membrane mitochondrial protein, cytochrome C-1, mitochondrial import receptor subunit TOM20, calnexin, heat shock protein 90kDa beta, member 1, heat shock 70kDa protein 5, Golgin A2, Autophagy Related 9A, actinin1, actinin4, cytokeratin 18, or a fragment thereof.

Further extracellular vesicle associated proteins suitable for the method and the synthetic extracellular vesicles disclosed herein are listed in Table 4.

According to a still more particular aspect of the present invention, the water phase of step a) comprises at least two lipids selected from the group comprising:
a neutral lipid selected from the group comprising ceramide, sphingomyelin, cephalin, cholesterol, cerebrosides, diacylglycerols, phosphatidylcholines, lysophosphatidylcholines, phosphatidylethanolamines, lysophosphatidylethanolamine, lysoethanolamines, inverted headgroup lipids, sphingosins, sterol-modified phospholipids, ether ester lipids, diether lipids, vinyl ether (plasmalogen);
an anionic lipid selected from the group comprising phosphatidic acids, lysophosphatidic acid derivatives, phosphatidylglycerols, lysophosphatidylglycerols, phosphatidylserines, lysophosphatidylserines, phosphatidylinositols, phosphatidylinositolphosphates, cardiolipins, Bis(Monoacylglycero)Phosphate derivatives;
a cationic lipid selected from the group comprising dioleyl-N,N-dimethylammonium chloride; N-(2,3-dioleyloxy)propyl)-N,N,N-trimethylammonium chloride; N,N-distearyl-N,N-dimethylammonium bromide; N-(2,3- dioleyloxy)propyl)-N,N,N-trimethylammonium chloride; 3β-(N-(N',N'-dimethylaminoethane)-carbamoyl)cholesterol; 1,2-dimyristyloxypropyl-3-dimethyl-hydroxy ethyl ammonium bromide; 2,3-dioleyloxy-N-[2(sperminecarboxamido) ethyl]-N,N-dimethyl-1-propanaminium trifluoroacetate; dioctadecylamidoglycyl carboxyspermine; N-(2,3-dioleyloxy)propyl)-N,N-dimethylammonium chloride and 1,2-Dioleoyl-3-dimethylammonium-propane;
a pH-sensitive lipid selected from the group comprising lipid N-(4-carboxybenzyl)-N,N-dimethyl-2,3-bis(oleoyloxy)propan-1-aminium, 1,2-distearoyl-3-dimethylammonium-propane, 1,2-dipalmitoyl-sn-glycero-3-succinate, 1,2-dioleoyl-sn-glycero-3-succinate, N-palmitoyl homocysteine ;
a photoswitchable lipid;
acylglycine derivatives, prenol derivatives, prostaglandine derivatives, glycosylated diacyl glycerols, eicosanoid derivatives, (palmitoyloxy)octadecanoic acid derivatives, diacetylene derivatives, diphytanoyl derivatives, fluorinated lipids, brominated lipids, lipopolysaccharides;
one of the aforementioned lipids coupled to a functional ligand selected from the group comprising biotin, N-hydroxysuccinimide ester, nitrilotriacetic acid - nickel, amine, carboxylic acid, maleimides, dithiopyridinyl, pyridyl disulfide , pyridyldithiopropionate, N-benzylguanine, carboxyacyl, cyanur, folate, square, galloyl, glycan, thiol, arginylglycylaspartic acid, a fluorescent dye molecule, a magnetic resonance imaging reagent, a chelator;
one of the aforementioned lipids coupled to polyethyleneglycol with a molecular weight comprised between 350 and 50,000 g/mole.

According to a further aspect of the present invention, step d) comprises producing polymer shell stabilized synthetic extracellular vesicles by emulsifying the combined phases at step c) using a mechanic or electronic emulsifier for at least 5 seconds at speed higher than 1,000 rpm.

A preferred embodiment of the present invention is directed to a synthetic extracellular vesicle having a diameter comprised between 70 and 5000 nm, comprising:
a lipid bilayer comprising at least two lipids selected from the group comprising:
   a neutral lipid selected from the group comprising ceramide, sphingomyelin, cephalin, cholesterol, cerebrosides, diacylglycerols, phosphatidylcholines, lysophosphatidylcholines, phosphatidylethanolamines, lysophosphatidylethanolamine, lysoethanolamines, inverted headgroup lipids, sphingosins, sterol-modified phospholipids, ether ester lipids, diether lipids, vinyl ether (plasmalogen);
   an anionic lipid selected from the group comprising phosphatidic acids, lysophosphatidic acid derivatives, phosphatidylglycerols, lysophosphatidylglycerols, phosphatidylserines, lysophosphatidylserines, phosphatidylinositols, phosphatidylinositolphosphates, cardiolipins, Bis(Monoacylglycero)Phosphate derivatives;
   a cationic lipid selected from the group comprising dioleyl-N,N-dimethylammonium chloride; N-(2,3-dioleyloxy)propyl)-N,N,N-trimethylammonium chloride; N,N-distearyl-N,N-dimethylammonium bromide; N-(2,3- dioleyloxy)propyl)-N,N,N-trimethylammonium chloride; 3β-(N-(N',N'-dimethylaminoethane)-carbamoyl)cholesterol; 1,2-dimyristyloxypropyl-3-dimethyl-hydroxy ethyl ammonium bromide; 2,3-dioleyloxy-N-[2(sperminecarboxamido) ethyl]-N,N-dimethyl-1-propanaminium trifluoroacetate; dioctadecylamidoglycyl carboxyspermine; N-(2,3-dioleyloxy)propyl)-N,N-dimethylammonium chloride and 1,2-Dioleoyl-3-dimethylammonium-propane;
   a pH- sensitive lipid selected from the group comprising lipid N-(4-carboxybenzyl)-N,N-dimethyl-2,3-bis(oleoyloxy)propan-1-aminium, 1,2-distearoyl-3-dimethylammonium-propane, 1,2-dipalmitoyl-sn-glycero-3-succinate, 1,2-dioleoyl-sn-glycero-3-succinate, N-palmitoyl homocysteine ;
   a photoswitchable lipid;
   acylglycine derivatives, prenol derivatives, prostaglandine derivatives, glycosylated diacyl glycerols, eicosanoid derivatives, (palmitoyloxy)octadecanoic acid derivatives, diacetylene derivatives, diphytanoyl derivatives, fluorinated lipids, brominated lipids, lipopolysaccharides;
   one of the aforementioned lipids coupled to a functional ligand selected from the group comprising biotin, N-hydroxysuccinimide ester, nitrilotriacetic acid - nickel, amine, carboxylic acid, maleimides, dithiopyridinyl, pyridyl disulfide , pyridyldithiopropionate, N-benzylguanine, carboxyacyl, cyanur, folate, square, galloyl, glycan, thiol, arginylglycylaspartic acid, a fluorescent dye molecule, a magnetic resonance imaging reagent, a chelator;
   one of the aforementioned lipids coupled to polyethyleneglycol with a molecular weight comprised between 350 and 50,000 g/mole.
   optionally one or more nucleic acids selected from the group comprising DNA, cDNA, mRNA, siRNA, antisense nucleotides, shRNA, piRNA, snRNA, IncRNA, PNA, left handed DNA, Clustered Regularly Interspaced Short Palindromic Repeats (CRISPR) guide RNA;
   optionally one or more nucleic acids selected from the group comprising miRNA molecules selected from the group comprising miR-17, miR-18a, miR-19a, miR-19b-1, miR-20a, miR-92a; miR-21, miR-30d-5p, miR-33b, miR-124, miR-125, miR-126, miR-130, miR-132, miR-133b, miR-140-5p, miR-191, miR-222, miR-451, miR-494, miR-575, miR-630, miR-638, miR-1202, miR-1207-5p, miR-1225-5p, miR-1268, miR-6087, miR-92a-3p-e, miR-K12-3, let-7a.
   one or more transmembrane proteins selected from the group comprising tetraspanin proteins CD9, CD37, CD47, CD53, CD63, CD81, CD82, CD151, Tspan8, heterotrimeric G protein subunit alpha, integrin α - chains and β-chains, transferrin receptor 1, transferrin receptor 2, lysosome associated membrane proteins, heparan sulfate proteoglycans, syndecans, extracellular matrix metalloproteinase inducer, A Disintegrin And Metalloproteinase Domain 10, CD3, CD11c, CD14, CD29, CD31, CD41, CD42a, CD44, CD45, CD50 or intercellular adhesion molecule 1, CD55, CD59, CD73, CD80, CD86, CD90, sonic hedgehog, major histocompatibility complex I, major histocompatibility complex II, epidermal growth factor receptor 2, epithelial cell adhesion molecule, Glycophorin A, Acetylcholinesterase S and E, amyloid beta precursor protein, multidrug resistance-associated protein 1, stem cells antigen-1, or a fragment thereof;
   optionally one or more cytosolic proteins selected from the group comprising the protein complexes endosomal sorting complexes required for transport I, II and III, tumour susceptibility gene 101, charged multivesicular body protein, Apoptosis-Linked Gene 2-Interacting Protein X, vacuolar protein sorting 4 homolog A 4A and 4B, arrestin domain-containing protein, flotillin-1, flotillin-2; caveolins, EH-domain containing 1 - 4, ras homolog family member A, annexins, heat shock proteins, ADP-ribosylation factor 6, syntenin, microtubule-associated protein Tau, or a fragment thereof;
   optionally one or more functional proteins selected from the group comprising cytokines, growth factors, interleukins, milk fat globule-EGF factor 8 protein, adhesion proteins, extracellular matrix proteins, nicotinamide phosphoribosyltransferase, signal transduction proteins, Wnta and Wntb, Fas, Fas Ligand, RANK, RANK Ligand, indolamin-2,3-dioxygenase, cytotoxic T-lymphocyte-associated protein 4 - immunoglobulin fusion protein, tumor necrosis factor-related apoptosis-inducing ligand, or a fragment thereof;
   optionally one or more proteins associated to intracellular compartments selected from the group comprising histone proteins, lamin A/C, inner membrane mitochondrial protein, cytochrome C-1, mitochondrial import receptor subunit TOM20, calnexin, heat shock protein 90kDa beta, member 1, heat shock 70kDa protein 5, Golgin A2, Autophagy Related 9A, actinin1, actinin4, cytokeratin 18, or a fragment thereof.

A particularly preferred embodiment of the present invention is directed to a synthetic extracellular vesicle with the composition described above and specifically comprising:
a lipid bilayer comprising cholesterol, N-stearoyl-D-erythro-sphingosylphosphorylcholine, 1,2-dioleoyl-sn-glycero-3-phosphocholine, 1,2-dioleoyl-sn-glycero-3-phospho-L-serine, 1,2-dioleoyl-sn-glycero-3-phosphoethanolamine, 1,2-dioleoyl-sn-glycero-3-phospho-rac-(1-glycerol), 1,2-dioleoyl-sn-glycero-3-phosphate (sodium salt), diacylglycerol, phosphatidylinositol, 1,2-dioleoyl-sn-glycero-3-phosphoethanolamine-N-(lissamine rhodamine B sulfonyl), 1,2-dioleoyl-sn-glycero-3-[(N-(5-amino-1-carboxypentyl)iminodiacetic acid) succinyl] (nickel salt),
one or more nucleic acid molecules selected from the group comprising miRNA miR-21, miR-124, miR-125, miR-126, miR-130 and miR-132, and one or more transmembrane proteins selected from the group comprising tetraspanin proteins CD9, CD63 and CD81.

Another particularly preferred embodiment of the present invention is directed to a synthetic extracellular vesicle with the composition described above and specifically comprising:
one or more functional protein nicotinamide phosphoribosyltransferase, or a fragment thereof;
one or more transmembrane proteins selected from the group comprising tetraspanin proteins CD9, CD63 and CD81, or a fragment thereof;
one or more cytosolic proteins selected from the group comprising Apoptosis-Linked Gene 2-Interacting Protein X (ALIX), tumour susceptibility gene 101 protein (TSG101),
wherein the synthetic extracellular vesicle do not comprise transferrin and albumin, or a fragment thereof.

Another particularly preferred embodiment of the present invention is directed to a synthetic extracellular vesicle with the composition described above and specifically comprising:
one or more transmembrane proteins selected from the group comprising MHCII, CD80, and CD86, or a fragment thereof;
optionally one or more transmembrane proteins selected from the group comprising CD11c, MHC I, integrin α and β-chains, intercellular adhesion molecule-1, and CD71, or a fragment thereof;
one or more functional proteins selected from the group comprising cytokines, interleukins, interleukin 4, milk fat globule-EGF factor 8 protein, growth factors, Fas, Fas Ligand, indolamin-2,3-dioxygenase, cytotoxic T-lymphocyte-associated protein 4 - immunoglobulin fusion protein, tumor necrosis factor-related apoptosis-inducing ligand, or a fragment thereof.

Another particularly preferred embodiment of the present invention is directed to a synthetic extracellular vesicle with the composition described above and specifically comprising:
a lipid bilayer comprising 1,2-dioleoyl-sn-glycero-3-phosphocholine, 1,2-dioleoyl-sn-glycero-3-phospho-rac-(1-glycerol), 1,2-dioleoyl-sn-glycero-3-phosphoethanolamine-N-(lissamine rhodamine B sulfonyl), 1,2-dioleoyl-sn-glycero-3-[(N-(5-amino-1-carboxypentyl)iminodiacetic acid) succinyl] (nickel salt);
functional protein Fas Ligand, or a fragment thereof;
optionally functional protein intercellular adhesion protein - 1, or a fragment thereof.

Another particularly preferred embodiment of the present invention is directed to a synthetic extracellular vesicle with the composition described above and specifically comprising:
one or more transmembrane proteins selected from the group comprising CD29, CD44, CD90, CD73, CD44, Sca-1, or a fragment thereof;
one or more transmembrane proteins selected from the group comprising tetraspanin proteins CD9, CD63, and CD81, or a fragment thereof;
one or more functional proteins selected from the group comprising Wnta and Wntb, or a fragment thereof;
at least one nucleic acid molecule selected from the group comprising miR-140-5p, miR-92a-3p-e;
one or more nucleic acid molecules selected from the group comprising miR-17, miR-18a, miR-19a, miR-19b-1, miR-20a, miR-92a, let-7a, miR-21, miR124, miR126, miR-133b, miR-191, miR-222, miR-494, miR-6087, miR-30d-5p ;
optionally one or more nucleic acid molecules selected from the group comprising miR-33b, miR-451, miR-575, miR-630, miR-638, miR-1202, miR-1207-5p, miR-1225-5p, miR-1268, miR-K12-3.

A further particularly preferred embodiment of the present invention is directed to a synthetic extracellular vesicle with the composition described above and specifically comprising:
a lipid bilayer comprising 1,2-dioleoyl-sn-glycero-3-phosphocholine, 1,2-dioleoyl-sn-glycero-3-phospho-rac-(1-glycerol), 1,2-dioleoyl-sn-glycero-3-phosphoethanolamine-N-(lissamine rhodamine B sulfonyl), 1,2-dioleoyl-sn-glycero-3-[(N-(5-amino-1-carboxypentyl)iminodiacetic acid) succinyl] (nickel salt),
functional protein RANK, or a fragment thereof.

A more particularly preferred embodiment of the present invention is directed to a synthetic extracellular vesicle as described above for use in the treatment of a disorder selected from the group comprising inflammation, cancer, rheumatic disorder, severe graft versus host disease, osteoarthritis, cardiovascular disorder, epithelial diseases,neurodegenerative disorders, autoimmune disorders, bone and cartilage disorders, osteoporosis, renal osteodystrophy, paget's disease of bone, osteopetrosis, rickets, neurological disorders, intoxication, neuroendocrinology disorders, endocrinology disorders, genetic disorders, infectious diseases, dental disorders, cosmetic procedures, coagulation disorders, dermatoses, diabetes, age-associated disorders.

### Description of the invention

### Definitions

Unless specifically noted, the embodiments describing **"cell-derived vesicles"** or **"extracellular vesicles"** shall include **"exosomes", "liposomes", "microvesicles"** and "apoptotic vesicles" alone or in combination. When the term **"exosome"** is used as an example, it is understood that liposomes and microvesicles can be substituted therein.

As used herein, the term **"extracellular vesicle"** refers to a cell-derived vesicle comprising a membrane that encloses an internal space. Extracellular vesicles comprise all membrane-bound vesicles that have a smaller diameter than the cell from which they are derived. Generally extracellular vesicles range in diameter from 20 nm to 1000 nm, and can comprise various macromolecular cargo either within the internal space, displayed on the external surface of the extracellular vesicle, and/or spanning the membrane. The cargo can comprise nucleic acids, proteins, carbohydrates, lipids, small molecules, and/or combinations thereof. Two types of extracellular vesicles are exosomes and microvesicles.

As used herein the term **"exosome"** refers to a cell -derived small (between 20-300 nm in diameter, more preferably 20-1000 nm in diameter) vesicle comprising a membrane that encloses an internal space, and which is generated from the cell by direct plasma membrane budding or by fusion of the late endosome with the plasma membrane. The exosome is a species of extracellular vesicle. The exosome comprises lipid or fatty acid and proteins and optionally comprises a), a polynucleotide (e.g., a nucleic acid, RNA, or DNA), a sugar (e.g., a simple sugar, polysaccharide, or glycan), a functional agent (e.g., a therapeutic agent) or other molecules. The exosome can be derived from a producer cell using a technique known in the prior art, and isolated from the producer cell based on its size, density, biochemical parameters, or a combination thereof.

**Microvesicles**, on the other hand, are released from a cell upon direct budding from the plasma membrane (PM). Microvesicles are typically larger than exosomes and range from approximately 100 nm to 1 µm.

Although these two vesicle-types, microvesicles, and exosomes, are separate classes of vesicles, due to the fact that they overlap in size, and since the commonly used non-specific protocols for exosome isolation and purification rely solely on the vesicle size differences, it is a fact that in many of the reports published, the exosome samples used are impure, since they probably also include microvesicles and large protein aggregates. Because of this, it has been proposed that the term **"extracellular vesicles"** (EVs) be used as a general term for all small vesicles/particles, including both vesicle types, and also apoptotic bodies or vesicles.

**"Liposomes"** are microscopic vesicles consisting of concentric lipid bilayers. Structurally, liposomes range in size and shape from long tubes to spheres, with dimensions from a few hundred Angstroms to fractions of a millimeter. Vesicle-forming lipids are selected to achieve a specified degree of fluidity or rigidity of the final complex providing the lipid composition of the outer layer.

**"Apoptotic bodies"** or **"apoptotic vesicles"** are released during cell death (apoptosis) and are heterogeneously shaped vesicles with sizes between 50-5000 nm. They are formed from the plasma membrane, and they contain DNA, RNA, histones, and signalling molecules. They usually have high amounts of phosphatidylserine in their membranes, since the outer membrane of apoptotic cells is enriched in PS.

**"Membrane"** as used herein comprises a lipid bilayer that separates an interior space from an exterior space and comprises one or more biological compounds, typically lipids, and optionally polypeptides and/or carbohydrates such as glycan and/or nucleic acids, and/or other macromolecules. In some embodiments, the membrane comprises lipids and fatty acids. In some embodiments, the membrane comprises phospholipids, glycolipids, fatty acids, sphingolipids, phosphoglycerides, sterols, cholesterols, and phosphatidylserines. The extracellular vesicle comprises a membrane as defined herein.

In some embodiments, the extracellular vesicle or exosome further comprises one or more macromolecule in their lumen.

The term **"macromolecule"** as used herein is selected from the group comprising an extracellular vesicle associated protein, a carbohydrate, a nucleic acid, a polypeptide, a cell receptor, an imaging probe.

As used herein, the term **"homogeneous"** in reference to a population of extracellular vesicles refers to population of vesicles that have the same or a similar amount of one or more proteins, or one or more nucleic acids, or one or more macromolecule. A homogenous population is one wherein about 65%, about 70%, about 75%, about 80%, about 85%, about 90%, about 95%, about 98%, or 100% of the vesicles share the one or more antigen binding domain(s) and/or payload.
As used herein, the term "heterogeneous" in reference to a population of engineered vesicles refers to population of vesicles that have differing identity or differing amount of one or more proteins, or one or more nucleic acids, or one or more macromolecule.

Moreover, as used herein, the term **"homogeneous"** in reference to a population of extracellular vesicles also refers to population of vesicles that have the same or a similar size. A homogenous population in size is one wherein the coefficient of variation calculated as [(standard deviation / average size) ^{∗} 100] is lower than 15 %, preferably lower than 13 %, preferably lower than 10 %, preferably lower than 8 %, preferably lower than 7 %, most preferably lower than 5 %.

The extracellular vesicle or exosome can interact with the target cell via membrane fusion and deliver nucleic acids or intracellular proteins or functional proteins to the surface or cytoplasm of a target cell. In some embodiments, membrane fusion occurs between the extracellular vesicle or exosome and the plasma membrane of a target cell. In other embodiments, membrane fusion occurs between the extracellular vesicle or exosome and an endosomal membrane of a target cell.

As used herein, the term **"modulate", "modulating", "modify", and/or "modulator"** generally refers to the ability to alter, by increase or decrease, e.g., directly or indirectly promoting, stimulating, up-regulating or interfering with/inhibiting/down-regulating a specific concentration, level, expression, function or behaviour, such as, e.g., to act as an antagonist or agonist. In some instances a modulator can increase and/or decrease a certain concentration, level, activity or function relative to a control, or relative to the average level of activity that would generally be expected or relative to a control level of activity.

In some embodiments, the extracellular vesicle has a diameter between 20 - 5000 nm, such as between about 20 - 150 nm, 20 - 500 nm, 20 - 1000 nm, 20 - 2000, nm, 20 - 3000 nm, 20 - 4000 nm, 20 - 5000 nm, 30 - 150 nm, 30 - 500 nm, 30 - 1000 nm, 30 - 2000, nm, 30 - 3000 nm, 30 - 4000 nm, 30 - 5000 nm, 40 - 150 nm, 40 - 500 nm, 40 - 1000 nm, 40 - 2000, nm, 40 - 3000 nm, 40 - 4000 nm, 40 - 5000 nm, 70 - 2000, nm, 70 - 3000 nm, 70 - 4000 nm, 70 - 5000 nm, 50 - 150 nm, 50 - 500 nm, 50 - 1000 nm, 50 - 2000, nm, 50 - 3000 nm, 50 - 4000 nm, 50 - 5000 nm, 100 - 150 nm, 100 - 500 nm, 100 - 1000 nm, 100 - 2000, nm, 100 - 3000 nm, 100 - 4000 nm, 100 - 5000 nm, 500 - 1000 nm, 500 - 2000, nm, 500 - 3000 nm, 500 - 4000 nm, 500 - 5000 nm.

In other embodiments, the extracellular vesicle has a diameter between about 20 - 1000 nm, such as between about 20 - 100 nm, 20 - 200 nm, 20 - 300 nm, 20 - 400 nm, 20 - 500 nm, 20 - 600 nm, 20 - 700 nm, 20 - 800 nm, 20 - 900 nm, 30 - 100 nm, 30 - 200 nm, 30 - 300 nm, 30 - 400 nm, 30 - 500 nm, 30 - 600 nm, 30 - 700 nm, 30 - 800 nm, 30 - 900 nm, 40 - 100 nm, 40 - 200 nm, 40 - 300 nm, 40 - 400 nm, 40 - 500 nm, 40 - 600 nm, 40 - 700 nm, 40 - 800 nm, 40 - 900 nm, 50 - 150 nm, 50 - 500 nm, 50 - 750 nm, 100 - 200 nm, 100 - 500 nm, or 500 - 1000 nm.

In another embodiment, a population of the extracellular vesicles described herein comprise a population wherein 90% of the extracellular vesicles have a diameter 20 - 5000 nm. In another embodiment, a population of the extracellular vesicles described herein comprise a population wherein 95% of the extracellular vesicles have a diameter 20 - 5000 nm. In another embodiment, a population of the extracellular vesicles described herein comprise a population wherein 99% of the extracellular vesicles have a diameter 20 - 5000 nm. In another embodiment, a population of the extracellular vesicles described herein comprise a population wherein 90% of the extracellular vesicles have a diameter 20 - 1000 nm. In another embodiment, a population of the extracellular vesicles described herein comprise a population wherein 95% of the extracellular vesicles have a diameter 20 - 1000 nm. In another embodiment, a population of the extracellular vesicles described herein comprise a population wherein 99% of the extracellular vesicles have a diameter 20 - 1000 nm. In another embodiment, a population of the extracellular vesicles described herein comprise a population wherein 90% of the extracellular vesicles have a diameter 20 - 500 nm. In another embodiment, a population of the extracellular vesicles described herein comprise a population wherein 95% of the extracellular vesicles have a diameter 20 - 500 nm. In another embodiment, a population of the extracellular vesicles described herein comprise a population wherein 99% of the extracellular vesicles have a diameter 20 - 500 nm.

In certain embodiments, the extracellular vesicle is an exosome. In certain embodiments, the extracellular vesicle is a microvesicle.

In some embodiments, the exosome has a diameter between about 20 - 5000 nm, such as between about 20 - 150 nm, 20 - 500 nm, 20 - 1000 nm, 20 - 2000, nm, 20 - 3000 nm, 20 - 4000 nm, 20 - 5000 nm, 30 - 150 nm, 30 - 500 nm, 30 - 1000 nm, 30 - 2000, nm, 30 - 3000 nm, 30 - 4000 nm, 30 - 5000 nm, 40 - 150 nm, 40 - 500 nm, 40 - 1000 nm, 40 - 2000, nm, 40 - 3000 nm, 40 - 4000 nm, 40 - 5000 nm,50 - 150 nm, 50 - 500 nm, 50 - 1000 nm, 50 - 2000, nm, 50 - 3000 nm, 50 - 4000 nm, 50 - 5000 nm, 70 - 2000, nm, 70 - 3000 nm, 70 - 4000 nm, 70 - 5000 nm, 100 - 150 nm, 100 - 500 nm, 100 - 1000 nm, 100 - 2000, nm, 100 - 3000 nm, 100 - 4000 nm, 100 - 5000 nm, 500 - 1000 nm, 500 - 2000, nm, 500 - 3000 nm, 500 - 4000 nm, 500 - 5000 nm.

In other embodiments, the exosome has a diameter between about 20 - 1000 nm, such as between about 20 - 100 nm, 20 - 200 nm, 20 - 300 nm, 20 - 400 nm, 20 - 500 nm, 20 - 600 nm, 20 - 700 nm, 20 - 800 nm, 20 - 900 nm, 30 - 100 nm, 30 - 200 nm, 30 - 300 nm, 30 - 400 nm, 30 - 500 nm, 30 - 600 nm, 30 - 700 nm, 30 - 800 nm, 30 - 900 nm, 40 - 100 nm, 40 - 200 nm, 40 - 300 nm, 40 - 400 nm, 40 - 500 nm, 40 - 600 nm, 40 - 700 nm, 40 - 800 nm, 40 - 900 nm, 50 - 150 nm, 50 - 500 nm, 50 - 750 nm, 100 - 200 nm, 100 - 500 nm, or 500 - 1000 nm.

In another embodiment, a population of the exosomes described herein comprise a population wherein 90% of the exosomes have a diameter 20 - 5000 nm. In another embodiment, a population of the exosomes described herein comprise a population wherein 95% of the exosomes have a diameter 20 - 5000 nm. In another embodiment, a population of the exosomes described herein comprise a population wherein 99% of the exosomes have a diameter 20 - 5000 nm. In another embodiment, a population of the exosomes described herein comprise a population wherein 90% of the exosomes have a diameter 20 - 1000 nm. In another embodiment, a population of the exosomes described herein comprise a population wherein 95% of the exosomes have a diameter 20 - 1000 nm. In another embodiment, a population of the exosomes described herein comprise a population wherein 99% of the exosomes have a diameter 20 - 1000 nm. In another embodiment, a population of the exosomes described herein comprise a population wherein 90% of the exosomes have a diameter 20 - 500 nm. In another embodiment, a population of the exosomes described herein comprise a population wherein 95% of the exosomes have a diameter 20 - 500 nm. In another embodiment, a population of the exosomes described herein comprise a population wherein 99% of the exosomes have a diameter 20 - 500 nm.

The present invention is directed to a method for producing synthetic extracellular vesicles comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for protein conjugation;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
wherein the amphiphilic copolymer forms a polymer shell stabilizing the synthetic extracellular vesicle, and
wherein the synthetic extracellular vesicles have a diameter comprised between 70 nm and 5000 nm.

A particular embodiment of the invention is directed to a method for producing synthetic extracellular vesicles comprising:
a) providing a water phase comprising at least two lipids, optionally one or more extracellular vesicle associated proteins, or fragments thereof, and optionally one or more nucleic acid molecules;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
wherein the amphiphilic copolymer forms a polymer shell stabilizing the synthetic extracellular vesicle, and
wherein the synthetic extracellular vesicles have a diameter comprised between 70 nm and 1000 nm.

In certain embodiments, the extracellular vesicle is an exosome. In certain embodiments, the extracellular vesicle is a microvesicle.

Therefore, a more particular embodiment of the invention is directed to a method for producing a synthetic exosome comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for protein conjugation;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
wherein the amphiphilic copolymer forms a polymer shell stabilizing the synthetic exosome, and wherein the synthetic exosome has a diameter comprised between 70 nm and 5000 nm.

Another more particular embodiment of the invention is directed to a method for producing a synthetic exosome comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for protein conjugation;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
wherein the amphiphilic copolymer forms a polymer shell stabilizing the synthetic exosome, and
wherein the synthetic exosome has a diameter comprised between 70 nm and 1000 nm.

Another still more particular embodiment of the invention is directed to a method for producing a synthetic exosome comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for protein conjugation;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
wherein the amphiphilic copolymer forms a polymer shell stabilizing the synthetic exosome, and
wherein the synthetic exosome has a diameter comprised between 70 nm and 700 nm.

### Release and purification procedure

In accordance with optional step d'), the polymer shell is removed from the polymer shell-stabilized synthetic extracellular vesicles. Since the polymer shell is not necessary any more after assembling the vesicles with all the required components, it is actually preferred to perform the step d') so as to obtain the synthetic extracellular vesicles into an aqueous phase.

The inventors have found that the synthetic extracellular vesicles can be efficiently released from the polymer shell by adding a deemulsifier to the polymer shell stabilized synthetic extracellular vesicles formed after emulsification. The deemulsifier destabilizes the structure of the surrounding polymer shell and thus, allows releasing the synthetic extracellular vesicles from the polymer shell into an aqueous buffer, also named "release buffer".
The deemulsifier is preferably selected from the group comprising 1H,1H,2H,2H-perfluoro-1-octanol; 1H,1H-perfluoro-1-pentanol; 1H,1H-perfluor-1-octanol; 1H, 1H,8H - perfluoro-1-octanol.

The deemulsifier is preferentially added at a ratio ranging from 1 : 1 to 10 : 1 with the intraluminal buffer (also named production buffer).

Thereafter, the synthetic extracellular vesicles are usually centrifuged after release from the polymer shell to allow purification from vesicles of unwanted dimensions and other impurities.

The centrifugation can be performed for a time comprised between 5 - 60 min and at acceleration comprised between 800 *g* - 100.000 g depending on the dimension of the synthetic extracellular vesicles of interest.

Moreover, for synthetic extracellular vesicles with diameter comprised between 100 - 1000 nm, the centrifugation is preferentially performed at acceleration comprised between 30,000 - 60,000 g and a time comprised between 10 - 60 min.
For synthetic extracellular vesicles with diameter comprised between 1000 - 3000 nm, the centrifugation is preferentially performed at acceleration comprised between 10,000 - 30,000 g and a time comprised between 10 - 60 min.
For synthetic extracellular vesicles with diameter comprised between 3000 - 5000 nm, the centrifugation is preferentially performed at acceleration comprised between 5,000 - 20,000 g and a time comprised between 10 - 60 min.

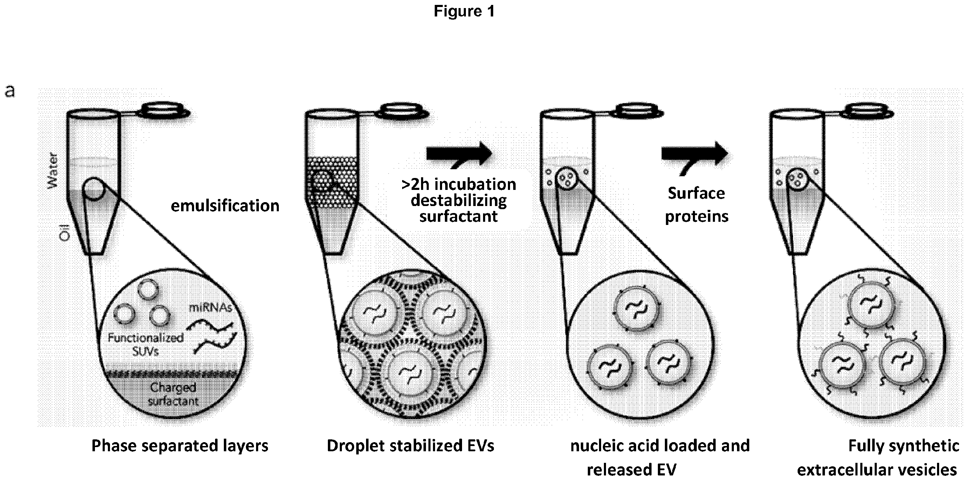

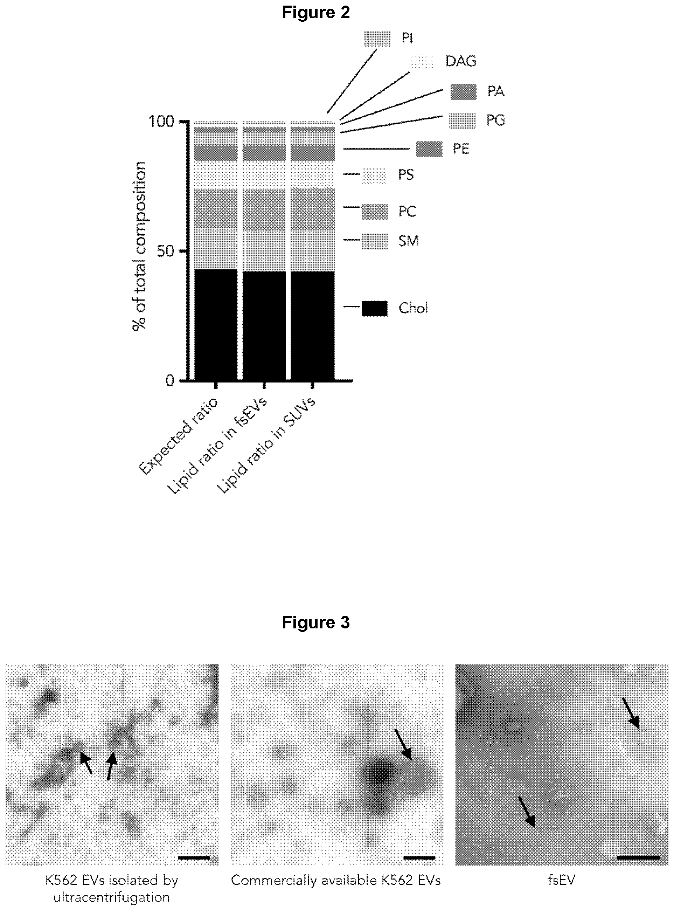

The synthetic extracellular vesicles synthetized following the inventive method, released into an aqueous medium and then purified by centrifugation (**Figure 1**), contained considerably less contaminating aggregates and non-vesicular particles compared to exosomes isolated by standard prior art methods (**Figure 3**), i.e. exosomes isolated by differential centrifugation from conditioned K562 erythroleukemia cell media or exosomes from the same cell line obtained from a commercial distributer.

Thus, present invention is directed to method for producing synthetic extracellular vesicles comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for protein conjugation;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
d') removing the polymer shell from the polymer shell-stabilized synthetic extracellular vesicles obtained in step d);
e) purifying the synthetic extracellular vesicles by centrifugation;
wherein the amphiphilic copolymer forms a polymer shell stabilizing the synthetic extracellular vesicle, and
wherein the synthetic extracellular vesicles have a diameter comprised between 70 nm and 5000 nm.

A particular embodiment of the invention is directed to a method for producing synthetic extracellular vesicles comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for protein conjugation;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
d') removing the polymer shell from the polymer shell-stabilized synthetic extracellular vesicles obtained in step d);
e) purifying the synthetic extracellular vesicles by centrifugation;
wherein the amphiphilic copolymer forms a polymer shell stabilizing the synthetic extracellular vesicle, and
wherein the synthetic extracellular vesicles have a diameter comprised between 70nm and 1000 nm.

A more particular embodiment of the invention is directed to a method for producing synthetic extracellular vesicles comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for protein conjugation,;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
d') removing the polymer shell from the polymer shell-stabilized synthetic extracellular vesicles obtained in step d) by adding a destabilizing agent;
e) purifying the synthetic extracellular vesicles by centrifugation;
wherein the amphiphilic copolymer forms a polymer shell stabilizing the synthetic extracellular vesicle, and
wherein the synthetic extracellular vesicles have a diameter comprised between 70nm and 5000 nm.

A more particular embodiment of the invention is directed to a method for producing synthetic extracellular vesicles comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for protein conjugation;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
d') removing the polymer shell from the polymer shell-stabilized synthetic extracellular vesicles obtained in step d);
e) purifying the synthetic extracellular vesicles by centrifugation, wherein the centrifugation is performed at acceleration comprised between 800 g - 100.000 *g* and a time comprised between 5 - 60 min;
wherein the amphiphilic copolymer forms a polymer shell stabilizing the synthetic extracellular vesicle, and
wherein the synthetic extracellular vesicles have a diameter comprised between 70nm and 5000 nm.

A particular embodiment of the invention is directed to a method for producing synthetic extracellular vesicles comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for protein conjugation;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
d') removing the polymer shell from the polymer shell-stabilized synthetic extracellular vesicles obtained in step d) by adding a destabilizing agent;
e) purifying the synthetic extracellular vesicles by centrifugation;
wherein the amphiphilic copolymer forms a polymer shell stabilizing the synthetic extracellular vesicle, and
wherein the synthetic extracellular vesicles have a diameter comprised between 70 nm and 1000 nm.

A more particular embodiment of the invention is directed to a method for producing synthetic extracellular vesicles comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for protein conjugation
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
d') removing the polymer shell from the polymer shell-stabilized synthetic extracellular vesicles obtained in step d) by adding a destabilizing agent;
e) purifying the synthetic extracellular vesicles by centrifugation, wherein the centrifugation is performed at acceleration comprised between 800 g - 100.000 g and a time comprised between 5 - 60 min ;
wherein the amphiphilic copolymer forms a polymer shell stabilizing the synthetic extracellular vesicle, and
wherein the synthetic extracellular vesicles have a diameter comprised between 70 nm and 5000 nm.

### Protein associated to the extracellular vesicles, functionalization procedure

In the inventive methods, the synthetic extracellular vesicles can be decorated with the proteins of interest after release into an aqueous solution, as described above, wherein the proteins are preferably known to be associated with extracellular vesicles.

The proteins can be coupled to the synthetic extracellular vesicles by applying bio-orthogonal surface chemistry such as N-hydroxysuccinimide ester and/or NTA - poly-histidine tag coupling, or they can be added to the water phase of step a) or can be integrated into or on the polymer shell stabilized synthetic extracellular vesicles by microfluidic technology such as pico-injection.

These procedures allow obtaining synthetic extracellular vesicles comprising proteins at a well-defined ratio protein: lipid, and with very low degree of variation in protein composition between different batches.

Preferred protein : lipid ratios as used herein are comprised between 1 : 20 to 1 : 100, 1 : 40 to 1 : 100, 1 : 50 to 1 : 100, 1 : 20 to 1 : 200, 1 : 40 to 1 : 200, 1 : 50 to 1 : 200, 1 : 75 to 1 : 200.

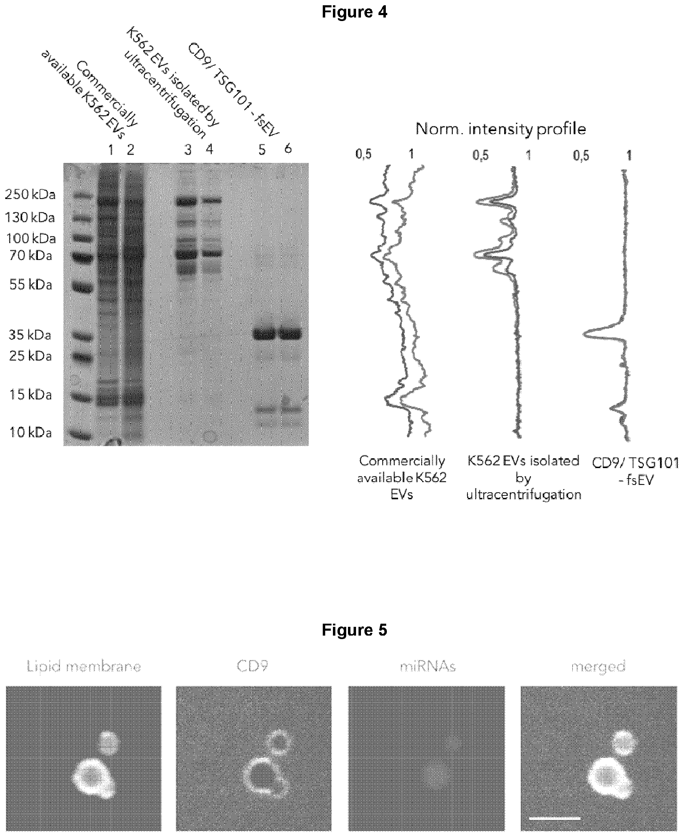

When assessing the exosome protein content, the inventor found that K562-derived exosomes isolated from conditioned media and those provided by a commercial distributer, differed greatly in their protein content, underscoring the degree of variation between different vesicle preparation methods (**Figure 4**)**.** Furthermore, when comparing between separately prepared batches of prior art exosomes, a substantial degree of variation in the protein composition could be observed. In contrast, the inventive synthetic exosomes equipped with purified recombinant human forms of exosome's surface markers CD9 and TSG101, attached by nitrilotriacetic acid (NTA)-poly histidine tag chemistry, appeared with a clearly defined band pattern and showed almost identical characteristics between separate preparations. Thus, the method disclosed herein allows obtaining synthetic exosomes that outperforms the exosomes obtained by prior art methods in terms of purity and reproducibility.

Important to mention, by applying bio-orthogonal surface chemistry such as N-hydroxysuccinimide ester and/or NTA - poly-histidine tag coupling, or by using microfluidic technologies, the protein to lipid ratio can be precisely adjusted. This ratio is also very homogenous among the vesicle population.
Thus, the inventive synthetic extracellular vesicles show an outstanding improvement in comparison to the non-adjustable extracellular vesicles obtained by prior art methods, such as differential centrifugation of cell culture medium, or membrane fragmentation of engineered cells.

The wording "extracellular vesicle associated protein" refers to proteins that are einriched in exosomes and extracellular vesicles in comparison to cells. Therefore "extracellular vesicle associated proteins" can also be used as marker of exosomes or other extracellular vesicles. Thus the term "extracellular vesicle associated proteins" has the same meaning as "extracellular vesicle protein marker" or "extracellular vesicle marker".

Therefore, in specific embodiments, the extracellular vesicles or exosomes comprise one or more proteins on their surface or in their lumen, wherein said proteins are selected from a group of proteins that was recently identified to be enriched on the surface or inside extracellular vesicles, and were thus defined as **"extracellular vesicle associated proteins"** (Thery et al., 2018, Minimal information for studies of extracellular vesicles 2018; Exocarta Top100 proteins). A list of extracellular vesicle associated proteins sutiable for the method and the extracellular vesicles disclosed herein are listed in Table 4.

As used herein the term **"fragment"** or **"active fragment"** of a protein refers to a fragment of that protein that retains the ability to be specifically coupled to the extracellular vesicle or exosome. The term **"fragment"** or **"active fragment"** of a protein also refers to a fragment of that protein that retains the ability to exert its function in the target cell.

For example, in the case of membrane proteins, a protein fragment refers to a cytosolic domain, a transmembrane domain or an extracellular domain of said protein.

For example, in the case of enzymes, a protein fragment refers to a catalytic domain of that enzyme.

For example, in the case of antibodies, a protein fragment refers to a fragment of the antibody that retains its capacity to bind specifically to the antigen. The antibody or antigen-binding fragment can be derived from natural sources, or partly or wholly synthetically produced. In some embodiments, the antibody is a monoclonal antibody. In some of these embodiments, the monoclonal antibody is an IgG antibody. In certain embodiments, the monoclonal antibody is an IgG₁, IgG₂, IgG₃, or IgG₄. In some other embodiments, the antibody is a polyclonal antibody. In certain embodiments, the antibody fragment, also named antigen-binding fragment, is selected from antigen-binding fragment (Fab), Fab', and F(ab')2, F(ab)2, a viable fragment (Fv), and Fd fragments. In certain embodiments, the antigen-binding fragment is a Single-chain variable fragment (scFv) or (ScFv)2 fragment. In certain other embodiments, the antibody or antigen-binding fragment is a single-domain antibody. In some embodiments, the antibody or antigen binding fragment is a bispecific or multispecific antibody.

For example, in the case of protein antigens, a protein fragment refers to a fragment of the antigen that retains its capacity to induce an immune response in a human or animal, and/or to be specifically recognized by an antibody.

Preferably, a suitable protein fragment of TSG101 (protein ID Q99816) comprises the amino acids 1 - 145, a suitable protein fragment of CD9 (protein ID P21926) comprises the amino acids 112 - 195, a suitable protein fragment of CD81 (protein ID P35762), comprises the amino acids 113-201, a suitable protein protein fragment of CD63 (protein ID P08962) comprises the amino acids 103 - 203, a suitable protein fragment of RANK (protein ID O35305) comprises the amino acids 31 - 214, a suitable protein fragment of FasL (protein ID NM_000639.1) comprises the amino acids 134 - 281, a suitable protein fragment of ICAM-1 (protein ID P05362) comprises the amino acids 1 - 480.

As used herein the term protein or a fragment thereof also include **"variant"** of a protein or of a fragment thereof, and refers to a protein or fragment that shares a certain amino acid sequence identity with the reference protein or fragment upon alignment by a method known in the art. A variant of a protein or of a fragment thereof can include a substitution, insertion, deletion, frameshift or rearrangement in another protein. In some embodiments variants share at least 70 %, 80 %, 85 %, 90 %, 95 % or 99 % sequence identity with the reference protein or with the fragment thereof.

Recitation of any protein provided herein encompasses a functional variant of the protein. The term **"functional variant"** of a protein refers to a variant of the protein that retains the ability to be specifically targeted to exosomes.

The percentage of **"sequence identity"** is determined by comparing two optimally aligned protein or polypeptide sequences over a "comparison window" on the full length of the reference sequence. A **"comparison window"** as used herein, refers to the optimal alignment between the reference and variant sequence after that the two sequences are optimally aligned, wherein the variant nucleic acid or polypeptide sequence in the comparison window may comprise additions or deletions (i.e., gaps) of 20 percent or less, usually 5 to 15 percent, or 10 to 12 percent, as compared to the reference sequences (which does not comprise additions or deletions) for optimal alignment. Identity percentage is calculated by determining the number of positions at which the identical amino acid residues occurs in both sequences to yield the number of matched positions, dividing the number of matched positions by the total number of positions in the reference sequence (i.e., the full length in amino acid or nucleotide) and multiplying the results by 100 to yield the percentage of sequence identity. Two protein or polypeptide sequences are said to be **"identical"** if the sequence of nucleotides or amino acids in the two sequences is the same when optimally aligned as described above.
The percentage of "sequence identity" can be determined on the comparison window defined above with the help of blastp with the "BLAST 2 Sequences" tool available at the NCBI website. (Tatusova A. et al., FEMS Microbiol Lett. 1999, 174:247-250).

Alternatively, a variant sequence may also be any amino acid sequence resulting from allowed substitutions at any number of positions of the parent sequence according to the formula below:
Ser substituted by Ser, Thr, Gly, and Asn;
Arg substituted by one of Arg, His, Gin, Lys, and Glu;
Leu substituted by one of Leu, Ile, Phe, Tyr, Met, and Val;
Pro substituted by one of Pro, Gly, Ala, and Thr;
Thr substituted by one of Thr, Pro, Ser, Ala, Gly, His, and Gin;
Ala substituted by one of Ala, Gly, Thr, and Pro;
Val substituted by one of Val, Met, Tyr, Phe, Ile, and Leu;
Gly substituted by one of Gly, Ala, Thr, Pro, and Ser;
Ile substituted by one of Ile, Met, Tyr, Phe, Val, and Leu;
Phe substituted by one of Phe, Trp, Met, Tyr, lie, Val, and Leu;
Tyr substituted by one of Tyr, Trp, Met, Phe, Ile, Val, and Leu;
His substituted by one of His, Glu, Lys, Gin, Thr, and Arg;
Gln substituted by one of Gin, Glu, Lys, Asn, His, Thr, and Arg;
Asn substituted by one of Asn, Glu, Asp, Gin, and Ser;
Lys substituted by one of Lys, Glu, Gln, His, and Arg;
Asp substituted by one of Asp, Glu, and Asn;
Glu substituted by one of Glu, Asp, Lys, Asn, Gln, His, and Arg;
Met substituted by one of Met, Phe, Ile, Val, Leu, and Tyr.

According to the present invention, the extracellular vesicle associated protein is preferentially selected from the group comprising:
a transmembrane proteins selected from the group comprising tetraspanin proteins CD9, CD37, CD47, CD53, CD63, CD81, CD82, CD151, Tspan8, heterotrimeric G protein subunit alpha (GNA), integrin α - chains and β-chains, transferrin receptor 1 (TfR1, CD71), transferrin receptor 2 (TFR2), lysosome associated membrane proteins (LAMP1, LAMP2), heparan sulfate proteoglycans, syndecans, extracellular matrix metalloproteinase inducer (EMMPRIN, BSG), A Disintegrin And Metalloproteinase Domain 10 (ADAM10), CD3, CD11c, CD14, CD29, CD31, CD41, CD42a, CD44, CD45, CD50 (intercellular adhesion molecule 1, ICAM-1), CD55, CD59, CD73, CD80, CD86, CD90, sonic hedgehog (SHH), major histocompatibility complex I (MHC I), major histocompatibility complex II (MHC II), epidermal growth factor receptor 2 (ERBB2), epithelial cell adhesion molecule (EpCAM), Glycophorin A (GYPA); Acetylcholinesterase S and E (AChE-S, AChE-E), amyloid beta precursor protein (APP), multidrug resistance-associated protein 1 (ABCC1), stem cells antigen-1 (Sca-1), or a fragment thereof;
a cytosolic protein selected from the group comprising the protein complexes endosomal sorting complexes required for transport ESCRT-I, ESCRT-II, and ESCRT-III, tumour susceptibility gene 101 (TSG101), charged multivesicular body protein (CHMP), Apoptosis-Linked Gene 2-Interacting Protein X (ALIX), vacuolar protein sorting 4 homolog A 4A and 4B, arrestin domain-containing protein (ARRDC1), flotillin-1, flotillin-2; caveolins, EH-domain containing 1 - 4 (EHD1 - EHD4), Ras homolog family member A (RHOA), annexins, heat shock proteins, ADP-ribosylation factor 6 (ARF6), syntenin, microtubule-associated protein Tau (MAPT), or a fragment thereof;
a functional protein selected from the group comprising cytokines, growth factors, interleukins, milk fat globule-EGF factor 8 protein (MFGE8), adhesion proteins, extracellular matrix proteins, nicotinamide phosphoribosyltransferase, signal transduction proteins Wnta and Wntb, Fas, Fas Ligand (FasL), RANK, RANK Ligand (RANKL), indolamin-2,3-dioxygenase, cytotoxic T-lymphocyte-associated protein 4 - immunoglobulin fusion protein, tumor necrosis factor-related apoptosis-inducing ligand, or a fragment thereof;
a protein associated to intracellular compartments selected from the group comprising histone proteins, lamin A/C, inner membrane mitochondrial protein (IMMT), cytochrome C-1 (CYC1), mitochondrial import receptor subunit TOM20, calnexin, heat shock protein 90kDa beta (Grp94), member 1 (HSP90B1), heat shock 70kDa protein 5 (HSPA5), Golgin A2 (GM130, GOLGA2), Autophagy Related 9A (ATG9A), actinin1, actinin4 (ACTN1, ACTN4), cytokeratin 18 (KRT18), or a fragment thereof.

Therefore, one embodiment of the present invention is directed to a method for producing synthetic extracellular vesicles comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for conjugation to an extracellular vesicle associated protein;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
wherein the amphiphilic copolymer forms a polymer shell stabilizing the synthetic extracellular vesicle, and
wherein the synthetic extracellular vesicles have a diameter between 70 nm and 5000 nm, and
wherein the extracellular vesicle associated protein, or a fragment thereof is selected from the group comprising:
a transmembrane proteins selected from the group comprising tetraspanin proteins CD9, CD37, CD47, CD53, CD63, CD81, CD82, CD151, Tspan8, heterotrimeric G protein subunit alpha (GNA), integrin α - chains and β-chains, transferrin receptor 1 (TfR1, CD71), transferrin receptor 2 (TFR2), lysosome associated membrane proteins (LAMP1, LAMP2), heparan sulfate proteoglycans, syndecans, extracellular matrix metalloproteinase inducer (EMMPRIN, BSG), A Disintegrin And Metalloproteinase Domain 10 (ADAM10), CD3, CD11c, CD14, CD29, CD31, CD41, CD42a, CD44, CD45, CD50 (intercellular adhesion molecule 1, ICAM-1), CD55, CD59, CD73, CD80, CD86, CD90, sonic hedgehog (SHH), major histocompatibility complex I (MHC I), major histocompatibility complex II (MHC II), epidermal growth factor receptor 2 (ERBB2), epithelial cell adhesion molecule (EpCAM), Glycophorin A (GYPA); Acetylcholinesterase S and E (AChE-S, AChE-E), amyloid beta precursor protein (APP), multidrug resistance-associated protein 1 (ABCC1), stem cells antigen-1 (Sca-1), or a fragment thereof;
a cytosolic protein selected from the group comprising the protein complexes endosomal sorting complexes required for transport ESCRT-I, ESCRT-II, and ESCRT-III, tumour susceptibility gene 101 (TSG101), charged multivesicular body protein (CHMP), Apoptosis-Linked Gene 2-Interacting Protein X (ALIX), vacuolar protein sorting 4 homolog A 4A and 4B, arrestin domain-containing protein (ARRDC1), flotillin-1, flotillin-2; caveolins, EH-domain containing 1 - 4 (EHD1 - EHD4), Ras homolog family member A (RHOA), annexins, heat shock proteins, ADP-ribosylation factor 6 (ARF6), syntenin, microtubule-associated protein Tau (MAPT), or a fragment thereof;
a functional protein selected from the group comprising cytokines, growth factors, interleukins, milk fat globule-EGF factor 8 protein (MFGE8), adhesion proteins, extracellular matrix proteins, nicotinamide phosphoribosyltransferase, signal transduction proteins Wnta and Wntb, Fas, Fas Ligand (FasL), RANK, RANK Ligand (RANKL), indolamin-2,3-dioxygenase, cytotoxic T-lymphocyte-associated protein 4 - immunoglobulin fusion protein, tumor necrosis factor-related apoptosis-inducing ligand, or a fragment thereof;
a protein associated to intracellular compartments selected from the group comprising histone proteins, lamin A/C, inner membrane mitochondrial protein (IMMT), cytochrome C-1 (CYC1), mitochondrial import receptor subunit TOM20, calnexin, heat shock protein 90kDa beta (Grp94), member 1 (HSP90B1), heat shock 70kDa protein 5 (HSPA5), Golgin A2 (GM130, GOLGA2), Autophagy Related 9A (ATG9A), actinin1, actinin4 (ACTN1, ACTN4), cytokeratin 18 (KRT18), or a fragment thereof.

A more particular embodiment of the invention is directed to a method for producing synthetic extracellular vesicles comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for conjugation to an extracellular vesicle associated protein;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
d') removing the polymer shell from the polymer shell-stabilized synthetic extracellular vesicles obtained in step d);
e) purifying the synthetic extracellular vesicles by centrifugation;
wherein the amphiphilic copolymer forms a polymer shell stabilizing the synthetic extracellular vesicle, and
wherein the synthetic extracellular vesicles have a diameter comprised between 70 nm and 5000 nm,
wherein the extracellular vesicle associated protein, or a fragment thereof is selected from the group comprising:
a transmembrane proteins selected from the group comprising tetraspanin proteins CD9, CD37, CD47, CD53, CD63, CD81, CD82, CD151, Tspan8, heterotrimeric G protein subunit alpha (GNA), integrin α - chains and β-chains, transferrin receptor 1 (TfR1, CD71), transferrin receptor 2 (TFR2), lysosome associated membrane proteins (LAMP1, LAMP2), heparan sulfate proteoglycans, syndecans, extracellular matrix metalloproteinase inducer (EMMPRIN, BSG), A Disintegrin And Metalloproteinase Domain 10 (ADAM10), CD3, CD11c, CD14, CD29, CD31, CD41, CD42a, CD44, CD45, CD50 (intercellular adhesion molecule 1, ICAM-1), CD55, CD59, CD73, CD80, CD86, CD90, sonic hedgehog (SHH), major histocompatibility complex I (MHC I), major histocompatibility complex II (MHC II), epidermal growth factor receptor 2 (ERBB2), epithelial cell adhesion molecule (EpCAM), Glycophorin A (GYPA); Acetylcholinesterase S and E (AChE-S, AChE-E), amyloid beta precursor protein (APP), multidrug resistance-associated protein 1 (ABCC1), stem cells antigen-1 (Sca-1), or a fragment thereof;
a cytosolic protein selected from the group comprising the protein complexes endosomal sorting complexes required for transport ESCRT-I, ESCRT-II, and ESCRT-III, tumour susceptibility gene 101 (TSG101), charged multivesicular body protein (CHMP), Apoptosis-Linked Gene 2-Interacting Protein X (ALIX), vacuolar protein sorting 4 homolog A 4A and 4B, arrestin domain-containing protein (ARRDC1), flotillin-1, flotillin-2; caveolins, EH-domain containing 1 - 4 (EHD1 - EHD4), Ras homolog family member A (RHOA), annexins, heat shock proteins, ADP-ribosylation factor 6 (ARF6), syntenin, microtubule-associated protein Tau (MAPT), or a fragment thereof;
a functional protein selected from the group comprising cytokines, growth factors, interleukins, milk fat globule-EGF factor 8 protein (MFGE8), adhesion proteins, extracellular matrix proteins, nicotinamide phosphoribosyltransferase, signal transduction proteins Wnta and Wntb, Fas, Fas Ligand (FasL), RANK, RANK Ligand (RANKL), indolamin-2,3-dioxygenase, cytotoxic T-lymphocyte-associated protein 4 - immunoglobulin fusion protein, tumor necrosis factor-related apoptosis-inducing ligand, or a fragment thereof;
a protein associated to intracellular compartments selected from the group comprising histone proteins, lamin A/C, inner membrane mitochondrial protein (IMMT), cytochrome C-1 (CYC1), mitochondrial import receptor subunit TOM20, calnexin, heat shock protein 90kDa beta (Grp94), member 1 (HSP90B1), heat shock 70kDa protein 5 (HSPA5), Golgin A2 (GM130, GOLGA2), Autophagy Related 9A (ATG9A), actinin1, actinin4 (ACTN1, ACTN4), cytokeratin 18 (KRT18), or a fragment thereof.

Further extracellular vesicle associated proteins suitable for the method and the synthetic extracellular vesicles disclosed herein are listed in Table 4.

For bio-orthogonal surface chemistry, suitable functional ligands are selected from the group comprising biotin, N-hydroxysuccinimide ester, N-Hydroxysulfosuccinimide, nitrilotriacetic acid -nickel, amine, pyridyl disulfide, pyridyldithiopropionate, carboxylic acid, maleimide, aromatic maleimid, dithiopyridinyl, N-benzylguanine, cyanuric chloride, and thiol.

Table 2 lists the possible functional ligands that can be attached to the lipids, their function and the interacting moieties.
These functional ligands react with particular moieties at high affinity, as for example the ligand biotin reacts with the moiety streptavidin or avidin. Thus, proteins and other macromolecules of interest can be coupled to the surface of the released extracellular vesicles by using the interaction at high affinity between a functional ligand and the respective reacting moiety.

Thus, a more particular embodiment of the invention is directed to a method for producing synthetic extracellular vesicles comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for conjugation to an extracellular vesicle associated protein;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
d') removing the polymer shell from the polymer shell-stabilized synthetic extracellular vesicles obtained in step d);
e) purifying the synthetic extracellular vesicles by centrifugation;
wherein the amphiphilic copolymer forms a polymer shell stabilizing the synthetic extracellular vesicle, and
wherein the synthetic extracellular vesicles have a diameter comprised between 70 nm and 5000 nm,
wherein the water phase of step a) comprises at least one lipid coupled to a functional ligand selected from biotin, N-hydroxysuccinimide ester, N-Hydroxysulfosuccinimide, nitrilotriacetic acid -nickel, amine, pyridyl disulfide , pyridyldithiopropionate, carboxylic acid, maleimide, aromatic maleimid, dithiopyridinyl, N-benzylguanine, cyanuric chloride, thiol and
wherein the method optionally comprises after step e) the following step:
f) coupling the synthetic extracellular vesicles with at least one macromolecule comprising at least one moiety reacting with one of said functional ligands, wherein the macromolecule is selected from the group comprising an extracellular vesicle associated protein, or a fragment thereof, a carbohydrate, a nucleic acid, a polypeptide, a cell receptor, an imaging probe.

A more particular embodiment of the invention is directed to a method for producing synthetic extracellular vesicles comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for conjugation to an extracellular vesicle associated protein;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
d') removing the polymer shell from the polymer shell-stabilized synthetic extracellular vesicles obtained in step d);
e) purifying the synthetic extracellular vesicles by centrifugation;
wherein the amphiphilic copolymer forms a polymer shell stabilizing the synthetic extracellular vesicle,
wherein the synthetic extracellular vesicles have a diameter comprised between 70 nm and 5000 nm,
wherein the water phase of step a) comprises at least one lipid coupled to a functional ligand selected from biotin, N-hydroxysuccinimide ester, N-Hydroxysulfosuccinimide, nitrilotriacetic acid -nickel, amine, pyridyl disulfide , pyridyldithiopropionate, carboxylic acid, maleimide, aromatic maleimid, dithiopyridinyl, N-benzylguanine, cyanuric chloride, thiol and
wherein the method optionally comprises after step e) the following step:
f) coupling the synthetic extracellular vesicles with at least one macromolecule comprising at least one moiety reacting with one of said functional ligands, wherein the macromolecule is selected from the group comprising an extracellular vesicle associated protein, or a fragment thereof, a carbohydrate, a nucleic acid, a polypeptide, a cell receptor, an imaging probe,
wherein the extracellular vesicle associated protein, or a fragment thereof is selected from the group comprising:
a transmembrane proteins selected from the group comprising tetraspanin proteins CD9, CD37, CD47, CD53, CD63, CD81, CD82, CD151, Tspan8, heterotrimeric G protein subunit alpha (GNA), integrin α - chains and β-chains, transferrin receptor 1 (TfR1, CD71), transferrin receptor 2 (TFR2), lysosome associated membrane proteins (LAMP1, LAMP2), heparan sulfate proteoglycans, syndecans, extracellular matrix metalloproteinase inducer (EMMPRIN, BSG), A Disintegrin And Metalloproteinase Domain 10 (ADAM10), CD3, CD11c, CD14, CD29, CD31, CD41, CD42a, CD44, CD45, CD50 (intercellular adhesion molecule 1, ICAM-1), CD55, CD59, CD73, CD80, CD86, CD90, sonic hedgehog (SHH), major histocompatibility complex I (MHC I), major histocompatibility complex II (MHC II), epidermal growth factor receptor 2 (ERBB2), epithelial cell adhesion molecule (EpCAM), Glycophorin A (GYPA); Acetylcholinesterase S and E (AChE-S, AChE-E), amyloid beta precursor protein (APP), multidrug resistance-associated protein 1 (ABCC1), stem cells antigen-1 (Sca-1), or a fragment thereof;
a cytosolic protein selected from the group comprising the protein complexes endosomal sorting complexes required for transport ESCRT-I, ESCRT-II, and ESCRT-III, tumour susceptibility gene 101 (TSG101), charged multivesicular body protein (CHMP), Apoptosis-Linked Gene 2-Interacting Protein X (ALIX), vacuolar protein sorting 4 homolog A 4A and 4B, arrestin domain-containing protein (ARRDC1), flotillin-1, flotillin-2; caveolins, EH-domain containing 1 - 4 (EHD1 - EHD4), Ras homolog family member A (RHOA), annexins, heat shock proteins, ADP-ribosylation factor 6 (ARF6), syntenin, microtubule-associated protein Tau (MAPT), or a fragment thereof;
a functional protein selected from the group comprising cytokines, growth factors, interleukins, milk fat globule-EGF factor 8 protein (MFGE8), adhesion proteins, extracellular matrix proteins, nicotinamide phosphoribosyltransferase, signal transduction proteins Wnta and Wntb, Fas, Fas Ligand (FasL), RANK, RANK Ligand (RANKL), indolamin-2,3-dioxygenase, cytotoxic T-lymphocyte-associated protein 4 - immunoglobulin fusion protein, tumor necrosis factor-related apoptosis-inducing ligand, or a fragment thereof;
a protein associated to intracellular compartments selected from the group comprising histone proteins, lamin A/C, inner membrane mitochondrial protein (IMMT), cytochrome C-1 (CYC1), mitochondrial import receptor subunit TOM20, calnexin, heat shock protein 90kDa beta (Grp94), member 1 (HSP90B1), heat shock 70kDa protein 5 (HSPA5), Golgin A2 (GM130, GOLGA2), Autophagy Related 9A (ATG9A), actinin1, actinin4 (ACTN1, ACTN4), cytokeratin 18 (KRT18), or a fragment thereof.

### Nucleic acid molecules and miRNA

The phrase **"nucleic acid molecule"** refers to a single or double-stranded polymer of deoxyribonucleotide or ribonucleotide bases. It includes chromosomal DNA and self- replicating plasmids, vectors, DNA, cDNA, mRNA, siRNA, antisense nucleotide sequence, shRNA, piRNA, snRNA, IncRNA, PNA, left handed DNA, Clustered Regularly Interspaced Short Palindromic Repeats (CRISPR) guide RNA.

Abbreviations used in this application include the following: "mRNA" refers to messenger RNA, "miRNA" refers to microRNA, "siRNA" refers to small interfering RNA, "antisense nucleotide sequence" refers to a single stranded sequence that is complementary to a nucleotide sequence of interest, "shRNA" refers to small or short hairpin RNA,"IncRNA" refers to long non-coding RNA, and "dsDNA" refers to double stranded DNA.

As used herein, the term **"microRNAs"** or **"miRNAs"** refers to post- transcriptional regulators that typically bind to complementary sequences in the three prime untranslated regions (3' UTRs) of target messenger RNA transcripts (mRNAs), usually resulting in gene silencing. Typically, miRNAs are short, non-coding ribonucleic acid (RNA) molecules, for example, 21 or 22 nucleotides long. The terms "microRNA" and "miRNA" are used interchangeably.

The content of miRNA molecules is preferably comprised between 75 pg/ 10¹² vesicles - 1000 pg/ 10¹² vesicles, between 75 pg/ 10¹² vesicles - 5000 pg/ 10¹² vesicles, 75pg/ 10¹² vesicles - 10,000 pg/ 10¹² vesicles, between 75 pg/ 10¹² vesicles - 20,000 pg/ 10¹² vesicle, between 75 pg/ 10¹² vesicles - 50,000 pg/ 10¹² vesicles, between 75 pg/ 10¹² vesicles - 100,000 pg/ 10¹² vesicles, between 75 pg/ 10¹² vesicles - 150,000 pg/ 10¹² vesicles, between 75 pg/ 10¹² vesicles - 200,000 pg/ 10¹² vesicles, between 75 pg/ 10¹² vesicles - 300,000 pg/ 10¹² vesicles, between 500 pg/ 10¹² vesicles - 1000 pg/ 10¹² vesicles, between 500 pg/ 10¹² vesicles - 5000 pg/ 10¹² vesicles, 500 pg/ 10¹² vesicles - 10,000 pg/ 10¹² vesicles, between 500 pg/ 10¹² vesicles - 20,000 pg/ 10¹² vesicle, 500 pg/ 10¹² vesicles - 50,000 pg/ 10¹² vesicles, between 500 pg/ 10¹² vesicles - 100,000 pg/ 10¹² vesicles, between 500 pg/ 10¹² vesicles - 150,000 pg/ 10¹² vesicles, between 500 pg/ 10¹² vesicles - 200,000 pg/ 10¹² vesicles, between 500 pg/ 10¹² vesicles - 300,000 pg/ 10¹² vesicles, between 5000 pg/ 10¹² vesicles - 10,000 pg/ 10¹² vesicles, between 5000 pg/ 10¹² vesicles - 20,000 pg/ 10¹² vesicle, 5000 pg/ 10¹² vesicles - 50,000 pg/ 10¹² vesicles, between 5000 pg/ 10¹² vesicles - 100,000 pg/ 10¹² vesicles, between 5000 pg/ 10¹² vesicles - 150,000 pg/ 10¹² vesicles, between 5000 pg/ 10¹² vesicles - 200,000 pg/ 10¹² vesicles, between 5000 pg/ 10¹² vesicles - 300,000 pg/ 10¹² vesicles.

In one aspect, the therapeutic agent is a short interfering RNA, also known as **siRNA.** Methods to prepare and screen interfering RNA and select for the ability to block polynucleotide expression are known in the art and non-limiting examples of which are shown below. These interfering RNA are provided by this invention alone or in combination with a suitable vector or within a host cell. Compositions containing the RNAi are further provided. RNAi is useful to knock-out or knock-down select functions in a cell or tissue as known in the art.

**siRNA sequences** can be designed by obtaining the target mRNA sequence and determining an appropriate siRNA complementary sequence. siRNAs of the invention are designed to interact with a target sequence, meaning they complement a target sequence sufficiently to hybridize to that sequence. An siRNA can be 100% identical to the target sequence. However, homology of the siRNA sequence to the target sequence can be less than 100%) as long as the siRNA can hybridize to the target sequence. Thus, for example, the siRNA molecule can be at least 70%, 75%, 80%, 85%, 90%, 95%, 96%, 97%, 98%, 99% or 100%) identical to the target sequence or the complement of the target sequence. Therefore, siRNA molecules with insertions, deletions or single point mutations relative to a target may also be used. The generation of several different siRNA sequences per target mRNA is recommended to allow screening for the optimal target sequence. A homology search, such as a BLAST search, should be performed to ensure that the siRNA sequence does not contain homology to any known mammalian gene.

As a general guide, siRNAs that include one or more of the following conditions are particularly useful in gene silencing in mammalian cells: GC ratio of between 45-55%, no runs of more than 9 G/C residues, G/C at the 5' end of the sense strand; A/U at the 5' end of the antisense strand; and at least 5 A/U residues in the first 7 bases of the 5' terminal of the antisense strand.

siRNA are, in general, from about 10 to about 30 nucleotides in length. For example, the siRNA can be 10 - 30 nucleotides long, 12 - 28 nucleotides long, 15 - 25 nucleotides long, 19 - 23 nucleotides long, or 21 - 23 nucleotides long. When an siRNA contains two strands of different lengths, the longer of the strands designates the length of the siRNA. In this situation, the unpaired nucleotides of the longer strand would form an overhang.

The term siRNA includes short hairpin RNAs (shRNAs). **shRNAs** comprise a single strand of RNA that forms a stem-loop structure, where the stem consists of the complementary sense and antisense strands that comprise a double-stranded siRNA, and the loop is a linker of varying size. The stem structure of shRNAs generally is from about 10 to about 30 nucleotides long. For example, the stem can be 10 - 30 nucleotides long, 12 - 28 nucleotides long, 15 - 25 nucleotides long, 19 - 23 nucleotides long, or 21 - 23 nucleotides long.

Tools to assist siRNA design are readily available to the public. For example, a computer-based siRNA design tool is available on the internet at www.dharmacon.com.

In some embodiments, the extracellular vesicle or exosome delivers their nucleic acid or intracellular protein or functional protein to a cell target. The delivery can occur in vitro or in a subject.

Preferentially, the extracellular vesicles disclosed herein include miRNA molecules selected from the group comprising miR-17, miR-18a, miR-19a, miR-19b-1, miR-20a, miR-92a; miR-21, miR-30d-5p, miR-33b, miR-124, miR-125, miR-126, miR-130, miR-132, miR-133b, miR-140-5p, miR-191, miR-222, miR-451, miR-494, miR-575, miR-630, miR-638, miR-1202, miR-1207-5p, miR-1225-5p, miR-1268, miR-6087, miR-92a-3p-e, miR-K12-3, let-7a.

Furthermore, the extracellular vesicles disclosed herein can include one or more miRNA molecules as listed in Table 3.

Thus, one embodiment of the present invention is directed to a method for producing synthetic extracellular vesicles comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for conjugation to an extracellular vesicle associated protein;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
wherein the amphiphilic copolymer forms a polymer shell stabilizing the synthetic extracellular vesicle, and
wherein the synthetic extracellular vesicles have a diameter between 70 nm and 5000 nm, and
wherein the water phase of step a) comprises one or more nucleic acid molecules selected from the group comprising miRNA molecules miR-17, miR-18a, miR-19a, miR-19b-1, miR-20a, miR-92a; miR-21, miR-30d-5p, miR-33b, miR-124, miR-125, miR-126, miR-130, miR-132, miR-133b, miR-140-5p, miR-191, miR-222, miR-451, miR-494, miR-575, miR-630, miR-638, miR-1202, miR-1207-5p, miR-1225-5p, miR-1268, miR-6087, miR-92a-3p-e, miR-K12-3, let-7a.

Another embodiment of the present invention is directed to a method for producing synthetic extracellular vesicles comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for conjugation to an extracellular vesicle associated protein;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
wherein the amphiphilic copolymer forms a polymer shell stabilizing the synthetic extracellular vesicle, and
wherein the synthetic extracellular vesicles have a diameter between 70 nm and 5000 nm, and
wherein the water phase of step a) comprises one or more nucleic acid molecules selected from the group comprising chromosomal DNA and self- replicating plasmids, vectors, DNA, cDNA, mRNA, siRNA, antisense nucleotide sequence, shRNA, piRNA, snRNA, IncRNA, PNA, left handed DNA, Clustered Regularly Interspaced Short Palindromic Repeats (CRISPR) guide RNA.

A further embodiment of the present invention is directed to a method for producing synthetic extracellular vesicles comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for conjugation to an extracellular vesicle associated protein;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
wherein the amphiphilic copolymer forms a polymer shell stabilizing the synthetic extracellular vesicle, and
wherein the synthetic extracellular vesicles have a diameter between 70 nm and 5000 nm, and
wherein the water phase of step a) comprises one or more nucleic acid molecules selected from the group comprising miRNA molecules miR-17, miR-18a, miR-19a, miR-19b-1, miR-20a, miR-92a; miR-21, miR-30d-5p, miR-33b, miR-124, miR-125, miR-126, miR-130, miR-132, miR-133b, miR-140-5p, miR-191, miR-222, miR-451, miR-494, miR-575, miR-630, miR-638, miR-1202, miR-1207-5p, miR-1225-5p, miR-1268, miR-6087, miR-92a-3p-e, miR-K12-3, let-7a, and
wherein the extracellular vesicle associated protein, or a fragment thereof is selected from the group comprising:
a transmembrane proteins selected from the group comprising tetraspanin proteins CD9, CD37, CD47, CD53, CD63, CD81, CD82, CD151, Tspan8, heterotrimeric G protein subunit alpha (GNA), integrin α - chains and β-chains, transferrin receptor 1 (TfR1, CD71), transferrin receptor 2 (TFR2), lysosome associated membrane proteins (LAMP1, LAMP2), heparan sulfate proteoglycans, syndecans, extracellular matrix metalloproteinase inducer (EMMPRIN, BSG), A Disintegrin And Metalloproteinase Domain 10 (ADAM10), CD3, CD11c, CD14, CD29, CD31, CD41, CD42a, CD44, CD45, CD50 (intercellular adhesion molecule 1, ICAM-1), CD55, CD59, CD73, CD80, CD86, CD90, sonic hedgehog (SHH), major histocompatibility complex I (MHC I), major histocompatibility complex II (MHC II), epidermal growth factor receptor 2 (ERBB2), epithelial cell adhesion molecule (EpCAM), Glycophorin A (GYPA); Acetylcholinesterase S and E (AChE-S, AChE-E), amyloid beta precursor protein (APP), multidrug resistance-associated protein 1 (ABCC1), stem cells antigen-1 (Sca-1), or a fragment thereof;
a cytosolic protein selected from the group comprising the protein complexes endosomal sorting complexes required for transport ESCRT-I, ESCRT-II, and ESCRT-III, tumour susceptibility gene 101 (TSG101), charged multivesicular body protein (CHMP), Apoptosis-Linked Gene 2-Interacting Protein X (ALIX), vacuolar protein sorting 4 homolog A 4A and 4B, arrestin domain-containing protein (ARRDC1), flotillin-1, flotillin-2; caveolins, EH-domain containing 1 - 4 (EHD1 - EHD4), Ras homolog family member A (RHOA), annexins, heat shock proteins, ADP-ribosylation factor 6 (ARF6), syntenin, microtubule-associated protein Tau (MAPT), or a fragment thereof;
a functional protein selected from the group comprising cytokines, growth factors, interleukins, milk fat globule-EGF factor 8 protein (MFGE8), adhesion proteins, extracellular matrix proteins, nicotinamide phosphoribosyltransferase, signal transduction proteins Wnta and Wntb, Fas, Fas Ligand (FasL), RANK, RANK Ligand (RANKL), indolamin-2,3-dioxygenase, cytotoxic T-lymphocyte-associated protein 4 - immunoglobulin fusion protein, tumor necrosis factor-related apoptosis-inducing ligand, or a fragment thereof;
a protein associated to intracellular compartments selected from the group comprising histone proteins, lamin A/C, inner membrane mitochondrial protein (IMMT), cytochrome C-1 (CYC1), mitochondrial import receptor subunit TOM20, calnexin, heat shock protein 90kDa beta (Grp94), member 1 (HSP90B1), heat shock 70kDa protein 5 (HSPA5), Golgin A2 (GM130, GOLGA2), Autophagy Related 9A (ATG9A), actinin1, actinin4 (ACTN1, ACTN4), cytokeratin 18 (KRT18), or a fragment thereof.

A more particular embodiment of the invention is directed to a method for producing synthetic extracellular vesicles comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for conjugation to an extracellular vesicle associated protein;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
d') removing the polymer shell from the polymer shell-stabilized synthetic extracellular vesicles obtained in step d);
e) purifying the synthetic extracellular vesicles by centrifugation;
wherein the amphiphilic copolymer forms a polymer shell stabilizing the synthetic extracellular vesicle, and
wherein the synthetic extracellular vesicles have a diameter comprised between 70 nm and 5000 nm,
wherein the water phase of step a) comprises at least one lipid coupled to a functional ligand selected from biotin, N-hydroxysuccinimide ester, N-Hydroxysulfosuccinimide, nitrilotriacetic acid -nickel, amine, pyridyl disulfide , pyridyldithiopropionate, carboxylic acid, maleimide, aromatic maleimid, dithiopyridinyl, N-benzylguanine, cyanuric chloride, thiol and
wherein the method optionally comprises after step e) the following step:
f) coupling the synthetic extracellular vesicles with at least one macromolecule comprising at least one moiety reacting with one of said functional ligands, wherein the macromolecule is selected from the group comprising an extracellular vesicle associated protein, or a fragment thereof, a carbohydrate, a nucleic acid, a polypeptide, a cell receptor, an imaging probe, and
wherein the water phase of step a) comprises one or more nucleic acid molecules selected from the group comprising miRNA molecules miR-17, miR-18a, miR-19a, miR-19b-1, miR-20a, miR-92a; miR-21, miR-30d-5p, miR-33b, miR-124, miR-125, miR-126, miR-130, miR-132, miR-133b, miR-140-5p, miR-191, miR-222, miR-451, miR-494, miR-575, miR-630, miR-638, miR-1202, miR-1207-5p, miR-1225-5p, miR-1268, miR-6087, miR-92a-3p-e, miR-K12-3, let-7a, and
wherein the extracellular vesicle associated protein, or a fragment thereof is selected from the group comprising:
a transmembrane proteins selected from the group comprising tetraspanin proteins CD9, CD37, CD47, CD53, CD63, CD81, CD82, CD151, Tspan8, heterotrimeric G protein subunit alpha (GNA), integrin α - chains and β-chains, transferrin receptor 1 (TfR1, CD71), transferrin receptor 2 (TFR2), lysosome associated membrane proteins (LAMP1, LAMP2), heparan sulfate proteoglycans, syndecans, extracellular matrix metalloproteinase inducer (EMMPRIN, BSG), A Disintegrin And Metalloproteinase Domain 10 (ADAM10), CD3, CD11c, CD14, CD29, CD31, CD41, CD42a, CD44, CD45, CD50 (intercellular adhesion molecule 1, ICAM-1), CD55, CD59, CD73, CD80, CD86, CD90, sonic hedgehog (SHH), major histocompatibility complex I (MHC I), major histocompatibility complex II (MHC II), epidermal growth factor receptor 2 (ERBB2), epithelial cell adhesion molecule (EpCAM), Glycophorin A (GYPA); Acetylcholinesterase S and E (AChE-S, AChE-E), amyloid beta precursor protein (APP), multidrug resistance-associated protein 1 (ABCC1), stem cells antigen-1 (Sca-1), or a fragment thereof;
a cytosolic protein selected from the group comprising the protein complexes endosomal sorting complexes required for transport ESCRT-I, ESCRT-II, and ESCRT-III, tumour susceptibility gene 101 (TSG101), charged multivesicular body protein (CHMP), Apoptosis-Linked Gene 2-Interacting Protein X (ALIX), vacuolar protein sorting 4 homolog A 4A and 4B, arrestin domain-containing protein (ARRDC1), flotillin-1, flotillin-2; caveolins, EH-domain containing 1 - 4 (EHD1 - EHD4), Ras homolog family member A (RHOA), annexins, heat shock proteins, ADP-ribosylation factor 6 (ARF6), syntenin, microtubule-associated protein Tau (MAPT), or a fragment thereof;
a functional protein selected from the group comprising cytokines, growth factors, interleukins, milk fat globule-EGF factor 8 protein (MFGE8), adhesion proteins, extracellular matrix proteins, nicotinamide phosphoribosyltransferase, signal transduction proteins Wnta and Wntb, Fas, Fas Ligand (FasL), RANK, RANK Ligand (RANKL), indolamin-2,3-dioxygenase, cytotoxic T-lymphocyte-associated protein 4 - immunoglobulin fusion protein, tumor necrosis factor-related apoptosis-inducing ligand, or a fragment thereof;
a protein associated to intracellular compartments selected from the group comprising histone proteins, lamin A/C, inner membrane mitochondrial protein (IMMT), cytochrome C-1 (CYC1), mitochondrial import receptor subunit TOM20, calnexin, heat shock protein 90kDa beta (Grp94), member 1 (HSP90B1), heat shock 70kDa protein 5 (HSPA5), Golgin A2 (GM130, GOLGA2), Autophagy Related 9A (ATG9A), actinin1, actinin4 (ACTN1, ACTN4), cytokeratin 18 (KRT18), or a fragment thereof.

### Lipid Composition

Lipids are the major scaffolding components of extracellular vesicles such as exosomes, and pivotal for their signaling capabilities. Therefore, synthetic extracellular vesicles were assembled with lipid compositions resembling those found in natural synthetic extracellular vesicles (**Figure 2**), although the technology allows for the integration of an almost unrestricted number of possible lipid types into synthetic extracellular vesicles membrane.

The lipid composition of the final synthetic extracellular vesicles can be easily fine-tuned on the basis of the composition of the initial lipid solution, as no lipid ratio change was observed during the emulsification and release procedures.

This technology also allows to finely regulating the charge of the synthetic extracellular vesicles by adjusting the ratio of cationic, neutral and anionic lipids.

In some embodiments, the molar percentage (mol %) of a **cationic lipid** typically comprises from 0% to 10%, from 10% to 20%, from 10% to 30%, from 10% to 40%, %, from 10% to 50%, from 10% to 60%, from 20% to 30%, from 20% to 40%, from 20% to 50%, from 20% to 60% of the total lipid present in vesicle.

In some embodiments, the molar percentage (mol %) of an **anionic lipid** typically comprises from 0% to 10%, from 10% to 20%, from 10% to 30%, from 10% to 40%, %, from 10% to 50%, from 10% to 60%, from 20% to 30%, from 20% to 40%, from 20% to 50%, from 20% to 60% of the total lipid present in vesicle.

In some embodiments, the molar percentage (mol %) of **neutral lipid** typically comprises from 49% to 99%, from 49% to 89%, from 49% to 79%, from 49% to 69%, %, from 59% to 99%, from 59% to 89%, from 59% to 79%, from 59% to 69% of the total lipid present in vesicle.

The present invention is not particularly limited concerning the chemical nature of the at least one **lipid** contained in the water phase of step a) and thus in the inner space of the **polymer shell stabilized synthetic extracellular vesicle**, as long as it is able to form a lipid bilayer. Good results are in particular achieved with phospholipids and in particular with a lipid being selected from the group comprising phosphocholine, phosphocholine derivatives, phosphoethanolamine, phosphoethanolamine derivatives, phosphatidylcholine, phosphatidylcholine derivatives, phosphatidylglycerol, phosphatidylglycerol derivatives and arbitrary combinations of two or more of the aforementioned lipids.

At least one of the lipids is an **amphiphilic lipid**, defined as having a hydrophilic and a hydrophobic portion, typically a hydrophilic head and a hydrophobic tail. The hydrophobic portion typically orients into a hydrophobic phase, e.g., within the bilayer, while the hydrophilic portion typically orients toward the aqueous phase, e.g., outside the bilayer, and possibly between adjacent apposed bilayer surfaces. The hydrophilic portion may comprise polar or charged groups such as carbohydrates, phosphate, carboxylic, sulfato, amino, sulfhydryl, nitro, hydroxy and other like groups. The hydrophobic portion may comprise apolar groups that include without limitation long chain saturated and unsaturated aliphatic hydrocarbon groups and groups substituted by one or more aromatic, cycloaliphatic or heterocyclic groups. Examples of amphipathic lipids include, but are not limited to, phospholipids, aminolipids and sphingolipids.

Typically, the lipids are phospholipids. Phospholipids include without limitation phosphatidylcholine, phosphatidylethanolamine, phosphatidylglycerol, phosphatidylinositol, phosphatidylserine, and their derivatives. It is to be understood that other lipid membrane components, such as cholesterol, sphingomyelin, cardiolipin, etc. may be used.

Lipids can be **"uncharged lipids"** or **"charged lipids." "Uncharged lipids"** refer to lipids that do not carry any charged or ionizable groups such as phosphate groups or choline groups. Examples of uncharged lipids include, but are not limited to, diacyl glycerols and prostaglandins.

**"Charged lipids"** include neutrally charged, i.e. **zwitterionic lipids, cationic lipids** and **anionic lipids.** Generally, lipids bearing a net positive or negative charge exhibit poor solubility in oil phases.

**Neutral lipids** exist in an uncharged or neutral zwitterionic form at a selected pH.

**"Zwitterionic lipids"** carry both positively-charged groups and ionizable groups such as amino groups and choline groups that bear a net positive charge, and negatively-charged groups and ionizable groups, such as phosphates, sulfates and carboxylates. Examples of zwitterionic lipids include, but are not limited to, phosphorylcholine and phosphorylethanolamine

**"Anionic lipids"** are lipids negatively charged at physiological pH. **"Cationic lipids"** are lipids positively charged at physiological pH.

Further suitable lipids are **pH sensitive lipids.** A **"pH-sensitive"** lipid refers to a lipid whose ability to form and/or maintain formation of a lipid bilayer depends at least in part on the pH of the surrounding environment. Synthetic extracellular vesicles containing such lipids are destabilized under acidic conditions of the endocytotic pathway. Therefore, the encapsulated content is delivered into the intracellular bio-environment through destabilization or its fusion with the endosomal membrane.

Specific examples of the lipids suitable to synthetize the synthetic extracellular vesicles according to the method disclosed herein are listed in Table 1.

Preferably, the **lipids are biodegradable** in order **to allow release of the internal proteins or nucleic acid molecules** in vivo and/or in vitro. Biodegradable lipids include but are not limited to 1,2-dioleoyl-sn-glycero-3-phosphocholine (dioleoyl-phosphocholine, DOPC), anionic 1,2-di-(9Z-octadecenoyl)-sn-glycero-3-phospho-(1'-rac-glycerol) (dioleoyl-phosphoglycerol, DOPG), and 1,2-distearoyl-sn-glycero-3-phosphoethanolamine (distearoyl-phosphoethanolamine, DSPE).

### Functionalized Lipids

According to an embodiment of the present invention, the at least one lipid comprised in the water phase of step a) is a lipid coupled with a **functional** ligand and/or with polyethylenglycol. Specific examples of the suitable functional ligands, the reacting moieties, and of the functionalized lipids containing are listed in Table 2.

Functionalized and non-functionalized lipids are available from a number of **commercial sources** including Avanti Polar Lipids (Alabaster, Alabama).

**Table 1: Suitable lipids**

| | **Class** | **Specific example** | **Abbreviation** |
|---|---|---|---|
| **Neutral lipids** | ceramide | | Cer |
| | sphingomyelin | Egg sphingomyelin, brain sphingomyelin, Milk sphingomyelin, Lyso sphingomyelin, | SM |
| | cholesterol | | Chol |
| | cerebrosides | Galactocerebroside, Glucocerebroside | Gal-Cer, Glc-Cer |
| | diacylglycerols | 1-oleoyl-2-acetyl-sn-glycerol | DAG, DG |
| | phosphatidylcholines | egg L-α-phosphatidylcholine | EggPC |
| | | distearoylphosphatidylcholine | DSPC |
| | | 1-palmitoyl-2-oleoyl-sn-glycero-3-phosphocholine | POPC |
| | | 1,2-dimyristoyl-sn-glycero-3-phosphocholine | DMPC |
| | | 1,2-dipalmitoyl-sn-glycero-3-phosphocholine | DPPC |
| | | 1,2-dioleoyl-sn-glycero-3-phosphocholine | 18:1 DOPC |
| | | dioleoylphosphatidylglycerol | DOPG |
| | | dipalmitoylphosphatidylglycerol | DPPG |
| | | palmitoyloleyolphosphatidylglycerol | POPG |
| | lysophosphatidylcholines | 1-palmitoyl-sn-glycero-3-phosphocholine | PC(16:0/0:0) |
| | phosphatidylethanolamines (also named "cephalin") | 1-stearoyl-2-oleoyl-sn-glycero-3-phosphoethanolamine | SOPE, 18:0-18:1 PE |
| | | 1,2-dimyristoyl-sn-glycero-3-phosphoethanolamine | DMPE, 14:0 PE |
| | | 1,2-dipalmitoyl-sn-glycero-3-phosphoethanolamine | DPPE |
| | | 1,2-dioleoyl-sn-glycero-3-phosphoethanolamine | 18:1 DOPE |
| | lysophosphatidylethanolamine | 1,2-Distearoyl-sn-glycero-3-phosphoethanolamine | DSPE |
| | | palmitoyloleoyl-phosphatidylethanolamine | POPE |
| | lysoethanolamines | 1-stearoyl-sn-glycero-3-phosphoethanolamine | 18:0 Lyso PE, egg Lyso PE |
| | Inverted Headgroups | 2-((2,3-bis(oleoyloxy)propyl)dimethylammonio)ethyl hydrogen phosphate | DOCP |
| | | 2-((2,3-bis(oleoyloxy)propyl)dimethylammonio)ethyl ethyl phosphate | DOCPe |
| | Sphingosin | (R,E)-2-aminooctadec-4-en-1-ol | 3-deoxy sphingosine |
| | | (2S,3S,4E)-2-aminooctadec-4-ene-1,3-diol | L-threo-sphingosine (d18:1) |
| | | D-erythro-sphingosine | Sphingosine (d18:1) |
| | | D-erythro-sphingosine (C17 base) | Sphingosine (d17:1) |
| | | D-erythro-sphingosine (C20 base) | Sphingosine (d20:1) |
| | | D-erythro-Sphingosine (C22 base) | Sphingosine (d22:1) |
| | | (2S,3R,4E,14Z)-2-aminooctadec-4,14-diene-1,3-diol | 4E,14Z-Sphingadiene |
| | | (2S,3R,4E,8Z)-2-aminooctadec-4,8-diene-1,3-diol | 4E,8Z-Sphingadiene |
| | | (2S,3R,4E,11Z)-2-aminooctadec-4,11-diene-1,3-diol | 4E,11Z-Sphingadiene |
| | | D-erythro-Sphingosine (C16 base) | Sphingosine (d16:1) |
| | | D-erythro-Sphingosine (C14 Base) | Sphingosine (d14:1) |
| | | Mito-Caged Sphingosine | Mito-So |
| | Sterol-modified phospholipids | 1-palmitoyl-2-cholesterylhemisuccinoyl-sn-glycero-3-phosphocholine | PChemsPC |
| | | 1-oleoyl-2-cholesterylhemisuccinoyl-sn-glycero-3-phosphocholine | OChems PC |
| | | 1-palmitoyl-2-cholesterylcarbonoyl-sn-glycero-3-phosphocholine, | PChcPC |
| | | 1,2-dicholesterylhemisuccinoyl-sn-glycero-3-phosphocholine, | DChemsPC |
| | Ether ester lipids | 1-O-heptadecyl-2-acetyl-sn-glycero-3-phosphocholine, | C17 PAF, C17-02:0 PC (Phosphocholine), |
| | | 1-O-hexadecyl-2-acetyl-sn-glycero-3-phosphocholine, | C16-02:0 PC, |
| | | 1-O-hexadecyl-2-oleoyl-sn-glycero-3-phosphocholine, | C16-18:1 PC, |
| | | 1-O-hexadecyl-2-arachidonoyl-sn-glycero-3-phosphocholine, | C16-20:4 PC, |
| | | 1-O-octadecyl-2-acetyl-sn-glycero-3-phosphocholine, | C18-02:0 PC, |
| | | 1-O-hexadecyl-2-butyryl-sn-glycero-3-phosphocholine, | C16-04:0 PC, |
| | | 1-O-octadecyl-2-butyryl-sn-glycero-3-phosphocholine, | C18-04:0 PC, |
| | | 1-O-hexadecyl-2-(8Z,11Z,14Z-eicosatrienoyl)-sn-glycero-3-phosphocholine, | C16-20:3 PC, |
| | | 1-O-hexadecyl-2-(5Z,8Z,11Z,14Z,17Z-eicosapentaenoyl)-sn-glycero-3-phosphocholine, | C16-20:5 PC, |
| | | 1-O-hexadecyl-2-docosahexaenoyl-sn-glycero-3-phosphocholine | C16-22:6 PC, |
| | | 1-hexadecyl-2-(9Z-octadecenoyl)-sn-glycero-3-phosphoethanolamine | C16-18:1 PE |
| | Diether Lipids | 1-O-hexadecanyl-2-O-(9Z-octadecenyl)-sn-glycero-3-phospho-(1'-rac-glycerol) (ammonium salt) | 16:0-18:1 Diether PG, |
| | | 1-O-hexadecanyl-2-O-(9Z-octadecenyl)-sn-glycero-3-phosphoethanolamine | 16:0-18:1 Diether PE |
| | | 1-O-hexadecanyl-2-O-(9Z-octadecenyl)-sn-glycero-3-phosphocholine | 16:0-18:1 Diether PC |
| | | 1,2-di-O-(9Z-octadecenyl)-sn-glycero-3-phosphocholine | 18:1 Diether PC |
| | | 1,2-di-O-octadecyl-sn-glycero-3-phosphocholine | 18:0 Diether PC |
| | | 1,2-di-O-hexadecyl-sn-glycero-3-phosphocholine | 16:0 Diether PC |
| | | 1-O-octadecyl-2-O-methyl-sn-glycero-3-phosphocholine | Edelfosine |
| | Vinyl Ether (Plasmalogen) | 1-(1Z-hexadecenyl)-sn-glycero-3-phosphocholine | C16(Plasm) LPC |
| | | 1-O-1'-(Z)-octadecenyl-2-hydroxy-sn-glycero-3-phosphocholine, | C18(Plasm) LPC |
| | | 1-(1Z-octadecenyl)-2-oleoyl-sn-glycero-3-phosphocholine | C18(Plasm)-18:1 PC |
| | | 1-(1Z-octadecenyl)-2-arachidonoyl-sn-glycero-3-phosphocholine | C18(Plasm)-20:4 PC |
| | | 1-O-1'-(Z)-octadecenyl-2-hydroxy-sn-glycero-3-phosphoethanolamine | C18(Plasm) LPE |
| | | 1-(1Z-octadecenyl)-2-docosahexaenoyl-sn-glycero-3-phosphocholine | C18(Plasm)-22:6 PC |
| | | 1-(1Z-octadecenyl)-2-oleoyl-sn-glycero-3-phosphoethanolamine | C18(Plasm)-18:1 PE |
| | | 1-(1Z-octadecenyl)-2-arachidonoyl-sn-glycero-3-phosphoethanolamine | C18(Plasm)-20:4 PE |
| | | 1-(1Z-octadecenyl)-2-docosahexaenoyl-sn-glycero-3-phosphoethanolamine | C18(Plasm)-22:6 PE |
| | N-Acylglycine | N-palmitoylglycine | |
| | | N-arachidonoylglycine | |
| | | N-oleoylglycine | |
| | Very Long Chain Fatty Acids (VLCFA) | 14Z,17Z,20Z,23Z,26Z,29Z-dotriacontahexaenoic acid | C32:6 fatty acid |
| | Prenols | Coenzyme Q6 (S. cerevisiae) | CoQ6 |
| | | Coenzyme Q8 (E. coli) | CoQ8 |
| | | Dolichol Mixture (13∼21) | |
| | | Polyprenol mixture (13∼21) | |
| | | Polyprenal mixture (13∼21) | |
| | Prostaglandins | Prostaglandin E1 | PGE1 |
| | | Prostaglandin F1α | PGF1α |
| | | Prostaglandin F2α (15 beta epimer) | 15-beta PGF2α |
| | | Prostaglandin F1β | PGF1β |
| | | Prostaglandin F1α(15 beta epimer) | 15beta-PGF1α |
| | | Prostaglandin F1α-d9 | PGF1α-d9 |
| | | Prostaglandin E1-d9 | PGE1-d9 |
| | | Prostaglandin E2 Ethanolamide | PGE2-EA |
| | | Prostaglandin A1 | PGA1 |
| | | Prostaglandin E2 | PGE2 |
| | | Prostaglandin F2β | PGF2β |
| | | Prostaglandin B1 | PGB1 |
| | | Prostaglandin F2α | PGF2α |
| | | 15-keto Prostaglandin F2α | 15-keto PGF2α |
| | Glycosylated Diacyl Glycerols | 1,2-diacyl-3-O-(α-D-glucopyranosyl)-sn-glycerol (E. coli) | MGlc-DAG |
| | | 1-oleoyl-2-palmitoyl-3-(α-D-galactosyl)-sn-glycerol | BbGL-2 |
| | | 1-palmitoyl-2-oleoyl-3-(β-D-glucosyl)-sn-glycerol | 16:0-18:1 DG glucose |
| | Eicosanoids | 5-Oxo-6E,8Z,11Z,14Z-eicosatetraenoic acid | 5-OxoETE |
| | | 17(S)-hydroxy Docosahexaenoic acid | 17(S)-HDHA |
| | | (±)14(15)-epoxy-5Z,8Z,11Z-eicosatrienoic acid | 14(15) EET |
| | | 15S-hydroxy-5Z,8Z,11Z,13E-eicosatetraenoic acid | 15(S)-HETE |
| | | 15(S)-hydroxy-N-(2-hydroxyethyl)-5Z,8Z,11Z,13E-eicosatetraenamide | 15(S)-HAEA |
| | | 13S-Hydroxy-9Z,11 E-octadecadienoic acid | 13(S)HODE |
| | | 13S-Hydroxy-N-(2-hydroxyethyl)-9Z,11 E-octadecadienamide | 13(S)HODE |
| | | | Ethanolamide |
| | Palmitic Acid-Hydroxy Stearic Acid, PAHSA | 9-(palmitoyloxy)octadecanoic acid | 9-PAHSA |
| | | 5-(palmitoyloxy)octadecanoic acid | 5-PAHSA |
| | | 9'-(palmitoyloxy)octadecanoic acid | 12-PAHSA |
| | | 1-palmitoyl-2-[9' -(palmitoyloxy)octadecanoyl]-sn-glycero-3-phosphoholine | 16:0-(12-PAHSA) PC |
| Anionic lipids | phosphatidic acids | 1-palmitoyl-2-oleoyl-sn-glycero-3-phosphate | 16:0-18:1 PA, POPA |
| | lysophosphatidic acids | 1-oleoyl-2-hydroxy-sn-glycero-3-phosphate | 18:1 Lyso PA |
| | | 1-stearoyl-2-hydroxy-sn-glycero-3-phosphate | 18:0 Lyso PA |
| | | 1-heptadecanoyl-2-hydroxy-sn-glycero-3-phosphate (sodium salt) | 17:0 Lyso PA |
| | | 1-arachidonoyl-2-hydroxy-sn-glycero-3-phosphate | 20:4 Lyso PA |
| | | 1-palmitoyl-2-hydroxy-sn-glycero-3-phosphate (sodium salt) | 16:0 Lyso PA |
| | | 1-myristoyl-2-hydroxy-sn-glycero-3-phosphate (sodium salt) | 14:0 Lyso PA |
| | phosphatidylglycerols | Egg L-α-phosphatidylglycerol | EggPG |
| | | 1,2-di-(9Z-octadecenoyl)-sn-glycero-3-phospho-(1'-rac-glycerol), or L-α-Phosphatidyl-DL-glycerol | 18:1 DOPG |
| | | 1-palmitoyl-2-oleoyl-sn-glycero-3-phospho-(1'-rac-glycerol) | POPG |
| | lysophosphatidylglycerols | 1-palmitoyl-2-hydroxy-sn-glycero-3-phospho-(1'-rac-glycerol) | 16:0 Lyso PG |
| | phosphatidylserines | 1,2-dioleoyl-sn-glycero-3-phospho-L-serine | DOPS |
| | | 1-stearoyl-2-oleoyl-sn-glycero-3-phospho-L-serine | SOPS |
| | lysophosphatidylserines | 1-stearoyl-sn-glycero-3-phospho-L-serine | PS (18:1/18:0) |
| | phosphatidylinositols | 1-stearoyl-2-arachidonoyl-sn-glycero-3-phospho-(1'-myo-inositol) | 18:0/20:4-PI |
| | phosphatidylinositolphosphates | 1-stearoyl-2-arachidonoyl-sn-glycero-3-phospho-(1'-myo-inositol-4'phosphate) | 18:0-20:4 PI(4)P |
| | | 1-stearoyl-2-arachidonoyl-sn-glycero-3-phospho-(1'-myo-inositol-4',5'-bisphosphate) | 18:0-20:4 PI(4,5)P2 |
| | | phosphatidylinositol 4,5-bisphosphate | PIP2 |
| | cardiolipins | 1',3'-bis[1,2-dilinoleoyl-sn-glycero-3-phospho]-sn-glycerol. | 18:1 Cardiolipin |
| | | 1',3'-bis[1,2-dimyristoleoyl-sn-glycero-3-phospho]-glycerol | 14:1 Cardiolipin |
| | | 1',3'-bis[1,2-dipalmitoyl-sn-glycero-3-phospho]-glycerol | 16:0 Cardiolipin |
| | | 1',3'-bis[1-Palmitoyl-2-oleoyl-sn-glycero-3-phospho]-glycerol | 16:0-18:1 Cardiolipin |
| | | 1',3'-bis[1,2-dipalmitoleoyl-sn-glycero-3-phospho]-glycerol | 16:1 Cardiolipin |
| | | 1',3'-bis[1,2-Distearoyl-sn-glycero-3-phospho]-glycerol | 18:0 Cardiolipin |
| | Bis(Monoacylglycero)Phosphate (BMP) | bis(monomyristoylglycero)phosphate (S,R Isomer) (ammonium salt), | 14:0 BMP (S,R) |
| | | sn-(3-myristoyl-2-hydroxy)-glycerol-1-phospho-sn-3'-(1',2'-dimyristoyl)-glycerol (ammonium salt) | 14:0 Hemi BMP (S,R) |
| | | bis(monooleoylglycero)phosphate (S,R Isomer) (ammonium salt) | 18:1 BMP (S,R) |
| | | sn-(3-oleoyl-2-hydroxy)-glycerol-1-phospho-sn-3'-(1',2'-dioleoyl)-glycerol (ammonium salt) | 18:1 Hemi BMP (S,R) |
| | | sn-(3-oleoyl-2-hydroxy)-glycerol-1-phospho-sn-1'-(3'-oleoyl-2'-hydroxy)-glycerol (ammonium salt) | 18:1 BMP (S,S) |
| | | sn-(1-oleoyl-2-hydroxy)-glycerol-3-phospho-sn-3'-(1'-oleoyl-2'-hydroxy)-glycerol (ammonium salt) | 18:1 BMP (R,R) |
| | | sn-[2,3-dioleoyl]-glycerol-1-phospho-sn-1'-[2',3'-dioleoyl]-glycerol (ammonium salt) | 18:1 BDP (S,S) |
| Cationic lipids | | 2,3-dioleyloxy-N-[2(sperminecarboxamido) ethyl]-N,N-dimethyl-1-propanaminium trifluoroacetate | DOSPA |
| | | 1,2-dimyristyloxypropyl-3-dimethyl-hydroxy ethyl ammonium bromide | DMRIE |
| | | N-(2,3-dioleyloxy)propyl)-N,N-dimethylammonium chloride | DODMA |
| | | dioctadecylamidoglycyl carboxyspermine | DOGS |
| | | 1,2-Dioleoyl-3-dimethylammonium-propane | DODAP |
| | | dioleyl-N,N-dimethylammonium chloride | DODAC |
| | | N-(2,3-dioleyloxy)propyl)-N,N,N-trimethylammonium chloride | DOTMA |
| | | N,N-distearyl-N,N-dimethylammonium bromide | DDAB |
| | | 1,2-dioleoyl-3-trimethylammonium-propane | 18:1 DOTAP |
| | | 3β-(N-(N',N'-dimethylaminoethane)-carbamoyl)cholesterol | DC-Chol |
| pH sensitive lipids | | N-(4-carboxybenzyl)-N,N-dimethyl-2,3-bis(oleoyloxy)propan-1-aminium | DOBAQ (cationic) |
| | | 1,2-distearoyl-3-dimethylammonium-propane | DAP (cationic) |
| | | 1,2-dipalmitoyl-sn-glycero-3-succinate | 16:0 DGS |
| | | 1,2-dioleoyl-sn-glycero-3-succinate | 18:1 DGS |
| | | N-palmitoyl homocysteine | PHC |
| **Biodegradable lipids** | | 1,2-dioleoyl-sn-glycero-3-phosphocholine | DOPC |
| | | 1,2-di-(9Z-octadecenoyl)-sn-glycero-3-phospho-(1'-rac-glycerol) | DOPG |
| | | 1,2-distearoyl-sn-glycero-3-phosphoethanolamine | DSPE |
| photoswitchable lipid | | N-[(E)-4-(4-((4-butylphenyl)diazenyl)phenyl)butanoyl]-D-erythro-sphingosine | ACe-1 |
| | | 1-stearoyl-2-[(E)-4-(4-((4-butylphenyl)diazenyl)phenyl)butanoyl]-sn-glycerol | 18:0-PhoDAG |
| | | 1-stearoyl-2-[(E)-4-(4-((4-butylphenyl)diazenyl)phenyl) butanoyl]-sn-glycero-3-phosphocholine | 18:0-azo PC |
| | | N-[(E)-4-(4-((4-butylphenyl)diazenyl)phenyl)butanoyl]-D-erythro-sphingosylphosphorylcholine | Azo SM |
| | | (E)-4-(4-((4-butylphenyl)diazenyl)phenyl)-N-(3-hydroxy-4-methoxybenzyl)butanamide | Trans-AzCA4 |
| | | 4-Butyl-Azo-4:0-Acid-1 | Trans-F AAzo-4 |
| | | 1-(E)-4-(4-((4-butylphenyl)diazenyl)phenyl)butanoyl]-2-hydroxy-sn-glycero-3-phosphate | Azo Lyso PA |

**Table 2: Functional moieties and examples of functionalized lipid**

| Functional Ligand | Example | Reacting moiety /Function |
|---|---|---|
| biotin | 1-oleoyl-2-(12-biotinyl-(aminododecanoyl))-sn-glycero-3-phosphoethanolamine (18:1-12:0 Biotin PE); | Avidin, Streptavidin |
| | 1,2-dipalmitoyl-sn-glycero-3-phosphoethanolamine-N-(biotinyl), 16:0 Biotinyl PE | |
| | 1,2-dioleoyl-sn-glycero-3-phosphoethanolamine-N-(biotinyl), 18:1 Biotinyl PE | |
| | 1,2-dioleoyl-sn-glycero-3-phosphoethanolamine-N-(cap biotinyl), 18:1 Biotinyl Cap PE; | |
| | 1,2-dipalmitoyl-sn-glycero-3-phosphoethanolamine-N-(cap biotinyl), 16:0 Biotinyl Cap PE | |
| N-hydroxysuccinimide ester (NHS), N-Hydroxysulfosuccinimide (sulfo-NHS) | NHS Palmitic acid N-hydroxysuccinimide ester | Amine |
| nitrilotriacetic acid (NTA)-nickel | 1,2-dioleoyl-sn-glycero-3- [(N-(5-amino-1 -carboxypentyl)iminodiacetic acid) succinyl], 18:1 DGS-NTA (Ni) | Histidine (His) tags, e.g. 6 x His-Tag |
| amines | 1,2-dioleoyl-sn-glycero-3-phosphoethanolamine-N-(hexanoylamine)18:1 Caproylamine PE | NHS, N-Hydroxysulfosuccinimide |
| | 1,2-Dipalmitoyl-sn-Glycero-3-Phosphoethanolamine-N-(hexanoylamine), 16:0 Caproylamine PE | |
| | 1,2-dipalmitoyl-sn-glycero-3-phosphoethanolamine-N-(dodecanylamine), 16:0 Dodecanylamine PE | |
| | 1,2-dioleoyl-sn-glycero-3-phosphoethanolamine-N-(dodecanylamine), 18:1 Dodecanylamine PE | |
| arginylglycylaspartic acid (RGD) | 1,2-distearoyl-sn-glycero-3-phosphoethanolamine-N-[4-(p-(cysarginylglycylaspartate-maleimidomethyl)-cyclohexane-carboxamide], DSPE-RGD | Integrin receptors on target cells |
| maleimides, aromatic maleimides | 1,2-dipalmitoyl-sn-glycero-3-phosphoethanolamine-N-[4-(p-maleimidomethyl) cyclohexane-carboxamide] (sodium salt), 16:0 PE MCC; | Thiol (e.g. thiolated antibodies) |
| N-[4-(p-maleimidophenyl)-butyryl], MPB; | 1,2-dioleoyl-sn-glycero-3-phosphoethanolamine-N-[4-(p-maleimidomethyl) cyclohexane-carboxamide] (sodium salt), 18:1 PE MCC; | |
| | 1,2-dioleoyl-sn-glycero-3-phosphocholine (N-aminoethyl), 18:1 aminoethyl PC; | |
| 4-(N-malei m idomethyl) cyclohexane-1-carboxylate, | 1,2-dioleoyl-sn-glycero-3-phosphoethanolamine-N-[4-(p-maleimidophenyl) butyramide] (sodium salt), 18:1 MPB PE | |
| MCC | 1,2-dipalmitoyl-sn-glycero-3-phosphoethanolamine-N-[4-(p-maleimidophenyl) butyramide] (sodium salt), 16:0 MPB PE | |
| pyridyldithiopropionate (PDP) | 1,2-dioleoyl-sn-glycero-3-phosphoethanolamine-N-[3-(2-pyridyldithio)propionate] (sodium salt), 18:1 PDP PE | maleimide-functionalized antibodies bind to sulfhydril group obtained after reduction of a PDP-phopholipid |
| | 1,2-dipalmitoyl-sn-glycero-3-phosphoethanolamine-N-[3-(2-pyridyldithio)propionate] (sodium salt), 16:0 PDP PE | |
| pyridyl disulfide (DPS) | | maleimide |
| dithiopyridinyl 4,4'-dithiodipyridine (4-PDS or 4-DTDP), | | maleimide |
| N-benzylguanine | 1,2-dioleoyl-sn-glycero-3-phosphoethanolamine-N-benzylguanine, 18:1 PE-benzylguanine | SNAP-tag |
| | 1,2-dioleoyl-sn-glycero-3-phosphoethanolamine-N-[benzylguanine(polyethylene glycol)-2000], 18:1 PE-PEG2000-benzylguanine | |
| fluorescent dye molecule, such as lissamine rhodamine B sulfonyl, Atto488, Alexa Fluor 488, Alexa Fluor 647, Fluorescein, N-(7-Nitrobenz-2-Oxa-1,3-Diazol-4-yl (NBD), Cy5, Cy5.5, Cy7, Topfluor® Alexa Fluor488, Topfluor® Alexa Fluor594 | 1,2- dioleoyl-sn-glycero-3-phosphoethanolamine-N-(lissamine rhodamine B sulfonyl) (RhB DOPE or LissRhod PE), | |
| | 1,1'-dioctadecyl-3,3,3',3'-tetramethylindotricarbocyanine iodide (DiR); | |
| | 1,1'-dioctadecyl-3,3,3',3'-tetramethylindodicarbocyanine (DiD) | |
| sulfhydryl / thiol group | 1,2-Dipalmitoyl-sn-Glycero-3-Phosphothioethanol (DPPTE) / 16:0 Ptd Thioethanol | Maleimides, lodoacetamides, benzylic halide, and bromomethylketones, |
| Carboxyacyl such as Succinyl, Glutaryl, dodecanoyl | 1,2-dioleoyl-sn-glycero-3-phosphoethanolamine-N-(succinyl) (sodium salt), 18:1 Succinyl PE; | |
| | 1,2-dipalmitoyl-sn-glycero-3-phosphoethanolamine-N-(succinyl) (sodium salt), 16:0 Succinyl PE; | |
| | 1,2-dioleoyl-sn-glycero-3-phosphoethanolamine-N-(glutaryl) (sodium salt), 18:1 Glutaryl PE; | |
| | 1,2-dipalmitoyl-sn-glycero-3-phosphoethanolamine-N-(glutaryl) (sodium salt), 16:0 Glutaryl PE; | |
| | 1,2-dioleoyl-sn-glycero-3-phosphoethanolamine-N-(dodecanoyl) (sodium salt), 18:1 Dodecanyl PE; | |
| | 1,2-dipalmitoyl-sn-glycero-3-phosphoethanolamine-N-(dodecanoyl) (sodium salt), 16:0 Dodecanoyl PE | |
| cyanuric chloride | cyanur-DSPE | coupling to amine-containing biomolecules such as peptides, antibodies, nanoparticles |
| | cyanur-PEG2000-PE (ammonium salt) | |
| | 1,2-distearoyl-sn-glycero-3-phosphoethanolamine-N-[cyanur(polyethylene glycol)-2000] (ammonium salt), DSPE-PEG(2000) Cyanur | |
| | 1,2-dipalmitoyl-sn-glycero-3-phosphoethanolamine-N-(cyanur), 16:0 Cyanur PE | |
| Folate | 1,2-dipalmitoyl-sn-glycero-3-phosphoethanolamine-N-(6-((folate)amino)hexanoyl), 16:0 Folate Cap PE, | Folate receptor on cancer cells (endocitosis) |
| Carbohydrate/Glycan: for example β -galactose, α-mannose-, β-mannose-, and α-fucose | 1,2-dipalmitoyl-sn-glycero-3-phospho((ethyl-1',2',3'-triazole)triethyleneglycolmannose), 16:0 PA-PEG3-mannose | Carbohydrate binding cell receptor |
| | β -galactose, α-mannose-, β-mannose-, and α-fucose; | |
| | 1,2-dioleoyl-sn-glycero-3-phosphoethanolamine-N-lactosyl (ammonium salt), (18:1 Lactosyl PE) | |
| | 1,2-diacyl-3-O-(α-D-glucopyranosyl)-sn-glycerol (E. coli), MGlc-DAG | |
| | 1-oleoyl-2-palmitoyl-3-(α-D-galactosyl)-sn-glycerol, BbGL-2 | |
| | 1-palmitoyl-2-oleoyl-3-(β-D-glucosyl)-sn-glycerol, 16:0-18:1 DG glucose | |
| Square | 1,2-dioleoyl-sn-glycero-3-phosphoethanolamine-N-square, 18:1 PE-Square | Square is a dye for |
| | 1,2-distearoyl-sn-glycero-3-phosphoethanolamine-N-square, 18:0 PE-square | Resonance Energy Transfer (RET), Flow Cytometry |
| Galloyl | 1,2-dipalmitoyl-sn-glycero-3-galloyl (16:0 DG Galloyl) | Self-adhering lipid so that bilayers strongly adhere to each other |
| Azide | 1,2-dioleoyl-sn-glycero-3-phosphoethanolamine-N-[azido(polyethylene glycol)-2000] (ammonium salt), DOPE-PEG(2000) Azide | Photochemically induced cross-linking with transmembrane peptides |
| Carboxylic acid | 1,2-dioleoyl-sn-glycero-3-phosphoethanolamine-N-[carboxy(polyethylene glycol)-2000] (sodium salt), DOPE-PEG(2000) Carboxylic acid | Amine moieties |
| Chelator: NTA, diethylenetriamine pentaacetic acid, DTPA | 1,2-dipalmitoyl-sn-glycero-3-phosphoethanolamine-N-diethylenetriaminepentaacetic acid (16:0 PE-DTPA), | gadolinium chelated with diethylenentriaminepentaacet yl (DTPA) provides contrast in magnetic resonance imaging |
| | 1,2-dioleoyl-sn-glycero-3-[(N-(5-amino-1-carboxypentyl)iminodiacetic acid) succinyl] (18:1 DGS-NTA), | |
| | 1,2-dioleoyl-sn-glycero-3- [(N-(5-amino-1 -carboxypentyl)iminodiacetic acid) succinyl] (Cobalt salt) (18:1 DGS-NTA)(Co), | |
| | 1,2-dioleoyl-sn-glycero-3- [(N-(5-amino-1 -carboxypentyl)iminodiacetic acid) succinyl] (nickel salt) (18:1 DGS-NTA)(Ni), | |
| | 1,2-dimyristoyl-sn-glycero-3-phosphoethanolamine-N-diethylenetriaminepentaacetic acid (copper salt), (14:0 PE-DTPA(Cu)), | |
| | DTPA-bis(stearylamide) (gadolinium salt), (DTPA-BSA (Gd)), | |
| | 1,2-distearoyl-sn-glycero-3-phosphoethanolamine-N-diethylenetriaminepentaacetic acid (gadolinium salt), (18:0 PE-DTPA (Gd)), | |
| | bis(1,2-dimyristoyl-sn-glycero-3-phosphoethanolamine)-N-N'-diethylenetriaminepentaacetic acid (gadolinium salt) (bis(14:0 PE)-DTPA(Gd)), | |
| | bis(1,2-dipalmitoyl-sn-glycero-3-phosphoethanolamine)-N-N'-diethylenetriaminepentaacetic acid (gadolinium salt) (bis(16:0 PE)-DTPA(Gd)), | |
| | bis(1,2-distearoyl-sn-glycero-3-phosphoethanolamine)-N-N'-diethylenetriaminepentaacetic acid (gadolinium salt) (bis(18:0 PE)-DTPA(Gd)), | |
| | 1,2-distearoyl-sn-glycero-3-phosphoethanolamine-N-diethylenetriaminepentaacetic acid (18:0 PE-DTPA). | |
| Magnetic resonance imaging, MRI imaging | 1,2-dipalmitoyl-sn-glycero-3-phosphoethanolamine-N-diethylenetriaminepentaacetic acid (gadolinium salt), (16:0 PE-DTPA (Gd)), | |
| | 1,2-distearoyl-sn-glycero-3-phosphoethanolamine-N-diethylenetriaminepentaacetic acid (gadolinium salt), (18:0 PE-DTPA (Gd)), | |
| | bis(1,2-dimyristoyl-sn-glycero-3-phosphoethanolamine)-N-N'-diethylenetriaminepentaacetic acid (gadolinium salt) (bis(14:0 PE)-DTPA(Gd)), | |
| | bis(1,2-dipalmitoyl-sn-glycero-3-phosphoethanolamine)-N-N'-diethylenetriaminepentaacetic acid (gadolinium salt) (bis(16:0 PE)-DTPA(Gd)), | |
| | bis(1,2-distearoyl-sn-glycero-3-phosphoethanolamine)-N-N'-diethylenetriaminepentaacetic acid (gadolinium salt) (bis(18:0 PE)-DTPA(Gd)), | |
| | 1,2-distearoyl-sn-glycero-3-phosphoethanolamine-N-diethylenetriaminepentaacetic acid (18:0 PE-DTPA). | |
| polyethylene glycol PEG200, PEG350, PEG550, PEG750, PEG1000, PEG2000, PEG3000, PEG5000, PEG20000, PEG50000, | 1,2-dipalmitoyl-sn-glycero-3-phosphoethanolamine-N-[methoxy(polyethylene glycol)-350], 18:1 PEG350 PE | Surface passivation |
| | 1,2-dioleoyl-sn-glycero-3-phosphoethanolamine-N-[methoxy(polyethylene glycol)-750], 18:1 PEG750 PE | |
| | 1,2-dioleoyl-sn-glycero-3-phosphoethanolamine-N-[methoxy(polyethylene glycol)-1000], 18:1 PEG1000 PE | |
| Diacetylene | 1,2-bis(10,12-tricosadiynoyl)-sn-glycero-3-phosphoethanolamine, 23:2 Diyne PE [DC(8,9)PE] | Photopolymerization |
| | 1-palmitoyl-2-(10,12-tricosadiynoyl)-sn-glycero-3-phosphocholine, 16:0-23:2 Diyne PC | |
| | 1-palmitoyl-2-(10,12-tricosadiynoyl)-sn-glycero-3-phosphoethanolamine, 16:0-23:2 Diyne PE | |
| | 1,2-bis(10,12-tricosadiynoyl)-sn-glycero-3-phosphocholine, 23:2 Diyne PC [DC(8,9)PC] | |
| Diphytanoyl Lipids | 1,2-diphytanoyl-sn-glycero-3-phosphoethanolamine-N-(7-nitro-2-1,3-benzoxadiazol-4-yl), 4ME 16:0 NBD PE (NBD-DPhPE) | Lipids containing diphytanoyl fatty acid chains allow to produce stable planar lipid membranes |
| | 1,2-diphytanoyl-sn-glycero-3-phosphocholine, 4ME 16:0 PC | |
| | 1,2-diphytanoyl-sn-glycero-3-phosphoethanolamine, 4ME 16:0 PE | |
| | 1,2-diphytanoyl-sn-glycero-3-phospho-(1'-rac-glycerol), 4ME 16:0 PG | |
| | 1,2-diphytanoyl-sn-glycero-3-phosphate, 4ME 16:0 PA | |
| | 1,2-diphytanoyl-sn-glycero-3-phospho-L-serine, 4ME 16:0 PS | |
| | phytanoyl Coenzyme A, 4ME 16:0 Coenzyme A | |
| | 1,2-di-O-phytanyl-sn-glycero-3-phosphocholine, 4ME 16:0 Diether PC | |
| | 1,2-di-O-phytanyl-sn-glycero-3-phosphoethanolamine, 4ME 16:0 Diether PE | |
| | 1,2-di-O-phytanyl-sn-glycerol, 4ME 16:0 Diether DG | |
| Fluorinated lipids | 1-palmitoyl-2-(16-fluoropalmitoyl)-sn-glycero-3-phosphocholine, 16:0-16:0 (16-F) PC | |
| Brominated Lipid | 1,2-di-(9,10-dibromo)stearoyl-sn-glycero-3-phosphocholine, 18:0 (9,10dibromo) PC | Fluorescence quenching |
| | 1-palmitoyl-2-stearoyl(4,5)dibromo-sn-glycero-3-phosphocholine, 16:0-18:0(4,5-dibromo) PC | |
| | 1-palmitoyl-2-(6,7-dibromo)stearoyl-sn-glycero-3-phosphocholine, 16:0-18:0 (6-7BR) PC | |
| | 1-palmitoyl-2-(9,10-dibromo)stearoyl-sn-glycero-3-phosphocholine, 16:0-18:0 (9-10BR) PC | |
| | 1-palmitoyl-2-(11,12-dibromo)stearoyl-sn-glycero-3-phosphocholine, 16:0-18:0 (11-12BR) PC | |

**Sulfhydryls**, also called **thiols**, exist in proteins in the side-chain of cysteine (Cys, C) amino acids. Sulfhydryl-reactive chemical groups include haloacetyls, maleimides, aziridines, acryloyls, arylating agents, vinylsulfones, pyridyl disulfides, TNB-thiols and disulfide reducing agents.
Different lipids which are offered for thioether conjugation contain maleimide, aromatic maleimides such as N-[4-(p-maleimidophenyl)-butyryl] (MPB) or 4-(N-maleimidomethyl)cyclohexane-1-carboxylate (MCC) group. The maleimide function group of MCC which contains an aliphatic cyclohexane ring is more stable toward hydrolysis in aqueous reaction environments rather than the aromatic phenyl group of MPB

**Carbohydrates** are selected from the group comprising β-galactose, α-mannose-, β-mannose-, and α-fucose. It has been shown that said carbohydrates can be conjugated to cholesterols to be incorporated into liposomes, and *in vitro* results showed that the sugar-conjugated liposomes are efficiently recognized by cells that overexpress carbohydrate-binding receptors on their surface (Rajabi and Mousa, 2016, Current Pharmaceutical Biotechnology, 17, 8).

**SNAP-tag** is a self-labeling protein tag commercially available in various expression vectors. SNAP-tag is a 182 residues polypeptide (19.4 kDa) that can be fused to any protein of interest and further specifically and covalently tagged with a suitable ligand, such as a fluorescent dye.

A functional ligand for coupling to lipids for carry out the present invention is preferably selected from the group comprising biotin, N-hydroxysuccinimide ester, nitrilotriacetic acid -nickel, amine, carboxylic acid, maleimides, dithiopyridinyl, pyridyl disulfide , pyridyldithiopropionate, N-benzylguanine, carboxyacyl, cyanur, folate, square, galloyl, glycan, thiol, arginylglycylaspartic acid, a fluorescent dye molecule, a magnetic resonance imaging reagent, a chelator.

Therefore, one embodiment of the present invention is directed to a method for producing synthetic extracellular vesicles comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for protein conjugation;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
wherein the amphiphilic copolymer forms a polymer shell stabilizing the synthetic extracellular vesicle, and
the synthetic extracellular vesicles have a diameter comprised between 70 nm and 5000 nm, and
wherein the water phase of step a) comprises at least two lipids selected from the group comprising:
a neutral lipid selected from the group comprising ceramide, sphingomyelin, cephalin, cholesterol, cerebrosides, diacylglycerols, phosphatidylcholines, lysophosphatidylcholines, phosphatidylethanolamines, lysophosphatidylethanolamine, lysoethanolamines, inverted headgroup lipids, sphingosins, sterol-modified phospholipids, ether ester lipids, diether lipids, vinyl ether (plasmalogen);
an anionic lipid selected from the group comprising phosphatidic acids, lysophosphatidic acid derivatives, phosphatidylglycerols, lysophosphatidylglycerols, phosphatidylserines, lysophosphatidylserines, phosphatidylinositols, phosphatidylinositolphosphates, cardiolipins, Bis(Monoacylglycero)Phosphate derivatives;
a cationic lipid selected from the group comprising dioleyl-N,N-dimethylammonium chloride; N-(2,3-dioleyloxy)propyl)-N,N,N-trimethylammonium chloride; N,N-distearyl-N,N-dimethylammonium bromide; N-(2,3- dioleyloxy)propyl)-N,N,N-trimethylammonium chloride; 3β-(N-(N',N'-dimethylaminoethane)-carbamoyl)cholesterol; 1,2-dimyristyloxypropyl-3-dimethyl-hydroxy ethyl ammonium bromide; 2,3-dioleyloxy-N-[2(sperminecarboxamido) ethyl]-N,N-dimethyl-1-propanaminium trifluoroacetate; dioctadecylamidoglycyl carboxyspermine; N-(2,3-dioleyloxy)propyl)-N,N-dimethylammonium chloride and 1,2-Dioleoyl-3-dimethylammonium-propane;
a pH- sensitive lipid selected from the group comprising lipid N-(4-carboxybenzyl)-N,N-dimethyl-2,3-bis(oleoyloxy)propan-1-aminium, 1,2-distearoyl-3-dimethylammonium-propane, 1,2-dipalmitoyl-sn-glycero-3-succinate, 1,2-dioleoyl-sn-glycero-3-succinate, N-palmitoyl homocysteine ;
a photoswitchable lipid;
acylglycine derivatives, prenol derivatives, prostaglandine derivatives, glycosylated diacyl glycerols, eicosanoid derivatives, (palmitoyloxy)octadecanoic acid derivatives, diacetylene derivatives, diphytanoyl derivatives, fluorinated lipids, brominated lipids, lipopolysaccharides;
one of the aforementioned lipids coupled to a functional ligand selected from the group comprising biotin, N-hydroxysuccinimide ester, nitrilotriacetic acid-nickel, amine, carboxylic acid, maleimides, dithiopyridinyl, pyridyl disulfide , pyridyldithiopropionate, N-benzylguanine, carboxyacyl, cyanur, folate, square, galloyl, glycan, thiol, arginylglycylaspartic acid, a fluorescent dye molecule, a magnetic resonance imaging reagent, a chelator;
one of the aforementioned lipids coupled to polyethyleneglycol with a molecular weight comprised between 350 and 50,000 g/mole.

In accordance with one embodiment, the present invention is directed to a method for producing synthetic extracellular vesicles comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for protein conjugation;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
d') removing the polymer shell from the polymer shell-stabilized synthetic extracellular vesicles obtained in step d);
e) purifying the synthetic extracellular vesicles by centrifugation;
wherein the amphiphilic copolymer forms a polymer shell stabilizing the synthetic extracellular vesicle, and
the synthetic extracellular vesicles have a diameter comprised between 70 nm and 5000 nm, and
wherein the water phase of step a) comprises at least two lipids selected from the group comprising:
a neutral lipid selected from the group comprising ceramide, sphingomyelin, cephalin, cholesterol, cerebrosides, diacylglycerols, phosphatidylcholines, lysophosphatidylcholines, phosphatidylethanolamines, lysophosphatidylethanolamine, lysoethanolamines, inverted headgroup lipids, sphingosins, sterol-modified phospholipids, ether ester lipids, diether lipids, vinyl ether (plasmalogen);
an anionic lipid selected from the group comprising phosphatidic acids, lysophosphatidic acid derivatives, phosphatidylglycerols, lysophosphatidylglycerols, phosphatidylserines, lysophosphatidylserines, phosphatidylinositols, phosphatidylinositolphosphates, cardiolipins, Bis(Monoacylglycero)Phosphate derivatives;
a cationic lipid selected from the group comprising dioleyl-N,N-dimethylammonium chloride; N-(2,3-dioleyloxy)propyl)-N,N,N-trimethylammonium chloride; N,N-distearyl-N,N-dimethylammonium bromide; N-(2,3- dioleyloxy)propyl)-N,N,N-trimethylammonium chloride; 3β-(N-(N',N'-dimethylaminoethane)-carbamoyl)cholesterol; 1,2-dimyristyloxypropyl-3-dimethyl-hydroxy ethyl ammonium bromide; 2,3-dioleyloxy-N-[2(sperminecarboxamido)ethyl]-N,N-dimethyl-1-propanaminium trifluoroacetate; dioctadecylamidoglycyl carboxyspermine; N-(2,3-dioleyloxy)propyl)-N,N-dimethylammonium chloride and 1,2-Dioleoyl-3-dimethylammonium-propane;
a pH- sensitive lipid selected from the group comprising lipid N-(4-carboxybenzyl)-N,N-dimethyl-2,3-bis(oleoyloxy)propan-1-aminium, 1,2-distearoyl-3-dimethylammonium-propane, 1,2-dipalmitoyl-sn-glycero-3-succinate, 1,2-dioleoyl-sn-glycero-3-succinate, N-palmitoyl homocysteine ;
a photoswitchable lipid;
acylglycine derivatives, prenol derivatives, prostaglandine derivatives, glycosylated diacyl glycerols, eicosanoid derivatives, (palmitoyloxy)octadecanoic acid derivatives, diacetylene derivatives, diphytanoyl derivatives, fluorinated lipids, brominated lipids, lipopolysaccharides;
one of the aforementioned lipids coupled to a functional ligand selected from the group comprising biotin, N-hydroxysuccinimide ester, nitrilotriacetic acid - nickel, amine, carboxylic acid, maleimides, dithiopyridinyl, pyridyl disulfide , pyridyldithiopropionate, N-benzylguanine, carboxyacyl, cyanur, folate, square, galloyl, glycan, thiol, arginylglycylaspartic acid, a fluorescent dye molecule, a magnetic resonance imaging reagent, a chelator;
one of the aforementioned lipids coupled to polyethyleneglycol with a molecular weight comprised between 350 and 50,000 g/mole.

### SUITABLE COPOLYMER TO STABILIZE THE EXTRACELLULAR VESICLES

In order to allow a good dispersion of the polymer shell stabilized vesicles in the oil phase and in order to allow a good dispersion of the lipid containing aqueous phase within the polymer shell of the vesicle, it is preferred that the polymer shell is made of an amphiphilic copolymer with a hydrophobic end arranged at the outer side and a hydrophilic end arranged at the inner side of the polymer shell.

This may be achieved by forming the polymer shell of the extracellular vesicle,from a diblock copolymer, or a triblock copolymer, to form a water-in-oil droplet.

Good results are particularly obtained, if the polymer shell of the droplet is made of a block copolymer comprising an hydrophobic block arranged at the outer side and a hydrophilic block arranged at the inner side of the polymer shell. The hydrophobic block may be, but is not restricted to members, e.g. selected from the group consisting of perfluorinated polymers, such as perfluorinated polyethers, polystyrene or poly(olefin oxides), such as poly(propylene oxide), whereas the hydrophilic block may be selected e.g. from polyether glycols, polyetheramine, polyacrylate acid, polymethylacrylate acid or poly[poly(ethylene glycol) methyl ether methacrylate]. Likewise, good results are obtained, if the polymer shell of the droplet is made of a **triblock copolymer** comprising two hydrophobic perfluorinated polymer end blocks and therebetween a hydrophilic polyether glycol block, wherein the triblock copolymer is folded so that the hydrophobic perfluorinated polymer blocks are arranged at the outer side and that the hydrophilic polyether glycol block is arranged at the inner side of the polymer shell. Examples for the hydrophobic blocks and the hydrophilic blocks are the same as those mentioned above.

Preferably, the perfluorinated polymer block is a perfluorinated polyether block (PFPE) and more preferably a perfluorinated polyether block having a weight average molecular weight of 1,000 to 10,000 g/mol. Likewise preferably, the polyether glycol (PEG) and polyetheramine (JEFFAMINE) blocks have preferably a weight average molecular weight of 100 to 50,000 g/mol. More specifically, suitable examples for the respective copolymers are PFPE-carboxylic acid (Krytox, MW 2500 or 7000 g/mol) and suitable examples for the respective diblock copolymers are PFPE(7000 g/mol)-PEG(1400 g/mol), PFPE(7000 g/mol)-PEG(600 g/mol), PFPE(2500 g/mol)- PEG(600 g/mol), PFPE(4000 g/mol)-PEG(600 g/mol), PFPE(4000 g/mol)- PEG(1400 g/mol), PFPE(2000 g/mol)-PEG(600 g/mol), PFPE(7000 g/mol)- JEFFAMINE(600 g/mol), PFPE(7000 g/mol)-JEFFAMINE (900 g/mol), PFPE(2500 g/mol)- JEFFAMINE(600 g/mol), PFPE(2500 g/mol)- JEFFAMINE(900 g/mol), PFPE(4000 g/mol)- JEFFAMINE(900 g/mol), PFPE(2500 g/mol)- JEFFAMINE(600 g/mol), PFPE(2000 g/mol)-JEFFAMINE (600 g/mol), PFPE(2000 g/mol)- JEFFAMINE (900 g/mol) and suitable examples for the respective triblock copolymers are PFPE(7000 g/mol)-PEG(1400 g/mol)-PFPE(7000 g/mol), PFPE(7000 g/mol)-PEG(600 g/mol)-PFPE(7000 g/mol), PFPE(4000 g/mol)- PEG(1400 g/mol)- PFPE(4000 g/mol) PFPE(2500 g/mol)- PEG(600 g/mol)- PFPE(2500 g/mol), PFPE(2000 g/mol)-PEG(600 g/mol)-PFPE(2000 g/mol), PFPE(7000 g/mol)- JEFFAMINE(900 g/mol)-PFPE(7000 g/mol) PFPE(7000 g/mol)- JEFFAMINE(600 g/mol)-PFPE(7000 g/mol), PFPE(4000 g/mol)- JEFFAM- INE(900 g/mol)-PFPE(4000 g/mol), PFPE(4000 g/mol)- JEFFAMINE(600 g/mol)- PFPE(4000 g/mol), PFPE(2500 g/mol)- JEFFAMINE(900 g/mol)-PFPE(2500 g/mol), PFPE(2500 g/mol)-JEFFAMINE(600 g/mol)-PFPE(2500 g/mol), PFPE(2000 g/mol)- JEFFAMINE(900 g/mol)-PFPE(2000 g/mol) and PFPE(2000 g/mol)- JEFFAMINE(600 g/mol)-PFPE(2000 g/mol). The molecular weight is determined by gel permeation chromatography using a polystyrene standard.

In one embodiment, the present invention is directed to a method for producing synthetic extracellular vesicles comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for conjugation to an extracellular vesicle associated protein;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
wherein the amphiphilic copolymer forms a polymer shell stabilizing the synthetic extracellular vesicle, and
wherein the synthetic extracellular vesicles have a diameter between 70 nm and 5000 nm, and
wherein the amphiphilic copolymer of step b) consists of a triblock copolymer consisting of two polyether glycol end blocks and one perfluorinated polymer end block, or of a triblock copolymer consisting of two perfluorinated polymer end blocks and one polyether glycol block, or of a diblock copolymer consisting of one perfluorinated polymer end block and a polyether glycol block, wherein the triblock or diblock copolymer is folded so that the perfluorinated polymer end blocks are arranged at the outer side and that the polyether glycol block is arranged at the inner side of the polymer shell.

### EMULSIFICATION CONDITIONS INFLUENCING EXTRACELLULAR VESICLE DIMENSION

One embodiment of the present invention is directed to a method for producing synthetic extracellular vesicles comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for conjugation to an extracellular vesicle associated protein;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier for at least 5 seconds at speed higher than 1,000 rpm;
wherein the amphiphilic copolymer forms a polymer shell stabilizing the synthetic extracellular vesicle, and
wherein the synthetic extracellular vesicles have a diameter between 70 nm and 5000 nm.

The emulsification procedure is usually performed with a mechanically or electronic emulsifier, for at least 5 seconds at speed higher than 1,000 rpm.
This procedure holds the considerable advantage to regulate the vesicle dimension by changing the time and shear stress of emulsification. As shown in **Example 2,** synthetic extracellular vesicles radii between 292 nm ± 12 nm, (coefficient of variation (CV)= 4.1 %; n= 3) were obtained by emulsification for 30 sec at 30,000 rpm and radii of 627 nm ± 15 nm, (CV= 2.4 %; n= 3) were obtained by emulsification for 30 sec at 14,000 rpm.

Notably, this procedure allowed obtaining extracellular vesicles very homogenous in size, as the coefficient of variation of the synthetized extracellular vesicles varied between 2.4 %, for vesicles of size 292 nm ± 12 nm, and 4.1 % for vesicles of size 627 nm ± 15 nm, which are variation levels much lower than observed in the natural exosome samples. Indeed the variation value of commercial K562 exosomes was CV = 42.5 % for dimensions 468 nm ± 199 nm, (n= 3), and that of exosomes isolated from conditioned K562 cell culture medium was CV = 13.3 %, for dimensions 240 nm ± 32 nm (n= 3).

Therefore, one particular embodiment of the present invention is directed to a method for producing synthetic extracellular vesicles comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for conjugation to an extracellular vesicle associated protein;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier for at least 20 seconds at speed between 1,000 - 20,000 rpm;
wherein the amphiphilic copolymer forms a polymer shell stabilizing the synthetic extracellular vesicle, and
wherein the synthetic extracellular vesicles have a diameter between 70 nm and 5000 nm.

One more particular embodiment of the present invention is directed to a method for producing synthetic extracellular vesicles comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for conjugation to an extracellular vesicle associated protein;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier **for at least 20 seconds at speed higher than 10,000 rpm;**
wherein the amphiphilic copolymer forms a polymer shell stabilizing the synthetic extracellular vesicle, and
wherein the synthetic extracellular vesicles have a diameter between 70 nm and 5000 nm.

One still more particular embodiment of the present invention is directed to a method for producing synthetic extracellular vesicles comprising:
a) providing a water phase comprising at least two lipids, optionally one or more extracellular vesicle associated proteins, or fragments thereof, and optionally one or more nucleic acid molecules, wherein the at least two lipids are a negative charged lipid, and a lipid coupled to a functional ligand for conjugation to an extracellular vesicle associated protein;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier for at least 20 seconds at speed higher than 14,000 rpm;wherein the amphiphilic copolymer forms a polymer shell stabilizing the synthetic extracellular vesicle, and
wherein the synthetic extracellular vesicles have a diameter between 70 nm and 5000 nm.

### Composition of synthetic extracellular vesicles

One particular embodiment of the present invention is directed to a synthetic extracellular vesicle having a diameter comprised between 70 and 5000 nm, comprising:
a lipid bilayer comprising at least two lipids selected from the group comprising:
a neutral lipid selected from the group comprising ceramide, sphingomyelin, cephalin, cholesterol, cerebrosides, diacylglycerols, phosphatidylcholines, lysophosphatidylcholines, phosphatidylethanolamines, lysophosphatidylethanolamine, lysoethanolamines, inverted headgroup lipids, sphingosins, sterol-modified phospholipids, ether ester lipids, diether lipids, vinyl ether (plasmalogen);
an anionic lipid selected from the group comprising phosphatidic acids, lysophosphatidic acid derivatives, phosphatidylglycerols, lysophosphatidylglycerols, phosphatidylserines, lysophosphatidylserines, phosphatidylinositols, phosphatidylinositolphosphates, cardiolipins, Bis(Monoacylglycero)Phosphate derivatives;
a cationic lipid selected from the group comprising dioleyl-N,N-dimethylammonium chloride; N-(2,3-dioleyloxy)propyl)-N,N,N-trimethylammonium chloride; N,N-distearyl-N,N-dimethylammonium bromide; N-(2,3-dioleyloxy)propyl)-N,N,N-trimethylammonium chloride; 3β-(N-(N',N'-dimethylaminoethane)-carbamoyl)cholesterol; 1,2-dimyristyloxypropyl-3-dimethyl-hydroxy ethyl ammonium bromide; 2,3-dioleyloxy-N-[2(sperminecarboxamido) ethyl]-N,N-dimethyl-1-propanaminium trifluoroacetate; dioctadecylamidoglycyl carboxyspermine; N-(2,3-dioleyloxy)propyl)-N,N-dimethylammonium chloride and 1,2-Dioleoyl-3-dimethylammonium-propane;
a pH- sensitive lipid selected from the group comprising lipid N-(4-carboxybenzyl)-N,N-dimethyl-2,3-bis(oleoyloxy)propan-1-aminium, 1,2-distearoyl-3-dimethylammonium-propane, 1,2-dipalmitoyl-sn-glycero-3-succinate, 1,2-dioleoyl-sn-glycero-3-succinate, N-palmitoyl homocysteine ;
a photoswitchable lipid;
acylglycine derivatives, prenol derivatives, prostaglandine derivatives, glycosylated diacyl glycerols, eicosanoid derivatives, (palmitoyloxy)octadecanoic acid derivatives, diacetylene derivatives, diphytanoyl derivatives, fluorinated lipids, brominated lipids, lipopolysaccharides;
one of the aforementioned lipids coupled to a functional ligand selected from the group comprising biotin, N-hydroxysuccinimide ester, nitrilotriacetic acid - nickel, amine, carboxylic acid, maleimides, dithiopyridinyl, pyridyl disulfide , pyridyldithiopropionate, N-benzylguanine, carboxyacyl, cyanur, folate, square, galloyl, glycan, thiol, arginylglycylaspartic acid, a fluorescent dye molecule, a magnetic resonance imaging reagent, a chelator;
one of the aforementioned lipids coupled to polyethyleneglycol with a molecular weight comprised between 350 and 50,000 g/mole.
optionally one or more nucleic acids selected from the group comprising DNA, cDNA, mRNA, siRNA, antisense nucleotides, shRNA, piRNA, snRNA, IncRNA, PNA, left handed DNA, Clustered Regularly Interspaced Short Palindromic Repeats (CRISPR) guide RNA;
optionally one or more nucleic acids selected from the group comprising miRNA molecules selected from the group comprising miR-17, miR-18a, miR-19a, miR-19b-1, miR-20a, miR-92a; miR-21, miR-30d-5p, miR-33b, miR-124, miR-125, miR-126, miR-130, miR-132, miR-133b, miR-140-5p, miR-191, miR-222, miR-451, miR-494, miR-575, miR-630, miR-638, miR-1202, miR-1207-5p, miR-1225-5p, miR-1268, miR-6087, miR-92a-3p-e, miR-K12-3, let-7a.
one or more transmembrane proteins selected from the group comprising tetraspanin proteins CD9, CD37, CD47, CD53, CD63, CD81, CD82, CD151, Tspan8, heterotrimeric G protein subunit alpha (GNA), integrin α-chains and β-chains, transferrin receptor 1 (TfR1, CD71), transferrin receptor 2 (TFR2), lysosome associated membrane proteins (LAMP1, LAMP2), heparan sulfate proteoglycans, syndecans, extracellular matrix metalloproteinase inducer (EMMPRIN, BSG), A Disintegrin And Metalloproteinase Domain 10 (ADAM10), CD3, CD11c, CD14, CD29, CD31, CD41, CD42a, CD44, CD45, CD55, CD59, CD73, CD80, CD86, CD90, sonic hedgehog (SHH), major histocompatibility complex I (MHC I), major histocompatibility complex II (MHC II), epidermal growth factor receptor 2 (ERBB2), epithelial cell adhesion molecule (EpCAM), Glycophorin A (GYPA); Acetylcholinesterase S and E (AChE-S, AChE-E), amyloid beta precursor protein (APP), multidrug resistance-associated protein 1 (ABCC1), intercellular adhesion molecule 1 (CD50, ICAM-1), stem cells antigen-1 (Sca-1), or a fragment thereof;
optionally one or more cytosolic proteins selected from the group comprising the protein complexes endosomal sorting complexes required for transport ESCRT-I, ESCRT-II, and ESCRT-III, tumour susceptibility gene 101 (TSG101), charged multivesicular body protein (CHMP), Apoptosis-Linked Gene 2-Interacting Protein X (ALIX), vacuolar protein sorting 4 homolog A 4A and 4B, arrestin domain-containing protein (ARRDC1), flotillin-1, flotillin-2; caveolins, EH-domain containing 1 - 4 (EHD1 - EHD4), Ras homolog family member A (RHOA), annexins, heat shock proteins, ADP-ribosylation factor 6 (ARF6), syntenin, microtubule-associated protein Tau (MAPT), or a fragment thereof;
optionally one or more functional proteins selected from the group comprising cytokines, growth factors, interleukins, milk fat globule-EGF factor 8 protein (MFGE8), adhesion proteins, extracellular matrix proteins, nicotinamide phosphoribosyltransferase, signal transduction proteins Wnta and Wntb, Fas, Fas Ligand (FasL), RANK, RANK Ligand (RANKL), indolamin-2,3-dioxygenase, cytotoxic T-lymphocyte-associated protein 4 - immunoglobulin fusion protein, tumor necrosis factor-related apoptosis-inducing ligand, or a fragment thereof;
optionally one or more proteins associated to intracellular compartments selected from the group comprising histone proteins, lamin A/C, inner membrane mitochondrial protein (IMMT), cytochrome C-1 (CYC1), mitochondrial import receptor subunit TOM20, calnexin, heat shock protein 90kDa beta (Grp94), member 1 (HSP90B1), heat shock 70kDa protein 5 (HSPA5), Golgin A2 (GM130, GOLGA2), Autophagy Related 9A (ATG9A), actinin1, actinin4 (ACTN1, ACTN4), cytokeratin 18 (KRT18), or a fragment thereof.

As mentioned above, in certain embodiments, the extracellular vesicle is an exosome. In certain embodiments, the extracellular vesicle is a microvesicle.

Another particular embodiment of the present invention is directed to a synthetic extracellular vesicle having a diameter comprised between 70 and 5000 nm, comprising:
a lipid bilayer comprising at least two lipids selected from the group comprising:
a neutral lipid selected from the group comprising ceramide, sphingomyelin, cephalin, cholesterol, cerebrosides, diacylglycerols, phosphatidylcholines, lysophosphatidylcholines, phosphatidylethanolamines, lysophosphatidylethanolamine, lysoethanolamines, inverted headgroup lipids, sphingosins, sterol-modified phospholipids, ether ester lipids, diether lipids, vinyl ether (plasmalogen);
an anionic lipid selected from the group comprising phosphatidic acids, lysophosphatidic acid derivatives, phosphatidylglycerols, lysophosphatidylglycerols, phosphatidylserines, lysophosphatidylserines, phosphatidylinositols, phosphatidylinositolphosphates, cardiolipins, Bis(Monoacylglycero)Phosphate derivatives;
a cationic lipid selected from the group comprising dioleyl-N,N-dimethylammonium chloride; N-(2,3-dioleyloxy)propyl)-N,N,N-trimethylammonium chloride; N,N-distearyl-N,N-dimethylammonium bromide; N-(2,3- dioleyloxy)propyl)-N,N,N-trimethylammonium chloride; 3β-(N-(N',N'-dimethylaminoethane)-carbamoyl)cholesterol; 1,2-dimyristyloxypropyl-3-dimethyl-hydroxy ethyl ammonium bromide; 2,3-dioleyloxy-N-[2(sperminecarboxamido) ethyl]-N,N-dimethyl-1-propanaminium trifluoroacetate; dioctadecylamidoglycyl carboxyspermine; N-(2,3-dioleyloxy)propyl)-N,N-dimethylammonium chloride and 1,2-Dioleoyl-3-dimethylammonium-propane;
a pH-sensitive lipid selected from the group comprising lipid N-(4-carboxybenzyl)-N,N-dimethyl-2,3-bis(oleoyloxy)propan-1-aminium, 1,2-distearoyl-3-dimethylammonium-propane, 1,2-dipalmitoyl-sn-glycero-3-succinate, 1,2-dioleoyl-sn-glycero-3-succinate, N-palmitoyl homocysteine ;
a photoswitchable lipid;
acylglycine derivatives, prenol derivatives, prostaglandine derivatives, glycosylated diacyl glycerols, eicosanoid derivatives, (palmitoyloxy)octadecanoic acid derivatives, diacetylene derivatives, diphytanoyl derivatives, fluorinated lipids, brominated lipids, lipopolysaccharides;
one of the aforementioned lipids coupled to a functional ligand selected from the group comprising biotin, N-hydroxysuccinimide ester, nitrilotriacetic acid - nickel, amine, carboxylic acid, maleimides, dithiopyridinyl, pyridyl disulfide , pyridyldithiopropionate, N-benzylguanine, carboxyacyl, cyanur, folate, square, galloyl, glycan, thiol, arginylglycylaspartic acid, a fluorescent dye molecule, a magnetic resonance imaging reagent, a chelator;
one of the aforementioned lipids coupled to polyethyleneglycol with a molecular weight comprised between 350 and 50,000 g/mole.
optionally one or more nucleic acids selected from the group comprising DNA, cDNA, mRNA, siRNA, antisense nucleotides, shRNA, piRNA, snRNA, IncRNA, PNA, left handed DNA, Clustered Regularly Interspaced Short Palindromic Repeats (CRISPR) guide RNA;
optionally one or more nucleic acids selected from the group comprising miRNA molecules selected from the group comprising miR-17, miR-18a, miR-19a, miR-19b-1, miR-20a, miR-92a; miR-21, miR-30d-5p, miR-33b, miR-124, miR-125, miR-126, miR-130, miR-132, miR-133b, miR-140-5p, miR-191, miR-222, miR-451, miR-494, miR-575, miR-630, miR-638, miR-1202, miR-1207-5p, miR-1225-5p, miR-1268, miR-6087, miR-92a-3p-e, miR-K12-3, let-7a.
one or more transmembrane proteins selected from the group comprising tetraspanin proteins CD9, CD37, CD47, CD53, CD63, CD81, CD82, CD151, Tspan8, heterotrimeric G protein subunit alpha (GNA), integrin α-chains and β-chains, transferrin receptor 1 (TfR1, CD71), transferrin receptor 2 (TFR2), lysosome associated membrane proteins (LAMP1, LAMP2), heparan sulfate proteoglycans, syndecans, extracellular matrix metalloproteinase inducer (EMMPRIN, BSG), A Disintegrin And Metalloproteinase Domain 10 (ADAM10), CD3, CD11c, CD14, CD29, CD31, CD41, CD42a, CD44, CD45, CD55, CD59, CD73, CD80, CD86, CD90, sonic hedgehog (SHH), major histocompatibility complex I (MHC I), major histocompatibility complex II (MHC II), epidermal growth factor receptor 2 (ERBB2), epithelial cell adhesion molecule (EpCAM), Glycophorin A (GYPA); Acetylcholinesterase S and E (AChE-S, AChE-E), amyloid beta precursor protein (APP), multidrug resistance-associated protein 1 (ABCC1), intercellular adhesion molecule 1 (CD50, ICAM-1), stem cells antigen-1 (Sca-1), or a fragment thereof;
optionally one or more cytosolic proteins selected from the group comprising the protein complexes endosomal sorting complexes required for transport ESCRT-I, ESCRT-II, and ESCRT-III, tumour susceptibility gene 101 (TSG101), charged multivesicular body protein (CHMP), Apoptosis-Linked Gene 2-Interacting Protein X (ALIX), vacuolar protein sorting 4 homolog A 4A and 4B, arrestin domain-containing protein (ARRDC1), flotillin-1, flotillin-2; caveolins, EH-domain containing 1 - 4 (EHD1 - EHD4), Ras homolog family member A (RHOA), annexins, heat shock proteins, ADP-ribosylation factor 6 (ARF6), syntenin, microtubule-associated protein Tau (MAPT), or a fragment thereof;
optionally one or more functional proteins selected from the group comprising cytokines, growth factors, interleukins, milk fat globule-EGF factor 8 protein (MFGE8), adhesion proteins, extracellular matrix proteins, nicotinamide phosphoribosyltransferase, signal transduction proteins Wnta and Wntb, Fas, Fas Ligand (FasL), RANK, RANK Ligand (RANKL), indolamin-2,3-dioxygenase, cytotoxic T-lymphocyte-associated protein 4 - immunoglobulin fusion protein, tumor necrosis factor-related apoptosis-inducing ligand, or a fragment thereof;
optionally one or more proteins associated to intracellular compartments selected from the group comprising histone proteins, lamin A/C, inner membrane mitochondrial protein (IMMT), cytochrome C-1 (CYC1), mitochondrial import receptor subunit TOM20, calnexin, heat shock protein 90kDa beta (Grp94), member 1 (HSP90B1), heat shock 70kDa protein 5 (HSPA5), Golgin A2 (GM130, GOLGA2), Autophagy Related 9A (ATG9A), actinin1, actinin4 (ACTN1, ACTN4), cytokeratin 18 (KRT18), or a fragment thereof.

Another more particular embodiment of the present invention is directed to a synthetic extracellular vesicle having a diameter comprised between 70 nm and 1000 nm, comprising:
a lipid bilayer comprising at least two lipids selected from the group comprising:
a neutral lipid selected from the group comprising ceramide, sphingomyelin, cephalin, cholesterol, cerebrosides, diacylglycerols, phosphatidylcholines, lysophosphatidylcholines, phosphatidylethanolamines, lysophosphatidylethanolamine, lysoethanolamines, inverted headgroup lipids, sphingosins, sterol-modified phospholipids, ether ester lipids, diether lipids, vinyl ether (plasmalogen);
an anionic lipid selected from the group comprising phosphatidic acids, lysophosphatidic acid derivatives, phosphatidylglycerols, lysophosphatidylglycerols, phosphatidylserines, lysophosphatidylserines, phosphatidylinositols, phosphatidylinositolphosphates, cardiolipins, Bis(Monoacylglycero)Phosphate derivatives;
a cationic lipid selected from the group comprising dioleyl-N,N-dimethylammonium chloride; N-(2,3-dioleyloxy)propyl)-N,N,N-trimethylammonium chloride; N,N-distearyl-N,N-dimethylammonium bromide; N-(2,3- dioleyloxy)propyl)-N,N,N-trimethylammonium chloride; 3β-(N-(N',N'-dimethylaminoethane)-carbamoyl)cholesterol; 1,2-dimyristyloxypropyl-3-dimethyl-hydroxy ethyl ammonium bromide; 2,3-dioleyloxy-N-[2(sperminecarboxamido) ethyl]-N,N-dimethyl-1-propanaminium trifluoroacetate; dioctadecylamidoglycyl carboxyspermine; N-(2,3-dioleyloxy)propyl)-N,N-dimethylammonium chloride and 1,2-Dioleoyl-3-dimethylammonium-propane;
a pH-sensitive lipid selected from the group comprising lipid N-(4-carboxybenzyl)-N,N-dimethyl-2,3-bis(oleoyloxy)propan-1-aminium, 1,2-distearoyl-3-dimethylammonium-propane, 1,2-dipalmitoyl-sn-glycero-3-succinate, 1,2-dioleoyl-sn-glycero-3-succinate, N-palmitoyl homocysteine ;
a photoswitchable lipid;
acylglycine derivatives, prenol derivatives, prostaglandine derivatives, glycosylated diacyl glycerols, eicosanoid derivatives, (palmitoyloxy)octadecanoic acid derivatives, diacetylene derivatives, diphytanoyl derivatives, fluorinated lipids, brominated lipids, lipopolysaccharides;
one of the aforementioned lipids coupled to a functional ligand selected from the group comprising biotin, N-hydroxysuccinimide ester, nitrilotriacetic acid - nickel, amine, carboxylic acid, maleimides, dithiopyridinyl, pyridyl disulfide , pyridyldithiopropionate, N-benzylguanine, carboxyacyl, cyanur, folate, square, galloyl, glycan, thiol, arginylglycylaspartic acid, a fluorescent dye molecule, a magnetic resonance imaging reagent, a chelator;
one of the aforementioned lipids coupled to polyethyleneglycol with a molecular weight comprised between 350 and 50,000 g/mole.
optionally one or more nucleic acids selected from the group comprising DNA, cDNA, mRNA, siRNA, antisense nucleotides, shRNA, piRNA, snRNA, IncRNA, PNA, left handed DNA, Clustered Regularly Interspaced Short Palindromic Repeats (CRISPR) guide RNA;
optionally one or more nucleic acids selected from the group comprising miRNA molecules selected from the group comprising miR-17, miR-18a, miR-19a, miR-19b-1, miR-20a, miR-92a; miR-21, miR-30d-5p, miR-33b, miR-124, miR-125, miR-126, miR-130, miR-132, miR-133b, miR-140-5p, miR-191, miR-222, miR-451, miR-494, miR-575, miR-630, miR-638, miR-1202, miR-1207-5p, miR-1225-5p, miR-1268, miR-6087, miR-92a-3p-e, miR-K12-3, let-7a.
one or more transmembrane proteins selected from the group comprising tetraspanin proteins CD9, CD37, CD47, CD53, CD63, CD81, CD82, CD151, Tspan8, heterotrimeric G protein subunit alpha (GNA), integrin α-chains and β-chains, transferrin receptor 1 (TfR1, CD71), transferrin receptor 2 (TFR2), lysosome associated membrane proteins (LAMP1, LAMP2), heparan sulfate proteoglycans, syndecans, extracellular matrix metalloproteinase inducer (EMMPRIN, BSG), A Disintegrin And Metalloproteinase Domain 10 (ADAM10), CD3, CD11c, CD14, CD29, CD31, CD41, CD42a, CD44, CD45, CD55, CD59, CD73, CD80, CD86, CD90, sonic hedgehog (SHH), major histocompatibility complex I (MHC I), major histocompatibility complex II (MHC II), epidermal growth factor receptor 2 (ERBB2), epithelial cell adhesion molecule (EpCAM), Glycophorin A (GYPA); Acetylcholinesterase S and E (AChE-S, AChE-E), amyloid beta precursor protein (APP), multidrug resistance-associated protein 1 (ABCC1), intercellular adhesion molecule 1 (CD50, ICAM-1), stem cells antigen-1 (Sca-1), or a fragment thereof;
optionally one or more cytosolic proteins selected from the group comprising the protein complexes endosomal sorting complexes required for transport ESCRT-I, ESCRT-II, and ESCRT-III, tumour susceptibility gene 101 (TSG101), charged multivesicular body protein (CHMP), Apoptosis-Linked Gene 2-Interacting Protein X (ALIX), vacuolar protein sorting 4 homolog A 4A and 4B, arrestin domain-containing protein (ARRDC1), flotillin-1, flotillin-2; caveolins, EH-domain containing 1 - 4 (EHD1 - EHD4), Ras homolog family member A (RHOA), annexins, heat shock proteins, ADP-ribosylation factor 6 (ARF6), syntenin, microtubule-associated protein Tau (MAPT), or a fragment thereof;
optionally one or more functional proteins selected from the group comprising cytokines, growth factors, interleukins, milk fat globule-EGF factor 8 protein (MFGE8), adhesion proteins, extracellular matrix proteins, nicotinamide phosphoribosyltransferase, signal transduction proteins Wnta and Wntb, Fas, Fas Ligand (FasL), RANK, RANK Ligand (RANKL), indolamin-2,3-dioxygenase, cytotoxic T-lymphocyte-associated protein 4 - immunoglobulin fusion protein, tumor necrosis factor-related apoptosis-inducing ligand, or a fragment thereof;
optionally one or more proteins associated to intracellular compartments selected from the group comprising histone proteins, lamin A/C, inner membrane mitochondrial protein (IMMT), cytochrome C-1 (CYC1), mitochondrial import receptor subunit TOM20, calnexin, heat shock protein 90kDa beta (Grp94), member 1 (HSP90B1), heat shock 70kDa protein 5 (HSPA5), Golgin A2 (GM130, GOLGA2), Autophagy Related 9A (ATG9A), actinin1, actinin4 (ACTN1, ACTN4), cytokeratin 18 (KRT18), or a fragment thereof.

A particularly preferred embodiment of the present invention is directed to a synthetic extracellular vesicle with the composition described above and specifically comprising:
a lipid bilayer comprising cholesterol, N-stearoyl-D-erythro-sphingosylphosphorylcholine (SM), 1,2-dioleoyl-sn-glycero-3-phosphocholine (DOPC), 1,2-dioleoyl-sn-glycero-3-phospho-L-serine (DOPS), 1,2-dioleoyl-sn-glycero-3-phospho-ethanolamine (DOPE), 1,2-dioleoyl-sn-glycero-3-phospho-rac-(1-glycerol) (DOPG), 1,2-dioleoyl-sn-glycero-3-phosphate (sodium salt) (PA), diacylglycerol, phosphatidylinositol, 1,2-dioleoyl-sn-glycero-3-phosphoethanolamine-N-(lissamine rhodamine B sulfonyl) (LissRhod PE), 1,2-dioleoyl-sn-glycero-3-[(N-(5-amino-1-carboxypentyl)iminodiacetic acid) succinyl] (nickel salt) (DGS-NTA(Ni²⁺));
one or more nucleic acid molecules selected from the group comprising miRNA miR-21, miR-124, miR-125, miR-126, miR-130 and miR-132, and one or more transmembrane proteins selected from the group comprising tetraspanin proteins CD9, CD63 and CD81, or a fragment thereof.

Another particularly preferred embodiment of the present invention is directed to a synthetic extracellular vesicle with the composition described above and specifically comprising:
one or more functional protein nicotinamide phosphoribosyltransferase, or a fragment thereof;
one or more transmembrane proteins selected from the group comprising tetraspanin proteins CD9, CD63 and CD81, or a fragment thereof;
one or more cytosolic proteins selected from the group comprising Apoptosis-Linked Gene 2-Interacting Protein X (ALIX), tumour susceptibility gene 101 protein (TSG101), or a fragment thereof,
wherein the synthetic extracellular vesicle do not comprise transferrin and albumin, or a fragment thereof.

Another particularly preferred embodiment of the present invention is directed to a synthetic extracellular vesicle with the composition described above and specifically comprising:
one or more transmembrane proteins selected from the group comprising MHCII, CD80, and CD86, or a fragment thereof;
optionally one or more transmembrane proteins selected from the group comprising CD11c, MHC I, integrin α and β-chains, ICAM-1, and CD71, or a fragment thereof;
one or more functional proteins selected from the group comprising cytokines, interleukins, interleukin 4, milk fat globule-EGF factor 8 protein (MFGE8), growth factors, Fas, Fas Ligand (FasL), indolamin-2,3-dioxygenase, cytotoxic T-lymphocyte-associated protein 4 - immunoglobulin fusion protein (CTLA4-Ig), tumor necrosis factor-related apoptosis-inducing ligand (Apo2L, TRAIL), or a fragment thereof.

Another particularly preferred embodiment of the present invention is directed to a synthetic extracellular vesicle with the composition described above and specifically comprising:
a lipid bilayer comprising 1,2-dioleoyl-sn-glycero-3-phosphocholine (DOPC), 1,2-dioleoyl-sn-glycero-3-phospho-rac-(1-glycerol) (DOPG), 1,2-dioleoyl-sn-glycero-3-phosphoethanolamine-N-(lissamine rhodamine B sulfonyl) (LissRhod PE), 1,2-dioleoyl-sn-glycero-3-[(N-(5-amino-1-carboxypentyl)iminodiacetic acid) succinyl] (nickel salt) (DGS-NTA(Ni2+)); functional protein Fas Ligand, or a fragment thereof;
optionally functional protein intercellular adhesion protein - 1, or a fragment thereof.

Another particularly preferred embodiment of the present invention is directed to a synthetic extracellular vesicle with the composition described above and specifically comprising:
a lipid bilayer comprising 1,2-dioleoyl-sn-glycero-3-phosphocholine (DOPC), 1,2-dioleoyl-sn-glycero-3-phospho-rac-(1-glycerol) (DOPG), 1,2-dioleoyl-sn-glycero-3-phosphoethanolamine-N-(lissamine rhodamine B sulfonyl) (LissRhod PE), 1,2-dioleoyl-sn-glycero-3-[(N-(5-amino-1-carboxypentyl)iminodiacetic acid) succinyl] (nickel salt) (DGS-NTA(Ni2+));
a fragment of functional protein Fas Ligand,
optionally functional protein intercellular adhesion protein - 1, or a fragment thereof,
wherein the Fas Ligand fragment comprises amino acids 134 - 281,
wherein the intercellular adhesion protein - 1 fragment comprises amino acids 1 - 480.

Another particularly preferred embodiment of the present invention is directed to a synthetic extracellular vesicle with the composition described above and specifically comprising:
one or more transmembrane proteins selected from the group comprising CD29, CD44, CD90, CD73, CD44, Sca-1, or a fragment thereof;
one or more transmembrane proteins selected from the group comprising tetraspanin proteins CD9, CD63, and CD81, or a fragment thereof;
one or more functional proteins selected from the group comprising Wnta and Wntb, or a fragment thereof;
at least one nucleic acid molecule selected from the group comprising miR-140-5p, miR-92a-3p-e;
one or more nucleic acid molecules selected from the group comprising miR-17, miR-18a, miR-19a, miR-19b-1, miR-20a, miR-92a, let-7a, miR-21, miR124, miR126, miR-133b, miR-191, miR-222, miR-494, miR-6087, miR-30d-5p ;
optionally one or more nucleic acid molecules selected from the group comprising miR-33b, miR-451, miR-575, miR-630, miR-638, miR-1202, miR-1207-5p, miR-1225-5p, miR-1268, miR-K12-3.

A further particularly preferred embodiment of the present invention is directed to a synthetic extracellular vesicle with the composition described above and specifically comprising:
a lipid bilayer comprising 1,2-dioleoyl-sn-glycero-3-phosphocholine (DOPC), 1,2-dioleoyl-sn-glycero-3-phospho-rac-(1-glycerol) (DOPG), 1,2-dioleoyl-sn-glycero-3-phosphoethanolamine-N-(lissamine rhodamine B sulfonyl) (LissRhod PE), 1,2-dioleoyl-sn-glycero-3-[(N-(5-amino-1-carboxypentyl)iminodiacetic acid) succinyl] (nickel salt) (DGS-NTA(Ni2+)), functional protein RANK, or a fragment thereof.

A further more particularly preferred embodiment of the present invention is directed to a synthetic extracellular vesicle with the composition described above and specifically comprising:
a lipid bilayer comprising 1,2-dioleoyl-sn-glycero-3-phosphocholine (DOPC), 1,2-dioleoyl-sn-glycero-3-phospho-rac-(1-glycerol) (DOPG), 1,2-dioleoyl-sn-glycero-3-phosphoethanolamine-N-(lissamine rhodamine B sulfonyl) (LissRhod PE), 1,2-dioleoyl-sn-glycero-3-[(N-(5-amino-1-carboxypentyl)iminodiacetic acid) succinyl] (nickel salt) (DGS-NTA(Ni2+)),
a fragment of functional protein RANK, wherein said fragment of functional protein RANK comprises amino acids 31 - 214.

### USES OF THE DISCLOSED EXTRACELLULAR VESICLES

The examples of the present invention show that the synthetic extracellular vesicles are able to deliver their protein and nucleic acid contents into target cells, thus affecting their gene expression, protein expression, signalling pathways and metabolism.
Thus, the inventive synthetic extracellular pathways can be used for therapy of a wide range of disorders by acting at cellular levels.
For example, it has been here shown that the synthetic extracellular vesicles resembling those of fibrocyte origin can stimulate epithelial cell proliferation, migration, and collagen deposition, ultimately leading to wound healing.

Therefore, one embodiment of the present invention is directed to a synthetic extracellular vesicle having a diameter comprised between 70 and 5000 nm, comprising:
a lipid bilayer comprising at least two lipids selected from the group comprising:
a neutral lipid selected from the group comprising ceramide, sphingomyelin, cephalin, cholesterol, cerebrosides, diacylglycerols, phosphatidylcholines, lysophosphatidylcholines, phosphatidylethanolamines, lysophosphatidylethanolamine, lysoethanolamines, inverted headgroup lipids, sphingosins, sterol-modified phospholipids, ether ester lipids, diether lipids, vinyl ether (plasmalogen);
an anionic lipid selected from the group comprising phosphatidic acids, lysophosphatidic acid derivatives, phosphatidylglycerols, lysophosphatidylglycerols, phosphatidylserines, lysophosphatidylserines, phosphatidylinositols, phosphatidylinositolphosphates, cardiolipins, Bis(Monoacylglycero)Phosphate derivatives;
a cationic lipid selected from the group comprising dioleyl-N,N-dimethylammonium chloride; N-(2,3-dioleyloxy)propyl)-N,N,N-trimethylammonium chloride; N,N-distearyl-N,N-dimethylammonium bromide; N-(2,3- dioleyloxy)propyl)-N,N,N-trimethylammonium chloride; 3β-(N-(N',N'-dimethylaminoethane)-carbamoyl)cholesterol; 1,2-dimyristyloxypropyl-3-dimethyl-hydroxy ethyl ammonium bromide; 2,3-dioleyloxy-N-[2(sperminecarboxamido)ethyl]-N,N-dimethyl-1-propanaminium trifluoroacetate; dioctadecylamidoglycyl carboxyspermine; N-(2,3-dioleyloxy)propyl)-N,N-dimethylammonium chloride and 1,2-Dioleoyl-3-dimethylammonium-propane;
a pH- sensitive lipid selected from the group comprising lipid N-(4-carboxybenzyl)-N,N-dimethyl-2,3-bis(oleoyloxy)propan-1-aminium, 1,2-distearoyl-3-dimethylammonium-propane, 1,2-dipalmitoyl-sn-glycero-3-succinate, 1,2-dioleoyl-sn-glycero-3-succinate, N-palmitoyl homocysteine ;
a photoswitchable lipid;
acylglycine derivatives, prenol derivatives, prostaglandine derivatives, glycosylated diacyl glycerols, eicosanoid derivatives, (palmitoyloxy)octadecanoic acid derivatives, diacetylene derivatives, diphytanoyl derivatives, fluorinated lipids, brominated lipids, lipopolysaccharides;
one of the aforementioned lipids coupled to a functional ligand selected from the group comprising biotin, N-hydroxysuccinimide ester, nitrilotriacetic acid - nickel, amine, carboxylic acid, maleimides, dithiopyridinyl, pyridyl disulfide , pyridyldithiopropionate, N-benzylguanine, carboxyacyl, cyanur, folate, square, galloyl, glycan, thiol, arginylglycylaspartic acid, a fluorescent dye molecule, a magnetic resonance imaging reagent, a chelator;
one of the aforementioned lipids coupled to polyethyleneglycol with a molecular weight comprised between 350 and 50,000 g/mole.
optionally one or more nucleic acids selected from the group comprising DNA, cDNA, mRNA, siRNA, antisense nucleotides, shRNA, piRNA, snRNA, IncRNA, PNA, left handed DNA, Clustered Regularly Interspaced Short Palindromic Repeats (CRISPR) guide RNA;
optionally one or more nucleic acids selected from the group comprising miRNA molecules selected from the group comprising miR-17, miR-18a, miR-19a, miR-19b-1, miR-20a, miR-92a; miR-21, miR-30d-5p, miR-33b, miR-124, miR-125, miR-126, miR-130, miR-132, miR-133b, miR-140-5p, miR-191, miR-222, miR-451, miR-494, miR-575, miR-630, miR-638, miR-1202, miR-1207-5p, miR-1225-5p, miR-1268, miR-6087, miR-92a-3p-e, miR-K12-3, let-7a.
one or more transmembrane proteins selected from the group comprising tetraspanin proteins CD9, CD37, CD47, CD53, CD63, CD81, CD82, CD151, Tspan8, heterotrimeric G protein subunit alpha (GNA), integrin α-chains and β-chains, transferrin receptor 1 (TfR1, CD71), transferrin receptor 2 (TFR2), lysosome associated membrane proteins (LAMP1, LAMP2), heparan sulfate proteoglycans, syndecans, extracellular matrix metalloproteinase inducer (EMMPRIN, BSG), A Disintegrin And Metalloproteinase Domain 10 (ADAM10), CD3, CD11c, CD14, CD29, CD31, CD41, CD42a, CD44, CD45, CD50 (intercellular adhesion molecule 1, ICAM-1), CD55, CD59, CD73, CD80, CD86, CD90, sonic hedgehog (SHH), major histocompatibility complex I (MHC I), major histocompatibility complex II (MHC II), epidermal growth factor receptor 2 (ERBB2), epithelial cell adhesion molecule (EpCAM), Glycophorin A (GYPA); Acetylcholinesterase S and E (AChE-S, AChE-E), amyloid beta precursor protein (APP), multidrug resistance-associated protein 1 (ABCC1), stem cells antigen-1 (Sca-1), or a fragment thereof;
optionally one or more cytosolic proteins selected from the group comprising the protein complexes endosomal sorting complexes required for transport ESCRT-I, ESCRT-II, and ESCRT-III, tumour susceptibility gene 101 (TSG101), charged multivesicular body protein (CHMP), Apoptosis-Linked Gene 2-Interacting Protein X (ALIX), vacuolar protein sorting 4 homolog A 4A and 4B, arrestin domain-containing protein (ARRDC1), flotillin-1, flotillin-2; caveolins, EH-domain containing 1 - 4 (EHD1 - EHD4), Ras homolog family member A (RHOA), annexins, heat shock proteins, ADP-ribosylation factor 6 (ARF6), syntenin, microtubule-associated protein Tau (MAPT), or a fragment thereof;
optionally one or more functional proteins selected from the group comprising cytokines, growth factors, interleukins, milk fat globule-EGF factor 8 protein (MFGE8), adhesion proteins, extracellular matrix proteins, nicotinamide phosphoribosyltransferase, signal transduction proteins Wnta and Wntb, Fas, Fas Ligand (FasL), RANK, RANK Ligand (RANKL), indolamin-2,3-dioxygenase, cytotoxic T-lymphocyte-associated protein 4 - immunoglobulin fusion protein, tumor necrosis factor-related apoptosis-inducing ligand, or a fragment thereof;
optionally one or more proteins associated to intracellular compartments selected from the group comprising histone proteins, lamin A/C, inner membrane mitochondrial protein (IMMT), cytochrome C-1 (CYC1), mitochondrial import receptor subunit TOM20, calnexin, heat shock protein 90kDa beta (Grp94), member 1 (HSP90B1), heat shock 70kDa protein 5 (HSPA5), Golgin A2 (GM130, GOLGA2), Autophagy Related 9A (ATG9A), actinin1, actinin4 (ACTN1, ACTN4), cytokeratin 18 (KRT18), or a fragment thereof,
for use in the treatment of a disorder selected from the group comprising inflammation, cancer, rheumatic disorder, severe graft versus host disease, osteoarthritis, cardiovascular disorder, epithelial diseases, neurodegenerative disorders, autoimmune disorders, bone and cartilage disorders, osteoporosis, renal osteodystrophy, paget's disease of bone, osteopetrosis, rickets, neurological disorders, intoxication, neuroendocrinology disorders, endocrinology disorders, genetic disorders, infectious diseases, dental disorders, cosmetic procedures, coagulation disorders, dermatoses, diabetes, age-associated disorders.

In certain embodiments, the extracellular vesicle is an exosome. In certain embodiments, the extracellular vesicle is a microvesicle.

One preferred embodiment of the present invention is directed to a synthetic extracellular vesicle with the composition described above and specifically comprising:
a lipid bilayer comprising cholesterol, N-stearoyl-D-erythro-sphingosylphosphorylcholine (SM), 1,2-dioleoyl-sn-glycero-3-phosphocholine (DOPC), 1,2-dioleoyl-sn-glycero-3-phospho-L-serine (DOPS), 1,2-dioleoyl-sn-glycero-3-phospho-ethanolamine (DOPE), 1,2-dioleoyl-sn-glycero-3-phospho-rac-(1-glycerol) (DOPG), 1,2-dioleoyl-sn-glycero-3-phosphate (sodium salt) (PA), diacylglycerol, phosphatidylinositol, 1,2-dioleoyl-sn-glycero-3-phosphoethanolamine-N-(lissamine rhodamine B sulfonyl) (LissRhod PE), 1,2-dioleoyl-sn-glycero-3-[(N-(5-amino-1-carboxypentyl)iminodiacetic acid) succinyl] (nickel salt) (DGS-NTA(Ni²⁺));
one or more nucleic acid molecules selected from the group comprising miRNA miR-21, miR-124, miR-125, miR-126, miR-130 and miR-132, and
one or more transmembrane proteins selected from the group comprising tetraspanin proteins CD9, CD63 and CD81, or a fragment thereof,
for use in the treatment of a disorder selected from the group comprising inflammation, cancer, rheumatic disorder, severe graft versus host disease, osteoarthritis, cardiovascular disorder, epithelial diseases, neurodegenerative disorders, autoimmune disorders, bone and cartilage disorders, osteoporosis, renal osteodystrophy, paget's disease of bone, osteopetrosis, rickets, neurological disorders, intoxication, neuroendocrinology disorders, endocrinology disorders, genetic disorders, infectious diseases, dental disorders, cosmetic procedures, coagulation disorders, dermatoses, diabetes, age-associated disorders.

A preferred embodiment of the present invention is directed to a synthetic extracellular vesicle with the composition disclosed herein and specifically comprising:
a lipid bilayer comprising cholesterol, N-stearoyl-D-erythro-sphingosylphosphorylcholine (SM), 1,2-dioleoyl-sn-glycero-3-phosphocholine (DOPC), 1,2-dioleoyl-sn-glycero-3-phospho-L-serine (DOPS), 1,2-dioleoyl-sn-glycero-3-phospho-ethanolamine (DOPE), 1,2-dioleoyl-sn-glycero-3-phospho-rac-(1-glycerol) (DOPG), 1,2-dioleoyl-sn-glycero-3-phosphate (sodium salt) (PA), diacylglycerol, phosphatidylinositol, 1,2-dioleoyl-sn-glycero-3-phosphoethanolamine-N-(lissamine rhodamine B sulfonyl) (LissRhod PE), 1,2-dioleoyl-sn-glycero-3-[(N-(5-amino-1-carboxypentyl)iminodiacetic acid) succinyl] (nickel salt) (DGS-NTA(Ni²⁺));
one or more nucleic acid molecules selected from the group comprising miRNA miR-21, miR-124, miR-125, miR-126, miR-130 and miR-132, and
one or more transmembrane proteins selected from the group comprising tetraspanin proteins CD9, CD63 and CD81, or a fragment thereof,
for use in the treatment of a disorder selected from the group comprising epithelial diseases, cosmetic procedures, coagulation disorders.

A particularly preferred embodiment of the present invention is directed to a synthetic extracellular vesicle with the composition described above and specifically comprising:
one or more functional protein nicotinamide phosphoribosyltransferase, or a fragment thereof;
one or more transmembrane proteins selected from the group comprising tetraspanin proteins CD9, CD63 and CD81, or a fragment thereof;
one or more cytosolic proteins selected from the group comprising Apoptosis-Linked Gene 2-Interacting Protein X (ALIX), tumour susceptibility gene 101 protein (TSG101), or a fragment thereof,
wherein the synthetic extracellular vesicle do not comprise transferrin and albumin, or a fragment thereof,
for use in the treatment of a disorder selected from the group comprising inflammation, cancer, rheumatic disorder, severe graft versus host disease, osteoarthritis, cardiovascular disorder, epithelial diseases, neurodegenerative disorders, autoimmune disorders, bone and cartilage disorders, osteoporosis, renal osteodystrophy, paget's disease of bone, osteopetrosis, rickets, neurological disorders, intoxication, neuroendocrinology disorders, endocrinology disorders, genetic disorders, infectious diseases, dental disorders, cosmetic procedures, coagulation disorders, dermatoses, diabetes, age-associated disorders.

A particularly preferred embodiment of the present invention is directed to a synthetic extracellular vesicle with the composition described above and specifically comprising:
one or more functional protein nicotinamide phosphoribosyltransferase, or a fragment thereof;
one or more transmembrane proteins selected from the group comprising tetraspanin proteins CD9, CD63 and CD81, or a fragment thereof;
one or more cytosolic proteins selected from the group comprising Apoptosis-Linked Gene 2-Interacting Protein X (ALIX), tumour susceptibility gene 101 protein (TSG101), or a fragment thereof,
wherein the synthetic extracellular vesicle do not comprise transferrin and albumin, or a fragment thereof,
for use in the treatment of age-associated disorders.

Another particularly preferred embodiment of the present invention is directed to a synthetic extracellular vesicle with the composition described above, and specifically comprising:
one or more transmembrane proteins selected from the group comprising MHCII, CD80, and CD86, or a fragment thereof;
optionally one or more transmembrane proteins selected from the group comprising CD11c, MHC I, integrin α and β-chains, ICAM-1, and CD71, or a fragment thereof;
one or more functional proteins selected from the group comprising cytokines, interleukins, interleukin 4, milk fat globule-EGF factor 8 protein (MFGE8), growth factors, Fas, Fas ligand (FasL), indolamin-2,3-dioxygenase, cytotoxic T-lymphocyte-associated protein 4 - immunoglobulin fusion protein (CTLA4-Ig), tumor necrosis factor-related apoptosis-inducing ligand (Apo2L, TRAIL), or a fragment thereof,
for use in the treatment of a disorder selected from the group comprising inflammation, cancer, rheumatic disorder, severe graft versus host disease, osteoarthritis, cardiovascular disorder, epithelial diseases, neurodegenerative disorders, autoimmune disorders, bone and cartilage disorders, osteoporosis, renal osteodystrophy, paget's disease of bone, osteopetrosis, rickets, neurological disorders, intoxication, neuroendocrinology disorders, endocrinology disorders, genetic disorders, infectious diseases, dental disorders, cosmetic procedures, coagulation disorders, dermatoses, diabetes, age-associated disorders.

Another particularly preferred embodiment of the present invention is directed to a synthetic extracellular vesicle with the composition described above, and specifically comprising:
one or more transmembrane proteins selected from the group comprising MHCII, CD80, and CD86, or a fragment thereof;
optionally one or more transmembrane proteins selected from the group comprising CD11c, MHC I, integrin α and β-chains, ICAM-1, and CD71, or a fragment thereof;
one or more functional proteins selected from the group comprising cytokines, interleukins, interleukin 4, milk fat globule-EGF factor 8 protein (MFGE8), growth factors, Fas, Fas ligand (FasL), indolamin-2,3-dioxygenase, cytotoxic T-lymphocyte-associated protein 4 - immunoglobulin fusion protein (CTLA4-Ig), tumor necrosis factor-related apoptosis-inducing ligand (Apo2L, TRAIL), or a fragment thereof,
for use in the treatment of a disorder selected from the group comprising inflammation, cancer, rheumatic disorder, severe graft versus host disease, osteoarthritis, epithelial diseases, autoimmune disorders, infectious diseases, diabetes, age-associated disorders.

Another particularly preferred embodiment of the present invention is directed to a synthetic extracellular vesicle with the composition described above, and specifically comprising:
a lipid bilayer comprising 1,2-dioleoyl-sn-glycero-3-phosphocholine (DOPC), 1,2-dioleoyl-sn-glycero-3-phospho-rac-(1-glycerol) (DOPG), 1,2-dioleoyl-sn-glycero-3-phosphoethanolamine-N-(lissamine rhodamine B sulfonyl) (LissRhod PE), 1,2-dioleoyl-sn-glycero-3-[(N-(5-amino-1-carboxypentyl)iminodiacetic acid) succinyl] (nickel salt) (DGS-NTA(Ni2+));
functional protein Fas Ligand, or a fragment thereof;
optionally functional protein intercellular adhesion protein - 1, or a fragment thereof,
for use in the treatment of a disorder selected from the group comprising inflammation, cancer, rheumatic disorder, severe graft versus host disease, osteoarthritis, cardiovascular disorder, epithelial diseases, neurodegenerative disorders, autoimmune disorders, bone and cartilage disorders, osteoporosis, renal osteodystrophy, paget's disease of bone, osteopetrosis, rickets, neurological disorders, intoxication, neuroendocrinology disorders, endocrinology disorders, genetic disorders, infectious diseases, dental disorders, cosmetic procedures, coagulation disorders, dermatoses, diabetes, age-associated disorders.

Another particularly preferred embodiment of the present invention is directed to a synthetic extracellular vesicle with the composition described above, and specifically comprising:
a lipid bilayer comprising 1,2-dioleoyl-sn-glycero-3-phosphocholine (DOPC), 1,2-dioleoyl-sn-glycero-3-phospho-rac-(1-glycerol) (DOPG), 1,2-dioleoyl-sn-glycero-3-phosphoethanolamine-N-(lissamine rhodamine B sulfonyl) (LissRhod PE), 1,2-dioleoyl-sn-glycero-3-[(N-(5-amino-1-carboxypentyl)iminodiacetic acid) succinyl] (nickel salt) (DGS-NTA(Ni2+));
functional protein Fas Ligand, or a fragment thereof;
optionally functional protein intercellular adhesion protein - 1, or a fragment thereof,
for use in the treatment of a disorder selected from the group comprising inflammation, cancer, rheumatic disorder, severe graft versus host disease, autoimmune disorders, infectious diseases.

Another particularly preferred embodiment of the present invention is directed to a synthetic extracellular vesicle with the composition described above, and specifically comprising:
one or more transmembrane proteins selected from the group comprising CD29, CD44, CD90, CD73, CD44, Sca-1, or a fragment thereof;
one or more transmembrane proteins selected from the group comprising tetraspanin proteins CD9, CD63, and CD81, or a fragment thereof;
one or more functional proteins selected from the group comprising Wnta and Wntb, or a fragment thereof;
at least one nucleic acid molecule selected from the group comprising miR-140-5p, miR-92a-3p-e;
one or more nucleic acid molecules selected from the group comprising miR-17, miR-18a, miR-19a, miR-19b-1, miR-20a, miR-92a, let-7a, miR-21, miR124, miR126, miR-133b, miR-191, miR-222, miR-494, miR-6087, miR-30d-5p ;
optionally one or more nucleic acid molecules selected from the group comprising miR-33b, miR-451, miR-575, miR-630, miR-638, miR-1202, miR-1207-5p, miR-1225-5p, miR-1268, miR-K12-3,
for use in the treatment of a disorder selected from the group comprising inflammation, cancer, rheumatic disorder, severe graft versus host disease, osteoarthritis, cardiovascular disorder, epithelial diseases, neurodegenerative disorders, autoimmune disorders, bone and cartilage disorders, osteoporosis, renal osteodystrophy, paget's disease of bone, osteopetrosis, rickets, neurological disorders, intoxication, neuroendocrinology disorders, endocrinology disorders, genetic disorders, infectious diseases, dental disorders, cosmetic procedures, coagulation disorders, dermatoses, diabetes, age-associated disorders.

Another more particularly preferred embodiment of the present invention is directed to a synthetic extracellular vesicle with the composition described above, and specifically comprising:
a lipid bilayer comprising 1,2-dioleoyl-sn-glycero-3-phosphocholine (DOPC), 1,2-dioleoyl-sn-glycero-3-phospho-rac-(1-glycerol) (DOPG), 1,2-dioleoyl-sn-glycero-3-phosphoethanolamine-N-(lissamine rhodamine B sulfonyl) (LissRhod PE), 1,2-dioleoyl-sn-glycero-3-[(N-(5-amino-1-carboxypentyl)iminodiacetic acid) succinyl] (nickel salt) (DGS-NTA(Ni2+)), functional protein RANK, or a fragment thereof,
for use in the treatment of a disorder selected from the group comprising inflammation, cancer, rheumatic disorder, severe graft versus host disease, osteoarthritis, cardiovascular disorder, epithelial diseases, neurodegenerative disorders, autoimmune disorders, bone and cartilage disorders, osteoporosis, renal osteodystrophy, paget's disease of bone, osteopetrosis, rickets, neurological disorders, intoxication, neuroendocrinology disorders, endocrinology disorders, genetic disorders, infectious diseases, dental disorders, cosmetic procedures, coagulation disorders, dermatoses, diabetes, age-associated disorders.

Another more particularly preferred embodiment of the present invention is directed to a synthetic extracellular vesicle with the composition described above, and specifically comprising:
a lipid bilayer comprising 1,2-dioleoyl-sn-glycero-3-phosphocholine (DOPC), 1,2-dioleoyl-sn-glycero-3-phospho-rac-(1-glycerol) (DOPG), 1,2-dioleoyl-sn-glycero-3-phosphoethanolamine-N-(lissamine rhodamine B sulfonyl) (LissRhod PE), 1,2-dioleoyl-sn-glycero-3-[(N-(5-amino-1-carboxypentyl)iminodiacetic acid) succinyl] (nickel salt) (DGS-NTA(Ni2+)), functional protein RANK, or a fragment thereof,
for use in the treatment of a disorder selected from the group comprising osteoarthritis, bone and cartilage disorders, osteoporosis, renal osteodystrophy, paget's disease of bone, osteopetrosis, rickets.

Also described herein is a **method for treating or ameliorating** a disorder comprising administering to a patient suffering from said disorder a therapeutically effective amount of a synthetic extracellular vesicle as disclosed herein, wherein the disorder is selected from the group comprising inflammation, cancer, rheumatic disorder, severe graft versus host disease, osteoarthritis, cardiovascular disorder, epithelial diseases, neurodegenerative disorders, autoimmune disorders, bone and cartilage disorders, osteoporosis, renal osteodystrophy, paget's disease of bone, osteopetrosis, rickets, neurological disorders, intoxication, neuroendocrinology disorders, endocrinology disorders, genetic disorders, infectious diseases, dental disorders, cosmetic procedures, coagulation disorders, dermatoses, diabetes, age-associated disorders.

Also described herein is a method for treating or ameliorating a disorder comprising administering to a patient suffering from said disorder a therapeutically effective amount of a synthetic extracellular vesicle having a diameter comprised between 70 and 5000 nm, comprising:
a lipid bilayer comprising at least two lipids selected from the group comprising:
a neutral lipid selected from the group comprising ceramide, sphingomyelin, cephalin, cholesterol, cerebrosides, diacylglycerols, phosphatidylcholines, lysophosphatidylcholines, phosphatidylethanolamines, lysophosphatidylethanolamine, lysoethanolamines, inverted headgroup lipids, sphingosins, sterol-modified phospholipids, ether ester lipids, diether lipids, vinyl ether (plasmalogen);
an anionic lipid selected from the group comprising phosphatidic acids, lysophosphatidic acid derivatives, phosphatidylglycerols, lysophosphatidylglycerols, phosphatidylserines, lysophosphatidylserines, phosphatidylinositols, phosphatidylinositolphosphates, cardiolipins, Bis(Monoacylglycero)Phosphate derivatives;
a cationic lipid selected from the group comprising dioleyl-N,N-dimethylammonium chloride; N-(2,3-dioleyloxy)propyl)-N,N,N-trimethylammonium chloride; N,N-distearyl-N,N-dimethylammonium bromide; N-(2,3- dioleyloxy)propyl)-N,N,N-trimethylammonium chloride; 3β-(N-(N',N'-dimethylaminoethane)-carbamoyl)cholesterol; 1,2-dimyristyloxypropyl-3-dimethyl-hydroxy ethyl ammonium bromide; 2,3-dioleyloxy-N-[2(sperminecarboxamido) ethyl]-N,N-dimethyl-1-propanaminium trifluoroacetate; dioctadecylamidoglycyl carboxyspermine; N-(2,3-dioleyloxy)propyl)-N,N-dimethylammonium chloride and 1,2-Dioleoyl-3-dimethylammonium-propane;
a pH- sensitive lipid selected from the group comprising lipid N-(4-carboxybenzyl)-N,N-dimethyl-2,3-bis(oleoyloxy)propan-1-aminium, 1,2-distearoyl-3-dimethylammonium-propane, 1,2-dipalmitoyl-sn-glycero-3-succinate, 1,2-dioleoyl-sn-glycero-3-succinate, N-palmitoyl homocysteine ;
a photoswitchable lipid;
acylglycine derivatives, prenol derivatives, prostaglandine derivatives, glycosylated diacyl glycerols, eicosanoid derivatives, (palmitoyloxy)octadecanoic acid derivatives, diacetylene derivatives, diphytanoyl derivatives, fluorinated lipids, brominated lipids, lipopolysaccharides;
one of the aforementioned lipids coupled to a functional ligand selected from the group comprising biotin, N-hydroxysuccinimide ester, nitrilotriacetic acid - nickel, amine, carboxylic acid, maleimides, dithiopyridinyl, pyridyl disulfide , pyridyldithiopropionate, N-benzylguanine, carboxyacyl, cyanur, folate, square, galloyl, glycan, thiol, arginylglycylaspartic acid, a fluorescent dye molecule, a magnetic resonance imaging reagent, a chelator;
one of the aforementioned lipids coupled to polyethyleneglycol with a molecular weight comprised between 350 and 50,000 g/mole.
optionally one or more nucleic acids selected from the group comprising DNA, cDNA, mRNA, siRNA, antisense nucleotides, shRNA, piRNA, snRNA, IncRNA, PNA, left handed DNA, Clustered Regularly Interspaced Short Palindromic Repeats (CRISPR) guide RNA;
optionally one or more nucleic acids selected from the group comprising miRNA molecules selected from the group comprising miR-17, miR-18a, miR-19a, miR-19b-1, miR-20a, miR-92a; miR-21, miR-30d-5p, miR-33b, miR-124, miR-125, miR-126, miR-130, miR-132, miR-133b, miR-140-5p, miR-191, miR-222, miR-451, miR-494, miR-575, miR-630, miR-638, miR-1202, miR-1207-5p, miR-1225-5p, miR-1268, miR-6087, miR-92a-3p-e, miR-K12-3, let-7a.
one or more transmembrane proteins selected from the group comprising tetraspanin proteins CD9, CD37, CD47, CD53, CD63, CD81, CD82, CD151, Tspan8, heterotrimeric G protein subunit alpha (GNA), integrin α-chains and β-chains, transferrin receptor 1 (TfR1, CD71), transferrin receptor 2 (TFR2), lysosome associated membrane proteins (LAMP1, LAMP2), heparan sulfate proteoglycans, syndecans, extracellular matrix metalloproteinase inducer (EMMPRIN, BSG), A Disintegrin And Metalloproteinase Domain 10 (ADAM10), CD3, CD11c, CD14, CD29, CD31, CD41, CD42a, CD44, CD45, CD50 (intercellular adhesion molecule 1, ICAM-1), CD55, CD59, CD73, CD80, CD86, CD90, sonic hedgehog (SHH), major histocompatibility complex I (MHC I), major histocompatibility complex II (MHC II), epidermal growth factor receptor 2 (ERBB2), epithelial cell adhesion molecule (EpCAM), Glycophorin A (GYPA); Acetylcholinesterase S and E (AChE-S, AChE-E), amyloid beta precursor protein (APP), multidrug resistance-associated protein 1 (ABCC1), stem cells antigen-1 (Sca-1), or a fragment thereof;
optionally one or more cytosolic proteins selected from the group comprising the protein complexes endosomal sorting complexes required for transport ESCRT-I, ESCRT-II, and ESCRT-III, tumour susceptibility gene 101 (TSG101), charged multivesicular body protein (CHMP), Apoptosis-Linked Gene 2-Interacting Protein X (ALIX), vacuolar protein sorting 4 homolog A 4A and 4B, arrestin domain-containing protein (ARRDC1), flotillin-1, flotillin-2; caveolins, EH-domain containing 1 - 4 (EHD1 - EHD4), Ras homolog family member A (RHOA), annexins, heat shock proteins, ADP-ribosylation factor 6 (ARF6), syntenin, microtubule-associated protein Tau (MAPT), or a fragment thereof;
one or more functional proteins selected from the group comprising cytokines, growth factors, interleukins, milk fat globule-EGF factor 8 protein (MFGE8), adhesion proteins, extracellular matrix proteins, nicotinamide phosphoribosyltransferase, signal transduction proteins Wnta and Wntb, Fas, Fas Ligand (FasL), RANK, RANK Ligand (RANKL), indolamin-2,3-dioxygenase, cytotoxic T-lymphocyte-associated protein 4 - immunoglobulin fusion protein, tumor necrosis factor-related apoptosis-inducing ligand, or a fragment thereof;
optionally one or more proteins associated to intracellular compartments selected from the group comprising histone proteins, lamin A/C, inner membrane mitochondrial protein (IMMT), cytochrome C-1 (CYC1), mitochondrial import receptor subunit TOM20, calnexin, heat shock protein 90kDa beta (Grp94), member 1 (HSP90B1), heat shock 70kDa protein 5 (HSPA5), Golgin A2 (GM130, GOLGA2), Autophagy Related 9A (ATG9A), actinin1, actinin4 (ACTN1, ACTN4), cytokeratin 18 (KRT18), or a fragment thereof,
wherein the disorder is selected from the group comprising inflammation, cancer, rheumatic disorder, severe graft versus host disease, osteoarthritis, cardiovascular disorder, epithelial diseases, neurodegenerative disorders, autoimmune disorders, bone and cartilage disorders, osteoporosis, renal osteodystrophy, paget's disease of bone, osteopetrosis, rickets, neurological disorders, intoxication, neuroendocrinology disorders, endocrinology disorders, genetic disorders, infectious diseases, dental disorders, cosmetic procedures, coagulation disorders, dermatoses, diabetes, age-associated disorders,

Also described herein is a method for treating or ameliorating a disorder comprising administering to a patient suffering from said disorder a therapeutically effective amount of a synthetic extracellular vesicle with the composition described above and specifically comprising:
a lipid bilayer comprising cholesterol, N-stearoyl-D-erythro-sphingosylphosphorylcholine (SM), 1,2-dioleoyl-sn-glycero-3-phosphocholine (DOPC), 1,2-dioleoyl-sn-glycero-3-phospho-L-serine (DOPS), 1,2-dioleoyl-sn-glycero-3-phospho-ethanolamine (DOPE), 1,2-dioleoyl-sn-glycero-3-phospho-rac-(1-glycerol) (DOPG), 1,2-dioleoyl-sn-glycero-3-phosphate (sodium salt) (PA), diacylglycerol, phosphatidylinositol, 1,2-dioleoyl-sn-glycero-3-phosphoethanolamine-N-(lissamine rhodamine B sulfonyl) (LissRhod PE), 1,2-dioleoyl-sn-glycero-3-[(N-(5-amino-1-carboxypentyl)iminodiacetic acid) succinyl] (nickel salt) (DGS-NTA(Ni²⁺));
one or more nucleic acid molecules selected from the group comprising miRNA miR-21, miR-124, miR-125, miR-126, miR-130 and miR-132, and
one or more transmembrane proteins selected from the group comprising tetraspanin proteins CD9, CD63 and CD81, or a fragment thereof,
wherein the disorder is selected from the group comprising inflammation, cancer, rheumatic disorder, severe graft versus host disease, osteoarthritis, cardiovascular disorder, epithelial diseases, neurodegenerative disorders, autoimmune disorders, bone and cartilage disorders, osteoporosis, renal osteodystrophy, paget's disease of bone, osteopetrosis, rickets, neurological disorders, intoxication, neuroendocrinology disorders, endocrinology disorders, genetic disorders, infectious diseases, dental disorders, cosmetic procedures, coagulation disorders, dermatoses, diabetes, age-associated disorders.

Also described herein is a method for treating or ameliorating a disorder comprising administering to a patient suffering from said disorder a therapeutically effective amount of a synthetic extracellular vesicle with the composition described above and specifically comprising:
one or more functional protein nicotinamide phosphoribosyltransferase, or a fragment thereof;
one or more transmembrane proteins selected from the group comprising tetraspanin proteins CD9, CD63 and CD81, or a fragment thereof;
one or more cytosolic proteins selected from the group comprising Apoptosis-Linked Gene 2-Interacting Protein X (ALIX), tumour susceptibility gene 101 protein (TSG101), or a fragment thereof,
wherein the synthetic extracellular vesicle do not comprise transferrin and albumin, or a fragment thereof,
wherein the disorder is selected from the group comprising inflammation, cancer, rheumatic disorder, severe graft versus host disease, osteoarthritis, cardiovascular disorder, epithelial diseases, neurodegenerative disorders, autoimmune disorders, bone and cartilage disorders, osteoporosis, renal osteodystrophy, paget's disease of bone, osteopetrosis, rickets, neurological disorders, intoxication, neuroendocrinology disorders, endocrinology disorders, genetic disorders, infectious diseases, dental disorders, cosmetic procedures, coagulation disorders, dermatoses, diabetes, age-associated disorders.

Also described herein is a method for treating or ameliorating a disorder comprising administering to a patient suffering from said disorder a therapeutically effective amount of a synthetic extracellular vesicle with the composition described above, and specifically comprising:
one or more transmembrane proteins selected from the group comprising MHCII, CD80, and CD86, or a fragment thereof;
optionally one or more transmembrane proteins selected from the group comprising CD11c, MHC I, integrin α and β-chains, ICAM-1, and CD71, or a fragment thereof;
one or more functional proteins selected from the group comprising cytokines, interleukins, interleukin 4, milk fat globule-EGF factor 8 protein (MFGE8), growth factors, Fas, Fas ligand (FasL), indolamin-2,3-dioxygenase, cytotoxic T-lymphocyte-associated protein 4 - immunoglobulin fusion protein (CTLA4-Ig), tumor necrosis factor-related apoptosis-inducing ligand (Apo2L, TRAIL), or a fragment thereof,
wherein the disorder is selected from the group comprising inflammation, cancer, rheumatic disorder, severe graft versus host disease, osteoarthritis, cardiovascular disorder, epithelial diseases, neurodegenerative disorders, autoimmune disorders, bone and cartilage disorders, osteoporosis, renal osteodystrophy, paget's disease of bone, osteopetrosis, rickets, neurological disorders, intoxication, neuroendocrinology disorders, endocrinology disorders, genetic disorders, infectious diseases, dental disorders, cosmetic procedures, coagulation disorders, dermatoses, diabetes, age-associated disorders.

Also described herein is a method for treating or ameliorating a disorder comprising administering to a patient suffering from said disorder a therapeutically effective amount of a synthetic extracellular vesicle with the composition described above, and specifically comprising:
a lipid bilayer comprising 1,2-dioleoyl-sn-glycero-3-phosphocholine (DOPC), 1,2-dioleoyl-sn-glycero-3-phospho-rac-(1-glycerol) (DOPG), 1,2-dioleoyl-sn-glycero-3-phosphoethanolamine-N-(lissamine rhodamine B sulfonyl) (LissRhod PE), 1,2-dioleoyl-sn-glycero-3-[(N-(5-amino-1-carboxypentyl)iminodiacetic acid) succinyl] (nickel salt) (DGS-NTA(Ni2+));
functional protein Fas Ligand, or a fragment thereof;
optionally functional protein intercellular adhesion protein - 1, or a fragment thereof,
wherein the disorder is selected from the group comprising inflammation, cancer, rheumatic disorder, severe graft versus host disease, osteoarthritis, cardiovascular disorder, epithelial diseases, neurodegenerative disorders, autoimmune disorders, bone and cartilage disorders, osteoporosis, renal osteodystrophy, paget's disease of bone, osteopetrosis, rickets, neurological disorders, intoxication, neuroendocrinology disorders, endocrinology disorders, genetic disorders, infectious diseases, dental disorders, cosmetic procedures, coagulation disorders, dermatoses, diabetes, age-associated disorders.

Also described herein is a method for treating or ameliorating a disorder comprising administering to a patient suffering from said disorder a therapeutically effective amount of a synthetic extracellular vesicle with the composition described above, and specifically comprising:
one or more transmembrane proteins selected from the group comprising CD29, CD44, CD90, CD73, CD44, Sca-1, or a fragment thereof;
one or more functional proteins selected from the group comprising Wnta and Wntb, or a fragment thereof;
at least one nucleic acid molecule selected from the group comprising miR-140-5p, miR-92a-3p-e;
one or more transmembrane proteins selected from the group comprising tetraspanin proteins CD9, CD63, and CD81, or a fragment thereof;
one or more nucleic acid molecules selected from the group comprising miR-17, miR-18a, miR-19a, miR-19b-1, miR-20a, miR-92a, let-7a, miR-21, miR124, miR126, miR-133b, miR-191, miR-222, miR-494, miR-6087, miR-30d-5p ;
optionally one or more nucleic acid molecules selected from the group comprising miR-33b, miR-451, miR-575, miR-630, miR-638, miR-1202, miR-1207-5p, miR-1225-5p, miR-1268, miR-K12-3,
wherein the disorder is selected from the group comprising inflammation, cancer, rheumatic disorder, severe graft versus host disease, osteoarthritis, cardiovascular disorder, epithelial diseases, neurodegenerative disorders, autoimmune disorders, bone and cartilage disorders, osteoporosis, renal osteodystrophy, paget's disease of bone, osteopetrosis, rickets, neurological disorders, intoxication, neuroendocrinology disorders, endocrinology disorders, genetic disorders, infectious diseases, dental disorders, cosmetic procedures, coagulation disorders, dermatoses, diabetes, age-associated disorders.

Further described herein is a method for treating or ameliorating a disorder comprising administering to a patient suffering from said disorder a therapeutically effective amount of a synthetic extracellular vesicle with the composition described above, and specifically comprising:
a lipid bilayer comprising 1,2-dioleoyl-sn-glycero-3-phosphocholine (DOPC), 1,2-dioleoyl-sn-glycero-3-phospho-rac-(1-glycerol) (DOPG), 1,2-dioleoyl-sn-glycero-3-phosphoethanolamine-N-(lissamine rhodamine B sulfonyl) (LissRhod PE), 1,2-dioleoyl-sn-glycero-3-[(N-(5-amino-1-carboxypentyl)iminodiacetic acid) succinyl] (nickel salt) (DGS-NTA(Ni2+)), functional protein RANK, or a fragment thereof,
wherein the disorder is selected from the group comprising inflammation, cancer, rheumatic disorder, severe graft versus host disease, osteoarthritis, cardiovascular disorder, epithelial diseases, neurodegenerative disorders, autoimmune disorders, bone and cartilage disorders, osteoporosis, renal osteodystrophy, paget's disease of bone, osteopetrosis, rickets, neurological disorders, intoxication, neuroendocrinology disorders, endocrinology disorders, genetic disorders, infectious diseases, dental disorders, cosmetic procedures, coagulation disorders, dermatoses, diabetes, age-associated disorders.

**"Disorder"** is any condition that would benefit from treatment with a substance/molecule or method described herein.

**"Cell proliferative disorder"** and **"proliferative disorder"** refer to disorders that are associated with some degree of abnormal cell proliferation, such as cancer.

**"Cancer"** and **"cancerous"** refer to, or describe a physiological condition in mammals that is typically characterized by a cell proliferative disorder. Cancer generally can include, but is not limited to, carcinoma, lymphoma (e.g., Hodgkin's and non-Hodgkin's lymphoma), blastoma, sarcoma, and leukemia. More specific examples of cancer can include, squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma, leukemia and other lymphoproliferative disorders, and various types of head and neck cancer.

**"Tumour"** refers to all neoplastic cell growth and proliferation, whether malignant or benign, and all pre-cancerous and cancerous cells and tissues. The terms "cancer", "cancerous", "cell proliferative disorder", "proliferative disorder", and "tumour" are not mutually exclusive as referred to herein.

**"Cardiovascular disorders"** include but are not limited to disorders of the heart and the vascular system like congestive heart failure, myocardial infarction, ischemic diseases of the heart, all kinds of atrial and ventricular arrhythmias, hypertensive vascular diseases, peripheral vascular diseases, and atherosclerosis.

**"Metastasis"** refers to the spread of cancer and/or tumour from its primary site to other places in the body of an individual.

The term **"neurodegenerative disease"** or **"neurological disorder"** or **"neuroinflammatory disorder"** refers to any disease, disorder, or condition affecting the central or peripheral nervous system. Preferred examples of neurodegenerative diseases and neuroinflammatory disorders are selected from the group comprising or consisting of: Alzheimer's disease, Parkinson's disease, Creutzfeldt Jakob disease (CJD), new variant of Creutzfeldt Jakobs disease (nvCJD), Hallervorden Spatz disease, Huntington's disease, multisystem atrophy, dementia, frontotemporal dementia, motor neuron disorders of multiple spontaneous or genetic background, amyotrophic lateral sclerosis (ALS), spinal muscular atrophy, spinocerebellar atrophies (SCAs), schizophrenia, affective disorders, major depression, meningoencephalitis, bacterial meningoencephalitis, viral meningoencephalitis, CNS autoimmune disorders, multiple sclerosis (MS), acute ischemic / hypoxic lesions, stroke, CNS and spinal cord trauma, head and spinal trauma, brain traumatic injuries, arteriosclerosis, atherosclerosis, microangiopathic dementia, Binswanger' disease (Leukoaraiosis), retinal degeneration, cochlear degeneration, macular degeneration, cochlear deafness, AIDS-related dementia, retinitis pigmentosa, fragile X-associated tremor/ataxia syndrome (FXTAS), progressive supranuclear palsy (PSP), striatonigral degeneration (SND), olivopontocerebellear degeneration (OPCD), Shy Drager syndrome (SDS), age dependant memory deficits, neurodevelopmental disorders associated with dementia, Down's Syndrome, synucleinopathies, superoxide dismutase mutations, trinucleotide repeat disorders as Huntington's Disease, trauma, hypoxia, vascular diseases, vascular inflammations, CNS-ageing. Also age dependant decrease of stem cell renewal may be addressed.

**"Aging-associated disorders** and **diseases"** are most often seen with increasing frequency with increasing senescence. Examples of aging-associated diseases are atherosclerosis and cardiovascular disease, cancer, arthritis, cataracts, osteoporosis, type 2 diabetes, hypertension and Alzheimer's disease. The incidence of all of these diseases increases exponentially with age.

**"Rheumatic diseases"** are characterized by inflammation that affects the connecting or supporting structures of the body; most commonly the joints, but also sometimes the tendons, ligaments, bones, and muscles. Some rheumatic diseases even affect the organs. These diseases can ultimately cause loss of function in those body parts. Preferred examples of **rheumatic diseases** and are selected from the group comprising or consisting of: osteoarthritis, rheumatoid arthritis, fibromyalgia, systemic lupus erythematosus, gout, juvenile idiopathic arthritis, arthritis, scleroderma.

**"Epithelial diseases"** include acne, atopic eczema, atopic dermatitis, contact dermatitis, impetigo, psoriasis, sunburn, sweating disorders, yeast infections of the mucous membranes.

**"Endocrinology disorders"** include diabetes, adrenal insufficiency, cushing's disease, gigantism, hyperthyroidism, hypothyroidism, hypopituitarism, polycystic ovary syndrome.
Neuroendocrine disorders are disorders that affect the interaction between the nervous system and the endocrine system. Examples of neuroendocrine disorders include diabetes insipidus, Kallman syndrome, neuroendocrine cancer, and neuroendocrine tumors (NETs), which are neoplasms that arise from cells of the endocrine and nervous systems.

**"Bone and cartilage disorders"** include diseases or injuries that affect human bones and cartilage.
**"Osteoarthritis"** is one of the leading causes of disability in adults worldwide. It is a degenerative disease of the joints secondary to many predisposing factors, most notably age, joint injury, altered mechanical stress, and obesity. All these processes cause a local chronic inflammatory response resulting in the progressive joint failure characteristic of osteoarthritis.
**"Osteoporosis"** is the result of cumulative bone loss during aging. Nevertheless, a wide variety of diseases, medications, and lifestyles can cause or contribute to the development of osteoporosis. In addition, the immune system participates in the regulation of bone homeostasis through production of cytokines and inflammatory mediators with subsequent activation of cartilage-degrading proteinases.
**"Paget's disease"** is a chronic skeletal disorder, caused by enhanced bone resorption followed by abnormal bone formation, in which a potential cross talk between the bone and the immune system takes place.
Other bone related disorders include renal osteodystrophy, osteopetrosis, rickets.

**"Cartilage disorders"** include osteoarthritis, costochondritis enchondromatosis, herniation, achondroplasia, relapsing polychondritis, chondroma, chondrosarcoma.

**"Treatment", "treat"** or **"treating"** refer to clinical intervention in an attempt to alter the natural course of a disorder in the individual being treated, and can be performed either for prophylaxis or during the course of clinical pathology. Desired results of treatment can include, but are not limited to, preventing occurrence or recurrence of the disorder, alleviation of symptoms, diminishment of any direct or indirect pathological consequences of the disorder, preventing metastasis, decreasing the rate of progression, amelioration or palliation of a disease state, and remission or improved prognosis. For example, treatment can include administration of a therapeutically effective amount of a pharmaceutical formulation comprising a synthetic extracellular vesicle disclosed herein to a subject to delay development or slow progression of a disorder, wherein the disorder is selected from the group comprising inflammation, cancer, rheumatic disorder, severe graft versus host disease, osteoarthritis, cardiovascular disorder, epithelial diseases, neurodegenerative disorders, autoimmune disorders, bone and cartilage disorders, osteoporosis, renal osteodystrophy, paget's disease of bone, osteopetrosis, rickets, neurological disorders, intoxication, neuroendocrinology disorders, endocrinology disorders, genetic disorders, infectious diseases, dental disorders, cosmetic procedures, coagulation disorders, dermatoses, diabetes, age-associated disorders.

**"Pharmaceutical formulation"** refers to a preparation in a form that allows the biological activity of the active ingredient (s) to be effective, and which contain no additional components which are toxic to the subjects to which the formulation is administered.

**"Pharmaceutically acceptable carrier"** refers to an ingredient in a pharmaceutical formulation, other than an active ingredient, which is nontoxic to the subject to whom it is administered. A pharmaceutically acceptable carrier includes, but is not limited to, a buffer, excipient, stabilizer, or preservative.

**"Therapeutically effective amount"** refers to the amount of an active ingredient or agent (e.g., a pharmaceutical formulation) to achieve a desired therapeutic or prophylactic result, e.g., to treat or prevent a disease or disorder in a subject. In the case of a cancer, the therapeutically effective amount of the therapeutic agent is an amount that reduces the number of cancer cells ; reduces the primary tumour size ; inhibits (i.e. slows to some extent and preferably stop) cancer cell infiltration into peripheral organs; inhibits (i.e. slows to some extent and preferably stop) tumour metastasis; inhibits, to some extent, tumour growth; and/or relieves to some extent one or more of the symptoms associated with the cancer. To the extent the drug may prevent growth and/or kill existing cancer cells, it may be cytostatic and/or cytotoxic. For cancer therapy, efficacy in vivo can, for example, be measured by assessing the duration of survival, time to disease progression (TTP), the response rates (RR), duration of response, and/or quality of life.

**"Individual"** or **"subject"** refers to a mammal, including but not limited to, domesticated animals (e.g., cows, sheep, cats, dogs, and horses), primates (e.g., humans and non-human primates such as monkeys), rabbits, and rodents (e.g., mice and rats).

A **"therapeutic agent"** or **"therapeutic molecule"** includes a compound or molecule that, when present in an effective amount, produces a desired therapeutic effect, pharmacologic and/or physiologic effect on a subject in need thereof. It includes any compound, e.g. , a small molecule drug, or a biologic (e.g. , a polypeptide drug or a nucleic acid drug) that when administered to a subject has a measurable or conveyable effect on the subject, e.g., it alleviates or decreases a symptom of a disease, disorder or condition.

As used herein, the term **"antibody"** encompasses an immunoglobulin whether natural or partly or wholly synthetically produced, and fragments thereof. The term also covers any protein having a binding domain that is homologous to an immunoglobulin binding domain. "Antibody" further includes a polypeptide comprising a framework region from an immunoglobulin gene or fragments thereof that specifically binds and recognizes an antigen. Use of the term antibody is meant to include whole antibodies, polyclonal, monoclonal and recombinant antibodies, fragments thereof, and further includes single-chain antibodies, humanized antibodies, murine antibodies, chimeric, mouse-human, mouse-primate, primate-human monoclonal antibodies, anti-idiotype antibodies, antibody fragments, such as, e.g., scFv, (scFv)2, Fab, Fab', and F(ab')2, F(abl)2, Fv, dAb, and Fd fragments, diabodies, and antibody-related polypeptides. Antibody includes bispecific antibodies and multispecific antibodies so long as they exhibit the desired biological activity or function.

### Description of the Figures

- Figure 1: shows a schematic representation of the production pipeline for bottom-up assembly of fully synthetic extracellular vesicles within a stabilizing polymer shell.
- Figure 2: shows quantification of lipid ratios (18:1 DOPI, 18:1 DAG, 18:1 PA, 18:1 DOPG, 18:1 DOPE, 18:1 DOPS, 18:1 DOPC, SM, Cholesterol) in the starting small unilamellar vesicles and in the produced synthetic extracellular vesicles as quantified by electrospray-ionization tandem mass spectrometry. These results are compared to the corresponding expected lipid ratio according to design of fully synthetic extracellular vesicles.
- Figure 3: shows transmission electron micrographs of uranyl acetate negative stained natural and synthetic vesicles. Left panel shows extracellular vesicles (black arrows) isolated from 48 hours conditioned K562 cell culture media by differential ultracentrifugation. Middle panel shows extracellular vesicles isolated from conditioned K562 media by a commercial distributer. Right panel shows fully synthetic extracellular vesicles produced with the lipid composition 70 mol% DOPC, 5 mol% DOPE, 20 mol% DOPG, 5 mol% DOPS according to one embodiment of the invention. Scale bars 100 nm, 100 nm and 1 µm, respectively.
- Figure 4: shows denaturating SDS polyacrylamide gel electrophoretic characterization of synthetic extracellular vesicles protein content. 3 µg of two different production batches of K562 Extracellular vesicles isolated by a commercial distributer were loaded on lane 1 and 2. 3 µg extracellular vesicles from two separate isolations of K562 cells were loaded on lane 3 and 4. 500 ng of synthetic extracellular vesicles from two separate assemblies and decorated with the extracellular domains of CD9 (Ser112-lle195) and TSG101 (Gly1-Pro145) were loaded on lane 5 and 6. Normalized Line profile intensities of respective lanes are shown on the right.
- Figure 5: shows representative confocal microscopy images of fully synthetic extracellular vesicles containing LissRhodamine B PE lipids, Alexa488 labeled CD9 (CD9) and Hoechst 33342 labeled miRNAs miR-21, miR-124, miR-125, miR-126, miR-130 and miR-132. Scale bar is 2 µm.

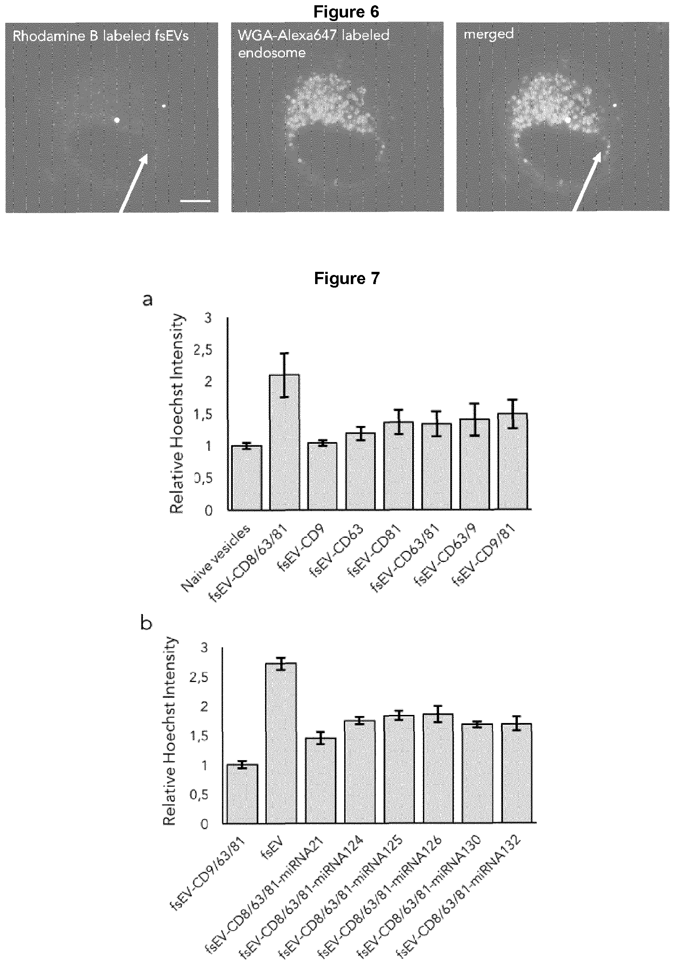

- Figure 6: shows single plan fluorescence confocal microscopy images of synthetic extracellular vesicles labelled with rhodamine B PE (left panel) and incubated with HaCaT keratynocytes stained with wheat germ agglutinin (WGA)-Alexa647 (middle panel) for 24 hours. Synthetic extracellular vesicles are internalized and co-localized with the WGA stained endosomes (right panel). Scale bar is 5 µm.
- Figure 7: shows (a and b) fluorescence intensity analysis of Hoechst 33342 stained HaCaT keratinocyte cultures to compare the effect of synthetic extracellular vesicles differing for the composition in tetraspanins or miRNAs, after treatment for 48 hours. Results are shown as mean ± SD, n=3 technical replicates.

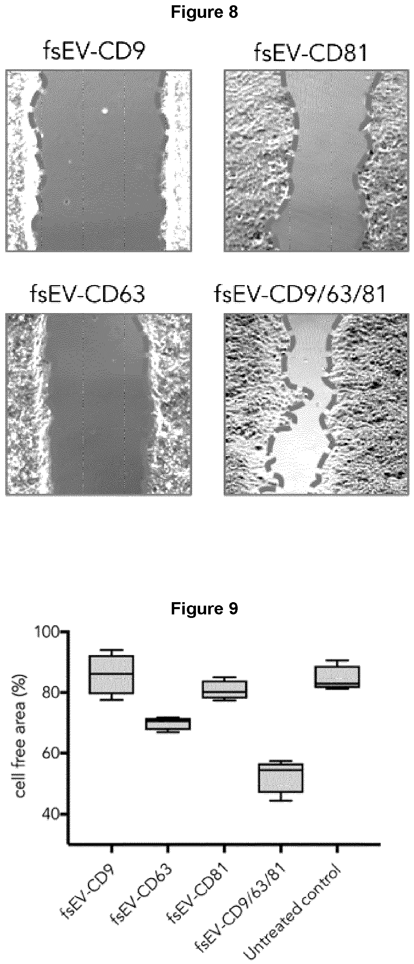

- Figure 8: shows phase contrast images of cell exclusion *in vitro* wound healing assays after 16 hours of migration of HaCaT monolayers pre-treated with the indicated different synthetic extracellular vesicles for 24 hours.
- Figure 9: shows *in vitro* quantification of wound healing migration assay of HaCaT keratinocyte monolayers treated for 24 hours with extracellular vesicles decorated with different tetraspanins, showed also in Figure 7. Box-plots show mean values, 0.75 and 0.25 quantile values, whiskers show maximum and minimum values, n=4 artificial wound sides.

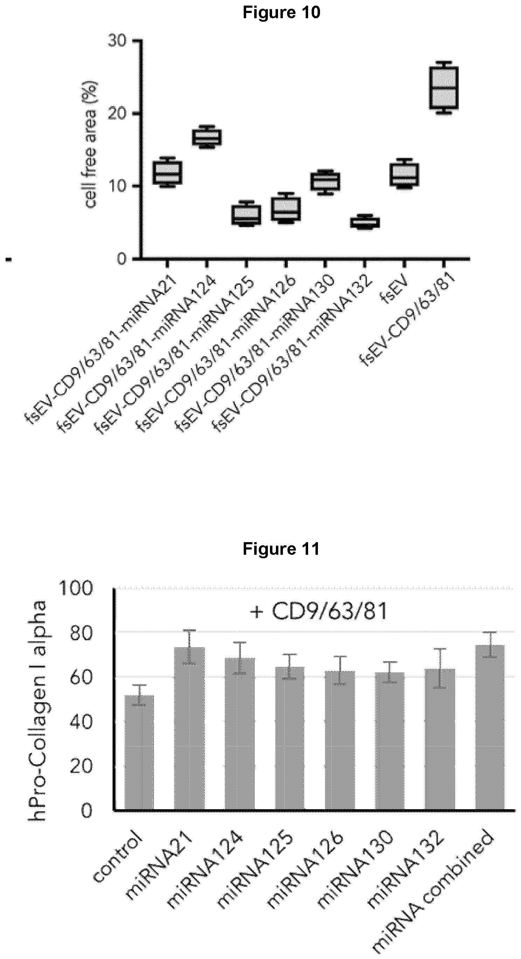

- Figure 10: shows *in vitro* quantification of wound healing migration assay of HaCaT keratinocyte monolayers treated for 24 hours with extracellular vesicles having different miRNAs, and showed also in Figure 7. Box-plots show mean values, 0.75 and 0.25 quantile values, whiskers show maximum and minimum values, n=4 artificial wound sides.
- Figure 11: shows enzyme-linked immunosorbent assay analysis of pro-collagen-Iα deposition of dermal fibroblasts after treatment for 24 hours with CD9, CD63 and CD81 decorated synthetic extracellular vesicles with different miRNA compositions. Results are shown as mean ± SD, n=3 technical replicates.

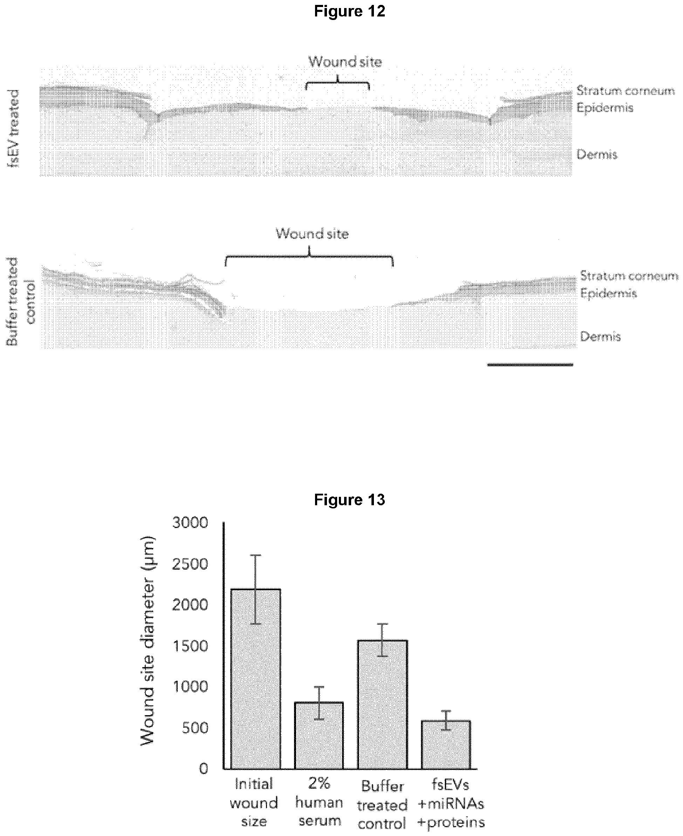

- Figure 12: shows hematoxilin/eosin stained histological sections of epidermal-wounded full thickness human organotypic skin models treated with the synthetic extracellular vesicles (fsEVs) or only the buffer control for 48 hours. Scale bar is 1 mm.
- Figure 13: shows quantification of epidermal wound-bed closure of full thickness human organotypic skin models from Figure 11 treated with 2% human serum (positive control), buffer treated controls (negative control) and synthetic extracellular vesicles loaded with miRNAs miR-21, miR-124, miR-125, miR-126, miR-130 and miR-132 and decorated with CD9, CD63 and CD81. Results are shown as mean ± SD, n=3 individual organotypic cultures.

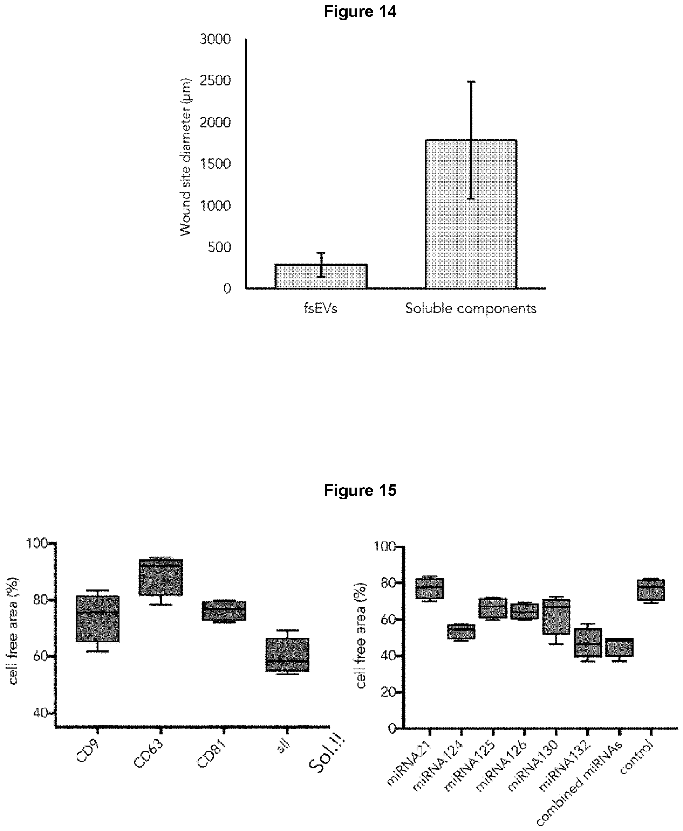

- Figure 14: shows quantification of epidermal wound-bed closure of full thickness human organotypic skin models treated with synthetic extracellular vesicles or only the soluble miRNA and tetraspanin components for 48 hours. Results are shown as mean ± SD, n=3 individual organotypic cultures.
- Figure 15: shows effect of synthetic extracellular vesicles on migration of A431 carcinoma cells, analysed by an *in vitro* wound healing migration assay of A431 carcinoma monolayers treated for 24 hours with synthetic extracellular vesicle variants. Box-plots show mean, 0.75 and 0.25 quantile, whiskers show maximum and minimum values, n=4 artificial wound sides

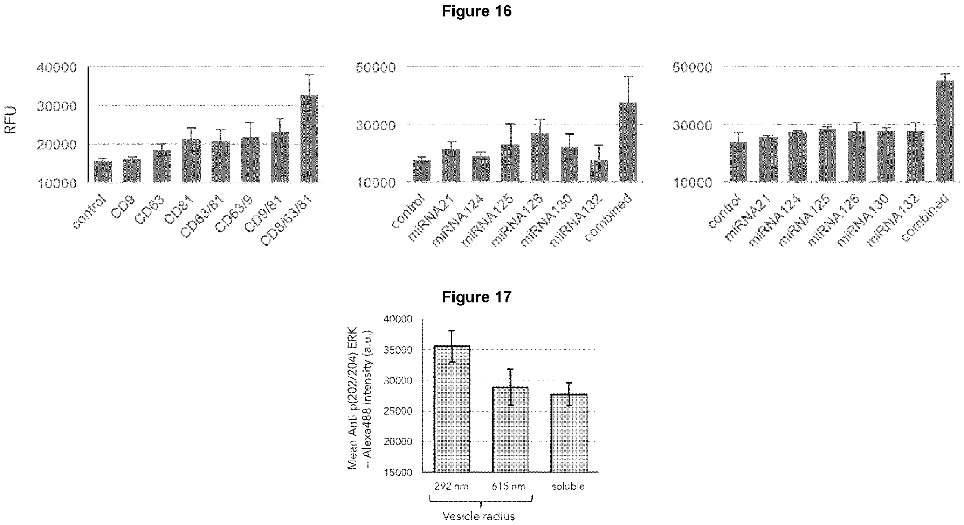

- Figure 16: shows effect of synthetic extracellular vesicles on proliferation of A431 carcinoma cells. Fluorescence intensity analysis of Hoechst 33342 stained A431 cells after treatment with synthetic extracellular vesicles of different composition for 48 hours. Results are shown as mean ± SD, n=3 technical replicates.
- Figure 17: shows quantification of ERK phosphorylation at amino acids 202/204 in MC-3T3 cells after incubation for 24 hours with RANK presenting synthetic extracellular vesicles of different radii (292 nm, 615 nm) or soluble RANK alone. Bars represent mean values ± SD, n=3 technical replicates.

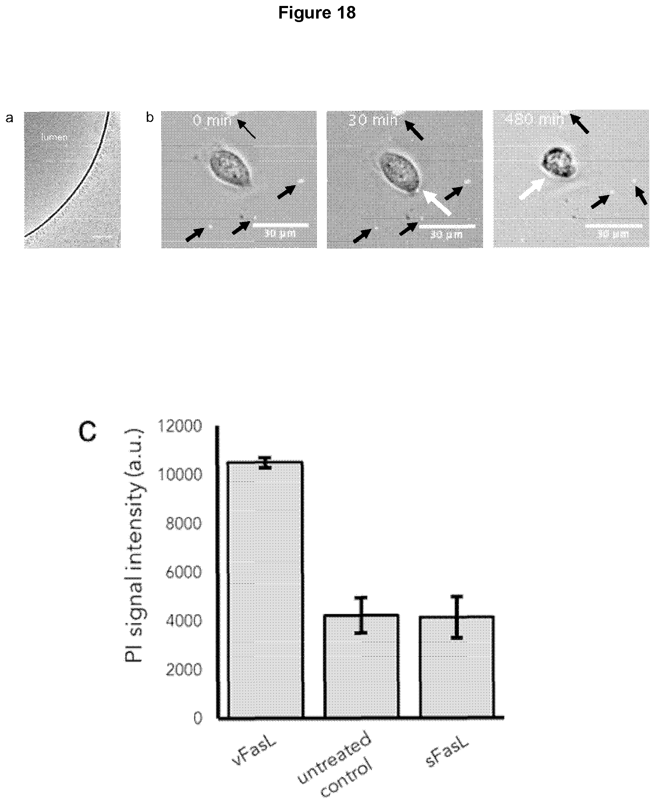

- Figure 18: shows bottom-up assembly of Fas Ligand (FasL) comprising synthetic extracellular vesicles. A) cryo-electron microscospy image of synthetic extracellular vesicles conjugated with recombinant extracellular domain of FasL. B) Live cell fluorescence time laps imaging of HaCaT cells incubated with FasL- synthetic extracellular vesicles (black arrow arrows). Upon contact formation with the synthetic extracellular vesicles (white arrow at 30 min), cells undergo progressive cells death, form blebs and stain positive for propidium iodide (white arrow at 480 min). C) Plate reader quantification of propidium iodide signals of Jurkat T-cells incubated with FasL- synthetic extracellular vesicles (vFasL), or with soluble FasL (sFasL), or left untreated as control. Bars represent mean values ± SD, *n*=3 biological replicates.

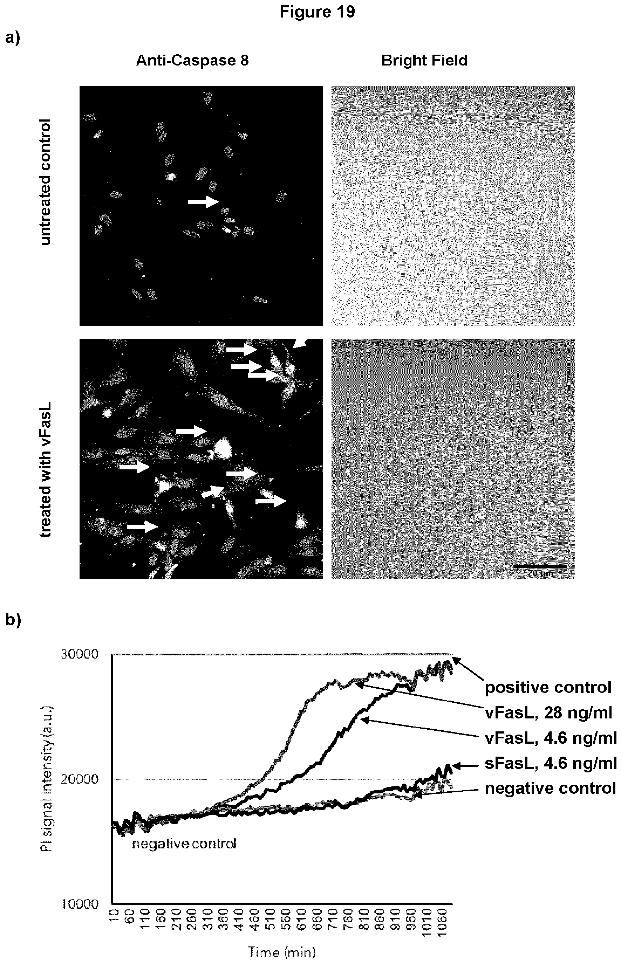

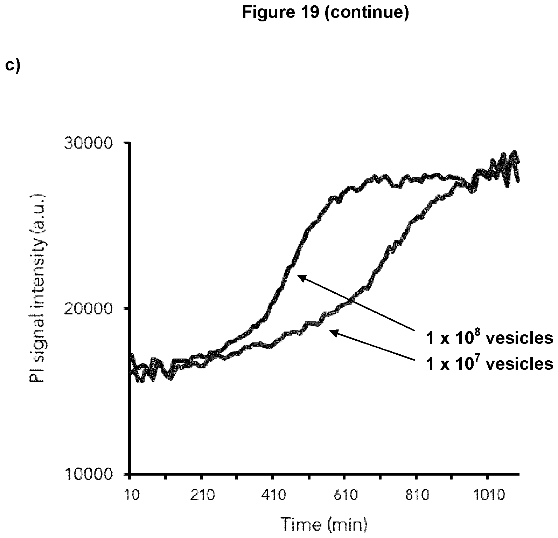

- Figure 19: shows caspase-8 activation in human dermal fibroblasts (BJ cells) incubated with Fas Ligand (FasL) comprising synthetic extracellular vesicles (vFasL). a) Analysis by fluorescence microscopy: left column shows staining for activated caspase 8, i.e. cleaved at Asp391 (white arrows), and DAPI stained nuclei. Right column shows corresponding bright field images. b) Time-analysis of staining intensity of propidium iodide of BJ cells incubated with FasL comprising synthetic extracellular vesicles (vFasL) at concentration 28 ng/ml and 4.6 ng/ml. c) Time-analysis of staining intensity of propidium iodide of BJ cells incubated with 10⁷ vesicles and 10⁸ vesicles of FasL comprising synthetic extracellular vesicles (vFasL).

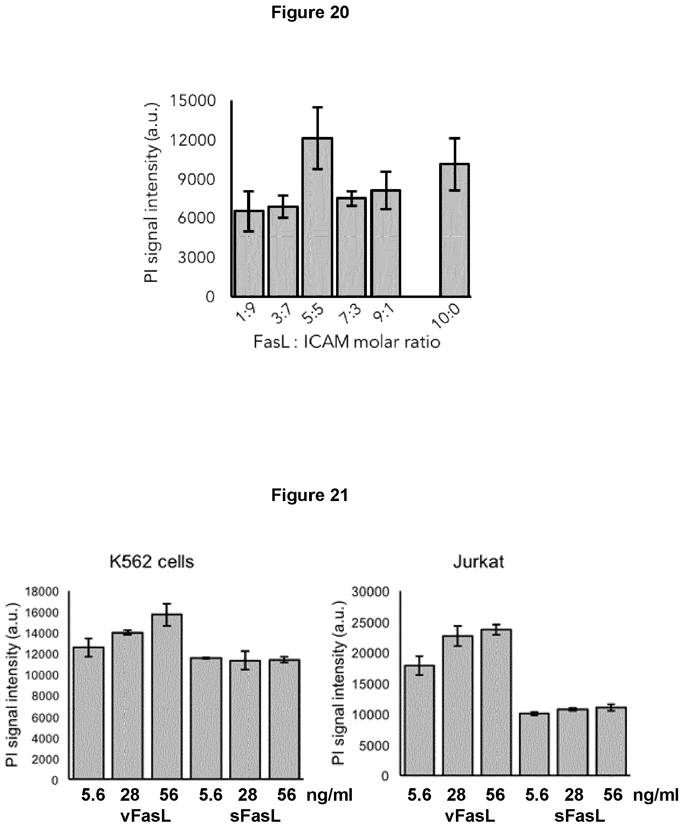

- Figure 20: Optimization of FasL-ICAM ratio present on surface of synthetic extracellular vesicles. Jurkat T-cells were incubated with synthetic extracellular vesicles having the different indicated FasL-ICAM ratios for 24 hours and cell death was quantified by propidium iodide (PI) staining. Bars represent mean values ± SD, *n*=3 biological replicates.
- Figure 21: shows assessment of FasL cytotoxicity on Jurkat and K562 cells at different concentrations of Fas Ligand (FasL) comprising synthetic extracellular vesicles (vFasL) or soluble FasL (sFasL). Cells were incubated with the different preparations and propidium iodide staining intensity was quantified after 24 hours.

### Examples

### Materials

18:1 DOPG 1,2-dioleoyl-sn-glycero-3-phospho-(1'-rac-glycerol), 18:1 DOPC 1,2-dioleoyl-sn-glycero-3-phosphocholesteroline, 18:1 DOPE 1,2-dioleoyl-sn-glycero-3-phosphoethanolamine, LissRhod PE 1,2-dioleoyl-sn-glycero-3-phosphoethanolamine-N-(lissamine rhodamine B sulfonyl), 18:1 DGS-NTA(Ni) 1,2-dioleoyl-sn-glycero-3-[(N-(5-amino-1-carboxypentyl)iminodiacetic acid)succinyl] (nickel salt), 18:1 1,2-dioleoyl-sn-glycero-3-phospho-(1'-myo-inositol) (ammonium salt), 18:1 1,2-di-(9Z-octadecenoyl)-sn-glycero-3-phospho-L-serine (sodium salt), 18:1 1,2-di-(9Z-octadecenoyl)-sn-glycero-3-phosphate (sodium salt), cholesterol, 18:1 1-2-di-(9Z-octadecenoyl)-sn-glycerol, 18:0 N-stearoyl-D-erythro-sphingosylphosphorylcholine and extrude set with 50 nm pore size polycarbonate filter membranes were purchased from Avanti Polar Lipids, USA. All lipids were stored in chloroform at -20°C and used without further purification. Hoechst 33342, CellTracker Green CMFDA dye, wheat germ agglutinin (WGA)-AlexaFluor conjugates (obtained from Thermo Fisher scientific, Invitrogen), Dulbecco's Modified Eagle Medium (DMEM) high Glucose, heat inactivated as well as exosome depleted fetal bovine serum, recombinant N-terminal His-tagged human CD9 (amino acids 103-203), penicillin-streptomycin (10,000 U/mL), L-Glutamine (200 mM), Alexa Fluor 488 NHS Ester, trypsin-EDTA (0.05 %) with phenol red and phosphate buffered saline were purchased from Thermo Fischer Scientific, Germany. 1H,1H,2H,2H-Perfluoro-1-octanol (PFO) de-emulsifier and human male plasma serum were purchased from Sigma Aldrich, Germany. Bovine albumin fraction V (BSA) was purchased from Carl Roth, Germany. HaCaT cells were obtained from CLS cell line service, Germany. A431, K562, MC 3T3, and BJ cell lines as well as Iscove's Modified Dulbecco's Medium were obtained from ATCC, USA. Atto488 conjugated 1,2-dipalmitoyl-sn-glycero-3-phosphoethanolamine was purchased from Atto-Tec GmbH, Germany. Caspase 8 (Cleaved Asp391) monoclonal antibody (clone S.147.8) was purchased from Thermo Fischer Scientific, Germany. Purified Mouse Anti-ERK1/2 (pT202/pY204) was purchased from BD biosciences (Cat. No. 612358). Recombinant N-terminal His-tagged human CD9 (protein ID P21926 amino acids 112-195) was purchased from Novus Biologicals, Germany. Recombinant N-terminal His-tagged human TSG101 (protein ID Q99816, amino acids 1-145) was purchased from Fitzgerald, USA. Recombinant N-terminal His-tagged human CD81 (protein ID P35762, amino acids 113-201) was purchased from MyBioSource, USA. Recombinant N-terminal His-tagged human CD63 (protein ID P08962, amino acids Ala 103-Val 203) was purchased from Thermo Fischer Scientific, Germany. Recombinant His-tagged RANK (protein ID O35305, amino acids 31-214) was obtained from Abcam, Germany. Recombinant his-tagged FasL amino acids N-Met-His8 (Pro134-Leu281) (protein ID NM_000639.1) was obtained from BioLegend, USA. His tagged ICAM-1 (protein ID P05362) recombinant human protein (Met 1 - Glu 480), was obtained from Thermo Fischer Scientific, Germany. miRIDIAN micro RNA mimics (hsa-miR-21-5p, hsa-miR124-3p, hsa-miR-125b-5p, hsa-miR-126-5p, hsa-miR-130a-3p, hsa-miR-132-3p) were purchased from Horizon Dharmacon, USA. K562 exosomes (HBM-K562) were obtained from Hansa BioMed Lonza, Switzerland. 4-well cell exclusion inserts were purchased from Ibidi, Germany. Pre-wounded full thickness human organotypic skin cultures, respective culture media and histological sample preparation services were purchased from MatTek Cooperation, USA. FC-40 oil was purchased from lolitec, Germany. ELISA kit for quantification of human pro-collagen I alpha was obtained from Abcam, UK.

### Methods

### Exosome isolation from K562 cell cultures

K562 extracellular vesicles were isolated from conditioned cell culture medium by differential centrifugation. For this, K562 cells were cultured in 50 ml of Iscove's modified Dulbecco's Medium for 48 hours in suspension with 10% exosome free serum at 37°C and 5% CO₂ atmosphere. The final cell concentration was 5x10⁵ cells/ ml. After incubation, the cell suspension was centrifuged at 300 g at 4°C for 10 minutes to remove the cells. The supernatant was filtered through a 0.22 µm filter and centrifuged at 125.000 g at 4°C for 75 min with a Beckmann Coulter Optima XE-100 ultracentrifuge in a JA-20 fixed angle rotor (k-factor 770). The pellet was washed with 50 ml ice-cold PBS and centrifuged again under the same conditions. The exosome pellet was resuspended in 1 ml PBS. The total protein concentration of this exosome suspension was assessed by measuring the absorbance at 280 nm with a Nanodrop ND-1000 spectrophotometer.

### Confocal and bright field microscopy

Confocal microscopy was performed with a laser scanning microscope LSM 800 (Carl Zeiss AG). Images were acquired with a 20x (Objective Plan-Apochromat 20x/ 0.8 M27, Carl Zeiss AG) and a 63x immersion oil objective (Plan-Apochromat 63x/ 1.40 Oil DIC, Carl Zeiss AG). Images were analyzed with ImageJ (NIH) and adjustments of image brightness and contrast or background corrections were performed always on the whole image and special care was taken not to obscure or eliminate any information from the original image. For bright field imaging a Leica DMi8 inverted fluorescent microscope equipped with a sCMOS camera and 10x HC PL Fluotar (NA 0.32, PH1) objective was used.

For analysis of extracellular vesicle uptake into HaCaT cells, rhodamine B labeled extracellular vesicles were incubated with HaCaT cells in Nunc LabTek 8-well chambers. Immediately after addition of the extracellular vesicles to the cells, 5 µg/ ml of AlexaFluor (obtained from Thermo Fischer Scientific, Invitrogen) conjugated wheat germ agglutinin (WGA) was added to the medium. Cells were incubated for 24 hours and subsequently imaged by confocal laser scanning microscopy. WGA binds to specific sugar residues on the outer cell membrane and is endocytosed along with them during membrane turn-over and endocytotic processes, staining intracellular endosomal vesicles.
Alexa488 labelled CD9 was produced by incubating NHS functionalized Alexa488 with recombinant CD9 in a twofold molar excess for 2 hours at 37°C in PBS. Subsequently, free NHS was quenched by adding a 10-fold molar excess of glycine.
Staining of HaCaT cells with CellTracker Green was performed by incubating 20 µM of cell Tracker Green CMFDA dye for 60 min. To remove excess dye and non-uptaken synthetic extracellular vesicles, cells were rinsed twice with PBS.

### Cell culture

HaCaT, BJ and A431 cells were cultured in Dulbecco's Modified Eagle Medium supplemented with 4.5 g/l glucose, 1 % L-glutamine, 1 % penicillin/streptomycin and 10 % fetal bovine serum. Cells were routinely cultured at 37 °C and 5 % CO₂ atmosphere and passaged at approx. 80 % confluency using 0.05 % trypsin/EDTA treatment. K562 cells were cultured in suspension in Iscove's modified Dulbecco's Medium supplemented with 10% exosomes free fetal bovine serum. K562 cells were splitted every other day by transferring 3 ml of cell suspension to 10 ml of fresh cell culture medium.

### Assessment of cell proliferation

For proliferation analysis, a previously reported Hoechst 33342 intensity analysis was applied. To this end, HaCaT and A431 cells were seeded at a density of 15.000 cells/ well in a flat-bottom transparent 96-well plate in 200 µl culture medium. Cells were seeded together with corresponding extracellular vesicles and incubated for 48 hours. Subsequently, cells were washed twice with 100 µl PBS and incubated for 10 min with icecold culture medium supplemented with 10 µM Hoechst 33342. After removal of the culture medium and 2x washing with PBS, Hoechst 33342 intensity was measured at four individual positions in each well using an Infinite M200 TECAN plate reader controlled by TECAN iControl software with an in-built gain optimization and excitation/emission setting adjusted to 380/460 nm. Measurements were performed in triplicates.

### Cell exclusion assay

For in vitro 2D wound healing assays, 4-well silicone cell exclusion cell culture inserts with a gap width of 500 µm were used in 12-well plastic plates. Cells were seeded at a cell density of 40.000 cells/ well and allowed to adhere overnight in 110 µl culture medium (2 ml of culture medium were added to the well outside of the inserts). Extracellular vesicles were incubated (at final lipid concentration of 10 µM) with the cell monolayer for 24 hours. Subsequently, the inserts were carefully removed using sterile tweezers and the wound was allowed to close for 16 hours. For quantification, culture medium was removed and cell layers were fixed with ice-cold 4% paraformaldehyde for a minimum of 20 min. The wound sides were then imaged by phase contract microscopy and the cell free area was quantified manually with ImageJ software.

### Organotypic dermal cultures

For analysis of human organotypic full thickness skin models, pre-wounded human epidermal keratinocytes (obtained from neonatal-foreskin normal tissue of a single donor) and fibroblasts 3D cultures were obtained from a commercial distributer (MatTek corporation). Skins were cultured at an air-liquid interface following manufacturer's suggestions. For wound closure analysis, tissues were allowed to equilibrate for 16 hours after arrival at 37°C in a 5% CO₂ atmosphere. Subsequently, 2 µl of the extracellular vesicle solution (or respective buffer controls) were pipetted onto the wound side and the wound was allowed to heal for 48 hours at 37°C in a 5% CO₂ atmosphere. Tissues were then fixed with 10% formalin solution overnight at 4°C. Wound size was quantified from histological H/E slices. For each wound, six individual slices and three individual wounds were analyzed.

### Protein analysis by gel-electrophoresis

For gel-electrophoretic analysis of protein content of K562 exosomes and synthetic extracellular vesicles, a NuPAGE bold Bis-Tris 4-12% gradient gel was used with MES running buffer. Electrophoresis was performed at 200V for 35 min under denaturating conditions with a total of 3 µg (for natural exosomes) or 500 ng (for synthetic extracellular vesicles) of protein loaded onto each lane. Protein staining was performed with Coomassie R250. Line intensity profiles of the respective lanes were measured by ImageJ software.

### Quantitative assessment of collagen deposition

For quantification of in vitro collagen deposition, BJ dermal fibroblast were seeded in 96-well flat bottom transparent cell culture plates at a density of 20.000 cells/ well. 24 hours after seeding, cells were washed twice with PBS and 200 µl of fresh cell culture medium was added together with synthetic extracellular vesicles (to a final lipids concentration of 10 µM) to the cells. Cells were incubated for 24 hours with synthetic extracellular vesicles. Subsequently human pro-collagen I alpha in the medium was quantified by enzyme-linked immunosorbent assay (Abcam ELISA Kit) following the manufacturer's instructions.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the scope of the invention as described in the following claims.

### Example 1. Production of the biomimetic fully synthetic extracellular vesicles

Synthetic extracellular vesicles were produced by shear stress emulsification (**Figure 1**). At first, a solution of small unilamellar vesicles was prepared as follows: lipids dissolved in chloroform stock solutions were mixed at the desired lipid ratio in glass vials and subsequently dried under a gentle nitrogen stream. The obtained lipid film was rehydrated to a final lipid concentration of 6 mM in PBS for 15 min and afterwards shaken for 5 min at 1000 rpm. This solution of small unilamellar vesicles was extruded at least 9 time through a 50 nm pore size filter.
The so obtained solution, representing the water phase of step a), was then diluted to a final concentration of 3 mM with PBS, or eventually PBS containing the desired miRIDIAN miRNA mimic components at concentration 40 - 145 nM.
In this example, polymer shell-stabilized extracellular vesicles were produced from small unilamellar vesicles containing 41 mol% cholesterol, 16 mol% SM, 15 mol% DOPC, 11 mol% DOPS, 6 mol% DOPE, 5 mol% DOPG, 2 mol%PA, 1 mol% DAG, 1 mol% PI, 1 mol% LissRhod PE, 1 mol% DGS-NTA(Ni²⁺) in PBS containing miRIDIAN RNA (40 - 145 nM). This lipid composition resembles that of natural extracellular vesicles. However, the technology allows for the integration of an almost unrestricted number of possible lipid types into synthetic exosome membrane.
This water phase was then combined with an oil phase at a ratio 1:2. The oil phase consisted of FC-40 oil containing the fluorosurfactant triblock PEG2500-PFPE600-PEG2500 at a final concentration of 1.25 mM.
The combined water phase and oil phase was then emulsified using an Ultra Turrax IKA T10 basic emulsifier for, exemplarily, 60 sec at approx. 26,300 rpm. The resulting polymer shell stabilized synthetic extracellular vesicles were incubated for at least 2 hours at 4°C in the dark.

Release of the polymer shell stabilized extracellular vesicles into an aqueous release buffer was performed by removing excess oil phase and adding the release buffer (PBS) and 1H,1H,2H,2H-perfluoro-1-octanol (PFO) to the mixture in a 1 : 1 : 1 ratio of aqueous release buffer: PFO.aqueous intraluminal buffer. After 30 min of equilibration, the layer containing the extracellular vesicles was transferred into a 2 ml microtube and PBS was added to a final volume of 2 ml.
This solution was centrifuged at >10.000 g for 15 min.
The supernatant was discarded and the extracellular vesicle pellet was resuspended in PBS. The extracellular vesicles were also released into PBS containing 0.1% BSA to block unspecific protein-lipid interactions

In order to conjugate the released extracellular vesicles with CD peptides, the total amount of NTA-Ni²⁺ functionalized lipids was calculated according to the lipid ratio. Thus, the His-tagged CD - peptides (CD9, TSG101, or CD63, or CD81) were added to the extracellular vesicle solution in a 1:2 excess and allowed to conjugate for 1 hour at 37°C protected from light. After this phase, 500 nM Hoechst33342 was eventually added to the extracellular vesicles to visualize nucleic acids by confocal microscopy analysis.
The extracellular vesicle solution was subsequently centrifuged at >10.000 rpm for 15 min. The supernatant containing unbound peptides was disposed and the extracellular vesicle pellet resuspended in PBS.

In order to compare the lipid ratio of the formed extracellular vesicles, and that of the small unilamellar vesicles, the total lipid concentration of the extracellular vesicles was determined by quantifying the fluorescence from the integrated rhodamine B or Atto488 conjugated lipids, which was referenced to a small unilamellar vesicle standard dilution curve.
The results of quantitative electrospray-ionization tandem mass spectrometry revealed that fully synthetic extracellular vesicles had the lipid composition cholesterol:SM:DOPC:DOPS:DOPE:DOPG:PA:DAG:PI 43:16:15:11:6:5:2:1:1, resembling not only the lipid composition of natural extracellular vesicles, but also that of the original small unilamellar vesicles (**Figure 2**), proving that no lipid ratio change occurs during the emulsification and release procedures. Therefore, the lipid composition of the extracellular vesicles can be easily fine-tuned by changing the lipid formulation of the original small unilamellar vesicles.

### Example 2. Comparison with exosomes obtained by state of the art methods.

The fully synthetic extracellular vesicles produced as described in Example 1 were compared to natural extracellular vesicles in term of purity, protein composition, dimension, and variability between different batches.

Interestingly, the fully synthetic extracellular vesicles produced according to the invention contained considerably less contaminating aggregates and non-vesicular particles compared to exosomes isolated by differential centrifugation from conditioned K562 erythroleukemia cell media or commercially available exosomes from the same cell line (see **Figure 3**).

Moreover, when assessing the protein content of the respective vesicles by denaturating polyacrylamide gel-electrophoresis, it was found that exosomes isolated from conditioned K562 media and K562 commercial exosomes, differed greatly in their protein content, underscoring the degree of variation between different vesicle preparation methods (**Figure 4**). Furthermore, when considering the variation between different exosome batches prepared with the same method (column 1 *vs* 2 and 3 *vs* 4 of **Figure 4**), a substantial degree of variation in the protein composition could be observed. In contrast, fully synthetic extracellular vesicles equipped with purified recombinant human forms of exosome's surface markers CD9 and TSG101, attached by nitrilotriacetic acid (NTA)-poly histidine tag chemistry, appeared with a clearly defined band pattern and showed almost identical characteristics between separate preparations (column 5 *vs* 6 of **Figure 4**). Thus, polymer shell stabilized extracellular vesicles can be considered as a more defined and robust platform for extracellular vesicles research that outperforms extracellular vesicles isolates from natural sources in terms of purity and reproducibility.

Additionally the biophysical similarity of fully synthetic extracellular vesicles to natural extracellular vesicles was evaluated by dynamic light scattering. The results showed that the size of polymer shell stabilized extracellular vesicles and therefore the hydro-dynamic radius of fully synthetic extracellular vesicles can be fine-tuned by adjusting the shear stress used during emulsification, producing fully synthetic extracellular vesicles radii between 292 nm ± 12 nm, (CV = 4.1 %; n = 3) by emulsification for 30 sec at 30.000 rpm, and radii between 627 nm ± 15 nm, (CV = 2.4 %; n = 3) by emulsification for 30 sec at 14.000 rpm. The zeta potential of synthetic extracellular vesicles containing the above-mentioned lipid formulation was -12,3 mV (± 0,7 mV, n = 3). Thus, size and zeta potential of the fully synthetic extracellular vesicles are comparable to those of natural extracellular vesicles reported in literature (Vogel, R. et al. High-Resolution Single Particle Zeta Potential Characterization of Biological Nanoparticles using Tunable Resistive Pulse Sensing, Scientific reports 7, 2017).

Moreover, the size values of the synthetic extracellular vesicles were very similar to those of commercial K562 exosomes (468 nm ± 199 nm, CV=42.5 %, and -11,8 mV ± 0,9 mV, n = 3) and of exosomes isolated from conditioned K562 cell culture medium (240 nm ± 32 nm, CV = 13.3 %, and -11,3 mV ± 0,5 mV, n = 3).

These results confirm that fully synthetic extracellular vesicles can be assembled from individually adjustable synthetic lipid precursors to precisely match the lipid composition and therefore biophysical characteristic (membrane charge, dimensions) of natural extracellular vesicles. Importantly, the fully synthetic extracellular vesicles prepared according to the inventive method showed the technical advantage to be much more homogenous in size in comparison with the exosome obtained according to prior art methods.

### Example 3. Building of fully synthetic extracellular vesicles resembling the protein and nucleic acid composition of natural exosomes.

Although lipids play an important role in extracellular vesicle communication, for therapeutic applications the main physiological functions of extracellular vesicles is commonly attributed to their micro RNA (miRNA) cargo and to the peripheral membrane proteins and receptor ligands on their surface.
The inventors have here produced synthetic exosomes miming fibrocyte-derived exosomes, in order to show the potential of the inventive method to produce synthetic exosomes for therapeutic application.

Natural fibrocyte-derived exosomes contain miRNAs including miR-21, miR-124, miR-125, miR-126, miR-130 and miR-132 and protein components including CD9, CD63 and CD81, and have been shown to promote wound-healing. Therefore, synthetic exosomes where prepared using synthetic miRIDIAN mimics of the miRNA described in the natural exosomes at a concentration typically found in natural exosomes (750 pg/ 1012 vesicles), by mixing the miRNA solution with the initial water phase containing the lipids (**Figure 1**). Following the release of the synthetic exosomes from the surrounding polymer shell into an aqueous solution, synthetic exosomes were decorated with recombinant extracellular domains of human tetraspanins CD9, CD63 and CD81 via poly-Histidine-tag chemistry at a 1:100 protein to lipid ratio, at a similar protein to lipid ratio as described for natural exosomes.
Confocal microscopy analysis of the prepared synthetic exosomes showed luminal distribution of the miRNAs and peripheral distribution of Alexa-488 labeled CD9, overlapping with the lipid fluorescence of Liss Rhodamin PE integrated into the lipid bilayer (**Figure 5**), thus demonstrating the correct bottom-up assembly of miRNA loaded and protein decorated extracellular vesicles.

### Example 4. Interaction between synthetic extracellular vesicles and target cells.

The interaction of fully synthetic extracellular vesicles synthetized as explained in Example 3 with target cells was analysed by incubating the keratinocytes HaCaT cells with said vesicles for 24 hour treatment. These experiments showed that fluorescently labeled synthetic extracellular vesicles are internalized by HaCaT cells via an endosomal pathway (**Figure 6**).

Importantly, these results suggest that the content of the fully synthetic extracellular vesicles is delivered into the target cells, so that they could be used for intracellular cargo delivery and for therapy of different disorders.

### Example 5. Effect of synthetic extracellular vesicles on wound healing.

It has been shown that human fibrocyte-derived exosomes can have pro-proliferative effect, accelerates the collective migratory behavior of dermal keratinocytes and enhances collagen deposition of dermal fibroblasts, ultimately promoting wound closure in a diabetic mice model. Therefore, the wound healing effect of the synthetic extracellular vesicles synthetized as in **Example 3** was tested studying their effect on cell proliferation, migration and collagen deposition, which is crucial for wound closure and healing.

The pro-proliferative effect on spontaneously immortalized keratinocytes Hacat cells, was assessed by quantifying keratinocyte number via nuclear staining after 48h of incubation with the fully synthetic extracellular vesicles.
In order to decipher the contribution of the individual biomolecular components of the fully synthetic extracellular vesicles, fully synthetic extracellular vesicles lacking miRNAs, but decorated with the single tetraspanins or combinations of them were produced and analysed. Interestingly, co-presentation of CD9 and CD63 or CD9 and CD81 leaded to a synergistic effect on proliferation (**Figure 7a**). However, the pro-proliferative effect was most pronounced when CD9, CD63 and CD81 where all co-presented, inducing a more than 2.7-fold increase compared to cultures treated with naive vesicles. The addition of the soluble tetraspanin protein variants alone to the HaCaT cells did not show a comparable effect. Therefore, these results reveal that the sole presentation of the CD9, CD63 and CD81 extracellular domain on synthetically assembled vesicles, has a pro-proliferative effect on keratinocytes, which can be considered as an essential requirement to promote wound healing.
The contribution of the single miRNAs was further evaluated by producing CD9, CD63 and CD81 biofunctionalized synthetic extracellular vesicles loaded with the individual human miRNA mimics (**Figure 7b**). Interestingly, the results revealed that each individual miRNA enhanced to some extend the pro-proliferative effect. Slightly higher enhancement (1.7-fold) was observed in case of miR-125 or miR-126 loaded fully synthetic extracellular vesicles. However, the highest pro-proliferative effect (2.5-fold) was achieved when fully synthetic extracellular vesicles were loaded with a combination of all six miRNAs compared to non-loaded CD9, CD63, CD81-biofunctionalized fully synthetic extracellular vesicles. Generally, miRNAs modulate a broad spectrum of cell activities by translational regulation of intracellular signaling pathways.

Thus, these results show that fully synthetic extracellular vesicles can act as appropriate carriers to convey miRNA-based signaling information and thereby acting on post-transcriptional gene regulation by which they mirror a central mechanism of extracellular vesicle signaling.

The effect of extracellular vesicles on epithelial cell migration was assessed performing in vitro cell exclusion wound healing assays of collectively migrating keratinocyte monolayers treated for 24h with the fully synthetic extracellular vesicles (**Figure 8**). By quantifying the cell free area 16 hours after removal of the exclusion-inserts, the fully synthetic extracellular vesicles resulted able to promote keratinocyte migration into the cell free area and therefore closure of the artificial wound side (**Figure 9**). Similarly to the results obtained for the pro-proliferative effect, co-presentation of all three tetraspanins on the fully synthetic extracellular vesicles showed a more pronounced enhancement of collective cell migration compared to single presentation of the proteins. When analyzing the effects of the individual miRNAs, miRNA132 resulted to especially improve the migratory behavior, and co-encapsulation of all six miRNAs could further increase collective cell migration compared to treatment with fully synthetic extracellular vesicles functionalized with CD9, CD63, CD81 only (**Figure 10**).

Building on these observation, the ratios of the different miRNAs and tetraspanins could be varied, in order to assess the influence of individual signaling pathways.

The effect of fully synthetic extracellular vesicles on pro-collagen-Iα deposition of BJ dermal fibroblasts was assessed by enzyme-linked immunosorbent assay (ELISA) after a 24 hours treatment with the synthetic extracellular vesicles (**Figure 11**). Although individual miRNA constituents showed no significant effect, a higher collagen deposition was obtained by treatment with fully synthetic extracellular vesicles containing all six miRNAs and tetraspanins.

Taken all these finding together, the *in vitro* assembled vesicles, like their natural equivalents, comprise the ability to boost three of the very fundamental processes critical for wound healing: proliferation, migration and collagen deposition.

### Example 6. Effect of biomimetic fully synthetic extracellular vesicles on epithelial regeneration.

In order to assess the ability of fully synthetic extracellular vesicles to promote epithelial regeneration of wounded skin, wounded organotypic full-thickness human skin models (**Figure 12**) were treated by applying synthetic extracellular vesicles for 48 hours on the wound site (see Methods). Closure of the 3 mm epidermal wound was assessed by quantification of the epithelial wound bed size from hematoxylin-eosin (H/E) stained histological samples (**Figure 13**). The treatment with synthetic extracellular vesicles substantially augmented the healing processes compared to buffer treated controls and application of the single soluble constituents was not as effective as the fully synthetic extracellular vesicles (**Figure 14**).

### Example 7. Migration promotion of fully synthetic extracellular vesicles.

It is known that proliferation, migration and collagen deposition play a critical role in development and progression of epithelial carcinomas. Therefore, the effect of fully synthetic extracellular vesicles miming the fibrocyte derived vesicle was additionally tested on A431 human vulvar squamous carcinoma cells.

The results revealed that A431 carcinoma cells responded to fully synthetic extracellular vesicle treatment by accelerated *in vitro* collective migration, a central requirement for tumor invasion and metastasis, but displayed an altered sensitivity for the respective fully synthetic extracellular vesicle surface proteins (**Figure 15**). Moreover, the fully synthetic extracellular vesicles increased A431 cells proliferation of 1.8-fold (**Figure 16**).

### Example 8. Extracellular vesicles for age-related disorders.

The inventive method was applied to produce synthetic extracellular vesicles containing a nicotinamide phosphoribosyltransferase intracellular protein, as previous studies showed that these vesicles play a role in treatment of age-related disorders, and increase of lifespan.

Therefore, the inventors have here produced synthetic extracellular vesicles according to the inventive method and specifically comprising the functional protein nicotinamide phosphoribosyltransferase, and cytosolic proteins such as Apoptosis-Linked Gene 2-Interacting Protein X (ALIX), tumour susceptibility gene 101 protein (TSG101). These synthetic extracellular vesicles did not comprise transferrin and albumin.

### Example 9. Synthesis of immunoregulatory extracellular vesicles.

The inventors aimed to synthetize synthetic extracellular vesicles with immunoregulatory properties that could be used to treat immune disorders, autoimmune disorders, inflammatory disorders, such as rheumatoid arthritis, and cancer, e.g. by cancer immunotherapy.

This type of synthetic extracellular vesicles can comprise transmembrane proteins such as MHCII, CD80, CD86, CD11c, MHC I, integrin α and β-chains, ICAM-1, and CD71; functional proteins such as cytokines, interleukins, IL4, growth factors, milk fat globule-EGF factor 8 protein (MFGE8), Fas, Fas Ligand (FasL), RANK, RANK Ligand (RANKL), indolamin-2,3-dioxygenase, cytotoxic T-lymphocyte-associated protein 4 - immunoglobulin fusion protein (CTLA4-Ig), tumor necrosis factor-related apoptosis-inducing ligand (Apo2L, TRAIL).

Fas (also named CD95, Apo-1) is a membrane receptor from the TNF family expressed on surface of almost all cells in the human body. Upon activation by Fas ligand (FasL), Fas induces apoptosis by activation of caspase signalling. Fas mediated apoptosis plays a major role in immunobiology, and especially by death induction of infected and cancerous cells mediated by cytotoxic T-cells and by natural killer cells (NK-cells). Moreover, Fas induced apoptosis is crucial for regulation of T-cell physiology in inflammatory diseases. Major producers of FasL are T- cells and NK-cells which release FasL primarily from intracellular reservoirs in a form bound to extracellular vesicles resembling exosomes.
Synthetic extracellular vesicles presenting FasL could therefore be useful not only to investigate further Fas-signalling, but also to provide new therapy option in immune disorders, inflammatory disorders, neurodegenerative disorders, or cancer.

In order to produce synthetic extracellular vesicles presenting FasL, synthetic extracellular vesicles were produced by the inventive method based on mechanical emulsification at 14,000 rpm for 30 sec with the following composition: 20 mol% DOPG, 78 mol% DOPC, 1 mol% DGS-NTA-(Ni²⁺) and 1 mol% LissRhod PE. Obtained synthetic extracellular vesicles had a mean size of 606 nm. Recombinant his-tagged FasL amino acids N-Met-His8 (BioLegend, USA) was coupled on the surface of these synthetic extracellular vesicles by applying bio-orthogonal surface chemistry NTA - poly-histidine tag coupling.
Cryo-electron microscopy analysis demonstrated correct coupling of FasL on vesicle surface (**Figure 18a**). The pro-apoptotic activity of FasL-synthetic extracellular vesicles was then tested on human keratinocytes by following cell positivity for propidium iodide by live-cell time lapse imaging (**Figure 18b**). Upon contact formation with the FasL presenting synthetic vesicles, target cells stained progressively positive for propidium iodide (PI), demonstrating the pro-apoptotic activity of FasL- synthetic vesicles on target cells.

FasL- synthetic vesicles were then tested for their pro-apoptotic activity on T-cells, which is important for therapy of inflammatory and autoimmune diseases. To this aim, Jurkat T-cells were incubated for 24 hours with FasL- synthetic vesicles and apoptotis was quantified by measuring propidium iodide (PI) staining intensity on a plate reader. Results showed that vesicle bound recombinant FasL is able to induce apoptosis in the T-cells, but not soluble FasL (**Figure 18c**).
Thereafter, FasL-synthetic vesicles were tested for their ability to activate caspase-8, which has been shown also for natural FasL-vesicles. To this aim, human dermal fibroblast BJ cells were incubated with FasL- synthetic vesicles for 2 hours and then stained with antibodies recognizing activated caspase 8, i.e. cleaved at Asp391 (**Figure 19**). The results confirmed activation of caspase 8 in the BJ cells treated with FasL- synthetic vesicles. Activation of caspase-8 was directly proportional to the vesicle concentration, and to the concentration of FasL on the vesicles after a given time

We further evaluated the pro-apoptotic concentration range of FasL presenting synthetic vesicles on Jurkat T-cells and on K562 granulocytic cells, which are frequently used to assess FasL mediated apoptosis on NK cells. We again found that the addition of soluble FasL (sFasL) did not display any cytotoxic effect at all tested concentrations (**Figure 21**). However, FasL bound on synthetic vesicle surface could induce substantial cell death in both cell line, at the lowest tested concentation of 5,6 ng/ml. Highest pro-apoptotic efficiency could be observed for the highest tested concentration of 56 ng/ml FasL presenting synthetic vesicle.

Thereafter, the inventive method was used to assemble pro-apoptotic synthetic vesicles exposing not only FasL but also the intercellular adhesion molecule ICAM-1, which is presented on natural occurring extracellular vesicles with immunological activity. Synthetic extracellular vesicles presenting FasL and ICAM-1, were produced as described above by mechanical emulsification at 14,000 rpm for 30 sec. Obtained synthetic extracellular vesicles had a mean size of 606 nm. The pro-apoptotic potential of these vesicles was tested on Jurkat T-cells using different FasL to ICAM-1 ratios, and measuring cell death by propidium iodide (PI) staining It was found that the killing efficiency of the synthetic vesicles depends on the FasL to ICAM-1 ratio (**Figure 20**). Such quantitative optimization of protein composition in extracellular vesicles is not feasible using natural derived extracellular vesicles. It was found that a FasL to ICAM-1 ratio of 5:5 is optimal and can induce cells apoptosis at a similar level as vesicles presenting only FasL (10:0 = double as much FasL on the vesicle surface). In other words, we could increase pro-apoptotic signalling by a factor of 2 through this initial optimization. Interestingly, ICAM - 1 is not cytotoxic by itself but when present as adhesion protein on cell derived vesicles. This demonstrates that adhesion proteins like ICAM - 1 can further increase the cytotoxicity of FasL exposing vesicles, probably because they mediate and facilitate the adhesion of the vesicles to the target cells.

### Example 10. Synthetic extracellular vesicles resembling mesenchymal stem-cell derived extracellular vesicles.

The inventive method was applied to produce synthetic extracellular vesicles resembling those derived from mesenchymal stem cells. Natural extracellular vesicles derived from mesenchymal stem cells have been shown to have a wide range of potential therapeutic effects, such as alleviation of severe graft versus host disease, osteoarthritis and promotion of cartilage extracellular matrix homeostasis. The synthetic extracellular vesicles resembling those of mesenchymal stem cell origin were prepared with transmembrane proteins such as CD29, CD44, CD90, CD73, Sca-1, tetraspanin proteins CD9, CD63, and CD81, functional proteins such as Wnta and Wntb, nucleic acid molecules such as miR-140-5p, miR-92a-3p-e, nucleic acid molecules such as miRNAs miR-33b, miR-451, miR-575, miR-630, miR-638, miR-1202, miR-1207-5p, miR-1225-5p, miR-1268, miR-K12-3.

### Example 11. Synthetic extracellular vesicles for treatment of stroke, angiogenesis, and other cardiovascular disorders.

Emerging evidence suggests that stem cell derived exosomes and their microRNA cargo mediate cell therapy derived neurorestorative effects in patients after stroke. In particular, these exosomes can play a role in angiogenesis, neurogenesis, vascular remodeling, white matter remodeling, and also modulate inflammatory and immune responses at the local and systemic level.

In order to reproduce exosomes with such properties, the inventive method was applied to produce synthetic extracellular vesicle specifically comprising: transmembrane proteins such asCD29, CD44, CD90, CD73, CD44, Sca-1,tetraspanin proteins CD9, CD63, and CD81; nucleic acid molecules such as miR-17, miR-18a, miR-19a, miR-19b-1, miR-20a, miR-92a, let-7a, miR-21, miR124, miR126, miR-133b, miR-191, miR-222, miR-494, miR-6087, miR-30d-5p ; miR-33b, miR-451, miR-575, miR-630, miR-638, miR-1202, miR-1207-5p, miR-1225-5p, miR-1268, miR-K12-3.

### Example 12. Synthetic extracellular vesicles for treatment of osteoporosis and other bone diseases.

Osteoclast to osteoblast signalling is crucial for bone homeostasis in order to assure correct bone formation and resorption. Receptor activator of nuclear factor-κB (RANK) is pivotal for this interaction. It has been shown, that maturing osteoclasts secrete vesicles presenting RANK on their surface, which then binds to RANKL on osteoblast surface, thus boosting mineralization of osteoblasts by activation of "reverse signalling". After RANK ligand (RANKL) - RANK binding, downstream MAP-kinase signalling is activated. The RANK-exposing vesicles have been shown to be crucial for physiological signalling and represent potential therapeutic targets to treat bone diseases such as osteoporosis.

Therefore, synthetic extracellular vesicles exposing RANK on their surface were produced applying the inventive method based on emulsification using a mechanic emulsifier, and tested for their activating effect on pre-osteoblast cells.

Synthetic extracellular vesicles were produced the following lipid composition: 20 mol% DOPG, 78 mol% DOPC, 1 mol% DGS-NTA(Ni²⁺) and 1 mol% LissRhod PE. Recombinant His-tagged RANK (amino acids 31-214) (protein ID O35305) was coupled on the surface of these synthetic extracellular vesicles by applying bio-orthogonal surface chemistry NTA - poly-histidine tag coupling, at a protein lipid (LissRhodPE) ratio of 1:100. Two samples of such synthetic extracellular vesicles were produced having diameters of 292 nm (emulsification at 30,000 rpm, 30 sec) and 615 nm (emulsification at 14,000 rpm, 30 sec), respectively, in order to optimize the vesicle dimension for maximal functionality. Synthetic extracellular vesicles were incubated with MC 3T3 cells, a model of pre-osteoblast cells, for 24 hours in 96 well plates at concentration 10µM (vesicle lipid moles/ medium volume). Cell samples were subsequently stained for p(202/204) ERK with an anti-p(202/204) ERK1/2 -Alexa488 conjugated antibody and staining intensity was quantified using an Infinite M200 TECAN plate reader controlled by TECAN iControl software with an in-built gain optimization and excitation/emission setting adjusted to 488/512 nm. Here, phosphorylation of ERK at amino acids 202/204 was quantified as a measure of vesicular RANK potency in specific pre-osteoblast cell activation. As a control, soluble RANK was added to control cells at the same concentration as used to produce vesicular RANK (soluble or vesicle RANK / medium volume = 100 ng/ml).
The results revealed that RANK exposed on synthetic extracellular vesicles can induce RANK signalling more strongly compared to soluble RANK (**Figure 17**). Moreover, we found that smaller RANK vesicles are stronger inducers compared to large vesicles. These data demonstrate that RANK presenting synthetic extracellular vesicles could be used for bone remodelling therapy in bone diseases such as osteoporosis. Moreover, the inventive RANK presenting synthetic extracellular vesicles could be used to further investigate the physiology of bone formation and remodeling.

**Table 3. Suitable miRNA molecules**

| MIRNA ID | ENTREZ ID | MIRNA ID | ENTREZ ID | MIRNA ID | ENTREZ ID |
|---|---|---|---|---|---|
| let-7a | 406881 | miR-30c-2* | 407032 | miR-141 | 406933 |
| let-7b | 406884 | miR-30d | 407033 | miR-143 | 406935 |
| let-7c | 406885 | miR-30e | 407034 | miR-145 | 406937 |
| let-7d | 406886 | miR-30e-3p | 407034 | miR-146a | 406938 |
| let-7e | 406887 | miR-31 | 407035 | miR-146b | 574447 |
| let-7f | 406888 | miR-32 | 407036 | miR-147 | 406939 |
| let-7g | 406890 | miR-33a | 407039 | miR-148a | 406940 |
| let-7i | 406891 | miR-33b | 693120 | miR-148b | 442892 |
| miR-1 | 406952 | miR-34a | 407040 | miR-149 | 406941 |
| miR-7-1* | 407043 | miR-34B | 407041 | miR-150 | 406942 |
| miR-10a | 406902 | miR-92a-1 | 407048 | miR-151 | 442893 |
| miR-10b | 406903 | miR-92A2 | 407049 | miR-152 | 406943 |
| miR-15a | 406948 | miR-92b | 693235 | miR-153-1 | 406944 |
| miR-15b | 406949 | miR-93 | 407050 | miR-153-2 | 406945 |
| miR-16 | 51573 | miR-95 | 407052 | miR-154 | 406946 |
| miR-16-1 | 406950 | miR-96 | 407053 | miR-155 | 406947 |
| miR-16-2 | 406951 | miR-98 | 407054 | miR-181a-2 | 406954 |
| miR-17 | 406952 | miR-99a | 407055 | miR-181a-1 | 406995 |
| miR-17-3p | 406952 | miR-99b | 407056 | miR-181B2 | 406956 |
| miR-17-5p | 406952 | miR-17-92a-1 | 407975 | miR-181c | 406957 |
| miR-18a | 406953 | miR-100 | 406892 | miR-181d | 574457 |
| miR-18b | 574033 | miR-101 | 406893 | miR-182 | 406958 |
| miR-19a | 406979 | miR-103A1 | 406895 | miR-183 | 406959 |
| miR-19b | 406980 | miR-103A2 | 406896 | miR-184 | 406960 |
| miR-19b-1* | 406980 | miR-103b-1 | 100302238 | miR-185 | 406961 |
| miR-19B2 | 406981 | miR-103b-2 | 100302282 | miR-186 | 406962 |
| miR-20a | 406982 | miR-105-1 | 406897 | miR-187 | 406963 |
| miR-20b | 574032 | miR-105-2 | 406898 | miR-188 | 406964 |
| miR-21 | 406991 | miR-106a | 406899 | miR-190 | 406965 |
| miR-22 | 407004 | miR-106b | 406900 | miR-190A | 406965 |
| miR-23a | 407010 | miR-107 | 406901 | miR-191 | 406966 |
| miR-23b | 407011 | miR-122 | 406906 | miR-192 | 406967 |
| miR-24 | 407012 | miR-125a | 406910 | miR-193a | 406968 |
| miR-24 | 407013 | miR-125B1 | 406911 | miR-193b | 574455 |
| miR-25 | 407014 | miR-125B2 | 406912 | miR-194 | 406969 |
| miR-26a | 407015 | miR-126 | 406913 | miR-194-2 | 406970 |
| miR-26A1 | 407015 | miR-128-1 | 406915 | miR-195 | 406971 |
| miR-26A2 | 407016 | miR-128-2 | 406916 | miR-196a | 406972 |
| miR-26b | 407017 | miR-130a | 406919 | miR-196A2 | 406973 |
| miR-27a | 407018 | miR-130b | 406920 | miR-196b | 442920 |
| miR-27b | 407019 | miR-132 | 406921 | miR-197 | 406974 |
| miR-28 | 407020 | miR-133a | 128826 | miR-198 | 406975 |
| miR-29a | 407021 | miR-134 | 406924 | miR-199A2 | 406977 |
| miR-29b | 407024 | miR-135a-1 | 406925 | miR-199B | 406978 |
| miR-29b-2 | 407025 | miR-135a-2 | 406926 | miR-200a | 406983 |
| miR-29B1 | 407024 | miR-135b | 442891 | miR-200b | 406984 |
| miR-29c | 407026 | miR-136 | 406927 | miR-200c | 406985 |
| miR-30a | 407029 | miR-137 | 406928 | miR-202 | 574448 |
| miR-30a-3p | 407029 | miR-138 | 406929 | miR-203 | 406986 |
| miR-30a-5p | 407029 | miR-138-2 | 406930 | miR-204 | 406987 |
| miR-30b | 407030 | miR-139 | 406931 | miR-205 | 406988 |
| miR-30C1 | 407031 | miR-140 | 406932 | miR-2052 | 100302260 |

| MIRNA ID | ENTREZ ID | MIRNA ID | ENTREZ ID | MIRNA ID | ENTREZ ID |
|---|---|---|---|---|---|
| miR-206 | 406989 | miR-373 | 442918 | miR-500 | 574502 |
| miR-210 | 406992 | miR-374a | 442919 | miR-500A | 574502 |
| miR-214 | 406996 | miR-374b | 100126317 | miR-501 | 574503 |
| miR-215 | 406997 | miR-375 | 494324 | miR-502 | 574504 |
| miR-217 | 406999 | miR-376A1 | 494325 | miR-503 | 574506 |
| miR-218-1 | 407000 | miR-376C | 442913 | miR-504 | 574507 |
| miR-219-1 | 407002 | miR-377 | 494326 | miR-505 | 574508 |
| miR-219b | 100616335 | miR-378 | 494327 | miR-507 | 574512 |
| miR-221 | 407006 | miR-378A | 494327 | miR-510 | 574515 |
| miR-222 | 407007 | miR-379 | 494328 | miR-511 | 574445 |
| miR-223 | 407008 | miR-380 | 494329 | miR-513 | 574510 |
| miR-224 | 407009 | miR-380-3p | 494329 | miR-513b | 100313822 |
| miR-296 | 407022 | miR-381 | 494330 | miR-513c | 100302114 |
| miR-297 | 100126354 | miR-382 | 494331 | miR-516b-2 | 574485 |
| miR-299 | 407023 | miR-383 | 494332 | miR-516b-1 | 574490 |
| miR-301a | 407027 | miR-384 | 494333 | miR-517c | 574492 |
| miR-301b | 100126318 | miR-409 | 574413 | miR-5189 | 100847057 |
| miR-302a | 407028 | miR-410 | 574434 | miR-518c* | 574477 |
| miR-320b-1 | 100302117 | miR-411 | 693121 | miR-518d-3p | 574489 |
| miR-320c-1 | 100302135 | miR-412 | 574433 | miR-518e* | 574487 |
| miR-320d-2 | 100302169 | miR-421 | 693122 | miR-518f | 574472 |
| miR-320c-2 | 100302195 | miR-422a | 494334 | miR-519b | 574469 |
| miR-320b-2 | 100313769 | miR-423 | 494335 | miR-520b | 574473 |
| miR-320d-1 | 100313896 | miR-424 | 494336 | miR-520e | 574461 |
| miR-320A | 407037 | miR-425 | 494337 | miR-521 | 574481 |
| miR-323A | 442897 | miR-429 | 554210 | miR-521 | 574494 |
| miR-323B | 574410 | miR-431* | 574038 | miR-522 | 574495 |
| miR-324 | 442898 | miR-432 | 574451 | miR-526a | 574475 |
| miR-324-3p | 442898 | miR-433 | 574034 | miR-526a | 574486 |
| miR-324-5p | 442898 | miR-448 | 554212 | miR-527 | 574497 |
| miR-325 | 442899 | miR-449a | 554213 | miR-532 | 693124 |
| miR-326 | 442900 | miR-450a-1 | 554214 | miR-539 | 664612 |
| miR-328 | 442901 | miR-450a-2 | 574505 | miR-541 | 100126308 |
| miR-331 | 442903 | miR-450B | 100126302 | miR-542 | 664617 |
| miR-335 | 442904 | miR-451 | 574411 | miR-543 | 100126335 |
| miR-335-5p | 442904 | miR-452 | 574412 | miR-548c-5p | 693129 |
| miR-337 | 442905 | miR-323b | 574410 | miR-548d-5p | 693130 |
| miR-338 | 442906 | miR-454 | 768216 | miR-548d-5p | 693131 |
| miR-339 | 442907 | miR-455 | 619556 | miR-550* | 693133 |
| miR-340 | 442908 | miR-483 | 619552 | miR-550a-1 | 693133 |
| miR-342 | 442909 | miR-484 | 619553 | miR-550a-2 | 693134 |
| miR-342-3p | 442909 | miR-485 | 574436 | miR-551b | 693136 |
| miR-345 | 442910 | miR-486 | 619554 | miR-557 | 693142 |
| miR-346 | 442911 | miR-486-5p | 619554 | miR-561 | 693146 |
| miR-361 | 494323 | miR-487A | 619555 | miR-564 | 693149 |
| miR-362 | 574030 | miR-487b | 664616 | miR-571 | 693156 |
| miR-363 | 574031 | miR-492 | 574449 | miR-573 | 693158 |
| miR-365 | 100126355 | miR-493 | 574450 | miR-574 | 693159 |
| miR-369 | 442914 | miR-494 | 574452 | miR-574-3p | 693159 |
| miR-369-3p | 442914 | miR-495 | 574453 | miR-575 | 693160 |
| miR-370 | 442915 | miR-496 | 574454 | miR-577 | 693162 |
| miR-371 | 442916 | miR-498 | 574460 | miR-578 | 693163 |

| MIRNA ID | ENTREZ ID | MIRNA ID | ENTREZ ID | MIRNA ID | ENTREZ ID |
|---|---|---|---|---|---|
| miR-581 | 693166 | miR-671 | 768213 | miR-1276 | 100302121 |
| miR-582 | 693167 | miR-708 | 100126333 | miR-1278 | 100302163 |
| miR-583 | 693168 | miR-718 | 100313781 | miR-1279 | 100302182 |
| miR-584 | 693169 | miR-744 | 100126313 | miR-1284 | 100302112 |
| miR-585 | 693170 | miR-758 | 768212 | miR-1286 | 100302118 |
| miR-588 | 693173 | miR-760 | 100126348 | miR-1288 | 100302124 |
| miR-589 | 693174 | miR-762 | 100313837 | miR-1287 | 100302133 |
| miR-590 | 693175 | miR-765 | 768220 | miR-1281 | 100302237 |
| miR-592 | 693177 | miR-766 | 768218 | miR-1282 | 100302254 |
| miR-593 | 693178 | miR-769 | 768217 | miR-1281 | 100302237 |
| miR-595 | 693180 | miR-770 | 768222 | miR-1295a | 100302178 |
| miR-598 | 693183 | miR-874 | 100126343 | miR-1290 | 100302276 |
| miR-601 | 693186 | miR-877 | 100126314 | miR-1291 | 100302221 |
| miR-603 | 693188 | miR-887 | 100126347 | miR-1293 | 100302220 |
| miR-609 | 693194 | miR-889 | 100126345 | miR-1294 | 100302181 |
| miR-610 | 693195 | miR-890 | 100126303 | miR-1296 | 100302150 |
| miR-612 | 693197 | miR-892b | 100126307 | miR-1297 | 100302187 |
| miR-615 | 693200 | miR-921 | 100126349 | miR-1298 | 100302153 |
| miR-617 | 693202 | miR-924 | 100126323 | miR-1299 | 100302167 |
| miR-618 | 693203 | miR-935 | 100126325 | miR-1301 | 100302246 |
| miR-622 | 693207 | miR-936 | 100126326 | miR-1305 | 100302270 |
| miR-623 | 693208 | miR-939 | 100126351 | miR-1321 | 100302171 |
| miR-624* | 693209 | miR-940 | 100126328 | miR-1322 | 100302166 |
| miR-625 | 693210 | miR-942 | 100126331 | miR-1323 | 100302255 |
| miR-627 | 693212 | miR-943 | 100126332 | miR-1343 | 100616437 |
| miR-628 | 693213 | miR-1180 | 100302256 | miR-1468 | 100302115 |
| miR-629 | 693214 | miR-1181 | 100302213 | miR-1471 | 100302126 |
| miR-630 | 693215 | miR-1182 | 100302132 | miR-1537 | 100302139 |
| miR-631 | 693216 | miR-1183 | 100302122 | miR-1538 | 100302119 |
| miR-632 | 693217 | miR-1185-1 | 100302157 | miR-1539 | 100302257 |
| miR-634 | 693219 | miR-1193 | 100422837 | miR-1587 | 100616251 |
| miR-637 | 693222 | miR-1197 | 100302250 | miR-1825 | 100302183 |
| miR-638 | 693223 | miR-1200 | 100302113 | miR-1827 | 100302217 |
| miR-639 | 693224 | miR-1202 | 100302259 | miR-1908 | 100302263 |
| miR-641 | 693226 | miR-1204 | 100302185 | miR-1910 | 100302261 |
| miR-642 | 693227 | miR-1205 | 100302161 | miR-1912 | 100302144 |
| miR-645 | 693230 | miR-1208 | 100302281 | miR-1913 | 100302141 |
| miR-647 | 693232 | miR-1225-5p | 100188847 | miR-1976 | 100302190 |
| miR-648 | 693233 | miR-1226* | 100302232 | miR-2110 | 100302224 |
| miR-649 | 693234 | miR-122a | 406906 | miR-2113 | 100302164 |
| miR-651 | 723779 | miR-1231 | 100302158 | miR-2116 | 100313886 |
| miR-652 | 724022 | miR-1244 | 100302285 | miR-2117 | 100313779 |
| miR-654 | 724024 | miR-1246 | 100302142 | miR-2278 | 100313780 |
| miR-655 | 724025 | miR-1248 | 100302143 | miR-2355 | 100423036 |
| miR-656 | 724026 | miR-1249 | 100302149 | miR-2467 | 100616360 |
| miR-657 | 724027 | miR-1251 | 100302289 | miR-2861 | 100422910 |
| miR-658 | 724028 | miR-1254 | 100302273 | miR-3115 | 100422866 |
| miR-659 | 724029 | miR-1261 | 100302228 | miR-3117 | 100422871 |
| miR-660 | 724030 | miR-1262 | 100302279 | miR-3125 | 100422986 |
| miR-662 | 724032 | miR-1264 | 100302251 | miR-3137 | 100422926 |
| miR-663 | 724033 | miR-1270 | 100302179 | miR-3138 | 100423011 |
| miR-665 | 100126315 | miR-1275 | 100302123 | miR-3143 | 100422934 |

| MIRNA ID | ENTREZ ID | MIRNA ID | ENTREZ ID | MIRNA ID | ENTREZ ID |
|---|---|---|---|---|---|
| miR-3146 | 100422967 | miR-4279 | 100422874 | miR-6087 | 102464835 |
| miR-3147 | 100422939 | miR-4281 | 100422962 | miR-6131 | 102465138 |
| miR-3149 | 100422921 | miR-4286 | 100422982 | miR-7112 | 102465906 |
| miR-3152 | 100422869 | miR-4290 | 100422963 | miR-7704 | 102465802 |
| miR-3155A | 100422989 | miR-4292 | 100422860 | miR-7705 | 102466854 |
| miR-3161 | 100423000 | miR-4297 | 100422873 | miR-7706 | 102465803 |
| miR-3168 | 100422878 | miR-4303 | 100422924 | miR-7974 | 102465856 |
| miR-3169 | 100422973 | miR-4306 | 100422861 | | |
| miR-3170 | 100422881 | miR-4311 | 100422905 | | |
| miR-3174 | 100422841 | miR-4312 | 100422971 | | |
| miR-3175 | 100422995 | miR-4316 | 100422851 | | |
| miR-3176 | 100423037 | miR-4317 | 100422840 | | |
| miR-3182 | 100422853 | miR-4322 | 100422925 | | |
| miR-3188 | 100422833 | miR-4323 | 100422980 | | |
| miR-3198 | 100423025 | miR-4325 | 100422883 | | |
| miR-3200 | 100422912 | miR-4326 | 100422945 | | |
| miR-3605 | 100500853 | miR-4327 | 100422891 | | |
| miR-3609 | 100500819 | miR-4424 | 100616328 | | |
| miR-3615 | 100500847 | miR-4429 | 100616469 | | |
| miR-3621 | 100500811 | miR-4443 | 100616407 | | |
| miR-3648 | 100500862 | miR-4447 | 100616485 | | |
| miR-3652 | 100500842 | miR-4448 | 100616127 | | |
| miR-3653 | 100500833 | miR-4454 | 100616234 | | |
| miR-3655 | 100500820 | miR-4455 | 100616111 | | |
| miR-3656 | 100500840 | miR-4461 | 100616209 | | |
| miR-3657 | 100500889 | miR-4467 | 100616367 | | |
| miR-3661 | 100500905 | miR-4482-1 | 100616323 | | |
| miR-3662 | 100500880 | miR-4485 | 100616263 | | |
| miR-3686 | 100500839 | miR-4488 | 100616470 | | |
| miR-3687 | 100500815 | miR-4492 | 100616376 | | |
| miR-3691 | 100500900 | miR-4497 | 100616454 | | |
| miR-3714 | 100500913 | miR-4508 | 100616275 | | |
| miR-3907 | 100500835 | miR-4520-1 | 100616401 | | |
| miR-3909 | 100500826 | miR-4520-2 | 100616466 | | |
| miR-3911 | 100500872 | miR-4524a | 100616316 | | |
| miR-3912 | 100500831 | miR-4536-1 | 100616155 | | |
| miR-3924 | 100500834 | miR-4644 | 100616430 | | |
| miR-3928 | 100500901 | miR-4645 | 100616285 | | |
| miR-3937 | 100500822 | miR-4657 | 100616393 | | |
| miR-3939 | 100500857 | miR-4710 | 100616300 | | |
| miR-3943 | 100500829 | miR-4785 | 100616364 | | |
| miR-3945 | 100500818 | miR-4792 | 100616448 | | |
| miR-3960 | 100616250 | miR-5000 | 100846995 | | |
| miR-3972 | 100616188 | miR-5094 | 100847059 | | |
| miR-4253 | 100422914 | miR-5100 | 100847014 | | |
| miR-4254 | 100423028 | miR-5190 | 100847080 | | |
| miR-4257 | 100422997 | miR-5192 | 100847087 | | |
| miR-4258 | 100423020 | miR-5572 | 100847042 | | |
| miR-4259 | 100422852 | miR-5690 | 100847048 | | |
| miR-4265 | 100422863 | miR-5698 | 100847024 | | |
| miR-4268 | 100422959 | miR-5704 | 100847040 | | |
| miR-4274 | 100422826 | miR-6089 | 102464837 | | |

**Table 4: Suitable extracellular vesicle associated proteins**

| Gene Name | Gene Symbol | Gene Name | Gene Symbol |
|---|---|---|---|
| alpha-1-B glycoprotein | A1BG | leukotriene B4 receptor | LTB4R |
| alpha-2-macroglobulin | A2M | latent transforming growth factor beta binding protein 1 | LTBP1 |
| alpha-2-macroglobulin-like 1 | A2ML1 | latent transforming growth factor beta binding protein 2 | LTBP2 |
| achalasia, adrenocortical insufficiency, alacrimia | AAAS | latent transforming growth factor beta binding protein 3 | LTBP3 |
| acetoacetyl-CoA synthetase | AACS | latent transforming growth factor beta binding protein 4 | LTBP4 |
| AAR2 splicing factor homolog (S. cerevisiae) | AAR2 | lactotransferrin | LTF |
| alanyl-tRNA synthetase | AARS | leukocyte receptor tyrosine kinase | LTK |
| alanyl-tRNA synthetase domain containing 1 | AARSD1 | listerin E3 ubiquitin protein ligase 1 | LTN1 |
| aminoadipate-semialdehyde dehydrogenase-phosphopantetheinyl transferase | AASDHPPT | LUC7-like (S. cerevisiae) | LUC7L |
| ATP-binding cassette, sub-family A (ABC1), member 7 | ABCA7 | lumican | LUM |
| ATP-binding cassette, sub-family B (MDR/TAP), member 1 | ABCB1 | lymphocyte antigen 6 complex, locus G6F | LY6G6F |
| ATP-binding cassette, sub-family B (MDR/TAP), member 11 | ABCB11 | lymphocyte antigen 75 | LY75 |
| ATP-binding cassette, sub-family B (MDR/TAP), member 4 | ABCB4 | LY75-CD302 readthrough | LY75-CD302 |
| ATP-binding cassette, sub-family B (MDR/TAP), member 6 (Langereis blood group) | ABCB6 | lymphocyte antigen 9 | LY9 |
| ATP-binding cassette, sub-family C (CFTR/MRP), member 1 | ABCC1 | Ly1 antibody reactive | LYAR |
| ATP-binding cassette, sub-family C (CFTR/MRP), member 11 | ABCC11 | LYN proto-oncogene, Src family tyrosine kinase | LYN |
| ATP-binding cassette, sub-family C (CFTR/MRP), member 2 | ABCC2 | LY6/PLAUR domain containing 3 | LYPD3 |
| ATP-binding cassette, sub-family C (CFTR/MRP), member 4 | ABCC4 | lysophospholipase I | LYPLA1 |
| ATP-binding cassette, sub-family C (CFTR/MRP), member 5 | ABCC5 | lysophospholipase II | LYPLA2 |
| ATP-binding cassette, sub-family C (CFTR/MRP), member 9 | ABCC9 | lysophospholipase-like 1 | LYPLAL1 |
| ATP-binding cassette, sub-family D (ALD), member 2 | ABCD2 | lysozyme | LYZ |
| ATP-binding cassette, sub-family E (OABP), member 1 | ABCE1 | leucine-zipper-like transcription regulator 1 | LZTR1 |
| ATP-binding cassette, sub-family F (GCN20), member 1 | ABCF1 | mannose-6-phosphate receptor (cation dependent) | M6PR |
| ATP-binding cassette, sub-family F (GCN20), member 2 | ABCF2 | microtubule-actin crosslinking factor 1 | MACF1 |
| ATP-binding cassette, sub-family F (GCN20), member 3 | ABCF3 | MAP-kinase activating death domain | MADD |
| ATP-binding cassette, sub-family G (WHITE), member 2 (Junior blood group) | ABCG2 | melanoma antigen family A4 | MAGEA4 |
| abhydrolase domain containing 11 | ABHD11 | melanoma antigen family B17 | MAGEB17 |
| abhydrolase domain containing 14B | ABHD14B | melanoma antigen family B2 | MAGEB2 |
| abhydrolase domain containing 16A | ABHD16A | melanoma antigen family D2 | MAGED2 |
| abhydrolase domain containing 17A | ABHD17A | membrane associated guanylate kinase, WW and PDZ domain containing 3 | MAGI3 |
| abhydrolase domain containing 17B | ABHD17B | mago-nashi homolog B (Drosophila) | MAGOHB |
| abl-interactor 1 | ABI1 | magnesium transporter 1 | MAGT1 |
| abl-interactor 2 | ABI2 | mal, T-cell differentiation protein 2 (gene/pseudogene) | MAL2 |
| ABI family, member 3 | ABI3 | MALT1 paracaspase | MALT1 |
| ABI family, member 3 (NESH) binding protein | ABI3BP | MAM domain containing 2 | MAMDC2 |
| activator of basal transcription 1 | ABT1 | mannosidase, alpha, class 1A, member 1 | MAN1A1 |
| acetyl-CoA acyltransferase 1 | ACAA1 | mannosidase, alpha, class 1A, member 2 | MAN1A2 |
| acetyl-CoA acyltransferase 2 | ACAA2 | mannosidase, alpha, class 1B, member 1 | MAN1B1 |
| acetyl-CoA carboxylase alpha | ACACA | mannosidase, alpha, class 2A, member 1 | MAN2A1 |
| acetyl-CoA carboxylase beta | ACACB | mannosidase, alpha, class 2B, member 1 | MAN2B1 |
| acyl-CoA dehydrogenase family, member 8 | ACAD8 | mesencephalic astrocyte-derived neurotrophic factor | MANF |
| acyl-CoA dehydrogenase, C-4 to C-12 straight chain | ACADM | microtubule-associated protein 1A | MAP1A |
| ArfGAP with coiled-coil, ankyrin repeat and PH domains 1 | ACAP1 | microtubule-associated protein 1B | MAP1B |
| acetyl-CoA acetyltransferase 1 | ACAT1 | microtubule-associated protein 1S | MAP1S |
| acetyl-CoA acetyltransferase 2 | ACAT2 | microtubule-associated protein 2 | MAP2 |
| | | microtubule-associated protein Tau | MAPT |
| acyl-CoA binding domain containing 3 | ACBD3 | mitogen-activated protein kinase kinase 1 | MAP2K1 |
| angiotensin I converting enzyme | ACE | mitogen-activated protein kinase kinase 2 | MAP2K2 |
| angiotensin I converting enzyme 2 | ACE2 | mitogen-activated protein kinase kinase 3 | MAP2K3 |
| ATP citrate lyase | ACLY | mitogen-activated protein kinase kinase 4 | MAP2K4 |
| aconitase 1, soluble | ACO1 | mitogen-activated protein kinase kinase 6 | MAP2K6 |
| aconitase 2, mitochondrial | ACO2 | mitogen-activated protein kinase kinase 7 | MAP2K7 |
| acyl-CoA thioesterase 1 | ACOT1 | mitogen-activated protein kinase kinase kinase 1, E3 ubiquitin protein ligase | MAP3K1 |
| acyl-CoA thioesterase 11 | ACOT11 | mitogen-activated protein kinase kinase kinase 19 | MAP3K19 |
| acyl-CoA thioesterase 7 | ACOT7 | microtubule-associated protein 4 | MAP4 |
| acid phosphatase 1, soluble | ACP1 | mitogen-activated protein kinase kinase kinase kinase 4 | MAP4K4 |
| acid phosphatase 2, lysosomal | ACP2 | microtubule-associated protein 7 | MAP7 |
| acid phosphatase, prostate | ACPP | mitogen-activated protein kinase 1 | MAPK1 |
| acyl-CoA synthetase long-chain family member 1 | ACSL1 | mitogen-activated protein kinase 14 | MAPK14 |
| acyl-CoA synthetase long-chain family member 3 | ACSL3 | mitogen-activated protein kinase 1 interacting protein 1-like | MAPK1 IP1 L |
| acyl-CoA synthetase long-chain family member 4 | ACSL4 | mitogen-activated protein kinase 3 | MAPK3 |
| acyl-CoA synthetase long-chain family member 5 | ACSL5 | mitogen-activated protein kinase 8 | MAPK8 |
| acyl-CoA synthetase long-chain family member 6 | ACSL6 | mitogen-activated protein kinase 8 interacting protein 1 | MAPK8IP1 |
| acyl-CoA synthetase medium-chain family member 1 | ACSM1 | mitogen-activated protein kinase 9 | MAPK9 |
| acyl-CoA synthetase short-chain family member 2 | ACSS2 | mitogen-activated protein kinase-activated protein kinase 2 | MAPKAPK2 |
| actin, alpha 1, skeletal muscle | ACTA1 | microtubule-associated protein, RP/EB family, member 1 | MAPRE1 |
| actin, alpha 2, smooth muscle, aorta | ACTA2 | microtubule-associated protein, RP/EB family, member 2 | MAPRE2 |
| actin, beta | ACTB | myristoylated alanine-rich protein kinase C substrate | MARCKS |
| actin, beta-like 2 | ACTBL2 | MARCKS-like 1 | MARCKSL1 |
| actin, alpha, cardiac muscle 1 | ACTC1 | MAP/microtubule affinity-regulating kinase 2 | MARK2 |
| actin gamma 1 | ACTG1 | MAP/microtubule affinity-regulating kinase 3 | MARK3 |
| actin, gamma 2, smooth muscle, enteric | ACTG2 | methionyl-tRNA synthetase | MARS |
| actin-like 6A | ACTL6A | MARVEL domain containing 2 | MARVELD2 |
| actinin, alpha 1 | ACTN1 | mannan-binding lectin serine peptidase 1 (C4/C2 activating component of Ra-reactive factor) | MASP1 |
| actinin, alpha 2 | ACTN2 | mannan-binding lectin serine peptidase 2 | MASP2 |
| actinin, alpha 3 (gene/pseudogene) | ACTN3 | methionine adenosyltransferase I, alpha | MAT1A |
| actinin, alpha 4 | ACTN4 | methionine adenosyltransferase II, alpha | MAT2A |
| actin-related protein 10 homolog (S. cerevisiae) | ACTR10 | methionine adenosyltransferase II, beta | MAT2B |
| ARP1 actin-related protein 1 homolog A, centractin alpha (yeast) | ACTR1A | matrilin 2 | MATN2 |
| ARP1 actin-related protein 1 homolog B, centractin beta (yeast) | ACTR1B | matrin 3 | MATR3 |
| ARP2 actin-related protein 2 homolog (yeast) | ACTR2 | mitochondrial antiviral signaling protein | MAVS |
| ARP3 actin-related protein 3 homolog (yeast) | ACTR3 | methyl-CpG binding domain protein 3 | MBD3 |
| ARP3 actin-related protein 3 homolog B (yeast) | ACTR3B | methyl-CpG binding domain protein 3-like 5 | MBD3L5 |
| ARP3 actin-related protein 3 homolog C (yeast) | ACTR3C | methyl-CpG binding domain protein 5 | MBD5 |
| ARP5 actin-related protein 5 homolog (yeast) | ACTR5 | mannose-binding lectin (protein C) 2, soluble | MBL2 |
| ARP8 actin-related protein 8 homolog (yeast) | ACTR8 | metallo-beta-lactamase domain containing 2 | MBLAC2 |
| activin A receptor, type I | ACVR1 | muscleblind-like splicing regulator 1 | MBNL1 |
| activin A receptor, type IB | ACVR1B | melanocortin 1 receptor (alpha melanocyte stimulating hormone receptor) | MC1R |
| aminoacylase 1 | ACY1 | melanoma cell adhesion molecule | MCAM |
| aminoacylase 3 | ACY3 | mutated in colorectal cancers | MCC |
| adenosine deaminase | ADA | methylcrotonoyl-CoA carboxylase 1 (alpha) | MCCC1 |
| ADAM metallopeptidase domain 10 | ADAM10 | MCF.2 cell line derived transforming sequence-like 2 | MCF2L2 |
| ADAM metallopeptidase domain 15 | ADAM15 | minichromosome maintenance complex component 10 | MCM10 |
| ADAM metallopeptidase domain 17 | ADAM17 | minichromosome maintenance complex component 2 | MCM2 |
| ADAM metallopeptidase domain 30 | ADAM30 | minichromosome maintenance complex component 3 | MCM3 |
| ADAM metallopeptidase domain 9 | ADAM9 | minichromosome maintenance complex component 4 | MCM4 |
| ADAM metallopeptidase with thrombospondin type 1 motif, 1 | ADAMTS1 | minichromosome maintenance complex component 5 | MCM5 |
| ADAM metallopeptidase with thrombospondin type 1 motif, 12 | ADAMTS12 | minichromosome maintenance complex component 6 | MCM6 |
| ADAM metallopeptidase with thrombospondin type 1 motif, 13 | ADAMTS13 | minichromosome maintenance complex component 7 | MCM7 |
| ADAM metallopeptidase with thrombospondin type 1 motif, 2 | ADAMTS2 | minichromosome maintenance complex binding protein | MCMBP |
| ADAM metallopeptidase with thrombospondin type 1 motif, 3 | ADAMTS3 | mucolipin 1 | MCOLN1 |
| adenosine deaminase, RNA-specific | ADAR | malignant T cell amplified sequence 1 | MCTS1 |
| adenylate cyclase 1 (brain) | ADCY1 | malate dehydrogenase 1, NAD (soluble) | MDH1 |
| adenylate cyclase 6 | ADCY6 | malate dehydrogenase 2, NAD (mitochondrial) | MDH2 |
| adenylate cyclase 9 | ADCY9 | midkine (neurite growth-promoting factor 2) | MDK |
| adducin 1 (alpha) | ADD1 | MDN1, midasin homolog (yeast) | MDN1 |
| adducin 2 (beta) | ADD2 | magnesium-dependent phosphatase 1 | MDP1 |
| adhesion G protein-coupled receptor E5 | ADGRE5 | malic enzyme 1, NADP(+)-dependent, cytosolic | ME1 |
| adhesion G protein-coupled receptor G1 | ADGRG1 | malic enzyme 3, NADP(+)-dependent, mitochondrial | ME3 |
| adhesion G protein-coupled receptor G2 | ADGRG2 | methyl CpG binding protein 2 | MECP2 |
| adhesion G protein-coupled receptor G4 | ADGRG4 | mediator complex subunit 20 | MED20 |
| adhesion G protein-coupled receptor G6 | ADGRG6 | mediator complex subunit 23 | MED23 |
| adhesion G protein-coupled receptor L2 | ADGRL2 | multiple EGF-like-domains 10 | MEGF10 |
| adhesion G protein-coupled receptor L3 | ADGRL3 | multiple EGF-like-domains 8 | MEGF8 |
| adhesion G protein-coupled receptor V1 | ADGRV1 | mediator of cell motility 1 | MEMO1 |
| alcohol dehydrogenase 1A (class I), alpha polypeptide | ADH1A | multiple endocrine neoplasia I | MEN1 |
| alcohol dehydrogenase 1B (class I), beta polypeptide | ADH1B | meprin A, alpha (PABA peptide hydrolase) | MEP1A |
| alcohol dehydrogenase 1C (class I), gamma polypeptide | ADH1C | meprin A, beta | MEP1B |
| alcohol dehydrogenase 5 (class III), chi polypeptide | ADH5 | mesoderm specific transcript | MEST |
| alcohol dehydrogenase 6 (class V) | ADH6 | MET proto-oncogene, receptor tyrosine kinase | MET |
| adipogenesis regulatory factor | ADIRF | methionyl aminopeptidase 2 | METAP2 |
| adenosine kinase | ADK | meteorin, glial cell differentiation regulator-like | METRNL |
| ADP-ribosylhydrolase like 2 | ADPRHL2 | methyltransferase like 13 | METTL13 |
| adrenergic, beta, receptor kinase 1 | ADRBK1 | methyltransferase like 16 | METTL16 |
| adhesion regulating molecule 1 | ADRM1 | mex-3 RNA binding family member B | MEX3B |
| adenylosuccinate lyase | ADSL | microfibrillar-associated protein 4 | MFAP4 |
| adenylosuccinate synthase | ADSS | milk fat globule-EGF factor 8 protein | MFGE8 |
| AE binding protein 1 | AEBP1 | antigen p97 (melanoma associated) identified by monoclonal antibodies 133.2 and 96.5 | MFI2 |
| afamin | AFM | major facilitator superfamily domain containing 1 | MFSD1 |
| alpha-fetoprotein | AFP | major facilitator superfamily domain containing 2B | MFSD2B |
| ATP/GTP binding protein-like 3 | AGBL3 | maltase-glucoamylase | MGAM |
| acylglycerol kinase | AGK | mannosyl (alpha-1,3-)-glycoprotein beta-1,2-N-acetylglucosaminyltransferase | MGAT1 |
| amylo-alpha-1,6-glucosidase, 4-alpha-glucanotransferase | AGL | mannosyl (alpha-1,3-)-glycoprotein beta-1,4-N-acetylglucosaminyltransferase , isozyme A | MGAT4A |
| argonaute RISC catalytic component 2 | AGO2 | mannosyl (alpha-1,6-)-glycoprotein beta-1,6-N-acetyl-glucosaminyltransferase | MGAT5 |
| alkylglycerone phosphate synthase | AGPS | monoglyceride lipase | MGLL |
| anterior gradient 2 | AGR2 | O-6-methylguanine-DNA methyltransferase | MGMT |
| anterior gradient 3 | AGR3 | matrix Gla protein | MGP |
| agrin | AGRN | mahogunin ring finger 1, E3 ubiquitin protein ligase | MGRN1 |
| angiotensinogen (serpin peptidase inhibitor, clade A, member 8) | AGT | microsomal glutathione S-transferase 2 | MGST2 |
| angiotensin II receptor-associated protein | AGTRAP | microsomal glutathione S-transferase 3 | MGST3 |
| AT hook containing transcription factor 1 | AHCTF1 | mindbomb E3 ubiquitin protein ligase 2 | MIB2 |
| adenosylhomocysteinase | AHCY | MHC class I polypeptide-related sequence A | MICA |
| adenosylhomocysteinase-like 1 | AHCYL1 | microtubule associated monooxygenase, calponin and LIM domain containing 1 | MICAL1 |
| adenosylhomocysteinase-like 2 | AHCYL2 | midline 2 | MID2 |
| AHNAK nucleoprotein | AHNAK | mesoderm induction early response 1, family member 3 | MIER3 |
| AHNAK nucleoprotein 2 | AHNAK2 | macrophage migration inhibitory factor (glycosylation-inhibiting factor) | MIF |
| aryl-hydrocarbon receptor repressor | AHRR | MIF antisense RNA 1 | MIF-AS1 |
| AHA1, activator of heat shock 90kDa protein ATPase homolog 1 (yeast) | AHSA1 | misshapen-like kinase 1 | MINK1 |
| alpha-2-HS-glycoprotein | AHSG | mitotic spindle positioning | MISP |
| axin interactor, dorsalization associated | AIDA | MIT, microtubule interacting and transport, domain containing 1 | MITD1 |
| allograft inflammatory factor 1-like | AIF1L | makorin ring finger protein 1 | MKRN1 |
| apoptosis-inducing factor, mitochondrion-associated, 2 | AIFM2 | melan-A | MLANA |
| aminoacyl tRNA synthetase complex-interacting multifunctional protein 1 | AIMP1 | megalencephalic leukoencephalopathy with subcortical cysts 1 | MLC1 |
| aminoacyl tRNA synthetase complex-interacting multifunctional protein 2 | AIMP2 | malectin | MLEC |
| adenylate kinase 1 | AK1 | myeloid leukemia factor 2 | MLF2 |
| adenylate kinase 2 | AK2 | mutL homolog 1 | MLH1 |
| adenylate kinase 4 | AK4 | myeloid/lymphoid or mixed-lineage leukemia (trithorax homolog, Drosophila); translocated to, 3 | MLLT3 |
| A kinase (PRKA) anchor protein 12 | AKAP12 | myeloid/lymphoid or mixed-lineage leukemia (trithorax homolog, Drosophila); translocated to, 4 | MLLT4 |
| A kinase (PRKA) anchor protein 9 | AKAP9 | MTOR associated protein, LST8 homolog (S. cerevisiae) | MLST8 |
| aldo-keto reductase family 1, member A1 (aldehyde reductase) | AKR1A1 | membrane metallo-endopeptidase | MME |
| aldo-keto reductase family 1, member B1 (aldose reductase) | AKR1B1 | matrix metallopeptidase 1 | MMP1 |
| aldo-keto reductase family 1, member B10 (aldose reductase) | AKR1B10 | matrix metallopeptidase 10 | MMP10 |
| aldo-keto reductase family 1, member C1 | AKR1C1 | matrix metallopeptidase 14 (membrane-inserted) | MMP14 |
| aldo-keto reductase family 1, member C3 | AKR1C3 | matrix metallopeptidase 15 (membrane-inserted) | MMP15 |
| aldo-keto reductase family 1, member E2 | AKR1E2 | matrix metallopeptidase 2 | MMP2 |
| aldo-keto reductase family 7, member A2 (aflatoxin aldehyde reductase) | AKR7A2 | matrix metallopeptidase 24 (membrane-inserted) | MMP24 |
| aldo-keto reductase family 7, member A3 (aflatoxin aldehyde reductase) | AKR7A3 | matrix metallopeptidase 25 | MMP25 |
| aldo-keto reductase family 7-like (gene/pseudogene) | AKR7L | matrix metallopeptidase 3 | MMP3 |
| v-akt murine thymoma viral oncogene homolog 1 | AKT1 | matrix metallopeptidase 7 | MMP7 |
| v-akt murine thymoma viral oncogene homolog 2 | AKT2 | matrix metallopeptidase 9 | MMP9 |
| aminolevulinate dehydratase | ALAD | multimerin 1 | MMRN1 |
| albumin | ALB | multimerin 2 | MMRN2 |
| activated leukocyte cell adhesion molecule | ALCAM | MMS19 nucleotide excision repair homolog (S. cerevisiae) | MMS19 |
| aldehyde dehydrogenase 16 family, member A1 | ALDH16A1 | myeloid cell nuclear differentiation antigen | MNDA |
| aldehyde dehydrogenase 18 family, member A1 | ALDH18A1 | MOB kinase activator 1A | MOB1A |
| aldehyde dehydrogenase 1 family, member A1 | ALDH1A1 | MOB kinase activator 1B | MOB1B |
| aldehyde dehydrogenase 1 family, member A3 | ALDH1A3 | MOB kinase activator 2 | MOB2 |
| aldehyde dehydrogenase 1 family, member L1 | ALDH1L1 | molybdenum cofactor synthesis 3 | MOCS3 |
| aldehyde dehydrogenase 2 family (mitochondrial) | ALDH2 | mannosyl-oligosaccharide glucosidase | MOGS |
| aldehyde dehydrogenase 3 family, member A1 | ALDH3A1 | MON2 homolog (S. cerevisiae) | MON2 |
| aldehyde dehydrogenase 3 family, member A2 | ALDH3A2 | MORC family CW-type zinc finger 3 | MORC3 |
| aldehyde dehydrogenase 3 family, member B1 | ALDH3B1 | v-mos Moloney murine sarcoma viral oncogene homolog | MOS |
| aldehyde dehydrogenase 5 family, member A1 | ALDH5A1 | Mov10 RISC complex RNA helicase | MOV10 |
| aldehyde dehydrogenase 6 family, member A1 | ALDH6A1 | monooxygenase, DBH-like 1 | MOXD1 |
| aldehyde dehydrogenase 7 family, member A1 | ALDH7A1 | mannose phosphate isomerase | MPI |
| aldehyde dehydrogenase 8 family, member A1 | ALDH8A1 | MPL proto-oncogene, thrombopoietin receptor | MPL |
| aldehyde dehydrogenase 9 family, member A1 | ALDH9A1 | myeloperoxidase | MPO |
| aldolase A, fructose-bisphosphate | ALDOA | membrane protein, palmitoylated 1, 55kDa | MPP1 |
| aldolase B, fructose-bisphosphate | ALDOB | membrane protein, palmitoylated 5 (MAGUK p55 subfamily member 5) | MPP5 |
| aldolase C, fructose-bisphosphate | ALDOC | membrane protein, palmitoylated 6 (MAGUK p55 subfamily member 6) | MPP6 |
| anaplastic lymphoma receptor tyrosine kinase | ALK | membrane protein, palmitoylated 7 (MAGUK p55 subfamily member 7) | MPP7 |
| AlkB family member 5, RNA demethylase | ALKBH5 | metallophosphoesterase 1 | MPPE1 |
| arachidonate 12-lipoxygenase | ALOX12 | mercaptopyruvate sulfurtransferase | MPST |
| arachidonate 12-lipoxygenase pseudogene 2 | ALOX12P2 | myelin protein zero-like 1 | MPZL1 |
| alkaline phosphatase, liver/bone/kidney | ALPL | myelin protein zero-like 2 | MPZL2 |
| alkaline phosphatase, placental | ALPP | mannose receptor, C type 2 | MRC2 |
| alkaline phosphatase, placental-like 2 | ALPPL2 | MRE11 meiotic recombination 11 homolog A (S. cerevisiae) | MRE11A |
| Aly/REF export factor | ALYREF | methylthioribose-1-phosphate isomerase 1 | MRI1 |
| alpha-1-microglobulin/bikunin precursor | AMBP | maestro heat-like repeat family member 1 | MROH1 |
| amnion associated transmembrane protein | AMN | mitochondrial ribosomal protein S17 | MRPS17 |
| antagonist of mitotic exit network 1 homolog (S. cerevisiae) | AMN1 | mitochondrial ribosomal protein S22 | MRPS22 |
| adenosine monophosphate deaminase 2 | AMPD2 | mRNA turnover 4 homolog (S. cerevisiae) | MRTO4 |
| amylase, alpha 1A (salivary) | AMY1A | murine retrovirus integration site 1 homolog | MRVI1 |
| amylase, alpha 1B (salivary) | AMY1 B | membrane-spanning 4-domains, subfamily A, member 1 | MS4A1 |
| amylase, alpha 1C (salivary) | AMY1C | mutS homolog 2 | MSH2 |
| amylase, alpha 2A (pancreatic) | AMY2A | mutS homolog 3 | MSH3 |
| amylase, alpha 2B (pancreatic) | AMY2B | mutS homolog 6 | MSH6 |
| anaphase promoting complex subunit 1 | ANAPC1 | musashi RNA-binding protein 2 | MSI2 |
| anaphase promoting complex subunit 2 | ANAPC2 | male-specific lethal 1 homolog (Drosophila) | MSL1 |
| anaphase promoting complex subunit 5 | ANAPC5 | mesothelin | MSLN |
| anaphase promoting complex subunit 7 | ANAPC7 | methylsterol monooxygenase 1 | MSMO1 |
| angiogenin, ribonuclease, RNase A family, 5 | ANG | microseminoprotein, prostate associated | MSMP |
| angiopoietin 1 | ANGPT1 | moesin | MSN |
| angiopoietin-like 1 | ANGPTL1 | methionine sulfoxide reductase A | MSRA |
| angiopoietin-like 2 | ANGPTL2 | macrophage stimulating 1 receptor | MST1R |
| angiopoietin-like 3 | ANGPTL3 | metastasis associated 1 family, member 2 | MTA2 |
| angiopoietin-like 4 | ANGPTL4 | metastasis associated 1 family, member 3 | MTA3 |
| angiopoietin-like 7 | ANGPTL7 | methylthioadenosine phosphorylase | MTAP |
| ankyrin 1, erythrocytic | ANK1 | mitochondrial carrier 2 | MTCH2 |
| ankyrin repeat and FYVE domain containing 1 | ANKFY1 | methylenetetrahydrofolate dehydrogenase (NADP+ dependent) 1, methenyltetrahydrofolate cyclohydrolase, formyltetrahydrofolate synthetase | MTHFD1 |
| ANKH inorganic pyrophosphate transport regulator | ANKH | methylenetetrahydrofolate dehydrogenase (NADP+ dependent) 1-like | MTHFD1L |
| ankyrin repeat domain 13A | ANKRD13A | myotubularin related protein 10 | MTMR10 |
| ankyrin repeat domain 18B | ANKRD18B | myotubularin related protein 12 | MTMR12 |
| ankyrin repeat domain 23 | ANKRD23 | mechanistic target of rapamycin (serine/threonine kinase) | MTOR |
| ankyrin repeat domain 24 | ANKRD24 | myotrophin | MTPN |
| ankyrin repeat domain 26 | ANKRD26 | metastasis suppressor 1 | MTSS1 |
| ankyrin repeat domain 28 | ANKRD28 | microsomal triglyceride transfer protein | MTTP |
| ankyrin repeat domain 36 | ANKRD36 | microtubule associated tumor suppressor candidate 2 | MTUS2 |
| ankyrin repeat domain 44 | ANKRD44 | mucin 1, cell surface associated | MUC1 |
| ankyrin repeat and sterile alpha motif domain containing 1A | ANKS1A | mucin 13, cell surface associated | MUC13 |
| ankyrin repeat and sterile alpha motif domain containing 1B | ANKS1B | mucin 16, cell surface associated | MUC16 |
| anillin, actin binding protein | ANLN | mucin 17, cell surface associated | MUC17 |
| anoctamin 1, calcium activated chloride channel | ANO1 | mucin 4, cell surface associated | MUC4 |
| anoctamin 6 | ANO6 | mucin 5AC, oligomeric mucus/gel-forming | MUC5AC |
| acidic (leucine-rich) nuclear phosphoprotein 32 family, member A | ANP32A | mucin 6, oligomeric mucus/gel-forming | MUC6 |
| acidic (leucine-rich) nuclear phosphoprotein 32 family, member B | ANP32B | mucin 7, secreted | MUC7 |
| acidic (leucine-rich) nuclear phosphoprotein 32 family, member E | ANP32E | melanoma associated antigen (mutated) 1-like 1 | MUM1L1 |
| alanyl (membrane) aminopeptidase | ANPEP | multivesicular body subunit 12A | MVB12A |
| anthrax toxin receptor 1 | ANTXR1 | multivesicular body subunit 12B | MVB12B |
| anthrax toxin receptor 2 | ANTXR2 | mevalonate (diphospho) decarboxylase | MVD |
| annexin A1 | ANXA1 | mevalonate kinase | MVK |
| annexin A11 | ANXA11 | major vault protein | MVP |
| annexin A13 | ANXA13 | MX dynamin-like GTPase 1 | MX1 |
| annexin A2 | ANXA2 | MX dynamin-like GTPase 2 | MX2 |
| annexin A2 pseudogene 1 | ANXA2P1 | matrix-remodelling associated 5 | MXRA5 |
| annexin A2 pseudogene 2 | ANXA2P2 | matrix-remodelling associated 8 | MXRA8 |
| annexin A3 | ANXA3 | myeloid-associated differentiation marker | MYADM |
| annexin A4 | ANXA4 | MYB binding protein (P160) 1a | MYBBP1A |
| annexin A5 | ANXA5 | myosin binding protein C, fast type | MYBPC2 |
| annexin A6 | ANXA6 | MYC binding protein 2, E3 ubiquitin protein ligase | MYCBP2 |
| annexin A7 | ANXA7 | MYCBP associated protein | MYCBPAP |
| annexin A8 | ANXA8 | myc target 1 | MYCT1 |
| amine oxidase, copper containing 1 | AOC1 | myeloid differentiation primary response 88 | MYD88 |
| aldehyde oxidase 1 | AOX1 | myeloid-derived growth factor | MYDGF |
| adaptor-related protein complex 1, beta 1 subunit | AP1B1 | myosin, heavy chain 10, non-muscle | MYH10 |
| adaptor-related protein complex 1, gamma 1 subunit | AP1G1 | myosin, heavy chain 11, smooth muscle | MYH11 |
| adaptor-related protein complex 1, mu 1 subunit | AP1M1 | myosin, heavy chain 13, skeletal muscle | MYH13 |
| adaptor-related protein complex 1, mu 2 subunit | AP1M2 | myosin, heavy chain 14, non-muscle | MYH14 |
| adaptor-related protein complex 1, sigma 1 subunit | AP1S1 | myosin, heavy chain 3, skeletal muscle, embryonic | MYH3 |
| adaptor-related protein complex 2, alpha 1 subunit | AP2A1 | myosin, heavy chain 7, cardiac muscle, beta | MYH7 |
| adaptor-related protein complex 2, alpha 2 subunit | AP2A2 | myosin, heavy chain 8, skeletal muscle, perinatal | MYH8 |
| adaptor-related protein complex 2, beta 1 subunit | AP2B1 | myosin, heavy chain 9, non-muscle | MYH9 |
| adaptor-related protein complex 2, mu 1 subunit | AP2M1 | myosin, light chain 1, alkali; skeletal, fast | MYL1 |
| adaptor-related protein complex 2, sigma 1 subunit | AP2S1 | myosin, light chain 12A, regulatory, non-sarcomeric | MYL12A |
| adaptor-related protein complex 3, beta 1 subunit | AP3B1 | myosin, light chain 12B, regulatory | MYL12B |
| adaptor-related protein complex 3, delta 1 subunit | AP3D1 | myosin, light chain 3, alkali; ventricular, skeletal, slow | MYL3 |
| adaptor-related protein complex 3, mu 1 subunit | AP3M1 | myosin, light chain 6, alkali, smooth muscle and non-muscle | MYL6 |
| adaptor-related protein complex 3, sigma 2 subunit | AP3S2 | myosin, light chain 6B, alkali, smooth muscle and non-muscle | MYL6B |
| adaptor-related protein complex 4, mu 1 subunit | AP4M1 | myosin, light chain 9, regulatory | MYL9 |
| adaptor-related protein complex 5, beta 1 subunit | AP5B1 | myosin light chain kinase | MYLK |
| adaptor-related protein complex 5, mu 1 subunit | AP5M1 | myosin light chain kinase 2 | MYLK2 |
| adaptor-related protein complex 5, zeta 1 subunit | AP5Z1 | myosin light chain kinase 3 | MYLK3 |
| apoptotic peptidase activating factor 1 | APAF1 | myosin XVA | MYO15A |
| amyloid beta (A4) precursor protein-binding, family B, member 1 interacting protein | APBB1IP | myosin XVI | MYO16 |
| amyloid P component, serum | APCS | myosin XVIIIA | MYO18A |
| acylaminoacyl-peptide hydrolase | APEH | myosin IA | MYO1A |
| APEX nuclease (multifunctional DNA repair enzyme) 1 | APEX1 | myosin IB | MYO1B |
| APH1A gamma secretase subunit | APH1A | myosin IC | MYO1C |
| apoptosis inhibitor 5 | API5 | myosin ID | MYO1 D |
| amyloid beta (A4) precursor-like protein 2 | APLP2 | myosin IE | MYO1E |
| adipocyte plasma membrane associated protein | APMAP | myosin IF | MYO1F |
| apolipoprotein A-I | APOA1 | myosin IG | MYO1G |
| apolipoprotein A-II | APOA2 | myosin IIIB | MYO3B |
| apolipoprotein A-IV | APOA4 | myosin VA (heavy chain 12, myoxin) | MYO5A |
| apolipoprotein A-V | APOA5 | myosin VB | MYO5B |
| apolipoprotein B | APOB | myosin VI | MYO6 |
| apolipoprotein B mRNA editing enzyme, catalytic polypeptide-like 3A | APOBEC3A | myosin VIIA | MYO7A |
| APOBEC3A and APOBEC3B deletion hybrid | APOBEC3A_B | myosin IXB | MYO9B |
| apolipoprotein B mRNA editing enzyme, catalytic polypeptide-like 3B | APOBEC3B | myocilin, trabecular meshwork inducible glucocorticoid response | MYOC |
| apolipoprotein B mRNA editing enzyme, catalytic polypeptide-like 3C | APOBEC3C | myoferlin | MYOF |
| apolipoprotein C-II | APOC2 | myozenin 2 | MYOZ2 |
| apolipoprotein C-III | APOC3 | NEDD4 binding protein 2-like 2 | N4BP2L2 |
| apolipoprotein D | APOD | N(alpha)-acetyltransferase 10, NatA catalytic subunit | NAA10 |
| apolipoprotein E | APOE | N(alpha)-acetyltransferase 15, NatA auxiliary subunit | NAA15 |
| apolipoprotein H (beta-2-glycoprotein I) | APOH | N(alpha)-acetyltransferase 16, NatA auxiliary subunit | NAA16 |
| apolipoprotein L, 1 | APOL1 | N(alpha)-acetyltransferase 50, NatE catalytic subunit | NAA50 |
| apolipoprotein L, 2 | APOL2 | N-acetylated alpha-linked acidic dipeptidase 2 | NAALAD2 |
| apolipoprotein M | APOM | NGFI-A binding protein 2 (EGR1 binding protein 2) | NAB2 |
| amyloid beta (A4) precursor protein | APP | nascent polypeptide-associated complex alpha subunit | NACA |
| adaptor protein, phosphotyrosine interaction, PH domain and leucine zipper containing 1 | APPL1 | nascent polypeptide-associated complex alpha subunit 2 | NACA2 |
| adaptor protein, phosphotyrosine interaction, PH domain and leucine zipper containing 2 | APPL2 | NEDD8 activating enzyme E1 subunit 1 | NAE1 |
| adenine phosphoribosyltransferase | APRT | N-acetylgalactosamin idase, alpha- | NAGA |
| aquaporin 1 (Colton blood group) | AQP1 | N-acetylglucosamine kinase | NAGK |
| aquaporin 2 (collecting duct) | AQP2 | N-acetylglucosaminidase, alpha | NAGLU |
| aquaporin 5 | AQP5 | nicotinamide phosphoribosyltransferase | NAMPT |
| aquarius intron-binding spliceosomal factor | AQR | N-acetylneuraminic acid synthase | NANS |
| androgen receptor | AR | nucleosome assembly protein 1-like 1 | NAP1L1 |
| A-Raf proto-oncogene, serine/threonine kinase | ARAF | nucleosome assembly protein 1-like 4 | NAP1 L4 |
| ArfGAP with RhoGAP domain, ankyrin repeat and PH domain 1 | ARAP1 | N-ethylmaleimide-sensitive factor attachment protein, alpha | NAPA |
| ArfGAP with RhoGAP domain, ankyrin repeat and PH domain 3 | ARAP3 | N-acyl phosphatidylethanolamine phospholipase D | NAPEPLD |
| archain 1 | ARCN1 | N-ethylmaleimide-sensitive factor attachment protein, gamma | NAPG |
| amphiregulin | AREG | nicotinate phosphoribosyltransferase | NAPRT |
| ADP-ribosylation factor 1 | ARF1 | napsin A aspartic peptidase | NAPSA |
| ADP-ribosylation factor 3 | ARF3 | asparaginyl-tRNA synthetase | NARS |
| ADP-ribosylation factor 4 | ARF4 | nuclear autoantigenic sperm protein (histone-binding) | NASP |
| ADP-ribosylation factor 5 | ARF5 | N-acetyltransferase 1 (arylamine N-acetyltransferase) | NAT1 |
| ADP-ribosylation factor 6 | ARF6 | N-acetyltransferase 10 (GCN5-related) | NAT10 |
| ADP-ribosylation factor GTPase activating protein 3 | ARFGAP3 | N-acetyltransferase 8-like (GCN5-related, putative) | NAT8L |
| ADP-ribosylation factor guanine nucleotide-exchange factor 2 (brefeldin A-inhibited) | ARFGEF2 | neuron navigator 1 | NAV1 |
| ADP-ribosylation factor interacting protein 1 | ARFIP1 | neurobeachin-like 2 | NBEAL2 |
| arginase 1 | ARG1 | neuroblastoma 1, DAN family BMP antagonist | NBL1 |
| Rho GTPase activating protein 1 | ARHGAP1 | neighbor of BRCA1 gene 1 | NBR1 |
| Rho GTPase activating protein 15 | ARHGAP15 | neurocalcin delta | NCALD |
| Rho GTPase activating protein 18 | ARHGAP18 | neural cell adhesion molecule 1 | NCAM1 |
| Rho GTPase activating protein 23 | ARHGAP23 | non-SMC condensin I complex, subunit D2 | NCAPD2 |
| Rho GTPase activating protein 26 | ARHGAP26 | non-SMC condensin I complex, subunit G | NCAPG |
| Rho GTPase activating protein 33 | ARHGAP33 | non-SMC condensin II complex, subunit G2 | NCAPG2 |
| Rho GTPase activating protein 35 | ARHGAP35 | non-SMC condensin I complex, subunit H | NCAPH |
| Rho GTPase activating protein 4 | ARHGAP4 | nuclear cap binding protein subunit 1, 80kDa | NCBP1 |
| Rho GTPase activating protein 6 | ARHGAP6 | nuclear cap binding protein subunit 2, 20kDa | NCBP2 |
| Rho GTPase activating protein 8 | ARHGAP8 | non-specific cytotoxic cell receptor protein 1 homolog (zebrafish) | NCCRP1 |
| Rho GTPase activating protein 9 | ARHGAP9 | neutral cholesterol ester hydrolase 1 | NCEH1 |
| Rho GDP dissociation inhibitor (GDI) alpha | ARHGDIA | neutrophil cytosolic factor 2 | NCF2 |
| Rho GDP dissociation inhibitor (GDI) beta | ARHGDIB | neutrophil cytosolic factor 4, 40kDa | NCF4 |
| Rho guanine nucleotide exchange factor (GEF) 1 | ARHGEF1 | NCK adaptor protein 1 | NCK1 |
| Rho guanine nucleotide exchange factor (GEF) 12 | ARHGEF12 | NCK adaptor protein 2 | NCK2 |
| Rho guanine nucleotide exchange factor (GEF) 16 | ARHGEF16 | NCK-associated protein 1 | NCKAP1 |
| Rho guanine nucleotide exchange factor (GEF) 17 | ARHGEF17 | NCK-associated protein 1-like | NCKAP1L |
| Rho/Rac guanine nucleotide exchange factor (GEF) 18 | ARHGEF18 | nucleolin | NCL |
| Rho/Rac guanine nucleotide exchange factor (GEF) 2 | ARHGEF2 | nuclear receptor coactivator 4 | NCOA4 |
| Rho guanine nucleotide exchange factor (GEF) 39 | ARHGEF39 | neuronal calcium sensor 1 | NCS1 |
| Rac/Cdc42 guanine nucleotide exchange factor (GEF) 6 | ARHGEF6 | nicastrin | NCSTN |
| Rho guanine nucleotide exchange factor (GEF) 7 | ARHGEF7 | nudE neurodevelopment protein 1 | NDE1 |
| AT rich interactive domain 1A (SWI-like) | ARID1A | necdin-like 2 | NDNL2 |
| AT rich interactive domain 5B (MRF1-like) | ARID5B | N-myc downstream regulated 1 | NDRG1 |
| ADP-ribosylation factor-like 1 | ARL1 | NDRG family member 2 | NDRG2 |
| ADP-ribosylation factor-like 15 | ARL15 | NDRG family member 3 | NDRG3 |
| ADP-ribosylation factor-like 2 | ARL2 | NADH dehydrogenase (ubiquinone) Fe-S protein 2, 49kDa (NADH-coenzyme Q reductase) | NDUFS2 |
| ADP-ribosylation factor-like 3 | ARL3 | nebulin | NEB |
| ADP-ribosylation factor-like 6 | ARL6 | nebulette | NEBL |
| ADP-ribosylation factor-like 6 interacting protein 1 | ARL61P1 | neural precursor cell expressed, developmentally down-regulated 1 | NEDD1 |
| ADP-ribosylation factor-like 6 interacting protein 5 | ARL6IP5 | neural precursor cell expressed, developmentally down-regulated 4, E3 ubiquitin protein ligase | NEDD4 |
| ADP-ribosylation factor-like 8A | ARL8A | neural precursor cell expressed, developmentally down-regulated 4-like, E3 ubiquitin protein ligase | NEDD4L |
| ADP-ribosylation factor-like 8B | ARL8B | neural precursor cell expressed, developmentally down-regulated 8 | NEDD8 |
| armadillo repeat containing 3 | ARMC3 | neural precursor cell expressed, developmentally down-regulated 9 | NEDD9 |
| armadillo repeat containing 5 | ARMC5 | neurofilament, heavy polypeptide | NEFH |
| armadillo repeat containing 6 | ARMC6 | neurofilament, light polypeptide | NEFL |
| armadillo repeat containing 8 | ARMC8 | neurofilament, medium polypeptide | NEFM |
| armadillo repeat containing 9 | ARMC9 | NIMA-related kinase 10 | NEK10 |
| age-related maculopathy susceptibility 2 | ARMS2 | NIMA-related kinase 7 | NEK7 |
| acidic residue methyltransferase 1 | ARMT1 | NIMA-related kinase 9 | NEK9 |
| actin related protein 2/3 complex, subunit 1A, 41kDa | ARPC1A | negative elongation factor complex member B | NELFB |
| actin related protein 2/3 complex, subunit 1B, 41kDa | ARPC1B | negative elongation factor complex member C/D | NELFCD |
| actin related protein 2/3 complex, subunit 2, 34kDa | ARPC2 | negative elongation factor complex member E | NELFE |
| actin related protein 2/3 complex, subunit 3, 21kDa | ARPC3 | neogenin 1 | NEO1 |
| actin related protein 2/3 complex, subunit 4, 20kDa | ARPC4 | nestin | NES |
| ARPC4-TTLL3 readthrough | ARPC4-TTLL3 | sialidase 1 (lysosomal sialidase) | NEU1 |
| actin related protein 2/3 complex, subunit 5, 16kDa | ARPC5 | neurofibromin 1 | NF1 |
| actin related protein 2/3 complex, subunit 5-like | ARPC5L | neurofibromin 2 (merlin) | NF2 |
| cAMP-regulated phosphoprotein, 19kDa | ARPP19 | nuclear factor of activated T-cells, cytoplasmic, calcineurin-dependent 1 | NFATC1 |
| arrestin 3, retinal (X-arrestin) | ARR3 | nuclear factor of activated T-cells, cytoplasmic, calcineurin-dependent 4 | NFATC4 |
| arrestin domain containing 1 | ARRDC1 | nuclear factor I/A | NFIA |
| arrestin domain containing 3 | ARRDC3 | nuclear factor, interleukin 3 regulated | NFIL3 |
| arylsulfatase B | ARSB | nuclear factor of kappa light polypeptide gene enhancer in B-cells 2 (p49/p100) | NFKB2 |
| arylsulfatase E (chondrodysplasia punctata 1) | ARSE | nerve growth factor receptor | NGFR |
| arylsulfatase F | ARSF | N-glycanase 1 | NGLY1 |
| armadillo repeat gene deleted in velocardiofacial syndrome | ARVCF | NHL repeat containing 2 | NHLRC2 |
| N-acylsphingosine amidohydrolase (acid ceramidase) 1 | ASAH1 | NHP2 ribonucleoprotein | NHP2 |
| ArfGAP with SH3 domain, ankyrin repeat and PH domain 1 | ASAP1 | NHP2 non-histone chromosome protein 2-like 1 (S. cerevisiae) | NHP2L1 |
| ArfGAP with SH3 domain, ankyrin repeat and PH domain 2 | ASAP2 | Nance-Horan syndrome (congenital cataracts and dental anomalies) | NHS |
| ankyrin repeat and SOCS box containing 15 | ASB15 | nidogen 1 | NID1 |
| activating signal cointegrator 1 complex subunit 3 | ASCC3 | nidogen 2 (osteonidogen) | NID2 |
| ash1 (absent, small, or homeotic)-like (Drosophila) | ASH1L | NIF3 NGG1 interacting factor 3-like 1 (S. cerevisiae) | NIF3L1 |
| argininosuccinate lyase | ASL | nucleolar protein interacting with the FHA domain of MKI67 | NIFK |
| acetylserotonin O-methyltransferase-like | ASMTL | ninein (GSK3B interacting protein) | NIN |
| arsA arsenite transporter, ATP-binding, homolog 1 (bacterial) | ASNA1 | ninjurin 1 | NINJ1 |
| asparagine synthetase (glutamine-hydrolyzing) | ASNS | Nipped-B homolog (Drosophila) | NIPBL |
| aspartate beta-hydroxylase | ASPH | nipsnap homolog 1 (C. elegans) | NIPSNAP1 |
| asporin | ASPN | nischarin | NISCH |
| argininosuccinate synthase 1 | ASS1 | nitrilase 1 | NIT1 |
| ATPase family, AAA domain containing 2 | ATAD2 | nitrilase family, member 2 | NIT2 |
| ATPase family, AAA domain containing 2B | ATAD2B | NFKB repressing factor | NKRF |
| ATPase family, AAA domain containing 3A | ATAD3A | NK6 homeobox 1 | NKX6-1 |
| ATPase family, AAA domain containing 3B | ATAD3B | NLR family, pyrin domain containing 8 | NLRP8 |
| ATPase family, AAA domain containing 3C | ATAD3C | NMD3 ribosome export adaptor | NMD3 |
| autophagy related 3 | ATG3 | NME/NM23 nucleoside diphosphate kinase 1 | NME1 |
| autophagy related 7 | ATG7 | NME1-NME2 readthrough | NME1-NME2 |
| autophagy related 9A | ATG9A | NME/NM23 nucleoside diphosphate kinase 2 | NME2 |
| 5-aminoimidazole-4-carboxamide ribonucleotide formyltransferase/IMP cyclohydrolase | ATIC | NME/NM23 nucleoside diphosphate kinase 2 pseudogene 1 | NME2P1 |
| atlastin GTPase 2 | ATL2 | NmrA-like family domain containing 1 | NMRAL1 |
| atlastin GTPase 3 | ATL3 | N-myristoyltransferase 1 | NMT1 |
| ATPase, class V, type 10A | ATP10A | N-myristoyltransferase 2 | NMT2 |
| ATPase, class V, type 10D | ATP10D | nucleotide-binding oligomerization domain containing 1 | NOD1 |
| ATPase, class VI, type 11A | ATP11A | nucleolar protein 9 | NOL9 |
| ATPase, class VI, type 11C | ATP11C | nucleolar and coiled-body phosphoprotein 1 | NOLC1 |
| ATPase, H+/K+ transporting, nongastric, alpha polypeptide | ATP12A | NODAL modulator 1 | NOMO1 |
| ATPase type 13A3 | ATP13A3 | NODAL modulator 2 | NOMO2 |
| ATPase, Na+/K+ transporting, alpha 1 polypeptide | ATP1A1 | NODAL modulator 3 | NOMO3 |
| ATPase, Na+/K+ transporting, alpha 2 polypeptide | ATP1A2 | non-POU domain containing, octamer-binding | NONO |
| ATPase, Na+/K+ transporting, alpha 3 polypeptide | ATP1A3 | NOP10 ribonucleoprotein | NOP10 |
| ATPase, Na+/K+ transporting, alpha 4 polypeptide | ATP1A4 | NOP14 nucleolar protein | NOP14 |
| ATPase, Na+/K+ transporting, beta 1 polypeptide | ATP1B1 | NOP16 nucleolar protein | NOP16 |
| ATPase, Na+/K+ transporting, beta 3 polypeptide | ATP1B3 | NOP2 nucleolar protein | NOP2 |
| ATPase, Ca++ transporting, cardiac muscle, slow twitch 2 | ATP2A2 | NOP56 ribonucleoprotein | NOP56 |
| ATPase, Ca++ transporting, ubiquitous | ATP2A3 | NOP58 ribonucleoprotein | NOP58 |
| ATPase, Ca++ transporting, plasma membrane 1 | ATP2B1 | NOP9 nucleolar protein | NOP9 |
| ATPase, Ca++ transporting, plasma membrane 2 | ATP2B2 | nitric oxide synthase trafficking | NOSTRIN |
| ATPase, Ca++ transporting, plasma membrane 3 | ATP2B3 | notch 1 | NOTCH1 |
| ATPase, Ca++ transporting, plasma membrane 4 | ATP2B4 | notch 2 | NOTCH2 |
| ATPase, Ca++ transporting, type 2C, member 1 | ATP2C1 | NADPH oxidase 3 | NOX3 |
| ATPase, H+/K+ exchanging, alpha polypeptide | ATP4A | Niemann-Pick disease, type C1 | NPC1 |
| ATP synthase, H+ transporting, mitochondrial F1 complex, alpha subunit 1, cardiac muscle | ATP5A1 | aminopeptidase puromycin sensitive | NPEPPS |
| ATP synthase, H+ transporting, mitochondrial F1 complex, beta polypeptide | ATP5B | nephrosis 1, congenital, Finnish type (nephrin) | NPHS1 |
| ATP synthase, H+ transporting, mitochondrial F1 complex, gamma polypeptide 1 | ATP5C1 | nephrosis 2, idiopathic, steroid-resistant (podocin) | NPHS2 |
| ATP synthase, H+ transporting, mitochondrial F1 complex, delta subunit | ATP5D | nuclear protein localization 4 homolog (S. cerevisiae) | NPLOC4 |
| ATP synthase, H+ transporting, mitochondrial Fo complex, subunit d | ATP5H | nucleophosmin (nucleolar phosphoprotein B23, numatrin) | NPM1 |
| ATP synthase, H+ transporting, mitochondrial Fo complex, subunit E | ATP5I | nucleophosmin/nucleoplasmin 3 | NPM3 |
| ATP synthase, H+ transporting, mitochondrial Fo complex, subunit G | ATP5L | nephronectin | NPNT |
| ATP synthase, H+ transporting, mitochondrial F1 complex, O subunit | ATP5O | natriuretic peptide receptor 1 | NPR1 |
| ATPase, H+ transporting, lysosomal accessory protein 1 | ATP6AP1 | natriuretic peptide receptor 3 | NPR3 |
| ATPase, H+ transporting, lysosomal accessory protein 2 | A TP6AP2 | neuroplastin | NPTN |
| ATPase, H+ transporting, lysosomal V0 subunit a1 | ATP6V0A1 | NAD(P)H dehydrogenase, quinone 1 | NQO1 |
| ATPase, H+ transporting, lysosomal V0 subunit a2 | ATP6V0A2 | NAD(P)H dehydrogenase, quinone 2 | NQO2 |
| ATPase, H+ transporting, lysosomal V0 subunit a4 | ATP6V0A4 | nuclear receptor subfamily 3, group C, member 1 (glucocorticoid receptor) | NR3C1 |
| ATPase, H+ transporting, lysosomal 16kDa, V0 subunit c | ATP6V0C | neuroblastoma RAS viral (v-ras) oncogene homolog | NRAS |
| ATPase, H+ transporting, lysosomal 38kDa, V0 subunit d1 | ATP6V0D1 | nuclear respiratory factor 1 | NRF1 |
| ATPase, H+ transporting, lysosomal 38kDa, V0 subunit d2 | ATP6V0D2 | neuregulin 2 | NRG2 |
| ATPase, H+ transporting, lysosomal 70kDa, V1 subunit A | ATP6V1A | neuropilin 1 | NRP1 |
| ATPase, H+ transporting, lysosomal 56/58kDa, V1 subunit B1 | ATP6V1B1 | neuropilin 2 | NRP2 |
| ATPase, H+ transporting, lysosomal 56/58kDa, V1 subunit B2 | ATP6V1B2 | NAD(P) dependent steroid dehydrogenase-like | NSDHL |
| ATPase, H+ transporting, lysosomal 42kDa, V1 subunit C1 | ATP6V1C1 | N-ethylmaleimide-sensitive factor | NSF |
| ATPase, H+ transporting, lysosomal 42kDa, V1 subunit C2 | ATP6V1C2 | NSFL1 (p97) cofactor (p47) | NSFL1 C |
| ATPase, H+ transporting, lysosomal 34kDa, V1 subunit D | ATP6V1D | NOP2/Sun RNA methyltransferase family, member 2 | NSUN2 |
| ATPase, H+ transporting, lysosomal 31kDa, V1 subunit E1 | ATP6V1E1 | 5', 3'-nucleotidase, cytosolic | NT5C |
| ATPase, H+ transporting, lysosomal 14kDa, V1 subunit F | ATP6V1F | 5'-nucleotidase, cytosolic II | NT5C2 |
| ATPase, H+ transporting, lysosomal 13kDa, V1 subunit G1 | ATP6V1G1 | 5'-nucleotidase domain containing 1 | NT5DC1 |
| ATPase, H+ transporting, lysosomal 50/57kDa, V1 subunit H | ATP6V1H | 5'-nucleotidase, ecto (CD73) | NT5E |
| ATPase, Cu++ transporting, alpha polypeptide | ATP7A | N-terminal Xaa-Pro-Lys N-methyltransferase 1 | NTMT1 |
| ATPase, Cu++ transporting, beta polypeptide | ATP7B | nucleoside-triphosphatase, cancer-related | NTPCR |
| ATPase, aminophospholipid transporter (APLT), class I, type 8A, member 1 | ATP8A1 | neurotrophic tyrosine kinase, receptor, type 2 | NTRK2 |
| ATPase, aminophospholipid transporter, class I, type 8A, member 2 | ATP8A2 | negative regulator of ubiquitin-like proteins 1 | NUB1 |
| ATPase, aminophospholipid transporter, class I, type 8B, member 1 | ATP8B1 | nucleotide binding protein 2 | NUBP2 |
| ATPase, aminophospholipid transporter, class I, type 8B, member 3 | ATP8B3 | nucleobindin 1 | NUCB1 |
| ATPase, class II, type 9A | ATP9A | nucleobindin 2 | NUCB2 |
| ATPase, class II, type 9B | ATP9B | nuclear casein kinase and cyclin-dependent kinase substrate 1 | NUCKS1 |
| attractin | ATRN | nudC nuclear distribution protein | NUDC |
| ataxin 1 | ATXN1 | NudC domain containing 1 | NUDCD1 |
| ataxin 10 | ATXN10 | NudC domain containing 2 | NUDCD2 |
| ataxin 2-like | ATXN2L | nudix (nucleoside diphosphate linked moiety X)-type motif 16-like 1 | NUDT16L1 |
| AXL receptor tyrosine kinase | AXL | nudix (nucleoside diphosphate linked moiety X)-type motif 21 | NUDT21 |
| alpha-2-glycoprotein 1, zinc-binding | AZGP1 | nudix (nucleoside diphosphate linked moiety X)-type motif 4 | NUDT4 |
| alpha-2-glycoprotein 1, zinc-binding pseudogene 1 | AZGP1P1 | nudix (nucleoside diphosphate linked moiety X)-type motif 5 | NUDT5 |
| azurocidin 1 | AZU1 | nudix (nucleoside diphosphate linked moiety X)-type motif 9 | NUDT9 |
| beta-2-microglobulin | B2M | nuclear mitotic apparatus protein 1 | NUMA1 |
| beta-1,3-glucuronyltransferase 3 | B3GAT3 | numb homolog (Drosophila) | NUMB |
| UDP-GlcNAc:betaGal beta-1,3-N-acetylglucosaminyltransferase 2 | B3GNT2 | numb homolog (Drosophila)-like | NUMBL |
| UDP-Gal:betaGlcNAc beta 1,4-galactosyltransferase, polypeptide 1 | B4GALT1 | nucleoporin 160kDa | NUP160 |
| UDP-Gal:betaGlcNAc beta 1,4-galactosyltransferase, polypeptide 3 | B4GALT3 | nucleoporin 205kDa | NUP205 |
| UDP-Gal:betaGlcNAc beta 1,4-galactosyltransferase, polypeptide 4 | B4GALT4 | nucleoporin 210kDa | NUP210 |
| UDP-Gal:betaGlcNAc beta 1,4-galactosyltransferase, polypeptide 5 | B4GALT5 | nucleoporin 43kDa | NUP43 |
| xylosylprotein beta 1,4-galactosyltransferase, polypeptide 7 | B4GALT7 | nucleoporin 88kDa | NUP88 |
| BRISC and BRCA1 A complex member 1 | BABAM1 | nucleoporin 93kDa | NUP93 |
| beta-site APP-cleaving enzyme 2 | BACE2 | nucleoporin like 2 | NUPL2 |
| BTB and CNC homology 1, basic leucine zipper transcription factor 2 | BACH2 | nucleolar and spindle associated protein 1 | NUSAP1 |
| BCL2-associated athanogene | BAG1 | nuclear transport factor 2 | NUTF2 |
| BCL2-associated athanogene 2 | BAG2 | nuclear RNA export factor 1 | NXF1 |
| BCL2-associated athanogene 5 | BAG5 | nucleoredoxin | NXN |
| BCL2-associated athanogene 6 | BAG6 | neurexophilin and PC-esterase domain family, member 4 | NXPE4 |
| BAI1-associated protein 2 | BAIAP2 | O-acyl-ADP-ribose deacylase 1 | OARD1 |
| BAI1-associated protein 2-like 1 | BAIAP2L1 | 2'-5'-oligoadenylate synthetase 2, 69/71 kDa | OAS2 |
| BAI1-associated protein 2-like 2 | BAIAP2L2 | 2'-5'-oligoadenylate synthetase 3, 100kDa | OAS3 |
| barrier to autointegration factor 1 | BANF1 | ornithine aminotransferase | OAT |
| brain abundant, membrane attached signal protein 1 | BASP1 | oligonucleotide/oligosaccharid e-binding fold containing 1 | OBFC1 |
| BCL2-associated X protein | BAX | odorant binding protein 2A | OBP2A |
| bromodomain adjacent to zinc finger domain, 1B | BAZ1B | obscurin, cytoskeletal calmodulin and titin-interacting RhoGEF | OBSCN |
| butyrobetaine (gamma), 2-oxoglutarate dioxygenase (gamma-butyrobetaine hydroxylase) 1 | BBOX1 | OCIA domain containing 2 | OCIAD2 |
| Bardet-Biedl syndrome 4 | BBS4 | occludin | OCLN |
| basal cell adhesion molecule (Lutheran blood group) | BCAM | oculocerebrorenal syndrome of Lowe | OCRL |
| B-cell receptor-associated protein 31 | BCAP31 | oral-facial-digital syndrome 1 | OFD1 |
| breast carcinoma amplified sequence 2 | BCAS2 | opioid growth factor receptor | OGFR |
| branched chain amino-acid transaminase 1, cytosolic | BCAT1 | osteoglycin | OGN |
| BRCA2 and CDKN1A interacting protein | BCCIP | O-linked N-acetylglucosamine (GlcNAc) transferase | OGT |
| butyrylcholinesterase | BCHE | oncoprotein induced transcript 3 | OIT3 |
| B-cell CLL/lymphoma 11A (zinc finger protein) | BCL11A | Obg-like ATPase 1 | OLA1 |
| BCL2-like 12 (proline rich) | BCL2L12 | olfactomedin 4 | OLFM4 |
| breakpoint cluster region | BCR | olfactomedin-like 3 | OLFML3 |
| 3-hydroxybutyrate dehydrogenase, type 2 | BDH2 | oxidized low density lipoprotein (lectin-like) receptor 1 | OLR1 |
| brain-derived neurotrophic factor | BDNF | optic atrophy 1 (autosomal dominant) | OPA1 |
| BEN domain containing 7 | BEND7 | optic atrophy 3 (autosomal recessive, with chorea and spastic paraplegia) | OPA3 |
| biglycan | BGN | opiate receptor-like 1 | OPRL1 |
| basic helix-loop-helix domain containing, class B, 9 | BHLHB9 | opioid receptor, mu 1 | OPRM1 |
| betaine--homocysteine S-methyltransferase | BHMT | optineurin | OPTN |
| betaine--homocysteine S-methyltransferase 2 | BHMT2 | olfactory receptor, family 11, subfamily L, member 1 | OR11L1 |
| BicC family RNA binding protein 1 | BICC1 | olfactory receptor, family 2, subfamily A, member 4 | OR2A4 |
| bridging integrator 1 | BIN1 | olfactory receptor, family 2, subfamily T, member 8 | OR2T8 |
| bridging integrator 2 | BIN2 | ORAI calcium release-activated calcium modulator 1 | ORAI1 |
| BLK proto-oncogene, Src family tyrosine kinase | BLK | orosomucoid 1 | ORM1 |
| Bloom syndrome, RecQ helicase-like | BLM | ORMDL sphingolipid biosynthesis regulator 1 | ORMDL1 |
| bleomycin hydrolase | BLMH | ORMDL sphingolipid biosynthesis regulator 2 | ORMDL2 |
| biogenesis of lysosomal organelles complex-1, subunit 5, muted | BLOC1S5 | ORMDL sphingolipid biosynthesis regulator 3 | ORMDL3 |
| biliverdin reductase A | BLVRA | osteosarcoma amplified 9, endoplasmic reticulum lectin | OS9 |
| biliverdin reductase B | BLVRB | oxysterol binding protein | OSBP |
| bone morphogenetic protein 15 | BMP15 | oxysterol binding protein-like 1A | OSBPL1A |
| bone morphogenetic protein 3 | BMP3 | O-sialoglycoprotein endopeptidase | OSGEP |
| bone morphogenetic protein 4 | BMP4 | oncostatin M | OSM |
| bone morphogenetic protein 7 | BMP7 | oncostatin M receptor | OSMR |
| bone morphogenetic protein receptor, type II (serine/threonine kinase) | BMPR2 | oligosaccharyltransferase complex subunit (non-catalytic) | OSTC |
| bolA family member 2 | BOLA2 | osteoclast stimulating factor 1 | OSTF1 |
| bolA family member 2B | BOLA2B | ornithine carbamoyltransferase | OTC |
| block of proliferation 1 | BOP1 | OTU deubiquitinase, ubiquitin aldehyde binding 1 | OTUB1 |
| bactericidal/permeability-increasing protein | BPI | OTU deubiquitinase 7A | OTUD7A |
| BPI fold containing family A, member 1 | BPIFA1 | OTU deubiquitinase 7B | OTUD7B |
| BPI fold containing family A, member 2 | BPIFA2 | oxidoreductase NAD-binding domain containing 1 | OXNAD1 |
| BPI fold containing family B, member 1 | BPIFB1 | oxidative stress responsive 1 | OXSR1 |
| 3'(2'),5'-bisphosphate nucleotidase 1 | BPNT1 | oxytocin receptor | OXTR |
| BRCA1-associated ATM activator 1 | BRAT1 | purinergic receptor P2X, ligand gated ion channel, 1 | P2RX1 |
| breast cancer 2, early onset | BRCA2 | purinergic receptor P2X, ligand gated ion channel, 4 | P2RX4 |
| bromodomain containing 4 | BRD4 | purinergic receptor P2X, ligand gated ion channel, 5 | P2RX5 |
| brain and reproductive organ-expressed (TNFRSF1A modulator) | BRE | purinergic receptor P2Y, G-protein coupled, 12 | P2RY12 |
| BRI3 binding protein | BRI3BP | purinergic receptor P2Y, G-protein coupled, 2 | P2RY2 |
| BRX1, biogenesis of ribosomes | BRIX1 | prolyl 3-hydroxylase 1 | P3H1 |
| BRICK1, SCAR/WAVE actin-nucleating complex subunit | BRK1 | prolyl 4-hydroxylase, alpha polypeptide I | P4HA1 |
| breast cancer metastasis suppressor 1 | BRMS1 | prolyl 4-hydroxylase, beta polypeptide | P4HB |
| BRO1 domain and CAAX motif containing | BROX | proliferation-associated 2G4, 38kDa | PA2G4 |
| basigin (Ok blood group) | BSG | poly(A) binding protein, cytoplasmic 1 | PABPC1 |
| bone marrow stromal cell antigen 1 | BST1 | poly(A) binding protein, cytoplasmic 1-like | PABPC1L |
| bone marrow stromal cell antigen 2 | BST2 | poly(A) binding protein, cytoplasmic 3 | PABPC3 |
| BTAF1 RNA polymerase II, B-TFIID transcription factor-associated, 170kDa | BTAF1 | poly(A) binding protein, cytoplasmic 4 (inducible form) | PABPC4 |
| basic transcription factor 3 | BTF3 | poly(A) binding protein, cytoplasmic 5 | PABPC5 |
| B-cell translocation gene 1, antiproliferative | BTG1 | protein kinase C and casein kinase substrate in neurons 2 | PACSIN2 |
| BTG family, member 2 | BTG2 | protein kinase C and casein kinase substrate in neurons 3 | PACSIN3 |
| Bruton agammaglobulinemia tyrosine kinase | BTK | peptidyl arginine deiminase, type II | PADI2 |
| butyrophilin, subfamily 1, member A1 | BTN1A1 | Paf1, RNA polymerase II associated factor, homolog (S. cerevisiae) | PAF1 |
| butyrophilin, subfamily 2, member A1 | BTN2A1 | platelet-activating factor acetylhydrolase 1b, regulatory subunit 1 (45kDa) | PAFAH1B1 |
| butyrophilin, subfamily 3, member A1 | BTN3A1 | platelet-activating factor acetylhydrolase 1b, catalytic subunit 2 (30kDa) | PAFAH1B2 |
| butyrophilin, subfamily 3, member A2 | BTN3A2 | platelet-activating factor acetylhydrolase 1b, catalytic subunit 3 (29kDa) | PAFAH1B3 |
| butyrophilin, subfamily 3, member A3 | BTN3A3 | P antigen family, member 2 (prostate associated) | PAGE2 |
| BUB3 mitotic checkpoint protein | BUB3 | phenylalanine hydroxylase | PAH |
| basic leucine zipper and W2 domains 1 | BZW1 | phosphoribosylaminoimidazol e carboxylase, phosphoribosylaminoimidazol e succinocarboxamide synthetase | PAICS |
| basic leucine zipper and W2 domains 2 | BZW2 | p21 protein (Cdc42/Rac)-activated kinase 2 | PAK2 |
| chromosome 10 open reading frame 54 | C10orf54 | p21 protein (Cdc42/Rac)-activated kinase 4 | PAK4 |
| chromosome 10 open reading frame 90 | C10orf90 | p21 protein (Cdc42/Rac)-activated kinase 6 | PAK6 |
| chromosome 11 open reading frame 52 | C11orf52 | phosphatase domain containing, paladin 1 | PALD1 |
| chromosome 11 open reading frame 54 | C11orf54 | paralemmin | PALM |
| chromosome 12 open reading frame 10 | C12orf10 | peptidylglycine alpha-amidating monooxygenase | PAM |
| chromosome 12 open reading frame 57 | C12orf57 | peptidase domain containing associated with muscle regeneration 1 | PAMR1 |
| chromosome 14 open reading frame 1 | C14orf1 | PAN3 poly(A) specific ribonuclease subunit | PAN3 |
| chromosome 14 open reading frame 166 | C14orf166 | pantothenate kinase 2 | PANK2 |
| chromosome 15 open reading frame 52 | C15orf52 | pantothenate kinase 4 | PANK4 |
| chromosome 16 open reading frame 13 | C16orf13 | pannexin 1 | PANX1 |
| chromosome 16 open reading frame 54 | C16orf54 | pregnancy-associated plasma protein A, pappalysin 1 | PAPPA |
| chromosome 16 open reading frame 62 | C16orf62 | PAPPA antisense RNA 1 | PAPPA-AS1 |
| chromosome 16 open reading frame 87 | C16orf87 | 3'-phosphoadenosine 5'-phosphosulfate synthase 1 | PAPSS1 |
| chromosome 16 open reading frame 89 | C16orf89 | 3'-phosphoadenosine 5'-phosphosulfate synthase 2 | PAPSS2 |
| chromosome 17 open reading frame 75 | C17orf75 | par-3 family cell polarity regulator | PARD3 |
| chromosome 17 open reading frame 80 | C17orf80 | par-6 family cell polarity regulator beta | PARD6B |
| chromosome 19 open reading frame 18 | C19orf18 | parkinson protein 7 | PARK7 |
| C1GALT1-specific chaperone 1 | C1GALT1C1 | poly (ADP-ribose) polymerase 1 | PARP1 |
| chromosome 1 open reading frame 116 | C1orf116 | poly (ADP-ribose) polymerase family, member 10 | PARP10 |
| chromosome 1 open reading frame 198 | C1orf198 | poly (ADP-ribose) polymerase family, member 12 | PARP12 |
| complement component 1, q subcomponent, A chain | C1QA | poly (ADP-ribose) polymerase family, member 14 | PARP14 |
| complement component 1, q subcomponent, B chain | C1QB | poly (ADP-ribose) polymerase family, member 16 | PARP16 |
| complement component 1, q subcomponent binding protein | C1QBP | poly (ADP-ribose) polymerase family, member 4 | PARP4 |
| complement component 1, q subcomponent, C chain | C1QC | poly (ADP-ribose) polymerase family, member 9 | PARP9 |
| C1q and tumor necrosis factor related protein 1 | C1QTNF1 | parvin, alpha | PARVA |
| C1q and tumor necrosis factor related protein 3 | C1QTNF3 | parvin, beta | PARVB |
| complement component 1, r subcomponent | C1R | parvin, gamma | PARVG |
| complement component 1, r subcomponent-like | C1RL | protein associated with topoisomerase II homolog 1 (yeast) | PATL1 |
| complement component 1, s subcomponent | C1S | phenazine biosynthesis-like protein domain containing | PBLD |
| chromosome 21 open reading frame 59 | C21orf59 | polybromo 1 | PBRM1 |
| C2 calcium-dependent domain containing 5 | C2CD5 | pyruvate carboxylase | PC |
| chromosome 2 open reading frame 16 | C2orf16 | pterin-4 alpha-carbinolamine dehydratase/dimerization cofactor of hepatocyte nuclear factor 1 alpha | PCBD1 |
| chromosome 2 open reading frame 74 | C2orf74 | poly(rC) binding protein 1 | PCBP1 |
| chromosome 2 open reading frame 88 | C2orf88 | poly(rC) binding protein 2 | PCBP2 |
| complement component 3 | C3 | poly(rC) binding protein 3 | PCBP3 |
| complement component 4A (Rodgers blood group) | C4A | protocadherin 1 | PCDH1 |
| complement component 4B (Chido blood group) | C4B | protocadherin gamma subfamily B, 5 | PCDHGB5 |
| complement component 4 binding protein, alpha | C4BPA | PCF11 cleavage and polyadenylation factor subunit | PCF11 |
| complement component 4 binding protein, beta | C4BPB | PCI domain containing 2 | PCID2 |
| complement component 5 | C5 | PDX1 C-terminal inhibiting factor 1 | PCIF1 |
| chromosome 5 open reading frame 15 | C5orf15 | phosphoenolpyruvate carboxykinase 1 (soluble) | PCK1 |
| chromosome 5 open reading frame 24 | C5orf24 | piccolo presynaptic cytomatrix protein | PCLO |
| chromosome 5 open reading frame 46 | C5orf46 | protein-L-isoaspartate (D-aspartate) O-methyltransferase | PCMT1 |
| chromosome 5 open reading frame 51 | C5orf51 | proliferating cell nuclear antigen | PCNA |
| complement component 6 | C6 | procollagen C-endopeptidase enhancer | PCOLCE |
| chromosome 6 open reading frame 10 | C6orf10 | proprotein convertase subtilisin/kexin type 6 | PCSK6 |
| chromosome 6 open reading frame 163 | C6orf163 | proprotein convertase subtilisin/kexin type 9 | PCSK9 |
| complement component 7 | C7 | prenylcysteine oxidase 1 | PCYOX1 |
| chromosome 7 open reading frame 50 | C7orf50 | phosphate cytidylyltransferase 1, choline, beta | PCYT1B |
| complement component 8, alpha polypeptide | C8A | phosphate cytidylyltransferase 2, ethanolamine | PCYT2 |
| complement component 8, gamma polypeptide | C8G | programmed cell death 10 | PDCD10 |
| chromosome 8 open reading frame 33 | C8orf33 | programmed cell death 2 | PDCD2 |
| complement component 9 | C9 | programmed cell death 2-like | PDCD2L |
| chromosome 9 open reading frame 64 | C9orf64 | programmed cell death 4 (neoplastic transformation inhibitor) | PDCD4 |
| chromosome 9 open reading frame 91 | C9orf91 | programmed cell death 5 | PDCD5 |
| carbonic anhydrase I | CA1 | programmed cell death 6 | PDCD6 |
| carbonic anhydrase XII | CA12 | programmed cell death 6 interacting protein (Apoptosis-Linked Gene 2-Interacting Protein X | PDCD6IP, ALIX |
| carbonic anhydrase XIV | CA14 | Parkinson disease 7 domain containing 1 | PDDC1 |
| carbonic anhydrase II | CA2 | phosphodiesterase 12 | PDE12 |
| carbonic anhydrase IV | CA4 | phosphodiesterase 1C, calmodulin-dependent 70kDa | PDE1C |
| carbonic anhydrase VI | CA6 | phosphodiesterase 4D interacting protein | PDE4DIP |
| carbonic anhydrase IX | CA9 | phosphodiesterase 5A, cGMP-specific | PDE5A |
| calcium binding protein 39 | CAB39 | phosphodiesterase 8A | PDE8A |
| calcium binding protein 39-like | CAB39L | platelet-derived growth factor alpha polypeptide | PDGFA |
| calcium binding protein 1 | CABP1 | platelet derived growth factor C | PDGFC |
| calcium channel, voltage-dependent, R type, alpha 1E subunit | CACNA1E | platelet-derived growth factor receptor, beta polypeptide | PDGFRB |
| calcium channel, voltage-dependent, L type, alpha 1S subunit | CACNA1S | pyruvate dehydrogenase (lipoamide) alpha 1 | PDHA1 |
| calcium channel, voltage-dependent, alpha 2/delta subunit 1 | CACNA2D1 | protein disulfide isomerase family A, member 2 | PDIA2 |
| calcium channel, voltage-dependent, alpha 2/delta subunit 2 | CACNA2D2 | protein disulfide isomerase family A, member 3 | PDIA3 |
| calcium channel, voltage-dependent, alpha 2/delta subunit 4 | CACNA2D4 | protein disulfide isomerase family A, member 4 | PDIA4 |
| calcyclin binding protein | CACYBP | protein disulfide isomerase family A, member 5 | PDIA5 |
| carbamoyl-phosphate synthetase 2, aspartate transcarbamylase, and dihydroorotase | CAD | protein disulfide isomerase family A, member 6 | PDIA6 |
| cell adhesion molecule 1 | CADM1 | PDZ and LIM domain 1 | PDLIM1 |
| cell adhesion molecule 4 | CADM4 | PDZ and LIM domain 5 | PDLIM5 |
| calbindin 1, 28kDa | CALB1 | PDZ and LIM domain 7 (enigma) | PDLIM7 |
| calmodulin 1 (phosphorylase kinase, delta) | CALM1 | PDS5 cohesin associated factor A | PDS5A |
| calmodulin 2 (phosphorylase kinase, delta) | CALM2 | PDS5 cohesin associated factor B | PDS5B |
| calmodulin 3 (phosphorylase kinase, delta) | CALM3 | pyridoxal-dependent decarboxylase domain containing 1 | PDXDC1 |
| calmodulin-like 3 | CALML3 | pyridoxal (pyridoxine, vitamin B6) kinase | PDXK |
| calmodulin-like 5 | CALML5 | PDZ domain containing 1 | PDZK1 |
| calreticulin | CALR | PDZK1 interacting protein 1 | PDZK1IP1 |
| calumenin | CALU | pseudopodium-enriched atypical kinase 1 | PEAK1 |
| calcium/calmodulin-dependent protein kinase II delta | CAMK2D | platelet endothelial aggregation receptor 1 | PEAR1 |
| calcium/calmodulin-dependent protein kinase II gamma | CAMK2G | phosphatidylethanolamine binding protein 1 | PEBP1 |
| calcium/calmodulin-dependent protein kinase IV | CAMK4 | platelet/endothelial cell adhesion molecule 1 | PECAM1 |
| calcium/calmodulin-dependent protein kinase kinase 2, beta | CAMKK2 | penta-EF-hand domain containing 1 | PEF1 |
| CaM kinase-like vesicle-associated | CAMKV | pellino E3 ubiquitin protein ligase 1 | PELI1 |
| cathelicidin antimicrobial peptide | CAMP | pellino E3 ubiquitin protein ligase family member 2 | PELI2 |
| calmodulin binding transcription activator 1 | CAMTA1 | pelota homolog (Drosophila) | PELO |
| cullin-associated and neddylation-dissociated 1 | CAND1 | proline, glutamate and leucine rich protein 1 | PELP1 |
| cullin-associated and neddylation-dissociated 2 (putative) | CAND2 | peptidase D | PEPD |
| calcium activated nucleotidase 1 | CANT1 | pescadillo ribosomal biogenesis factor 1 | PES1 |
| calnexin | CANX | peroxisomal biogenesis factor 1 | PEX1 |
| CAP, adenylate cyclase-associated protein 1 (yeast) | CAP1 | peroxisomal biogenesis factor 3 | PEX3 |
| capping protein (actin filament), gelsolin-like | CAPG | phosphoribosylformylglycinam idine synthase | PFAS |
| calpain 1, (mu/l) large subunit | CAPN1 | prefoldin subunit 2 | PFDN2 |
| calpain 2, (m/ll) large subunit | CAPN2 | prefoldin subunit 4 | PFDN4 |
| calpain 5 | CAPN5 | prefoldin subunit 5 | PFDN5 |
| calpain 7 | CAPN7 | 6-phosphofructo-2-kinase/fructose-2,6-biphosphatase 2 | PFKFB2 |
| calpain, small subunit 1 | CAPNS1 | 6-phosphofructo-2-kinase/fructose-2,6-biphosphatase 3 | PFKFB3 |
| calpain, small subunit 2 | CAPNS2 | phosphofructokinase, liver | PFKL |
| cell cycle associated protein 1 | CAPRIN1 | phosphofructokinase, muscle | PFKM |
| calcyphosine | CAPS | phosphofructokinase, platelet | PFKP |
| calcyphosine 2 | CAPS2 | profilin 1 | PFN1 |
| capping protein (actin filament) muscle Z-line, alpha 1 | CAPZA1 | profilin 2 | PFN2 |
| capping protein (actin filament) muscle Z-line, alpha 2 | CAPZA2 | phosphoglycerate mutase 1 (brain) | PGAM1 |
| capping protein (actin filament) muscle Z-line, beta | CAPZB | phosphoglycerate mutase 2 (muscle) | PGAM2 |
| caspase recruitment domain family, member 9 | CARD9 | phosphoglycerate mutase family member 4 | PGAM4 |
| calcium regulated heat stable protein 1, 24kDa | CARHSP1 | phosphoglycerate mutase family member 5 | PGAM5 |
| carbohydrate kinase domain containing | CARKD | phosphogluconate dehydrogenase | PGD |
| cysteinyl-tRNA synthetase | CARS | phosphoglycerate kinase 1 | PGK1 |
| cancer susceptibility candidate 4 | CASC4 | phosphoglycerate kinase 2 | PGK2 |
| calcium/calmodulin-dependent serine protein kinase (MAGUK family) | CASK | 6-phosphogluconolactonase | PGLS |
| caspase 14, apoptosis-related cysteine peptidase | CASP14 | peptidoglycan recognition protein 1 | PGLYRP1 |
| caspase 3, apoptosis-related cysteine peptidase | CASP3 | peptidoglycan recognition protein 2 | PGLYRP2 |
| caspase 6, apoptosis-related cysteine peptidase | CASP6 | phosphoglucomutase 1 | PGM1 |
| caspase 9, apoptosis-related cysteine peptidase | CASP9 | phosphoglucomutase 2 | PGM2 |
| catalase | CAT | phosphoglucomutase 2-like 1 | PGM2L1 |
| caveolin 1, caveolae protein, 22kDa | CAV1 | phosphoglucomutase 3 | PGM3 |
| caveolin 2 | CAV2 | phosphoglucomutase 5 | PGM5 |
| Cbl proto-oncogene, E3 ubiquitin protein ligase | CBL | progesterone receptor membrane component 1 | PGRMC1 |
| carbonyl reductase 1 | CBR1 | progesterone receptor membrane component 2 | PGRMC2 |
| carbonyl reductase 3 | CBR3 | phosphatase and actin regulator 4 | PHACTR4 |
| cystathionine-beta-synthase | CBS | prohibitin | PHB |
| chromobox homolog 1 | CBX1 | prohibitin 2 | PHB2 |
| chromobox homolog 3 | CBX3 | PHD finger protein 23 | PHF23 |
| chromobox homolog 5 | CBX5 | PHD finger protein 5A | PHF5A |
| chromobox homolog 8 | CBX8 | phosphoglycerate dehydrogenase | PHGDH |
| coiled-coil and C2 domain containing 1A | CC2D1A | phosphorylase kinase, alpha 1 (muscle) | PHKA1 |
| coiled-coil and C2 domain containing 1B | CC2D1B | phosphorylase kinase, alpha 2 (liver) | PHKA2 |
| cell division cycle and apoptosis regulator 1 | CCAR1 | phosphorylase kinase, beta | PHKB |
| cell cycle and apoptosis regulator 2 | CCAR2 | pleckstrin homology-like domain, family A, member 1 | PHLDA1 |
| cysteine conjugate-beta lyase 2 | CCBL2 | pleckstrin homology-like domain, family A, member 2 | PHLDA2 |
| coiled-coil domain containing 105 | CCDC105 | pleckstrin homology-like domain, family B, member 1 | PHLDB1 |
| coiled-coil domain containing 115 | CCDC115 | pleckstrin homology-like domain, family B, member 2 | PHLDB2 |
| coiled-coil domain containing 129 | CCDC129 | phosphohistidine phosphatase 1 | PHPT1 |
| coiled-coil domain containing 132 | CCDC132 | phosphatidylinositol 4-kinase type 2 alpha | PI4K2A |
| coiled-coil domain containing 158 | CCDC158 | phosphatidylinositol 4-kinase, catalytic, alpha | PI4KA |
| coiled-coil domain containing 171 | CCDC171 | phosphatidylinositol 4-kinase, catalytic, beta | PI4KB |
| coiled-coil domain containing 175 | CCDC175 | phosphatidylinositol binding clathrin assembly protein | PICALM |
| coiled-coil domain containing 22 | CCDC22 | polymeric immunoglobulin receptor | PIGR |
| coiled-coil domain containing 25 | CCDC25 | phosphatidylinositol-4-phosphate 3-kinase, catalytic subunit type 2 alpha | PIK3C2A |
| coiled-coil domain containing 33 | CCDC33 | phosphatidylinositol-4-phosphate 3-kinase, catalytic subunit type 2 beta | PIK3C2B |
| coiled-coil domain containing 47 | CCDC47 | phosphatidylinositol 3-kinase, catalytic subunit type 3 | PIK3C3 |
| coiled-coil domain containing 50 | CCDC50 | phosphatidylinositol-4,5-bisphosphate 3-kinase, catalytic subunit alpha | PIK3CA |
| coiled-coil domain containing 64B | CCDC64B | phosphatidylinositol-4,5-bisphosphate 3-kinase, catalytic subunit beta | PIK3CB |
| coiled-coil domain containing 88B | CCDC88B | phosphoinositide-3-kinase, regulatory subunit 1 (alpha) | PIK3R1 |
| coiled-coil domain containing 93 | CCDC93 | phosphoinositide-3-kinase, regulatory subunit 4 | PIK3R4 |
| chemokine (C-C motif) ligand 2 | CCL2 | paired immunoglobin-like type 2 receptor alpha | PILRA |
| chemokine (C-C motif) ligand 20 | CCL20 | peptidylprolyl cis/trans isomerase, NIMA-interacting 1 | PIN1 |
| chemokine (C-C motif) ligand 22 | CCL22 | peptidylprolyl cis/trans isomerase, NIMA-interacting 4 | PIN4 |
| chemokine (C-C motif) ligand 28 | CCL28 | prolactin-induced protein | PIP |
| chemokine (C-C motif) ligand 7 | CCL7 | phosphatidylinositol-5-phosphate 4-kinase, type II, alpha | PIP4K2A |
| cerebral cavernous malformation 2 | CCM2 | phosphatidylinositol-5-phosphate 4-kinase, type II, beta | PIP4K2B |
| cyclin D-type binding-protein 1 | CCNDBP1 | phosphatidylinositol-5-phosphate 4-kinase, type II, gamma | PIP4K2C |
| cyclin Y | CCNY | phosphatidylinositol-4-phosphate 5-kinase, type I, alpha | PIP5K1A |
| cyclin Y-like 1 | CCNYL1 | pirin (iron-binding nuclear protein) | PIR |
| cell cycle progression 1 | CCPG1 | PITH (C-terminal proteasome-interacting domain of thioredoxin-like) domain containing 1 | PITHD1 |
| chemokine (C-C motif) receptor 4 | CCR4 | phosphatidylinositol transfer protein, alpha | PITPNA |
| chemokine (C-C motif) receptor 5 (gene/pseudogene) | CCR5 | phosphatidylinositol transfer protein, beta | PITPNB |
| copper chaperone for superoxide dismutase | CCS | phosphatidylinositol transfer protein, membrane-associated 1 | PITPNM1 |
| chaperonin containing TCP1, subunit 2 (beta) | CCT2 | polycystic kidney disease 1 (autosomal dominant) | PKD1 |
| chaperonin containing TCP1, subunit 3 (gamma) | CCT3 | polycystic kidney disease 1-like 3 | PKD1L3 |
| chaperonin containing TCP1, subunit 4 (delta) | CCT4 | polycystic kidney disease 2 (autosomal dominant) | PKD2 |
| chaperonin containing TCP1, subunit 5 (epsilon) | CCT5 | polycystic kidney disease 2-like 2 | PKD2L2 |
| chaperonin containing TCP1, subunit 6A (zeta 1) | CCT6A | polycystic kidney and hepatic disease 1 (autosomal recessive) | PKHD1 |
| chaperonin containing TCP1, subunit 6B (zeta 2) | CCT6B | polycystic kidney and hepatic disease 1 (autosomal recessive)-like 1 | PKHD1L1 |
| chaperonin containing TCP1, subunit 7 (eta) | CCT7 | pyruvate kinase, liver and RBC | PKLR |
| chaperonin containing TCP1, subunit 8 (theta) | CCT8 | pyruvate kinase, muscle | PKM |
| CD101 molecule | CD101 | protein kinase N2 | PKN2 |
| CD109 molecule | CD109 | protein kinase N3 | PKN3 |
| CD14 molecule | CD14 | plakophilin 2 | PKP2 |
| CD151 molecule (Raph blood group) | CD151 | plakophilin 3 | PKP3 |
| CD163 molecule | CD163 | plakophilin 4 | PKP4 |
| CD163 molecule-like 1 | CD163L1 | phospholipase A2, group IIA (platelets, synovial fluid) | PLA2G2A |
| CD19 molecule | CD19 | phospholipase A2-activating protein | PLAA |
| CD1a molecule | CD1A | plasminogen activator, tissue | PLAT |
| CD1b molecule | CD1B | plasminogen activator, urokinase | PLAU |
| CD1c molecule | CD1C | plasminogen activator, urokinase receptor | PLAUR |
| CD2 molecule | CD2 | phospholipase B domain containing 2 | PLBD2 |
| CD200 molecule | CD200 | phospholipase C, beta 1 (phosphoinositide-specific) | PLCB1 |
| CD209 molecule | CD209 | phospholipase C, beta 3 (phosphatidylinositol-specific) | PLCB3 |
| CD22 molecule | CD22 | phospholipase C, beta 4 | PLCB4 |
| CD226 molecule | CD226 | phospholipase C, delta 1 | PLCD1 |
| CD24 molecule | CD24 | phospholipase C, delta 3 | PLCD3 |
| CD247 molecule | CD247 | phospholipase C, epsilon 1 | PLCE1 |
| CD248 molecule, endosialin | CD248 | phospholipase C, gamma 1 | PLCG1 |
| CD274 molecule | CD274 | phospholipase C, gamma 2 (phosphatidylinositol-specific) | PLCG2 |
| CD276 molecule | CD276 | phospholipase C-like 1 | PLCL1 |
| CD2-associated protein | CD2AP | phospholipase C-like 2 | PLCL2 |
| CD2 (cytoplasmic tail) binding protein 2 | CD2BP2 | phospholipase D1, phosphatidylcholine-specific | PLD1 |
| CD300a molecule | CD300A | phospholipase D family, member 3 | PLD3 |
| CD320 molecule | CD320 | plectin | PLEC |
| CD33 molecule | CD33 | pleckstrin | PLEK |
| CD36 molecule (thrombospondin receptor) | CD36 | pleckstrin 2 | PLEK2 |
| CD37 molecule | CD37 | pleckstrin homology domain containing, family A (phosphoinositide binding specific) member 1 | PLEKHA1 |
| CD38 molecule | CD38 | pleckstrin homology domain containing, family A (phosphoinositide binding specific) member 4 | PLEKHA4 |
| CD3d molecule, delta (CD3-TCR complex) | CD3D | pleckstrin homology domain containing, family A member 7 | PLEKHA7 |
| CD3e molecule, epsilon (CD3-TCR complex) | CD3E | pleckstrin homology domain containing, family B (evectins) member 2 | PLEKHB2 |
| CD3g molecule, gamma (CD3-TCR complex) | CD3G | pleckstrin homology domain containing, family F (with FYVE domain) member 1 | PLEKHF1 |
| CD4 molecule | CD4 | pleckstrin homology domain containing, family F (with FYVE domain) member 2 | PLEKHF2 |
| CD40 molecule, TNF receptor superfamily member 5 | CD40 | pleckstrin homology domain containing, family G (with RhoGef domain) member 3 | PLEKHG3 |
| CD40 ligand | CD40LG | pleckstrin homology domain containing, family G (with RhoGef domain) member 4B | PLEKHG4B |
| CD44 molecule (Indian blood group) | CD44 | pleckstrin homology domain containing, family O member 2 | PLEKHO2 |
| CD46 molecule, complement regulatory protein | CD46 | plasminogen | PLG |
| CD47 molecule | CD47 | perilipin 2 | PLIN2 |
| CD48 molecule | CD48 | perilipin 3 | PLIN3 |
| CD5 molecule | CD5 | procollagen-lysine, 2-oxoglutarate 5-dioxygenase 1 | PLOD1 |
| CD53 molecule | CD53 | procollagen-lysine, 2-oxoglutarate 5-dioxygenase 2 | PLOD2 |
| CD55 molecule, decay accelerating factor for complement (Cromer blood group) | CD55 | procollagen-lysine, 2-oxoglutarate 5-dioxygenase 3 | PLOD3 |
| CD58 molecule | CD58 | proteolipid protein 1 | PLP1 |
| CD59 molecule, complement regulatory protein | CD59 | proteolipid protein 2 (colonic epithelium-enriched) | PLP2 |
| CD5 molecule-like | CD5L | pleiotropic regulator 1 | PLRG1 |
| CD6 molecule | CD6 | plastin 1 | PLS1 |
| CD63 molecule | CD63 | plastin 3 | PLS3 |
| CD68 molecule | CD68 | phospholipid scramblase 1 | PLSCR1 |
| CD70 molecule | CD70 | phospholipid scramblase 3 | PLSCR3 |
| CD74 molecule, major histocompatibility complex, class II invariant chain | CD74 | phospholipid transfer protein | PLTP |
| CD79b molecule, immunoglobulin-associated beta | CD79B | plasmalemma vesicle associated protein | PLVAP |
| CD80 molecule | CD80 | plexin domain containing 2 | PLXDC2 |
| CD81 molecule | CD81 | plexin A1 | PLXNA1 |
| CD82 molecule | CD82 | plexin A2 | PLXNA2 |
| CD84 molecule | CD84 | plexin A3 | PLXNA3 |
| CD86 molecule | CD86 | plexin A4 | PLXNA4 |
| CD8a molecule | CD8A | plexin B1 | PLXNB1 |
| CD8b molecule | CD8B | plexin B2 | PLXNB2 |
| CD9 molecule | CD9 | plexin B3 | PLXNB3 |
| CD99 molecule | CD99 | plexin C1 | PLXNC1 |
| CD99 molecule-like 2 | CD99L2 | plexin D1 | PLXND1 |
| cell division cycle 16 | CDC16 | peptidase M20 domain containing 1 | PM20D1 |
| cell division cycle 23 | CDC23 | peptidase M20 domain containing 2 | PM20D2 |
| cell division cycle 25B | CDC25B | premelanosome protein | PMEL |
| cell division cycle 27 | CDC27 | polyamine modulated factor 1 binding protein 1 | PMFBP1 |
| cell division cycle 37 | CDC37 | phosphomannomutase 2 | PMM2 |
| cell division cycle 42 | CDC42 | phosphomevalonate kinase | PMVK |
| CDC42 binding protein kinase alpha (DMPK-like) | CDC42BPA | polynucleotide kinase 3'-phosphatase | PNKP |
| CDC42 binding protein kinase beta (DMPK-like) | CDC42BPB | pinin, desmosome associated protein | PNN |
| CDC42 binding protein kinase gamma (DMPK-like) | CDC42BPG | partner of NOB1 homolog (S. cerevisiae) | PNO1 |
| cell division cycle 42 pseudogene 6 | CDC42P6 | purine nucleoside phosphorylase | PNP |
| CDC42 small effector 2 | CDC42SE2 | patatin-like phospholipase domain containing 1 | PNPLA1 |
| cell division cycle 5-like | CDC5L | patatin-like phospholipase domain containing 6 | PNPLA6 |
| cell division cycle associated 3 | CDCA3 | podocan | PODN |
| cell division cycle associated 8 | CDCA8 | podocalyxin-like | PODXL |
| CUB domain containing protein 1 | CDCP1 | protein O-fucosyltransferase 2 | POFUT2 |
| cadherin 1, type 1, E-cadherin (epithelial) | CDH1 | polymerase (DNA directed), beta | POLB |
| cadherin 11, type 2, OB-cadherin (osteoblast) | CDH11 | polymerase (DNA directed), delta 1, catalytic subunit | POLD1 |
| cadherin 13 | CDH13 | polymerase (DNA directed), delta 2, accessory subunit | POLD2 |
| cadherin 17, LI cadherin (liver-intestine) | CDH17 | polymerase (DNA directed) nu | POLN |
| cadherin 2, type 1, N-cadherin (neuronal) | CDH2 | polymerase (RNA) I polypeptide C, 30kDa | POLR1C |
| cadherin-related 23 | CDH23 | polymerase (RNA) I polypeptide D, 16kDa | POLR1D |
| cadherin 3, type 1, P-cadherin (placental) | CDH3 | polymerase (RNA) II (DNA directed) polypeptide A, 220kDa | POLR2A |
| cadherin 6, type 2, K-cadherin (fetal kidney) | CDH6 | polymerase (RNA) II (DNA directed) polypeptide B, 140kDa | POLR2B |
| cadherin 9, type 2 (T1-cadherin) | CDH9 | polymerase (RNA) II (DNA directed) polypeptide C, 33kDa | POLR2C |
| cadherin-related family member 2 | CDHR2 | polymerase (RNA) II (DNA directed) polypeptide E, 25kDa | POLR2E |
| cadherin-related family member 5 | CDHR5 | polymerase (RNA) II (DNA directed) polypeptide H | POLR2H |
| CDP-diacylglycerol--inositol 3-phosphatidyltransferase | CDIPT | polymerase (RNA) II (DNA directed) polypeptide L, 7.6kDa | POLR2L |
| cyclin-dependent kinase 1 | CDK1 | polymerase (RNA) III (DNA directed) polypeptide A, 155kDa | POLR3A |
| cyclin-dependent kinase 11 B | CDK11B | polymerase (RNA) III (DNA directed) polypeptide B | POLR3B |
| cyclin-dependent kinase 12 | CDK12 | polymerase (RNA) III (DNA directed) polypeptide C (62kD) | POLR3C |
| cyclin-dependent kinase 13 | CDK13 | polymerase (RNA) III (DNA directed) polypeptide E (80kD) | POLR3E |
| cyclin-dependent kinase 14 | CDK14 | polymerase (RNA) mitochondrial (DNA directed) | POLRMT |
| cyclin-dependent kinase 16 | CDK16 | paraoxonase 1 | PON1 |
| cyclin-dependent kinase 17 | CDK17 | paraoxonase 3 | PON3 |
| cyclin-dependent kinase 18 | CDK18 | processing of precursor 1, ribonuclease P/MRP subunit (S. cerevisiae) | POP1 |
| cyclin-dependent kinase 2 | CDK2 | P450 (cytochrome) oxidoreductase | POR |
| cyclin-dependent kinase 3 | CDK3 | periostin, osteoblast specific factor | POSTN |
| cyclin-dependent kinase 4 | CDK4 | POTE ankyrin domain family, member B2 | POTEB2 |
| cyclin-dependent kinase 5 | CDK5 | POTE ankyrin domain family, member B3 | POTEB3 |
| cyclin-dependent kinase 5, regulatory subunit 2 (p39) | CDK5R2 | POTE ankyrin domain family, member E | POTEE |
| CDK5 regulatory subunit associated protein 2 | CDK5RAP2 | POTE ankyrin domain family, member F | POTEF |
| cyclin-dependent kinase 6 | CDK6 | POTE ankyrin domain family, member I | POTEI |
| cyclin-dependent kinase 9 | CDK9 | POTE ankyrin domain family, member J | POTEJ |
| CDP-diacylglycerol synthase (phosphatidate cytidylyltransferase) 2 | CDS2 | POTE ankyrin domain family, member K, pseudogene | POTEKP |
| corneodesmosin | CDSN | POTE ankyrin domain family, member M | POTEM |
| CDV3 homolog (mouse) | CDV3 | POU class 2 homeobox 2 | POU2F2 |
| carcinoembryonic antigen-related cell adhesion molecule 1 (biliary glycoprotein) | CEACAM1 | pyrophosphatase (inorganic) 1 | PPA1 |
| carcinoembryonic antigen-related cell adhesion molecule 5 | CEACAM5 | pyrophosphatase (inorganic) 2 | PPA2 |
| carcinoembryonic antigen-related cell adhesion molecule 8 | CEACAM8 | phosphatidic acid phosphatase type 2A | PPAP2A |
| cat eye syndrome chromosome region, candidate 5 | CECR5 | phosphatidic acid phosphatase type 2B | PPAP2B |
| carboxyl ester lipase | CEL | phosphatidic acid phosphatase type 2C | PPAP2C |
| CUGBP, Elav-like family member 1 | CELF1 | peroxisome proliferator-activated receptor gamma | PPARG |
| CUGBP, Elav-like family member 2 | CELF2 | phosphoribosyl pyrophosphate amidotransferase | PPAT |
| cell migration inducing protein, hyaluronan binding | CEMIP | pro-platelet basic protein (chemokine (C-X-C motif) ligand 7) | PPBP |
| centromere protein E, 312kDa | CENPE | protein tyrosine phosphatase, receptor type, f polypeptide (PTPRF), interacting protein (liprin), alpha 1 | PPFIA1 |
| centromere protein V | CENPV | protein tyrosine phosphatase, receptor type, f polypeptide (PTPRF), interacting protein (liprin), alpha 2 | PPFIA2 |
| centrosomal protein 131kDa | CEP131 | protein tyrosine phosphatase, receptor type, f polypeptide (PTPRF), interacting protein (liprin), alpha 3 | PPFIA3 |
| centrosomal protein 250kDa | CEP250 | PTPRF interacting protein, binding protein 1 (liprin beta 1) | PPFIBP1 |
| centrosomal protein 350kDa | CEP350 | PTPRF interacting protein, binding protein 2 (liprin beta 2) | PPFIBP2 |
| centrosomal protein 55kDa | CEP55 | peptidylprolyl isomerase A (cyclophilin A) | PPIA |
| centrosomal protein 97kDa | CEP97 | peptidylprolyl isomerase A (cyclophilin A) pseudogene 22 | PPIAP22 |
| ceramide synthase 1 | CERS1 | peptidylprolyl isomerase A (cyclophilin A) pseudogene 31 | PPIAP31 |
| carboxylesterase 2 | CES2 | peptidylprolyl isomerase B (cyclophilin B) | PPIB |
| carboxylesterase 3 | CES3 | peptidylprolyl isomerase C (cyclophilin C) | PPIC |
| cholesteryl ester transfer protein, plasma | CETP | peptidylprolyl isomerase D | PPID |
| cilia and flagella associated protein 20 | CFAP20 | peptidylprolyl isomerase E (cyclophilin E) | PPIE |
| cilia and flagella associated protein 43 | CFAP43 | peptidylprolyl isomerase H (cyclophilin H) | PPIH |
| cilia and flagella associated protein 44 | CFAP44 | peptidylprolyl isomerase (cyclophilin)-like 1 | PPIL1 |
| cilia and flagella associated protein 58 | CFAP58 | diphosphoinositol pentakisphosphate kinase 2 | PPIP5K2 |
| cilia and flagella associated protein 70 | CFAP70 | periplakin | PPL |
| complement factor B | CFB | protein phosphatase, Mg2+/Mn2+ dependent, 1A | PPM1A |
| complement factor D (adipsin) | CFD | protein phosphatase, Mg2+/Mn2+ dependent, 1B | PPM1B |
| complement factor H | CFH | protein phosphatase, Mg2+/Mn2+ dependent, 1G | PPM1G |
| complement factor H-related 3 | CFHR3 | protein phosphatase, Mg2+/Mn2+ dependent, 1L | PPM1L |
| complement factor I | CFI | protein phosphatase methylesterase 1 | PPME1 |
| cofilin 1 (non-muscle) | CFL1 | protein phosphatase 1, catalytic subunit, alpha isozyme | PPP1CA |
| cofilin 2 (muscle) | CFL2 | protein phosphatase 1, catalytic subunit, beta isozyme | PPP1CB |
| cystic fibrosis transmembrane conductance regulator (ATP-binding cassette sub-family C, member 7) | CFTR | protein phosphatase 1, catalytic subunit, gamma isozyme | PPP1CC |
| coiled-coil-helix-coiled-coil-helix domain containing 3 | CHCHD3 | protein phosphatase 1, regulatory subunit 12B | PPP1R12B |
| chromodomain helicase DNA binding protein 1-like | CHD1L | protein phosphatase 1, regulatory subunit 21 | PPP1R21 |
| chromodomain helicase DNA binding protein 4 | CHD4 | protein phosphatase 1, regulatory subunit 7 | PPP1R7 |
| chromodomain helicase DNA binding protein 6 | CHD6 | protein phosphatase 2, catalytic subunit, alpha isozyme | PPP2CA |
| chromodomain helicase DNA binding protein 9 | CHD9 | protein phosphatase 2, catalytic subunit, beta isozyme | PPP2CB |
| chitinase domain containing 1 | CHID1 | protein phosphatase 2, regulatory subunit A, alpha | PPP2R1A |
| charged multivesicular body protein 1A | CHMP1A | protein phosphatase 2, regulatory subunit A, beta | PPP2R1B |
| charged multivesicular body protein 1B | CHMP1B | protein phosphatase 2, regulatory subunit B, alpha | PPP2R2A |
| charged multivesicular body protein 2A | CHMP2A | protein phosphatase 2, regulatory subunit B, beta | PPP2R2B |
| charged multivesicular body protein 2B | CHMP2B | protein phosphatase 2, regulatory subunit B, gamma | PPP2R2C |
| charged multivesicular body protein 3 | CHMP3 | protein phosphatase 2, regulatory subunit B, delta | PPP2R2D |
| charged multivesicular body protein 4A | CHMP4A | protein phosphatase 2A activator, regulatory subunit 4 | PPP2R4 |
| charged multivesicular body protein 4B | CHMP4B | protein phosphatase 2, regulatory subunit B', gamma | PPP2R5C |
| charged multivesicular body protein 4C | CHMP4C | protein phosphatase 2, regulatory subunit B', epsilon isoform | PPP2R5E |
| charged multivesicular body protein 5 | CHMP5 | protein phosphatase 3, catalytic subunit, alpha isozyme | PPP3CA |
| charged multivesicular body protein 6 | CHMP6 | protein phosphatase 4, catalytic subunit | PPP4C |
| cysteine and histidine-rich domain (CHORD) containing 1 | CHORDC1 | protein phosphatase 4, regulatory subunit 1 | PPP4R1 |
| calcineurin-like EF-hand protein 1 | CHP1 | protein phosphatase 5, catalytic subunit | PPP5C |
| chordin-like 2 | CHRDL2 | protein phosphatase 6, catalytic subunit | PPP6C |
| cholinergic receptor, nicotinic, alpha 3 (neuronal) | CHRNA3 | protein phosphatase 6, regulatory subunit 1 | PPP6R1 |
| carbohydrate (keratan sulfate Gal-6) sulfotransferase 1 | CHST1 | palmitoyl-protein thioesterase 1 | PPT1 |
| carbohydrate (chondroitin 4) sulfotransferase 12 | CHST12 | prolylcarboxypeptidase (angiotensinase C) | PRCP |
| carbohydrate (N-acetylgalactosamine 4-0) sulfotransferase 14 | CHST14 | PR domain containing 16 | PRDM16 |
| CTF18, chromosome transmission fidelity factor 18 homolog (S. cerevisiae) | CHTF18 | peroxiredoxin 1 | PRDX1 |
| conserved helix-loop-helix ubiquitous kinase | CHUK | peroxiredoxin 2 | PRDX2 |
| cytosolic iron-sulfur assembly component 1 | CIAO1 | peroxiredoxin 3 | PRDX3 |
| calcium and integrin binding 1 (calmyrin) | CIB1 | peroxiredoxin 4 | PRDX4 |
| cold inducible RNA binding protein | CIRBP | peroxiredoxin 5 | PRDX5 |
| CDGSH iron sulfur domain 2 | CISD2 | peroxiredoxin 6 | PRDX6 |
| citron rho-interacting serine/threonine kinase | CIT | proline/arginine-rich end leucine-rich repeat protein | PRELP |
| Cbp/p300-interacting transactivator, with Glu/Asp-rich carboxy-terminal domain, 1 | CITED1 | prolyl endopeptidase | PREP |
| cytoskeleton-associated protein 4 | CKAP4 | proteoglycan 4 | PRG4 |
| cytoskeleton associated protein 5 | CKAP5 | protein kinase, AMP-activated, alpha 1 catalytic subunit | PRKAA1 |
| creatine kinase, brain | CKB | protein kinase, AMP-activated, alpha 2 catalytic subunit | PRKAA2 |
| creatine kinase, mitochondrial 1A | CKMT1A | protein kinase, AMP-activated, beta 2 non-catalytic subunit | PRKAB2 |
| creatine kinase, mitochondrial 1B | CKMT1B | protein kinase, cAMP-dependent, catalytic, alpha | PRKACA |
| cytoplasmic linker associated protein 1 | CLASP1 | protein kinase, cAMP-dependent, catalytic, beta | PRKACB |
| cytoplasmic linker associated protein 2 | CLASP2 | protein kinase, AMP-activated, gamma 1 non-catalytic subunit | PRKAG1 |
| Charcot-Leyden crystal galectin | CLC | protein kinase, AMP-activated, gamma 2 non-catalytic subunit | PRKAG2 |
| chloride channel accessory 4 | CLCA4 | protein kinase, cAMP-dependent, regulatory, type I, alpha | PRKAR1A |
| chloride channel, voltage-sensitive 3 | CLCN3 | protein kinase, cAMP-dependent, regulatory, type II, alpha | PRKAR2A |
| chloride channel, voltage-sensitive 4 | CLCN4 | protein kinase, cAMP-dependent, regulatory, type II, beta | PRKAR2B |
| chloride channel, voltage-sensitive 5 | CLCN5 | protein kinase C, alpha | PRKCA |
| chloride channel, voltage-sensitive 7 | CLCN7 | protein kinase C, beta | PRKCB |
| claudin 1 | CLDN1 | protein kinase C, delta | PRKCD |
| claudin 12 | CLDN12 | protein kinase C, gamma | PRKCG |
| claudin 18 | CLDN18 | protein kinase C, eta | PRKCH |
| claudin 2 | CLDN2 | protein kinase C, iota | PRKCI |
| claudin 3 | CLDN3 | protein kinase C, theta | PRKCQ |
| claudin 4 | CLDN4 | protein kinase C substrate 80K-H | PRKCSH |
| claudin 5 | CLDN5 | protein kinase C, zeta | PRKCZ |
| claudin 6 | CLDN6 | protein kinase D1 | PRKD1 |
| claudin 7 | CLDN7 | protein kinase D2 | PRKD2 |
| claudin domain containing 1 | CLDND1 | protein kinase D3 | PRKD3 |
| C-type lectin domain family 11, member A | CLEC11A | protein kinase, DNA-activated, catalytic polypeptide | PRKDC |
| C-type lectin domain family 1, member B | CLEC1B | protein kinase, interferon-inducible double stranded RNA dependent activator | PRKRA |
| C-type lectin domain family 3, member B | CLEC3B | PRKR interacting protein 1 (IL11 inducible) | PRKRIP1 |
| chloride intracellular channel 1 | CLIC1 | protein kinase, X-linked | PRKX |
| chloride intracellular channel 2 | CLIC2 | protein arginine methyltransferase 1 | PRMT1 |
| chloride intracellular channel 3 | CLIC3 | protein arginine methyltransferase 5 | PRMT5 |
| chloride intracellular channel 4 | CLIC4 | prion protein | PRNP |
| chloride intracellular channel 5 | CLIC5 | protein C receptor, endothelial | PROCR |
| chloride intracellular channel 6 | CLIC6 | prominin 1 | PROM1 |
| clathrin interactor 1 | CLINT1 | prominin 2 | PROM2 |
| CAP-GLY domain containing linker protein 2 | CLIP2 | protein S (alpha) | PROS1 |
| ceroid-lipofuscinosis, neuronal 3 | CLN3 | protein Z, vitamin K-dependent plasma glycoprotein | PROZ |
| chloride channel, nucleotide-sensitive, 1A | CLNS1A | pre-mRNA processing factor 19 | PRPF19 |
| caseinolytic mitochondrial matrix peptidase chaperone subunit | CLPX | pre-mRNA processing factor 31 | PRPF31 |
| clarin 3 | CLRN3 | pre-mRNA processing factor 38B | PRPF38B |
| calsyntenin 1 | CLSTN1 | pre-mRNA processing factor 4 | PRPF4 |
| clathrin, light chain A | CLTA | PRP40 pre-mRNA processing factor 40 homolog A (S. cerevisiae) | PRPF40A |
| clathrin, light chain B | CLTB | pre-mRNA processing factor 4B | PRPF4B |
| clathrin, heavy chain (Hc) | CLTC | pre-mRNA processing factor 6 | PRPF6 |
| clathrin, heavy chain-like 1 | CLTCL1 | pre-mRNA processing factor 8 | PRPF8 |
| clusterin | CLU | peripherin | PRPH |
| clustered mitochondria (cluA/CLU1) homolog | CLUH | peripherin 2 (retinal degeneration, slow) | PRPH2 |
| clavesin 2 | CLVS2 | phosphoribosyl pyrophosphate synthetase 1 | PRPS1 |
| carboxymethylenebutenolidase homolog (Pseudomonas) | CMBL | phospho ribosyl pyrophosphate synthetase 1-like 1 | PRPS1L1 |
| c-Maf inducing protein | CMIP | phosphoribosyl pyrophosphate synthetase 2 | PRPS2 |
| cytidine monophosphate (UMP-CMP) kinase 1, cytosolic | CMPK1 | phosphoribosyl pyrophosphate synthetase-associated protein 1 | PRPSAP1 |
| cytidine monophosphate (UMP-CMP) kinase 2, mitochondrial | CMPK2 | phosphoribosyl pyrophosphate synthetase-associated protein 2 | PRPSAP2 |
| cap methyltransferase 1 | CMTR1 | proline rich 14-like | PRR14L |
| cardiomyopathy associated 5 | CMYA5 | proline rich 27 | PRR27 |
| CNDP dipeptidase 2 (metallopeptidase M20 family) | CNDP2 | proline rich 36 | PRR36 |
| cyclic nucleotide gated channel beta 1 | CNGB1 | proline rich 4 (lacrimal) | PRR4 |
| connector enhancer of kinase suppressor of Ras 2 | CNKSR2 | proline-rich coiled-coil 2A | PRRC2A |
| CNKSR family member 3 | CNKSR3 | proline rich Gla (G-carboxyglutamic acid) 1 | PRRG1 |
| calponin 1, basic, smooth muscle | CNN1 | proline-rich transmembrane protein 3 | PRRT3 |
| calponin 2 | CNN2 | protease, serine, 16 (thymus) | PRSS16 |
| calponin 3, acidic | CNN3 | protease, serine, 22 | PRSS22 |
| cyclin and CBS domain divalent metal cation transport mediator 2 | CNNM2 | protease, serine, 23 | PRSS23 |
| cyclin and CBS domain divalent metal cation transport mediator 3 | CNNM3 | protease, serine, 3 | PRSS3 |
| cyclin and CBS domain divalent metal cation transport mediator 4 | CNNM4 | protease, serine, 48 | PRSS48 |
| CCR4-NOT transcription complex, subunit 1 | CNOT1 | protease, serine, 8 | PRSS8 |
| CCR4-NOT transcription complex, subunit 11 | CNOT11 | proteinase 3 | PRTN3 |
| CCR4-NOT transcription complex, subunit 7 | CNOT7 | prosaposin | PSAP |
| 2',3'-cyclic nucleotide 3' phosphodiesterase | CNP | phosphoserine aminotransferase 1 | PSAT1 |
| canopy FGF signaling regulator 2 | CNPY2 | prostate stem cell antigen | PSCA |
| ciliary neurotrophic factor receptor | CNTFR | PC4 and SFRS1 interacting protein 1 | PSIP1 |
| centlein, centrosomal protein | CNTLN | proteasome (prosome, macropain) subunit, alpha type, 1 | PSMA1 |
| contactin 1 | CNTN1 | proteasome (prosome, macropain) subunit, alpha type, 2 | PSMA2 |
| contactin 5 | CNTN5 | proteasome (prosome, macropain) subunit, alpha type, 3 | PSMA3 |
| contactin associated protein-like 4 | CNTNAP4 | proteasome (prosome, macropain) subunit, alpha type, 4 | PSMA4 |
| CoA synthase | COASY | proteasome (prosome, macropain) subunit, alpha type, 5 | PSMA5 |
| cordon-bleu WH2 repeat protein-like 1 | COBLL1 | proteasome (prosome, macropain) subunit, alpha type, 6 | PSMA6 |
| cochlin | COCH | proteasome (prosome, macropain) subunit, alpha type, 7 | PSMA7 |
| component of oligomeric golgi complex 1 | COG1 | proteasome (prosome, macropain) subunit, alpha type, 8 | PSMA8 |
| component of oligomeric golgi complex 2 | COG2 | proteasome (prosome, macropain) subunit, beta type, 1 | PSMB1 |
| component of oligomeric golgi complex 3 | COG3 | proteasome (prosome, macropain) subunit, beta type, 10 | PSMB10 |
| component of oligomeric golgi complex 4 | COG4 | proteasome (prosome, macropain) subunit, beta type, 11 | PSMB11 |
| component of oligomeric golgi complex 5 | COG5 | proteasome (prosome, macropain) subunit, beta type, 2 | PSMB2 |
| component of oligomeric golgi complex 6 | COG6 | proteasome (prosome, macropain) subunit, beta type, 3 | PSMB3 |
| component of oligomeric golgi complex 7 | COG7 | proteasome (prosome, macropain) subunit, beta type, 4 | PSMB4 |
| collagen, type XI, alpha 1 | COL11A1 | proteasome (prosome, macropain) subunit, beta type, 5 | PSMB5 |
| collagen, type XII, alpha 1 | COL12A1 | proteasome (prosome, macropain) subunit, beta type, 6 | PSMB6 |
| collagen, type XIV, alpha 1 | COL14A1 | proteasome (prosome, macropain) subunit, beta type, 7 | PSMB7 |
| collagen, type XV, alpha 1 | COL15A1 | proteasome (prosome, macropain) subunit, beta type, 8 | PSMB8 |
| collagen, type XVI, alpha 1 | COL16A1 | proteasome (prosome, macropain) subunit, beta type, 9 | PSMB9 |
| collagen, type XVIII, alpha 1 | COL18A1 | proteasome (prosome, macropain) 26S subunit, ATPase, 1 | PSMC1 |
| collagen, type I, alpha 1 | COL1A1 | proteasome (prosome, macropain) 26S subunit, ATPase, 2 | PSMC2 |
| collagen, type I, alpha 2 | COL1A2 | proteasome (prosome, macropain) 26S subunit, ATPase, 3 | PSMC3 |
| collagen, type XXI, alpha 1 | COL21A1 | proteasome (prosome, macropain) 26S subunit, ATPase, 4 | PSMC4 |
| collagen, type XXIV, alpha 1 | COL24A1 | proteasome (prosome, macropain) 26S subunit, ATPase, 5 | PSMC5 |
| collagen, type II, alpha 1 | COL2A1 | proteasome (prosome, macropain) 26S subunit, ATPase, 6 | PSMC6 |
| collagen, type III, alpha 1 | COL3A1 | proteasome (prosome, macropain) 26S subunit, non-ATPase, 1 | PSMD1 |
| collagen, type IV, alpha 1 | COL4A1 | proteasome (prosome, macropain) 26S subunit, non-ATPase, 10 | PSMD10 |
| collagen, type IV, alpha 2 | COL4A2 | proteasome (prosome, macropain) 26S subunit, non-ATPase, 11 | PSMD11 |
| collagen, type IV, alpha 3 (Goodpasture antigen) | COL4A3 | proteasome (prosome, macropain) 26S subunit, non-ATPase, 12 | PSMD12 |
| collagen, type V, alpha 1 | COL5A1 | proteasome (prosome, macropain) 26S subunit, non-ATPase, 13 | PSMD13 |
| collagen, type V, alpha 2 | COL5A2 | proteasome (prosome, macropain) 26S subunit, non-ATPase, 14 | PSMD14 |
| collagen, type VI, alpha 1 | COL6A1 | proteasome (prosome, macropain) 26S subunit, non-ATPase, 2 | PSMD2 |
| collagen, type VI, alpha 2 | COL6A2 | proteasome (prosome, macropain) 26S subunit, non-ATPase, 3 | PSMD3 |
| collagen, type VI, alpha 3 | COL6A3 | proteasome (prosome, macropain) 26S subunit, non-ATPase, 4 | PSMD4 |
| collagen, type VII, alpha 1 | COL7A1 | proteasome (prosome, macropain) 26S subunit, non-ATPase, 5 | PSMD5 |
| collectin sub-family member 10 (C-type lectin) | COLEC10 | proteasome (prosome, macropain) 26S subunit, non-ATPase, 6 | PSMD6 |
| collectin sub-family member 12 | COLEC12 | proteasome (prosome, macropain) 26S subunit, non-ATPase, 7 | PSMD7 |
| collagen beta(1-O)galactosyltransferase 1 | COLGALT 1 | proteasome (prosome, macropain) 26S subunit, non-ATPase, 8 | PSMD8 |
| COMM domain containing 3 | COMMD3 | proteasome (prosome, macropain) 26S subunit, non-ATPase, 9 | PSMD9 |
| cartilage oligomeric matrix protein | COMP | proteasome (prosome, macropain) activator subunit 1 (PA28 alpha) | PSME1 |
| catechol-O-methyltransferase | COMT | proteasome (prosome, macropain) activator subunit 2 (PA28 beta) | PSME2 |
| coatomer protein complex, subunit alpha | COPA | proteasome (prosome, macropain) activator subunit 3 (PA28 gamma; Ki) | PSME3 |
| coatomer protein complex, subunit beta 1 | COPB1 | proteasome (prosome, macropain) activator subunit 4 | PSME4 |
| coatomer protein complex, subunit beta 2 (beta prime) | COPB2 | proteasome (prosome, macropain) assembly chaperone 1 | PSMG1 |
| coatomer protein complex, subunit epsilon | COPE | proteasome (prosome, macropain) assembly chaperone 2 | PSMG2 |
| coatomer protein complex, subunit gamma 1 | COPG1 | paraspeckle component 1 | PSPC1 |
| coatomer protein complex, subunit gamma 2 | COPG2 | phosphoserine phosphatase | PSPH |
| COP9 signalosome subunit 2 | COPS2 | proline-serine-threonine phosphatase interacting protein 1 | PSTPIP1 |
| COP9 signalosome subunit 3 | COPS3 | proline-serine-threonine phosphatase interacting protein 2 | PSTPIP2 |
| COP9 signalosome subunit 4 | COPS4 | polypyrimidine tract binding protein 1 | PTBP1 |
| COP9 signalosome subunit 5 | COPS5 | polypyrimidine tract binding protein 3 | PTBP3 |
| COP9 signalosome subunit 6 | COPS6 | phosphotriesterase related | PTER |
| COP9 signalosome subunit 8 | COPS8 | prostaglandin E synthase 2 | PTGES2 |
| coatomer protein complex, subunit zeta 1 | COPZ1 | prostaglandin E synthase 3 (cytosolic) | PTGES3 |
| coenzyme Q6 monooxygenase | COQ6 | PTGES3L-AARSD1 readthrough | PTGES3L-AARSD1 |
| coronin, actin binding protein, 1A | CORO1A | prostaglandin F2 receptor inhibitor | PTGFRN |
| coronin, actin binding protein, 1B | CORO1B | prostaglandin reductase 1 | PTGR1 |
| coronin, actin binding protein, 1C | CORO1C | prostaglandin reductase 2 | PTGR2 |
| coronin 7 | CORO7 | prostaglandin-endoperoxide synthase 1 (prostaglandin G/H synthase and cyclooxygenase) | PTGS1 |
| CORO7-PAM16 readthrough | CORO7-PAM16 | protein tyrosine kinase 2 | PTK2 |
| coactosin-like F-actin binding protein 1 | COTL1 | protein tyrosine kinase 2 beta | PTK2B |
| cytochrome c oxidase subunit IV isoform 1 | COX4I1 | protein tyrosine kinase 7 (inactive) | PTK7 |
| cytochrome c oxidase subunit Vb | COX5B | prothymosin, alpha | PTMA |
| ceruloplasmin (ferroxidase) | CP | parathymosin | PTMS |
| carboxypeptidase A1 (pancreatic) | CPA1 | protein tyrosine phosphatase type IVA, member 1 | PTP4A1 |
| carboxypeptidase B1 (tissue) | CPB1 | protein tyrosine phosphatase type IVA, member 2 | PTP4A2 |
| carboxypeptidase D | CPD | protein tyrosine phosphatase, non-receptor type 1 | PTPN1 |
| carboxypeptidase M | CPM | protein tyrosine phosphatase, non-receptor type 11 | PTPN11 |
| carboxypeptidase N, polypeptide 2 | CPN2 | protein tyrosine phosphatase, non-receptor type 13 (APO-1/CD95 (Fas)-associated phosphatase) | PTPN13 |
| copine I | CPNE1 | protein tyrosine phosphatase, non-receptor type 18 (brain-derived) | PTPN18 |
| copine II | CPNE2 | protein tyrosine phosphatase, non-receptor type 2 | PTPN2 |
| copine III | CPNE3 | protein tyrosine phosphatase, non-receptor type 23 | PTPN23 |
| copine V | CPNE5 | protein tyrosine phosphatase, non-receptor type 6 | PTPN6 |
| copine VI (neuronal) | CPNE6 | protein tyrosine phosphatase, non-receptor type 7 | PTPN7 |
| copine VIII | CPNE8 | protein tyrosine phosphatase, non-receptor type 9 | PTPN9 |
| coproporphyrinogen oxidase | CPOX | protein tyrosine phosphatase, receptor type, A | PTPRA |
| carbamoyl-phosphate synthase 1, mitochond rial | CPS1 | protein tyrosine phosphatase, receptor type, B | PTPRB |
| cleavage and polyadenylation specific factor 1, 160kDa | CPSF1 | protein tyrosine phosphatase, receptor type, C | PTPRC |
| cleavage and polyadenylation specific factor 3, 73kDa | CPSF3 | protein tyrosine phosphatase, receptor type, C-associated protein | PTPRCAP |
| cleavage and polyadenylation specific factor 3-like | CPSF3L | protein tyrosine phosphatase, receptor type, F | PTPRF |
| cleavage and polyadenylation specific factor 6, 68kDa | CPSF6 | protein tyrosine phosphatase, receptor type, G | PTPRG |
| cleavage and polyadenylation specific factor 7, 59kDa | CPSF7 | protein tyrosine phosphatase, receptor type, J | PTPRJ |
| carboxypeptidase, vitellogenic-like | CPVL | protein tyrosine phosphatase, receptor type, K | PTPRK |
| complement component (3b/4b) receptor 1 (Knops blood group) | CR1 | protein tyrosine phosphatase, receptor type, O | PTPRO |
| complement component (3d/Epstein Barr virus) receptor 2 | CR2 | protein tyrosine phosphatase, receptor type, S | PTPRS |
| cellular retinoic acid binding protein 2 | CRABP2 | polymerase I and transcript release factor | PTRF |
| crumbs family member 2 | CRB2 | peptidyl-tRNA hydrolase 2 | PTRH2 |
| crumbs family member 3 | CRB3 | pituitary tumor-transforming 1 interacting protein | PTTG1 IP |
| cAMP responsive element binding protein 5 | CREB5 | pentraxin 3, long | PTX3 |
| cellular repressor of E1A-stimulated genes 1 | CREG1 | poly-U binding splicing factor 60KDa | PUF60 |
| cysteine-rich protein 2 | CRIP2 | purine-rich element binding protein A | PURA |
| cysteine-rich PDZ-binding protein | CRIPT | purine-rich element binding protein B | PURB |
| cysteine-rich secretory protein LCCL domain containing 1 | CRISPLD1 | pseudouridylate synthase 1 | PUS1 |
| v-crk avian sarcoma virus CT10 oncogene homolog | CRK | poliovirus receptor | PVR |
| v-crk avian sarcoma virus CT10 oncogene homolog-like | CRKL | poliovirus receptor-related 2 (herpesvirus entry mediator B) | PVRL2 |
| cytokine receptor-like factor 3 | CRLF3 | peroxidasin | PXDN |
| collapsin response mediator protein 1 | CRMP1 | paxillin | PXN |
| crooked neck pre-mRNA splicing factor 1 | CRNKL1 | pyrroline-5-carboxylate reductase-like | PYCRL |
| cornulin | CRNN | phosphorylase, glycogen; brain | PYGB |
| ciliary rootlet coiled-coil, rootletin | CROCC | phosphorylase, glycogen, liver | PYGL |
| ciliary rootlet coiled-coil, rootletin family member 2 | CROCC2 | phosphorylase, glycogen, muscle | PYGM |
| cartilage associated protein | CRTAP | pregnancy-zone protein | PZP |
| CREB regulated transcription coactivator 2 | CRTC2 | glutaminyl-tRNA synthetase | QARS |
| crystallin, alpha A | CRYAA | quinoid dihydropteridine reductase | QDPR |
| crystallin, alpha B | CRYAB | glutaminyl-peptide cyclotransferase | QPCT |
| crystallin, lambda 1 | CRYL1 | glutaminyl-peptide cyclotransferase-like | QPCTL |
| crystallin, mu | CRYM | quinolinate phosphoribosyltransferase | QPRT |
| crystallin, zeta (quinone reductase) | CRYZ | quiescin Q6 sulfhydryl oxidase 1 | QSOX1 |
| crystallin, zeta (quinone reductase)-like 1 | CRYZL1 | quiescin Q6 sulfhydryl oxidase 2 | QSOX2 |
| citrate synthase | CS | queuine tRNA-ribosyltransferase 1 | QTRT1 |
| cold shock domain containing E1, RNA-binding | CSDE1 | RAB10, member RAS oncogene family | RAB10 |
| CSE1 chromosome segregation 1-like (yeast) | CSE1 L | RAB11A, member RAS oncogene family | RAB11A |
| colony stimulating factor 1 (macrophage) | CSF1 | RAB11B, member RAS oncogene family | RAB11B |
| colony stimulating factor 2 (granulocyte-macrophage) | CSF2 | RAB11 family interacting protein 1 (class I) | RAB11FIP1 |
| colony stimulating factor 3 (granulocyte) | CSF3 | RAB12, member RAS oncogene family | RAB12 |
| c-src tyrosine kinase | CSK | RAB13, member RAS oncogene family | RAB13 |
| CUB and Sushi multiple domains 2 | CSMD2 | RAB14, member RAS oncogene family | RAB14 |
| casein alpha s1 | CSN1S1 | RAB15, member RAS oncogene family | RAB15 |
| casein beta | CSN2 | RAB17, member RAS oncogene family | RAB17 |
| casein kappa | CSN3 | RAB18, member RAS oncogene family | RAB18 |
| casein kinase 1, alpha 1 | CSNK1A1 | RAB19, member RAS oncogene family | RAB19 |
| casein kinase 1, delta | CSNK1D | RAB1A, member RAS oncogene family | RAB1A |
| casein kinase 1, gamma 1 | CSNK1G1 | RAB1B, member RAS oncogene family | RAB1B |
| casein kinase 1, gamma 3 | CSNK1G3 | RAB1C, member RAS oncogene family pseudogene | RAB1C |
| casein kinase 2, alpha 1 polypeptide | CSNK2A1 | RAB20, member RAS oncogene family | RAB20 |
| casein kinase 2, alpha prime polypeptide | CSNK2A2 | RAB21, member RAS oncogene family | RAB21 |
| casein kinase 2, beta polypeptide | CSNK2B | RAB22A, member RAS oncogene family | RAB22A |
| chondroitin sulfate proteoglycan 4 | CSPG4 | RAB23, member RAS oncogene family | RAB23 |
| chondroitin sulfate proteoglycan 5 (neuroglycan C) | CSPG5 | RAB25, member RAS oncogene family | RAB25 |
| cysteine and glycine-rich protein 1 | CSRP1 | RAB27A, member RAS oncogene family | RAB27A |
| cysteine and glycine-rich protein 2 | CSRP2 | RAB27B, member RAS oncogene family | RAB27B |
| cystatin C | CST3 | RAB29, member RAS oncogene family | RAB29 |
| cystatin S | CST4 | RAB2A, member RAS oncogene family | RAB2A |
| cystatin D | CST5 | RAB2B, member RAS oncogene family | RAB2B |
| cystatin A (stefin A) | CSTA | RAB30, member RAS oncogene family | RAB30 |
| cystatin B (stefin B) | CSTB | RAB32, member RAS oncogene family | RAB32 |
| cleavage stimulation factor, 3' pre-RNA, subunit 1, 50kDa | CSTF1 | RAB33A, member RAS oncogene family | RAB33A |
| cleavage stimulation factor, 3' pre-RNA, subunit 3, 77kDa | CSTF3 | RAB33B, member RAS oncogene family | RAB33B |
| C-terminal binding protein 1 | CTBP1 | RAB34, member RAS oncogene family | RAB34 |
| C-terminal binding protein 2 | CTBP2 | RAB35, member RAS oncogene family | RAB35 |
| CTD nuclear envelope phosphatase 1 | CTDNEP1 | RAB37, member RAS oncogene family | RAB37 |
| CTD (carboxy-terminal domain, RNA polymerase II, polypeptide A) small phosphatase 1 | CTDSP1 | RAB38, member RAS oncogene family | RAB38 |
| CTD (carboxy-terminal domain, RNA polymerase II, polypeptide A) small phosphatase-like | CTDSPL | RAB39A, member RAS oncogene family | RAB39A |
| cystathionine gamma-lyase | CTH | RAB39B, member RAS oncogene family | RAB39B |
| cytotoxic T-lymphocyte-associated protein 4 - immunoglobulin fusion protein | CTLA4-Ig | RAB3A, member RAS oncogene family | RAB3A |
| catenin (cadherin-associated protein), alpha 1, 102kDa | CTNNA1 | | |
| catenin (cadherin-associated protein), alpha 2 | CTNNA2 | RAB3B, member RAS oncogene family | RAB3B |
| catenin (cadherin-associated protein), beta 1, 88kDa | CTNNB1 | RAB3C, member RAS oncogene family | RAB3C |
| catenin (cadherin-associated protein), delta 1 | CTNND1 | RAB3D, member RAS oncogene family | RAB3D |
| catenin (cadherin-associated protein), delta 2 | CTNND2 | RAB3 GTPase activating protein subunit 1 (catalytic) | RAB3GAP1 |
| cystinosin, lysosomal cystine transporter | CTNS | RAB3 GTPase activating protein subunit 2 (non-catalytic) | RAB3GAP2 |
| CTP synthase 1 | CTPS1 | RAB43, member RAS oncogene family | RAB43 |
| CTP synthase 2 | CTPS2 | RAB4A, member RAS oncogene family | RAB4A |
| CTR9, Paf1/RNA polymerase II complex component | CTR9 | RAB4B, member RAS oncogene family | RAB4B |
| cathepsin A | CTSA | RAB5A, member RAS oncogene family | RAB5A |
| cathepsin B | CTSB | RAB5B, member RAS oncogene family | RAB5B |
| cathepsin C | CTSC | RAB5C, member RAS oncogene family | RAB5C |
| cathepsin D | CTSD | RAB6A, member RAS oncogene family | RAB6A |
| cathepsin G | CTSG | RAB6B, member RAS oncogene family | RAB6B |
| cathepsin H | CTSH | RAB6C, member RAS oncogene family | RAB6C |
| cathepsin L | CTSL | RAB7A, member RAS oncogene family | RAB7A |
| cathepsin V | CTSV | RAB8A, member RAS oncogene family | RAB8A |
| cathepsin Z | CTSZ | RAB8B, member RAS oncogene family | RAB8B |
| cortactin | CTTN | RAB9A, member RAS oncogene family | RAB9A |
| cubilin (intrinsic factor-cobalamin receptor) | CUBN | RAB9B, member RAS oncogene family | RAB9B |
| cullin 1 | CUL1 | RAB GTPase activating protein 1 | RABGAP1 |
| cullin 2 | CUL2 | ras-related C3 botulinum toxin substrate 1 (rho family, small GTP binding protein Rac1) | RAC1 |
| cullin 3 | CUL3 | ras-related C3 botulinum toxin substrate 2 (rho family, small GTP binding protein Rac2) | RAC2 |
| cullin 4A | CUL4A | ras-related C3 botulinum toxin substrate 3 (rho family, small GTP binding protein Rac3) | RAC3 |
| cullin 4B | CUL4B | Rac GTPase activating protein 1 | RACGAP1 |
| cullin 5 | CUL5 | RAD21 homolog (S. pombe) | RAD21 |
| cutA divalent cation tolerance homolog (E. coli) | CUTA | RAD23 homolog B (S. cerevisiae) | RAD23B |
| cut-like homeobox 2 | CUX2 | RAD50 homolog (S. cerevisiae) | RAD50 |
| CWC25 spliceosome-associated protein homolog (S. cerevisiae) | CWC25 | ribonucleic acid export 1 | RAE1 |
| CWF19-like 1, cell cycle control (S. pombe) | CWF19L1 | Raf-1 proto-oncogene, serine/threonine kinase | RAF1 |
| coxsackie virus and adenovirus receptor | CXADR | retinoic acid induced 14 | RAI14 |
| chemokine (C-X-C motif) ligand 16 | CXCL16 | v-ral simian leukemia viral oncogene homolog A (ras related) | RALA |
| chemokine (C-X-C motif) ligand 2 | CXCL2 | v-ral simian leukemia viral oncogene homolog B | RALB |
| chemokine (C-X-C motif) ligand 8 | CXCL8 | Ral GTPase activating protein, beta subunit (non-catalytic) | RALGAPB |
| chemokine (C-X-C motif) receptor 4 | CXCR4 | RALY heterogeneous nuclear ribonucleoprotein | RALY |
| cytochrome b5 type A (microsomal) | CYB5A | RALY RNA binding protein-like | RALYL |
| cytochrome b5 type B (outer mitochondrial membrane) | CYB5B | RAN, member RAS oncogene family | RAN |
| cytochrome b5 reductase 1 | CYB5R1 | RAN binding protein 1 | RANBP1 |
| cytochrome b5 reductase 3 | CYB5R3 | RAN binding protein 10 | RANBP10 |
| cytochrome b-245, beta polypeptide | CYBB | RAN binding protein 2 | RANBP2 |
| cytochrome b reductase 1 | CYBRD1 | RAN binding protein 3 | RANBP3 |
| cytochrome c-1 | CYC1 | RAN binding protein 9 | RANBP9 |
| cytoplasmic FMR1 interacting protein 1 | CYFIP1 | Ran GTPase activating protein 1 | RANGAP1 |
| cytoplasmic FMR1 interacting protein 2 | CYFIP2 | RAN, member RAS oncogene family pseudogene 1 | RANP1 |
| cytochrome P450, family 17, subfamily A, polypeptide 1 | CYP17A1 | RAP1A, member of RAS oncogene family | RAP1A |
| cytochrome P450, family 2, subfamily D, polypeptide 6 | CYP2D6 | RAP1B, member of RAS oncogene family | RAP1 B |
| cysteine-rich, angiogenic inducer, 61 | CYR61 | RAP1B, member of RAS oncogene family pseudogene | RAP1BL |
| cysteine-rich tail protein 1 | CYSRT1 | RAP1 GTPase activating protein 2 | RAP1GAP2 |
| cysteine-rich transmembrane module containing 1 | CYSTM1 | RAP1, GTP-GDP dissociation stimulator 1 | RAP1GDS1 |
| cytohesin 2 | CYTH2 | RAP2A, member of RAS oncogene family | RAP2A |
| cytohesin 3 | CYTH3 | RAP2B, member of RAS oncogene family | RAP2B |
| dishevelled associated activator of morphogenesis 1 | DAAM1 | RAP2C, member of RAS oncogene family | RAP2C |
| Dab, mitogen-responsive phosphoprotein, homolog 2 (Drosophila) | DAB2 | Rap guanine nucleotide exchange factor (GEF) 1 | RAPGEF1 |
| dishevelled-binding antagonist of beta-catenin 1 | DACT1 | Rap guanine nucleotide exchange factor (GEF) 3 | RAPGEF3 |
| defender against cell death 1 | DAD1 | Rap guanine nucleotide exchange factor (GEF) 6 | RAPGEF6 |
| dystroglycan 1 (dystrophin-associated glycoprotein 1) | DAG1 | retinoic acid receptor, alpha | RARA |
| dual adaptor of phosphotyrosine and 3-phosphoinositides | DAPP1 | retinoic acid receptor responder (tazarotene induced) 1 | RARRES1 |
| aspartyl-tRNA synthetase | DARS | arginyl-tRNA synthetase | RARS |
| DBF4 zinc finger B | DBF4B | RAS p21 protein activator (GTPase activating protein) 1 | RASA1 |
| dopamine beta-hydroxylase (dopamine beta-monooxygenase) | DBH | RAS p21 protein activator 2 | RASA2 |
| diazepam binding inhibitor (GABA receptor modulator, acyl-CoA binding protein) | DBI | RAS p21 protein activator 3 | RASA3 |
| drebrin 1 | DBN1 | RAS p21 protein activator 4 | RASA4 |
| drebrin-like | DBNL | RAS p21 protein activator 4C, pseudogene | RASA4CP |
| DDB1 and CUL4 associated factor 7 | DCAF7 | RAS protein activator like 1 (GAP1 like) | RASAL1 |
| discoidin, CUB and LCCL domain containing 2 | DCBLD2 | RAS protein activator like 3 | RASAL3 |
| dermcidin | DCD | RAS guanyl releasing protein 2 (calcium and DAG-regulated) | RASGRP2 |
| dachsous cadherin-related 2 | DCHS2 | Ras association (RaIGDS/AF-6) domain family member 2 | RASSF2 |
| deoxycytidine kinase | DCK | RB1-inducible coiled-coil 1 | RB1CC1 |
| doublecortin-like kinase 1 | DCLK1 | retinoblastoma binding protein 4 | RBBP4 |
| doublecortin-like kinase 2 | DCLK2 | retinoblastoma binding protein 6 | RBBP6 |
| decorin | DCN | retinoblastoma binding protein 7 | RBBP7 |
| dopachrome tautomerase | DCT | retinoblastoma-like 2 | RBL2 |
| dynactin 1 | DCTN1 | RNA binding motif protein 12 | RBM12 |
| dynactin 2 (p50) | DCTN2 | RNA binding motif protein 14 | RBM14 |
| dynactin 3 (p22) | DCTN3 | RBM14-RBM4 readthrough | RBM14-RBM4 |
| dCTP pyrophosphatase 1 | DCTPP1 | RNA binding motif protein 19 | RBM19 |
| DCN1, defective in cullin neddylation 1, domain containing 3 | DCUN1D3 | RNA binding motif protein 22 | RBM22 |
| dicarbonyl/L-xylulose reductase | DCXR | RNA binding motif protein 25 | RBM25 |
| dimethylarginine dimethylaminohydrolase 1 | DDAH1 | RNA binding motif protein 28 | RBM28 |
| dimethylarginine dimethylaminohydrolase 2 | DDAH2 | RNA binding motif (RNP1, RRM) protein 3 | RBM3 |
| damage-specific DNA binding protein 1, 127kDa | DDB1 | RNA binding motif protein 39 | RBM39 |
| dopa decarboxylase (aromatic L-amino acid decarboxylase) | DDC | RNA binding motif protein 4 | RBM4 |
| dendrin | DDN | RNA binding motif protein 6 | RBM6 |
| dolichyl-diphosphooligosaccharide--protein glycosyltransferase subunit (non-catalytic) | DDOST | RNA binding motif protein 8A | RBM8A |
| discoidin domain receptor tyrosine kinase 1 | DDR1 | RNA binding motif protein, X-linked | RBMX |
| discoidin domain receptor tyrosine kinase 2 | DDR2 | retinol binding protein 1, cellular | RBP1 |
| DDRGK domain containing 1 | DDRGK1 | retinol binding protein 4, plasma | RBP4 |
| D-dopachrome tautomerase | DDT | retinol binding protein 5, cellular | RBP5 |
| DEAD (Asp-Glu-Ala-Asp) box helicase 1 | DDX1 | ring-box 1, E3 ubiquitin protein ligase | RBX1 |
| DEAD (Asp-Glu-Ala-Asp) box helicase 17 | DDX17 | ring finger and CCCH-type domains 1 | RC3H1 |
| DEAD (Asp-Glu-Ala-Asp) box polypeptide 18 | DDX18 | regulator of chromosome condensation 1 | RCC1 |
| DEAD (Asp-Glu-Ala-Asp) box polypeptide 19A | DDX19A | regulator of chromosome condensation 2 | RCC2 |
| DEAD (Asp-Glu-Ala-Asp) box polypeptide 19B | DDX19B | RNA terminal phosphate cyclase-like 1 | RCL1 |
| DEAD (Asp-Glu-Ala-Asp) box helicase 21 | DDX21 | reticulocalbin 1, EF-hand calcium binding domain | RCN1 |
| DEAD (Asp-Glu-Ala-Asp) box polypeptide 23 | DDX23 | reticulocalbin 2, EF-hand calcium binding domain | RCN2 |
| DEAD (Asp-Glu-Ala-Asp) box polypeptide 27 | DDX27 | REST corepressor 2 | RCOR2 |
| DEAD (Asp-Glu-Ala-Asp) box polypeptide 39A | DDX39A | retinol dehydrogenase 11 (all-trans/9-cis/11-cis) | RDH11 |
| DEAD (Asp-Glu-Ala-Asp) box polypeptide 39B | DDX39B | retinol dehydrogenase 14 (all-trans/9-cis/11-cis) | RDH14 |
| DEAD (Asp-Glu-Ala-Asp) box helicase 3, X-linked | DDX3X | retinol dehydrogenase 5 (11-cis/9-cis) | RDH5 |
| DEAD (Asp-Glu-Ala-Asp) box helicase 3, Y-linked | DDX3Y | radixin | RDX |
| DEAD (Asp-Glu-Ala-Asp) box polypeptide 4 | DDX4 | RecQ helicase-like | RECQL |
| DEAD (Asp-Glu-Ala-Asp) box polypeptide 46 | DDX46 | receptor accessory protein 5 | REEP5 |
| DEAD (Asp-Glu-Ala-Asp) box polypeptide 47 | DDX47 | regenerating islet-derived family, member 4 | REG4 |
| DEAD (Asp-Glu-Ala-Asp) box polypeptide 49 | DDX49 | v-rel avian reticuloendotheliosis viral oncogene homolog | REL |
| DEAD (Asp-Glu-Ala-Asp) box helicase 5 | DDX5 | RELT-like 1 | RELL1 |
| DEAD (Asp-Glu-Ala-Asp) box polypeptide 50 | DDX50 | reelin | RELN |
| DEAD (Asp-Glu-Ala-Asp) box polypeptide 58 | DDX58 | renin binding protein | RENBP |
| DEAD (Asp-Glu-Ala-Asp) box helicase 6 | DDX6 | retention in endoplasmic reticulum sorting receptor 1 | RER1 |
| DEAD (Asp-Glu-Ala-Asp) box polypeptide 60 | DDX60 | ret proto-oncogene | RET |
| 2,4-dienoyl CoA reductase 1, mitochondrial | DECR1 | resistin | RETN |
| differentially expressed in FDCP 6 homolog (mouse) | DEF6 | replication factor C (activator 1) 1, 145kDa | RFC1 |
| defensin, alpha 1 | DEFA1 | replication factor C (activator 1) 2, 40kDa | RFC2 |
| defensin, alpha 1B | DEFA1B | replication factor C (activator 1) 3, 38kDa | RFC3 |
| defensin, alpha 3, neutrophil-specific | DEFA3 | replication factor C (activator 1) 4, 37kDa | RFC4 |
| DEK proto-oncogene | DEK | replication factor C (activator 1) 5, 36.5kDa | RFC5 |
| DENN/MADD domain containing 3 | DENND3 | ring finger and FYVE-like domain containing E3 ubiquitin protein ligase | RFFL |
| DENN/MADD domain containing 6A | DENND6A | raftlin, lipid raft linker 1 | RFTN1 |
| density-regulated protein | DENR | regucalcin | RGN |
| DEP domain containing 1B | DEPDC1B | regulator of G-protein signaling 10 | RGS10 |
| deoxyribose-phosphate aldolase (putative) | DERA | regulator of G-protein signaling 16 | RGS16 |
| derlin 1 | DERL1 | regulator of G-protein signaling 18 | RGS18 |
| desmin | DES | regulator of G-protein signaling 20 | RGS20 |
| deafness, autosomal dominant 5 | DFNA5 | regulator of G-protein signaling 3 | RGS3 |
| diacylglycerol kinase, alpha 80kDa | DGKA | regulator of G-protein signaling 6 | RGS6 |
| 24-dehydrocholesterol reductase | DHCR24 | rhomboid 5 homolog 1 (Drosophila) | RHBDF1 |
| 7-dehydrocholesterol reductase | DHCR7 | rhomboid 5 homolog 2 (Drosophila) | RHBDF2 |
| dihydrofolate reductase | DHFR | Rh family, C glycoprotein | RHCG |
| dehydrogenase/reductase (SDR family) member 1 | DHRS1 | Ras homolog enriched in brain | RHEB |
| dehydrogenase/reductase (SDR family) member 7 | DHRS7 | ras homolog family member A | RHOA |
| DEAH (Asp-Glu-Ala-His) box helicase 15 | DHX15 | ras homolog family member B | RHOB |
| DEAH (Asp-Glu-Ala-His) box polypeptide 16 | DHX16 | Rho-related BTB domain containing 3 | RHOBTB3 |
| DEAH (Asp-Glu-Ala-His) box helicase 30 | DHX30 | ras homolog family member C | RHOC |
| DEAH (Asp-Glu-Ala-His) box polypeptide 34 | DHX34 | ras homolog family member F (in filopodia) | RHOF |
| DEAH (Asp-Glu-Ala-His) box polypeptide 36 | DHX36 | ras homolog family member G | RHOG |
| DEAH (Asp-Glu-Ala-His) box helicase 9 | DHX9 | ras homolog family member Q | RHOQ |
| diaphanous-related formin 1 | DIAPH1 | rhophilin, Rho GTPase binding protein 2 | RHPN2 |
| diaphanous-related formin 3 | DIAPH3 | RIC8 guanine nucleotide exchange factor A | RIC8A |
| DIM1 dimethyladenosine transferase 1 homolog (S. cerevisiae) | DIMT1 | RIC8 guanine nucleotide exchange factor B | RIC8B |
| DIP2 disco-interacting protein 2 homolog A (Drosophila) | DIP2A | RPTOR independent companion of MTOR, complex 2 | RICTOR |
| DIP2 disco-interacting protein 2 homolog B (Drosophila) | DIP2B | RIO kinase 3 | RIOK3 |
| DIP2 disco-interacting protein 2 homolog C (Drosophila) | DIP2C | RGD motif, leucine rich repeats, tropomodulin domain and proline-rich containing | RLTPR |
| DIRAS family, GTP-binding RAS-like 2 | DIRAS2 | regulator of microtubule dynamics 2 | RMDN2 |
| DIS3 exosome endoribonuclease and 3'-5' exoribonuclease | DIS3 | required for meiotic nuclear division 5 homolog A (S. cerevisiae) | RMND5A |
| DIS3 like 3'-5' exoribonuclease 2 | DIS3L2 | ribonuclease, RNase A family, 2 (liver, eosinophil-derived neurotoxin) | RNASE2 |
| disrupted in schizophrenia 1 | DISC1 | ribonuclease, RNase A family, 4 | RNASE4 |
| dyskeratosis congenita 1, dyskerin | DKC1 | ribonuclease, RNase A family, 7 | RNASE7 |
| dickkopf WNT signaling pathway inhibitor 1 | DKK1 | ribonuclease H2, subunit A | RNASEH2A |
| dickkopf WNT signaling pathway inhibitor 3 | DKK3 | RNF103-CHMP3 readthrough | RNF103-CHMP3 |
| dihydrolipoamide S-acetyltransferase | DLAT | ring finger protein 11 | RNF11 |
| DLC1 Rho GTPase activating protein | DLC1 | ring finger protein 123 | RNF123 |
| dihydrolipoamide dehydrogenase | DLD | ring finger protein 149 | RNF149 |
| discs, large homolog 1 (Drosophila) | DLG1 | ring finger protein 20, E3 ubiquitin protein ligase | RNF20 |
| discs, large homolog 2 (Drosophila) | DLG2 | ring finger protein 213 | RNF213 |
| discs, large homolog 3 (Drosophila) | DLG3 | ring finger protein 40, E3 ubiquitin protein ligase | RNF40 |
| dihydrolipoamide S-succinyltransferase (E2 component of 2-oxo-glutarate complex) | DLST | RNA guanylyltransferase and 5'-phosphatase | RNGTT |
| DNA methyltransferase 1 associated protein 1 | DMAP1 | ribonuclease/angiogenin inhibitor 1 | RNH1 |
| deleted in malignant brain tumors 1 | DMBT1 | arginyl aminopeptidase (aminopeptidase B) | RNPEP |
| dystrophin | DMD | RNA binding protein S1, serine-rich domain | RNPS1 |
| dynein, axonemal, assembly factor 5 | DNAAF5 | roundabout, axon guidance receptor, homolog 2 (Drosophila) | ROBO2 |
| dynein, axonemal, heavy chain 12 | DNAH12 | Rho-associated, coiled-coil containing protein kinase 1 | ROCK1 |
| dynein, axonemal, heavy chain 17 | DNAH17 | Rho-associated, coiled-coil containing protein kinase 2 | ROCK2 |
| dynein, axonemal, heavy chain 2 | DNAH2 | rogdi homolog (Drosophila) | ROGDI |
| dynein, axonemal, heavy chain 5 | DNAH5 | receptor tyrosine kinase-like orphan receptor 1 | ROR1 |
| dynein, axonemal, heavy chain 7 | DNAH7 | retinitis pigmentosa 2 (X-linked recessive) | RP2 |
| dynein, axonemal, heavy chain 8 | DNAH8 | replication protein A1, 70kDa | RPA1 |
| DnaJ (Hsp40) homolog, subfamily A, member 1 | DNAJA1 | replication protein A2, 32kDa | RPA2 |
| DnaJ (Hsp40) homolog, subfamily A, member 2 | DNAJA2 | RPGRIP1-like | RPGRIP1L |
| DnaJ (Hsp40) homolog, subfamily B, member 1 | DNAJB1 | ribose 5-phosphate isomerase A | RPIA |
| DnaJ (Hsp40) homolog, subfamily B, member 11 | DNAJB11 | ribosomal protein L10 | RPL10 |
| DnaJ (Hsp40) homolog, subfamily B, member 3 | DNAJB3 | ribosomal protein L10a | RPL10A |
| DnaJ (Hsp40) homolog, subfamily B, member 6 | DNAJB6 | ribosomal protein L10a pseudogene 6 | RPL10AP6 |
| DnaJ (Hsp40) homolog, subfamily B, member 9 | DNAJB9 | ribosomal protein L10a pseudogene 9 | RPL10AP9 |
| DnaJ (Hsp40) homolog, subfamily C, member 10 | DNAJC10 | ribosomal protein L10-like | RPL10L |
| DnaJ (Hsp40) homolog, subfamily C, member 13 | DNAJC13 | ribosomal protein L11 | RPL11 |
| DnaJ (Hsp40) homolog, subfamily C, member 19 | DNAJC19 | ribosomal protein L12 | RPL12 |
| DnaJ (Hsp40) homolog, subfamily C, member 2 | DNAJC2 | ribosomal protein L12 pseudogene 14 | RPL12P14 |
| DnaJ (Hsp40) homolog, subfamily C, member 3 | DNAJC3 | ribosomal protein L12 pseudogene 19 | RPL12P19 |
| DnaJ (Hsp40) homolog, subfamily C, member 5 | DNAJC5 | ribosomal protein L12 pseudogene 2 | RPL12P2 |
| DnaJ (Hsp40) homolog, subfamily C, member 7 | DNAJC7 | ribosomal protein L12 pseudogene 32 | RPL12P32 |
| DnaJ (Hsp40) homolog, subfamily C, member 8 | DNAJC8 | ribosomal protein L12 pseudogene 35 | RPL12P35 |
| DnaJ (Hsp40) homolog, subfamily C, member 9 | DNAJC9 | ribosomal protein L12 pseudogene 6 | RPL12P6 |
| deoxyribonuclease I-like 1 | DNASE1 L 1 | ribosomal protein L13 | RPL13 |
| dynamin 1 | DNM1 | ribosomal protein L13a | RPL13A |
| dynamin 1-like | DNM1L | ribosomal protein L14 | RPL14 |
| dynamin 2 | DNM2 | ribosomal protein L15 | RPL15 |
| dynamin 3 | DNM3 | ribosomal protein L15 pseudogene 17 | RPL15P17 |
| DNA (cytosine-5-)-methyltransferase 1 | DNMT1 | ribosomal protein L15 pseudogene 18 | RPL15P18 |
| DNA (cytosine-5-)-methyltransferase 3 alpha | DNMT3A | ribosomal protein L15 pseudogene 22 | RPL15P22 |
| aspartyl aminopeptidase | DNPEP | ribosomal protein L15 pseudogene 3 | RPL15P3 |
| 2'-deoxynucleoside 5'-phosphate N-hydrolase 1 | DNPH1 | ribosomal protein L15 pseudogene 7 | RPL15P7 |
| DNA nucleotidylexotransferase | DNTT | ribosomal protein L17 | RPL17 |
| dedicator of cytokinesis 1 | DOCK1 | RPL17-C18orf32 readthrough | RPL17-C18orf32 |
| dedicator of cytokinesis 10 | DOCK10 | ribosomal protein L18 | RPL18 |
| dedicator of cytokinesis 11 | DOCK11 | ribosomal protein L18a | RPL18A |
| dedicator of cytokinesis 2 | DOCK2 | ribosomal protein L19 | RPL19 |
| dedicator of cytokinesis 5 | DOCK5 | ribosomal protein L21 | RPL21 |
| dedicator of cytokinesis 7 | DOCK7 | ribosomal protein L22 | RPL22 |
| dedicator of cytokinesis 8 | DOCK8 | ribosomal protein L22-like 1 | RPL22L1 |
| dedicator of cytokinesis 9 | DOCK9 | ribosomal protein L23 | RPL23 |
| docking protein 1, 62kDa (downstream of tyrosine kinase 1) | DOK1 | ribosomal protein L23a | RPL23A |
| docking protein 2, 56kDa | DOK2 | ribosomal protein L23a pseudogene 32 | RPL23AP32 |
| docking protein 3 | DOK3 | ribosomal protein L23a pseudogene 42 | RPL23AP42 |
| docking protein 7 | DOK7 | ribosomal protein L23 pseudogene 6 | RPL23P6 |
| dopey family member 2 | DOPEY2 | ribosomal protein L24 | RPL24 |
| dipeptidase 1 (renal) | DPEP1 | ribosomal protein L26 | RPL26 |
| dipeptidase 3 | DPEP3 | ribosomal protein L26-like 1 | RPL26L1 |
| dipeptidyl-peptidase 3 | DPP3 | ribosomal protein L27 | RPL27 |
| dipeptidyl-peptidase 4 | DPP4 | ribosomal protein L27a | RPL27A |
| dipeptidyl-peptidase 7 | DPP7 | ribosomal protein L28 | RPL28 |
| dpy-30 homolog (C. elegans) | DPY30 | ribosomal protein L29 | RPL29 |
| dihydropyrimidinase | DPYS | ribosomal protein L29 pseudogene 11 | RPL29P11 |
| dihydropyrimidinase-like 2 | DPYSL2 | ribosomal protein L29 pseudogene 12 | RPL29P12 |
| dihydropyrimidinase-like 3 | DPYSL3 | ribosomal protein L29 pseudogene 26 | RPL29P26 |
| developmentally regulated GTP binding protein 1 | DRG1 | ribosomal protein L29 pseudogene 9 | RPL29P9 |
| developmentally regulated GTP binding protein 2 | DRG2 | ribosomal protein L3 | RPL3 |
| desmocollin 1 | DSC1 | ribosomal protein L30 | RPL30 |
| desmocollin 2 | DSC2 | ribosomal protein L31 | RPL31 |
| desmocollin 3 | DSC3 | ribosomal protein L32 | RPL32 |
| Down syndrome critical region 3 | DSCR3 | ribosomal protein L34 | RPL34 |
| desmoglein 1 | DSG1 | ribosomal protein L35 | RPL35 |
| desmoglein 2 | DSG2 | ribosomal protein L35a | RPL35A |
| desmoglein 3 | DSG3 | ribosomal protein L36 | RPL36 |
| DSN1, MIS12 kinetochore complex component | DSN1 | ribosomal protein L37a | RPL37A |
| desmoplakin | DSP | ribosomal protein L38 | RPL38 |
| dystonin | DST | ribosomal protein L4 | RPL4 |
| destrin (actin depolymerizing factor) | DSTN | ribosomal protein L5 | RPL5 |
| deltex 3 like, E3 ubiquitin ligase | DTX3L | ribosomal protein L6 | RPL6 |
| deoxythymidylate kinase (thymidylate kinase) | DTYMK | ribosomal protein L7 | RPL7 |
| dual oxidase 2 | DUOX2 | ribosomal protein L7a | RPL7A |
| dihydrouridine synthase 3-like (S. cerevisiae) | DUS3L | ribosomal protein L8 | RPL8 |
| dual specificity phosphatase 26 (putative) | DUSP26 | ribosomal protein L9 | RPL9 |
| dual specificity phosphatase 3 | DUSP3 | ribosomal protein, large, P0 | RPLP0 |
| deoxyuridine triphosphatase | DUT | ribosomal protein, large, P0 pseudogene 2 | RPLP0P2 |
| dynein, cytoplasmic 1, heavy chain 1 | DYNC1 H1 | ribosomal protein, large, P0 pseudogene 3 | RPLP0P3 |
| dynein, cytoplasmic 1, intermediate chain 2 | DYNC112 | ribosomal protein, large, P0 pseudogene 6 | RPLP0P6 |
| dynein, cytoplasmic 1, light intermediate chain 1 | DYNC1LI1 | ribosomal protein, large, P1 | RPLP1 |
| dynein, cytoplasmic 1, light intermediate chain 2 | DYNC1LI2 | ribosomal protein, large, P2 | RPLP2 |
| dynein, cytoplasmic 2, heavy chain 1 | DYNC2H1 | ribophorin I | RPN1 |
| dynein, light chain, LC8-type 1 | DYNLL1 | ribophorin II | RPN2 |
| dynein, light chain, roadblock-type 1 | DYNLRB1 | ribonuclease P/MRP 30kDa subunit | RPP30 |
| dynein, light chain, Tctex-type 1 | DYNLT1 | regulation of nuclear pre-mRNA domain containing 1B | RPRD1B |
| dysferlin | DYSF | ribosomal protein S10 | RPS10 |
| dystrotelin | DYTN | ribosomal protein S10 pseudogene 11 | RPS10P11 |
| EBNA1 binding protein 2 | EBNA1BP 2 | ribosomal protein S10 pseudogene 13 | RPS10P13 |
| endothelin converting enzyme 1 | ECE1 | ribosomal protein S10 pseudogene 22 | RPS10P22 |
| enoyl CoA hydratase 1, peroxisomal | ECH1 | ribosomal protein S10 pseudogene 4 | RPS10P4 |
| ethylmalonyl-CoA decarboxylase 1 | ECHDC1 | ribosomal protein S10 pseudogene 7 | RPS10P7 |
| enoyl CoA hydratase, short chain, 1, mitochond rial | ECHS1 | ribosomal protein S11 | RPS11 |
| extracellular matrix protein 1 | ECM1 | ribosomal protein S12 | RPS12 |
| enhancer of mRNA decapping 3 | EDC3 | ribosomal protein S13 | RPS13 |
| enhancer of mRNA decapping 4 | EDC4 | ribosomal protein S14 | RPS14 |
| endothelial differentiation-related factor 1 | EDF1 | ribosomal protein S15 | RPS15 |
| EGF-like repeats and discoidin I-like domains 3 | EDIL3 | ribosomal protein S15a | RPS15A |
| endothelin receptor type B | EDNRB | ribosomal protein S16 | RPS16 |
| early endosome antigen 1 | EEA1 | ribosomal protein S16 pseudogene 1 | RPS16P1 |
| eukaryotic translation elongation factor 1 alpha 1 | EEF1A1 | ribosomal protein S16 pseudogene 10 | RPS16P10 |
| eukaryotic translation elongation factor 1 alpha 1 pseudogene 5 | EEF1A1P5 | ribosomal protein S17 | RPS17 |
| eukaryotic translation elongation factor 1 alpha 2 | EEF1A2 | ribosomal protein S18 | RPS18 |
| eukaryotic translation elongation factor 1 beta 2 | EEF1B2 | ribosomal protein S18 pseudogene 12 | RPS18P12 |
| eukaryotic translation elongation factor 1 delta (guanine nucleotide exchange protein) | EEF1D | ribosomal protein S18 pseudogene 5 | RPS18P5 |
| eukaryotic translation elongation factor 1 epsilon 1 | EEF1E1 | ribosomal protein S19 | RPS19 |
| eukaryotic translation elongation factor 1 gamma | EEF1G | ribosomal protein S2 | RPS2 |
| eukaryotic translation elongation factor 2 | EEF2 | ribosomal protein S20 | RPS20 |
| eukaryotic elongation factor, selenocysteine-tRNA-specific | EEFSEC | ribosomal protein S21 | RPS21 |
| EF-hand calcium binding domain 5 | EFCAB5 | ribosomal protein S23 | RPS23 |
| EGF containing fibulin-like extracellular matrix protein 1 | EFEMP1 | ribosomal protein S24 | RPS24 |
| EGF containing fibulin-like extracellular matrix protein 2 | EFEMP2 | ribosomal protein S25 | RPS25 |
| EF-hand domain family, member D2 | EFHD2 | ribosomal protein S26 | RPS26 |
| ephrin-B1 | EFNB1 | ribosomal protein S27 | RPS27 |
| EFR3 homolog A (S. cerevisiae) | EFR3A | ribosomal protein S27a | RPS27A |
| elongation factor Tu GTP binding domain containing 1 | EFTUD1 | ribosomal protein S27a pseudogene 11 | RPS27AP11 |
| elongation factor Tu GTP binding domain containing 2 | EFTUD2 | ribosomal protein S27a pseudogene 12 | RPS27AP12 |
| epidermal growth factor | EGF | ribosomal protein S27a pseudogene 16 | RPS27AP16 |
| EGF-like-domain, multiple 7 | EGFL7 | ribosomal protein S27-like | RPS27L |
| epidermal growth factor receptor | EGFR | ribosomal protein S28 | RPS28 |
| EH domain binding protein 1-like 1 | EHBP1L1 | ribosomal protein S29 | RPS29 |
| EH-domain containing 1 | EHD1 | ribosomal protein S2 pseudogene 11 | RPS2P11 |
| EH-domain containing 2 | EHD2 | ribosomal protein S2 pseudogene 12 | RPS2P12 |
| EH-domain containing 3 | EHD3 | ribosomal protein S2 pseudogene 17 | RPS2P17 |
| EH-domain containing 4 | EHD4 | ribosomal protein S2 pseudogene 20 | RPS2P20 |
| enoyl-CoA, hydratase/3-hydroxyacyl CoA dehydrogenase | EHHADH | ribosomal protein S2 pseudogene 5 | RPS2P5 |
| euchromatic histone-lysine N-methyltransferase 2 | EHMT2 | ribosomal protein S2 pseudogene 51 | RPS2P51 |
| eukaryotic translation initiation factor 1A, Y-linked | EIF1AY | ribosomal protein S2 pseudogene 55 | RPS2P55 |
| eukaryotic translation initiation factor 2A, 65kDa | EIF2A | ribosomal protein S2 pseudogene 8 | RPS2P8 |
| eukaryotic translation initiation factor 2-alpha kinase 2 | EIF2AK2 | ribosomal protein S3 | RPS3 |
| eukaryotic translation initiation factor 2B, subunit 1 alpha, 26kDa | EIF2B1 | ribosomal protein S3A | RPS3A |
| eukaryotic translation initiation factor 2B, subunit 2 beta, 39kDa | EIF2B2 | ribosomal protein S3 pseudogene 3 | RPS3P3 |
| eukaryotic translation initiation factor 2B, subunit 3 gamma, 58kDa | EIF2B3 | ribosomal protein S4, X-linked | RPS4X |
| eukaryotic translation initiation factor 2B, subunit 4 delta, 67kDa | EIF2B4 | ribosomal protein S4X pseudogene 13 | RPS4XP13 |
| eukaryotic translation initiation factor 2B, subunit 5 epsilon, 82kDa | EIF2B5 | ribosomal protein S4X pseudogene 6 | RPS4XP6 |
| eukaryotic translation initiation factor 2D | EIF2D | ribosomal protein S4, Y-linked 1 | RPS4Y1 |
| eukaryotic translation initiation factor 2, subunit 1 alpha, 35kDa | EIF2S1 | ribosomal protein S4, Y-linked 2 | RPS4Y2 |
| eukaryotic translation initiation factor 2, subunit 2 beta, 38kDa | EIF2S2 | ribosomal protein S5 | RPS5 |
| eukaryotic translation initiation factor 2, subunit 3 gamma, 52kDa | EIF2S3 | ribosomal protein S6 | RPS6 |
| eukaryotic translation initiation factor 3, subunit A | EIF3A | ribosomal protein S6 kinase, 90kDa, polypeptide 1 | RPS6KA1 |
| eukaryotic translation initiation factor 3, subunit B | EIF3B | ribosomal protein S6 kinase, 90kDa, polypeptide 3 | RPS6KA3 |
| eukaryotic translation initiation factor 3, subunit C | EIF3C | ribosomal protein S6 kinase, 90kDa, polypeptide 4 | RPS6KA4 |
| eukaryotic translation initiation factor 3, subunit C-like | EIF3CL | ribosomal protein S6 kinase, 90kDa, polypeptide 5 | RPS6KA5 |
| eukaryotic translation initiation factor 3, subunit D | EIF3D | ribosomal protein S6 kinase, 90kDa, polypeptide 6 | RPS6KA6 |
| eukaryotic translation initiation factor 3, subunit E | EIF3E | ribosomal protein S7 | RPS7 |
| eukaryotic translation initiation factor 3, subunit F | EIF3F | ribosomal protein S8 | RPS8 |
| eukaryotic translation initiation factor 3, subunit G | EIF3G | ribosomal protein S9 | RPS9 |
| eukaryotic translation initiation factor 3, subunit H | EIF3H | ribosomal protein SA | RPSA |
| eukaryotic translation initiation factor 3, subunit I | EIF3I | ribosomal protein SA pseudogene 12 | RPSAP12 |
| eukaryotic translation initiation factor 3, subunit J | EIF3J | ribosomal protein SA pseudogene 15 | RPSAP15 |
| eukaryotic translation initiation factor 3, subunit K | EIF3K | ribosomal protein SA pseudogene 18 | RPSAP18 |
| eukaryotic translation initiation factor 3, subunit L | EIF3L | ribosomal protein SA pseudogene 19 | RPSAP19 |
| eukaryotic translation initiation factor 3, subunit M | EIF3M | ribosomal protein SA pseudogene 29 | RPSAP29 |
| eukaryotic translation initiation factor 4A1 | EIF4A1 | ribosomal protein SA pseudogene 58 | RPSAP58 |
| eukaryotic translation initiation factor 4A2 | EIF4A2 | ribosomal protein SA pseudogene 61 | RPSAP61 |
| eukaryotic translation initiation factor 4A3 | EIF4A3 | ribosomal protein SA pseudogene 8 | RPSAP8 |
| eukaryotic translation initiation factor 4B | EIF4B | ribosomal protein SA pseudogene 9 | RPSAP9 |
| eukaryotic translation initiation factor 4E | EIF4E | repetin | RPTN |
| eukaryotic translation initiation factor 4 gamma, 1 | EIF4G1 | regulatory associated protein of MTOR, complex 1 | RPTOR |
| eukaryotic translation initiation factor 4H | EIF4H | RCD1 required for cell differentiation1 homolog (S. pombe) | RQCD1 |
| eukaryotic translation initiation factor 5 | EIF5 | Ras-related GTP binding A | RRAGA |
| eukaryotic translation initiation factor 5A | EIF5A | Ras-related GTP binding B | RRAGB |
| eukaryotic translation initiation factor 5A2 | EIF5A2 | Ras-related GTP binding C | RRAGC |
| eukaryotic translation initiation factor 5Alike 1 | EIF5AL1 | Ras-related GTP binding D | RRAGD |
| eukaryotic translation initiation factor 5B | EIF5B | related RAS viral (r-ras) oncogene homolog | RRAS |
| eukaryotic translation initiation factor 6 | EIF6 | related RAS viral (r-ras) oncogene homolog 2 | RRAS2 |
| elastase, neutrophil expressed | ELANE | ras responsive element binding protein 1 | RREB1 |
| ELAV like RNA binding protein 1 | ELAVL1 | ribonucleotide reductase M1 | RRM1 |
| engulfment and cell motility 1 | ELMO1 | ribonucleotide reductase M2 | RRM2 |
| engulfment and cell motility 2 | ELMO2 | ribosomal RNA processing 12 homolog (S. cerevisiae) | RRP12 |
| engulfment and cell motility 3 | ELMO3 | ribosomal RNA processing 1B | RRP1B |
| elongator acetyltransferase complex subunit 2 | ELP2 | ribosomal RNA processing 9, small subunit (SSU) processome component, homolog (yeast) | RRP9 |
| elongator acetyltransferase complex subunit 3 | ELP3 | RRS1 ribosome biogenesis regulator homolog (S. cerevisiae) | RRS1 |
| embigin | EMB | ribosomal L1 domain containing 1 | RSL1 D1 |
| emerin | EMD | ring finger and SPRY domain containing 1 | RSPRY1 |
| essential meiotic structure-specific endonuclease subunit 2 | EME2 | Ras suppressor protein 1 | RSU1 |
| EMG1 N1-specific pseudouridine methyltransferase | EMG1 | RNA 3'-terminal phosphate cyclase | RTCA |
| elastin microfibril interfacer 1 | EMILIN1 | RNA 2',3'-cyclic phosphate and 5'-OH ligase | RTCB |
| elastin microfibril interfacer 2 | EMILIN2 | regulator of telomere elongation helicase 1 | RTEL1 |
| echinoderm microtubule associated protein like 3 | EML3 | rhotekin | RTKN |
| echinoderm microtubule associated protein like 4 | EML4 | retrotransposon-like 1 | RTL1 |
| echinoderm microtubule associated protein like 5 | EML5 | reticulon 2 | RTN2 |
| enabled homolog (Drosophila) | ENAH | reticulon 3 | RTN3 |
| endonuclease domain containing 1 | ENDOD1 | reticulon 4 | RTN4 |
| endoglin | ENG | reticulon 4 receptor | RTN4R |
| endo-beta-N-acetylglucosaminidase | ENGASE | RUN and FYVE domain containing 1 | RUFY1 |
| enolase 1, (alpha) | ENO1 | RUN and SH3 domain containing 2 | RUSC2 |
| enolase 2 (gamma, neuronal) | ENO2 | RuvB-like AAA ATPase 1 | RUVBL1 |
| enolase 3 (beta, muscle) | ENO3 | RuvB-like AAA ATPase 2 | RUVBL2 |
| glutamyl aminopeptidase (aminopeptidase A) | ENPEP | ryanodine receptor 1 (skeletal) | RYR1 |
| ectonucleotide pyrophosphatase/phosphodiesterase 1 | ENPP1 | ryanodine receptor 2 (cardiac) | RYR2 |
| ectonucleotide pyrophosphatase/phosphodiesterase 3 | ENPP3 | S100 calcium binding protein A10 | S100A10 |
| ectonucleotide pyrophosphatase/phosphodiesterase 4 (putative) | ENPP4 | S100 calcium binding protein A11 | S100A11 |
| ectonucleotide pyrophosphatase/phosphodiesterase 6 | ENPP6 | S100 calcium binding protein A11 pseudogene 1 | S100A11P1 |
| ectonucleotide pyrophosphatase/phosphodiesterase 7 | ENPP7 | S100 calcium binding protein A13 | S100A13 |
| ectonucleoside triphosphate diphosphohydrolase 1 | ENTPD1 | S100 calcium binding protein A14 | S100A14 |
| ectonucleoside triphosphate diphosphohydrolase 2 | ENTPD2 | S100 calcium binding protein A16 | S100A16 |
| ectonucleoside triphosphate diphosphohydrolase 4 | ENTPD4 | S100 calcium binding protein A4 | S100A4 |
| erythrocyte membrane protein band 4.1 | EPB41 | S100 calcium binding protein A6 | S100A6 |
| erythrocyte membrane protein band 4.1-like 1 | EPB41L1 | S100 calcium binding protein A7 | S100A7 |
| erythrocyte membrane protein band 4.1-like 2 | EPB41L2 | S100 calcium binding protein A7A | S100A7A |
| erythrocyte membrane protein band 4.1-like 3 | EPB41L3 | S100 calcium binding protein A8 | S100A8 |
| erythrocyte membrane protein band 4.1 like 4B | EPB41L4B | S100 calcium binding protein A9 | S100A9 |
| erythrocyte membrane protein band 4.1 like 5 | EPB41L5 | S100 calcium binding protein P | S100P |
| erythrocyte membrane protein band 4.2 | EPB42 | S100P binding protein | S100PBP |
| epithelial cell adhesion molecule | EPCAM | serum amyloid A1 | SAA1 |
| EPH receptor A1 | EPHA1 | serum amyloid A2 | SAA2 |
| EPH receptor A2 | EPHA2 | serum amyloid A4, constitutive | SAA4 |
| EPH receptor A3 | EPHA3 | SAC1 suppressor of actin mutations 1-like (yeast) | SACM1 L |
| EPH receptor A4 | EPHA4 | sacsin molecular chaperone | SACS |
| EPH receptor A5 | EPHA5 | SUMO1 activating enzyme subunit 1 | SAE1 |
| EPH receptor B1 | EPHB1 | S-antigen; retina and pineal gland (arrestin) | SAG |
| EPH receptor B2 | EPHB2 | spalt-like transcription factor 1 | SALL1 |
| EPH receptor B3 | EPHB3 | sterile alpha motif domain containing 4A | SAMD4A |
| EPH receptor B4 | EPHB4 | sterile alpha motif domain containing 9 | SAMD9 |
| epoxide hydrolase 1, microsomal (xenobiotic) | EPHX1 | sterile alpha motif domain containing 9-like | SAMD9L |
| epoxide hydrolase 2, cytoplasmic | EPHX2 | SAM domain and HD domain 1 | SAMHD1 |
| epsin 2 | EPN2 | SAMM50 sorting and assembly machinery component | SAMM50 |
| epsin 3 | EPN3 | Sin3A-associated protein, 18kDa | SAP18 |
| erythropoietin | EPO | Sin3A-associated protein, 30kDa | SAP30 |
| epiplakin 1 | EPPK1 | secretion associated, Ras related GTPase 1A | SAR1A |
| glutamyl-prolyl-tRNA synthetase | EPRS | secretion associated, Ras related GTPase 1B | SAR1B |
| epidermal growth factor receptor pathway substrate 15 | EPS15 | seryl-tRNA synthetase | SARS |
| epidermal growth factor receptor pathway substrate 15-like 1 | EPS15L1 | squamous cell carcinoma antigen recognized by T cells 3 | SART3 |
| epidermal growth factor receptor pathway substrate 8 | EPS8 | spindle assembly 6 homolog (C. elegans) | SASS6 |
| EPS8-like 1 | EPS8L1 | spermidine/spermine N1-acetyltransferase family member 2 | SAT2 |
| EPS8-like 2 | EPS8L2 | SATB homeobox 1 | SATB1 |
| EPS8-like 3 | EPS8L3 | Shwachman-Bodian-Diamond syndrome | SBDS |
| eosinophil peroxidase | EPX | SET binding factor 1 | SBF1 |
| endoplasmic reticulum aminopeptidase 1 | ERAP1 | suprabasin | SBSN |
| erb-b2 receptor tyrosine kinase 2 | ERBB2 | secretory carrier membrane protein 1 | SCAMP1 |
| erbb2 interacting protein | ERBB2IP | secretory carrier membrane protein 2 | SCAMP2 |
| erb-b2 receptor tyrosine kinase 3 | ERBB3 | secretory carrier membrane protein 3 | SCAMP3 |
| erb-b2 receptor tyrosine kinase 4 | ERBB4 | secretory carrier membrane protein 4 | SCAMP4 |
| excision repair cross-complementation group 2 | ERCC2 | scavenger receptor class B, member 1 | SCARB1 |
| endoplasmic reticulum-golgi intermediate compartment (ERGIC) 1 | ERGIC1 | scavenger receptor class B, member 2 | SCARB2 |
| ERGIC and golgi 2 | ERGIC2 | saccharopine dehydrogenase (putative) | SCCPDH |
| ER lipid raft associated 1 | ERLIN1 | sciellin | SCEL |
| ER lipid raft associated 2 | ERLIN2 | sec1 family domain containing 1 | SCFD1 |
| erythroblast membrane-associated protein (Scianna blood group) | ERMAP | single-chain Fv fragment | SCFV |
| ermin, ERM-like protein | ERMN | secretoglobin, family 2A, member 1 | SCGB2A1 |
| endoplasmic reticulum metallopeptidase 1 | ERMP1 | secretoglobin, family 3A, member 1 | SCGB3A1 |
| ERO1-like (S. cerevisiae) | ERO1L | scinderin | SCIN |
| endoplasmic reticulum protein 29 | ERP29 | sodium channel, voltage gated, type X alpha subunit | SCN10A |
| endoplasmic reticulum protein 44 | ERP44 | sodium channel, voltage gated, type XI alpha subunit | SCN11A |
| endogenous retrovirus group FRD, member 2 | ERVFRD-2 | sodium channel, voltage gated, type V alpha subunit | SCN5A |
| endogenous retrovirus group K, member 6 | ERVK-6 | short coiled-coil protein | SCOC |
| endothelial cell adhesion molecule | ESAM | serine carboxypeptidase 1 | SCPEP1 |
| esterase D | ESD | scribbled planar cell polarity protein | SCRIB |
| endothelial cell-specific molecule 1 | ESM1 | secernin 1 | SCRN1 |
| epithelial splicing regulatory protein 1 | ESRP1 | secernin 2 | SCRN2 |
| epithelial splicing regulatory protein 2 | ESRP2 | scratch family zinc finger 1 | SCRT1 |
| extended synaptotagmin-like protein 1 | ESYT1 | SCY1-like 1 (S. cerevisiae) | SCYL1 |
| extended synaptotagmin-like protein 2 | ESYT2 | SCY1-like 2 (S. cerevisiae) | SCYL2 |
| eukaryotic translation termination factor 1 | ETF1 | syndecan 1 | SDC1 |
| electron-transfer-flavoprotein, alpha polypeptide | ETFA | syndecan 2 | SDC2 |
| electron-transfer-flavoprotein, beta polypeptide | ETFB | syndecan 4 | SDC4 |
| ethylmalonic encephalopathy 1 | ETHE1 | syndecan binding protein (syntenin) | SDCBP |
| eva-1 homolog A (C. elegans) | EVA1A | syndecan binding protein (syntenin) 2 | SDCBP2 |
| eva-1 homolog B (C. elegans) | EVA1B | stromal cell-derived factor 2-like 1 | SDF2L1 |
| ecotropic viral integration site 2B | EVI2B | stromal cell derived factor 4 | SDF4 |
| Enah/Vasp-like | EVL | succinate dehydrogenase complex, subunit A, flavoprotein (Fp) | SDHA |
| envoplakin | EVPL | sidekick cell adhesion molecule 2 | SDK2 |
| exocyst complex component 1 | EXOC1 | serum deprivation response | SDPR |
| exocyst complex component 2 | EXOC2 | SEC11 homolog A (S. cerevisiae) | SEC11A |
| exocyst complex component 3 | EXOC3 | SEC13 homolog (S. cerevisiae) | SEC13 |
| exocyst complex component 3-like 4 | EXOC3L4 | SEC14-like 4 (S. cerevisiae) | SEC14L4 |
| exocyst complex component 4 | EXOC4 | SEC16 homolog A (S. cerevisiae) | SEC16A |
| exocyst complex component 5 | EXOC5 | SEC22 vesicle trafficking protein homolog B (S. cerevisiae) (gene/pseudogene) | SEC22B |
| exocyst complex component 6 | EXOC6 | Sec23 homolog A (S. cerevisiae) | SEC23A |
| exocyst complex component 6B | EXOC6B | Sec23 homolog B (S. cerevisiae) | SEC23B |
| exocyst complex component 7 | EXOC7 | SEC23 interacting protein | SEC231P |
| exocyst complex component 8 | EXOC8 | SEC24 family member A | SEC24A |
| exosome component 1 | EXOSC1 | SEC24 family member B | SEC24B |
| exosome component 3 | EXOSC3 | SEC24 family member C | SEC24C |
| exosome component 4 | EXOSC4 | SEC24 family member D | SEC24D |
| exosome component 5 | EXOSC5 | SEC31 homolog A (S. cerevisiae) | SEC31A |
| exosome component 6 | EXOSC6 | Sec61 beta subunit | SEC61 B |
| exosome component 7 | EXOSC7 | secreted and transmembrane 1 | SECTM1 |
| exostosin glycosyltransferase 2 | EXT2 | SEH1-like (S. cerevisiae) | SEH1L |
| exostosin-like glycosyltransferase 2 | EXTL2 | selenium binding protein 1 | SELENBP1 |
| eyes shut homolog (Drosophila) | EYS | selectin L | SELL |
| ezrin | EZR | selectin P (granule membrane protein 140kDa, antigen CD62) | SELP |
| coagulation factor X | F10 | selectin P ligand | SELPLG |
| coagulation factor XI | F11 | sema domain, immunoglobulin domain (Ig), short basic domain, secreted, (semaphorin) 3C | SEMA3C |
| F11 receptor | F11R | sema domain, immunoglobulin domain (Ig), short basic domain, secreted, (semaphorin) 3F | SEMA3F |
| coagulation factor XIII, A1 polypeptide | F13A1 | sema domain, immunoglobulin domain (Ig), short basic domain, secreted, (semaphorin) 3G | SEMA3G |
| coagulation factor II (thrombin) | F2 | sema domain, immunoglobulin domain (Ig), transmembrane domain (TM) and short cytoplasmic domain, (semaphorin) 4C | SEMA4C |
| coagulation factor II (thrombin) receptor | F2R | sema domain, seven thrombospondin repeats (type 1 and type 1-like), transmembrane domain (TM) and short cytoplasmic domain, (semaphorin) 5A | SEMA5A |
| coagulation factor II (thrombin) receptor-like 3 | F2RL3 | semenogelin I | SEMG1 |
| coagulation factor III (thromboplastin, tissue factor) | F3 | semenogelin II | SEMG2 |
| coagulation factor V (proaccelerin, labile factor) | F5 | selenoprotein P, plasma, 1 | SEPP1 |
| coagulation factor VII (serum prothrombin conversion accelerator) | F7 | septin 1 | SEPT1 |
| coagulation factor VIII, procoagulant component | F8 | septin 10 | SEPT10 |
| coagulation factor IX | F9 | septin 11 | SEPT11 |
| fatty acid binding protein 1, liver | FABP1 | septin 2 | SEPT2 |
| fatty acid binding protein 3, muscle and heart | FABP3 | septin 5 | SEPT5 |
| fatty acid binding protein 5 (psoriasis-associated) | FABP5 | septin 6 | SEPT6 |
| fatty acid binding protein 5 pseudogene 7 | FABP5P7 | septin 7 | SEPT7 |
| Fas (TNFRSF6)-associated via death domain | FADD | septin 8 | SEPT8 |
| Fas (TNFRSF6) associated factor 1 | FAF1 | septin 9 | SEPT9 |
| fumarylacetoacetate hydrolase (fumarylacetoacetase) | FAH | SERPINE1 mRNA binding protein 1 | SERBP1 |
| fumarylacetoacetate hydrolase domain containing 2A | FAHD2A | serine incorporator 1 | SERINC1 |
| family with sequence similarity 104, member A | FAM104A | serine incorporator 2 | SERINC2 |
| family with sequence similarity 120A | FAM120A | serine incorporator 3 | SERINC3 |
| family with sequence similarity 120B | FAM120B | serine incorporator 5 | SERINC5 |
| family with sequence similarity 129, member A | FAM129A | serpin peptidase inhibitor, clade A (alpha-1 antiproteinase, antitrypsin), member 1 | SERPINA1 |
| family with sequence similarity 129, member B | FAM129B | serpin peptidase inhibitor, clade A (alpha-1 antiproteinase, antitrypsin), member 3 | SERPINA3 |
| family with sequence similarity 151, member A | FAM151A | serpin peptidase inhibitor, clade A (alpha-1 antiproteinase, antitrypsin), member 4 | SERPINA4 |
| family with sequence similarity 160, member A2 | FAM160A 2 | serpin peptidase inhibitor, clade A (alpha-1 antiproteinase, antitrypsin), member 5 | SERPINA5 |
| family with sequence similarity 171, member A1 | FAM171A 1 | serpin peptidase inhibitor, clade A (alpha-1 antiproteinase, antitrypsin), member 7 | SERPINA7 |
| family with sequence similarity 171, member A2 | FAM171A 2 | serpin peptidase inhibitor, clade B (ovalbumin), member 1 | SERPINB1 |
| family with sequence similarity 174, member A | FAM174A | serpin peptidase inhibitor, clade B (ovalbumin), member 12 | SERPINB12 |
| family with sequence similarity 174, member B | FAM174B | serpin peptidase inhibitor, clade B (ovalbumin), member 13 | SERPINB13 |
| family with sequence similarity 175, member B | FAM175B | serpin peptidase inhibitor, clade B (ovalbumin), member 3 | SERPINB3 |
| family with sequence similarity 177, member A1 | FAM177A 1 | serpin peptidase inhibitor, clade B (ovalbumin), member 4 | SERPINB4 |
| family with sequence similarity 178, member B | FAM178B | serpin peptidase inhibitor, clade B (ovalbumin), member 5 | SERPINB5 |
| family with sequence similarity 179, member B | FAM179B | serpin peptidase inhibitor, clade B (ovalbumin), member 6 | SERPINB6 |
| family with sequence similarity 184, member A | FAM184A | serpin peptidase inhibitor, clade B (ovalbumin), member 9 | SERPINB9 |
| family with sequence similarity 186, member A | FAM186A | serpin peptidase inhibitor, clade C (antithrombin), member 1 | SERPINC1 |
| family with sequence similarity 193, member B | FAM193B | serpin peptidase inhibitor, clade D (heparin cofactor), member 1 | SERPIND1 |
| family with sequence similarity 208, member B | FAM208B | serpin peptidase inhibitor, clade E (nexin, plasminogen activator inhibitor type 1), member 1 | SERPINE1 |
| family with sequence similarity 209, member A | FAM209A | serpin peptidase inhibitor, clade E (nexin, plasminogen activator inhibitor type 1), member 2 | SERPINE2 |
| family with sequence similarity 20, member A | FAM20A | serpin peptidase inhibitor, clade F (alpha-2 antiplasmin, pigment epithelium derived factor), member 1 | SERPINF1 |
| family with sequence similarity 20, member C | FAM20C | serpin peptidase inhibitor, clade F (alpha-2 antiplasmin, pigment epithelium derived factor), member 2 | SERPINF2 |
| family with sequence similarity 213, member A | FAM213A | serpin peptidase inhibitor, clade G (C1 inhibitor), member 1 | SERPING1 |
| family with sequence similarity 213, member B | FAM213B | serpin peptidase inhibitor, clade H (heat shock protein 47), member 1, (collagen binding protein 1) | SERPINH1 |
| family with sequence similarity 3, member B | FAM3B | SEC14 and spectrin domains 1 | SESTD1 |
| family with sequence similarity 3, member C | FAM3C | SET nuclear proto-oncogene | SET |
| family with sequence similarity 49, member A | FAM49A | SET domain containing 4 | SETD4 |
| family with sequence similarity 49, member B | FAM49B | splicing factor 3a, subunit 1, 120kDa | SF3A1 |
| family with sequence similarity 63, member A | FAM63A | splicing factor 3a, subunit 2, 66kDa | SF3A2 |
| family with sequence similarity 63, member B | FAM63B | splicing factor 3a, subunit 3, 60kDa | SF3A3 |
| family with sequence similarity 64, member A | FAM64A | splicing factor 3b, subunit 1, 155kDa | SF3B1 |
| family with sequence similarity 65, member A | FAM65A | splicing factor 3b, subunit 2, 145kDa | SF3B2 |
| family with sequence similarity 65, member C | FAM65C | splicing factor 3b, subunit 3, 130kDa | SF3B3 |
| family with sequence similarity 71, member F1 | FAM71 F1 | splicing factor 3b, subunit 4, 49kDa | SF3B4 |
| family with sequence similarity 83, member F | FAM83F | splicing factor 3b, subunit 6, 14kDa | SF3B6 |
| family with sequence similarity 83, member H | FAM83H | Sfi1 homolog, spindle assembly associated (yeast) | SFI1 |
| family with sequence similarity 84, member B | FAM84B | Scm-like with four mbt domains 1 | SFMBT1 |
| family with sequence similarity 91, member A1 | FAM91A1 | stratifin | SFN |
| family with sequence similarity 98, member A | FAM98A | splicing factor proline/glutamine-rich | SFPQ |
| family with sequence similarity 98, member B | FAM98B | secreted frizzled-related protein 1 | SFRP1 |
| Fanconi anemia, complementation group I | FANCI | secreted frizzled-related protein 4 | SFRP4 |
| Fanconi anemia, complementation group M | FANCM | SFT2 domain containing 2 | SFT2D2 |
| fibroblast activation protein, alpha | FAP | sideroflexin 1 | SFXN1 |
| FERM, RhoGEF (ARHGEF) and pleckstrin domain protein 1 (chondrocyte-derived) | FARP1 | sphingosine-1-phosphate lyase 1 | SGPL1 |
| phenylalanyl-tRNA synthetase, alpha subunit | FARSA | small glutamine-rich tetratricopeptide repeat (TPR)-containing, alpha | SGTA |
| phenylalanyl-tRNA synthetase, beta subunit | FARSB | SH2B adaptor protein 1 | SH2B1 |
| Fas cell surface death receptor | FAS | SH2 domain containing 1A | SH2D1A |
| Fas ligand (TNF superfamily, member 6) | FASLG | SH3 domain binding glutamate-rich protein like | SH3BGRL |
| fatty acid synthase | FASN | SH3 domain binding glutamate-rich protein like 3 | SH3BGRL3 |
| FAT atypical cadherin 1 | FAT1 | SH3-domain binding protein 4 | SH3BP4 |
| FAT atypical cadherin 2 | FAT2 | SH3-domain GRB2-like 1 | SH3GL1 |
| FAT atypical cadherin 4 | FAT4 | SH3-domain GRB2-like endophilin B1 | SH3GLB1 |
| fibrillarin | FBL | SH3-domain kinase binding protein 1 | SH3KBP1 |
| fibulin 1 | FBLN1 | SH3 and multiple ankyrin repeat domains 3 | SHANK3 |
| fibulin 2 | FBLN2 | sex hormone-binding globulin | SHBG |
| fibrillin 1 | FBN1 | SHC (Src homology 2 domain containing) transforming protein 1 | SHC1 |
| fibrillin 2 | FBN2 | SHC (Src homology 2 domain containing) transforming protein 2 | SHC2 |
| fibrillin 3 | FBN3 | shisa family member 2 | SHISA2 |
| fructose-1,6-bisphosphatase 1 | FBP1 | serine hydroxymethyltransferase 1 (soluble) | SHMT1 |
| fructose-1,6-bisphosphatase 2 | FBP2 | serine hydroxymethyltransferase 2 (mitochondrial) | SHMT2 |
| F-box and leucine-rich repeat protein 12 | FBXL12 | soc-2 suppressor of clear homolog (C. elegans) | SHOC2 |
| F-box and leucine-rich repeat protein 20 | FBXL20 | sedoheptulokinase | SHPK |
| F-box and leucine-rich repeat protein 4 | FBXL4 | shroom family member 1 | SHROOM1 |
| F-box and leucine-rich repeat protein 8 | FBXL8 | shroom family member 2 | SHROOM2 |
| F-box protein 15 | FBXO15 | shroom family member 3 | SHROOM3 |
| F-box protein 17 | FBXO17 | sucrase-isomaltase (alpha-glucosidase) | SI |
| F-box protein 2 | FBXO2 | sialic acid acetylesterase | SIAE |
| F-box protein 22 | FBXO22 | single immunoglobulin and toll-interleukin 1 receptor (TIR) domain | SIGIRR |
| F-box protein 45 | FBXO45 | sialic acid binding Ig-like lectin 1, sialoadhesin | SIGLEC1 |
| F-box protein 6 | FBXO6 | signal-induced proliferation-associated 1 like 1 | SIPA1L1 |
| F-box and WD repeat domain containing 8 | FBXW8 | signal-regulatory protein alpha | SIRPA |
| Fc fragment of IgE, high affinity I, receptor for; gamma polypeptide | FCER1G | signal-regulatory protein beta 1 | SIRPB1 |
| Fc fragment of IgE, low affinity II, receptor for (CD23) | FCER2 | signaling threshold regulating transmembrane adaptor 1 | SIT1 |
| Fc fragment of IgG binding protein | FCGBP | src kinase associated phosphoprotein 1 | SKAP1 |
| Fc fragment of IgG, low affinity IIa, receptor (CD32) | FCGR2A | src kinase associated phosphoprotein 2 | SKAP2 |
| Fc fragment of IgG, low affinity IIc, receptor for (CD32) (gene/pseudogene) | FCGR2C | superkiller viralicidic activity 2-like (S. cerevisiae) | SKIV2L |
| Fc fragment of IgG, receptor, transporter, alpha | FCGRT | superkiller viralicidic activity 2-like 2 (S. cerevisiae) | SKIV2L2 |
| ficolin (collagen/fibrinogen domain containing) 1 | FCN1 | SKI family transcriptional corepressor 2 | SKOR2 |
| ficolin (collagen/fibrinogen domain containing lectin) 2 | FCN2 | S-phase kinase-associated protein 1 | SKP1 |
| ficolin (collagen/fibrinogen domain containing) 3 | FCN3 | Src-like-adaptor 2 | SLA2 |
| Fc receptor-like A | FCRLA | SLAIN motif family, member 1 | SLAIN1 |
| farnesyl-diphosphate farnesyltransferase 1 | FDFT1 | signaling lymphocytic activation molecule family member 1 | SLAMF1 |
| farnesyl diphosphate synthase | FDPS | SLAM family member 6 | SLAMF6 |
| flap structure-specific endonuclease 1 | FEN1 | solute carrier family 10, member 3 | SLC10A3 |
| fermitin family member 2 | FERMT2 | solute carrier family 12 (sodium/potassium/chloride transporter), member 1 | SLC12A1 |
| fermitin family member 3 | FERMT3 | solute carrier family 12 (sodium/potassium/chloride transporter), member 2 | SLC12A2 |
| FES proto-oncogene, tyrosine kinase | FES | solute carrier family 12 (sodium/chloride transporter), member 3 | SLC12A3 |
| fibrinogen alpha chain | FGA | solute carrier family 12 (potassium/chloride transporter), member 4 | SLC12A4 |
| fibrinogen beta chain | FGB | solute carrier family 12 (potassium/chloride transporter), member 5 | SLC12A5 |
| FYVE, RhoGEF and PH domain containing 2 | FGD2 | solute carrier family 12 (potassium/chloride transporter), member 6 | SLC12A6 |
| FYVE, RhoGEF and PH domain containing 4 | FGD4 | solute carrier family 12 (potassium/chloride transporter), member 7 | SLC12A7 |
| fibroblast growth factor 16 | FGF16 | solute carrier family 12, member 9 | SLC12A9 |
| fibroblast growth factor 18 | FGF18 | solute carrier family 13 (sodium-dependent dicarboxylate transporter), member 2 | SLC13A2 |
| fibroblast growth factor 19 | FGF19 | solute carrier family 13 (sodium-dependent dicarboxylate transporter), member 3 | SLC13A3 |
| fibroblast growth factor binding protein 1 | FGFBP1 | solute carrier family 15 (oligopeptide transporter), member 2 | SLC15A2 |
| fibroblast growth factor receptor 1 | FGFR1 | solute carrier family 16 (monocarboxylate transporter), member 1 | SLC16A1 |
| fibroblast growth factor receptor 2 | FGFR2 | solute carrier family 16 (aromatic amino acid transporter), member 10 | SLC16A10 |
| fibroblast growth factor receptor 3 | FGFR3 | solute carrier family 16 (monocarboxylate transporter), member 3 | SLC16A3 |
| fibroblast growth factor receptor 4 | FGFR4 | solute carrier family 16, member 6 | SLC16A6 |
| fibroblast growth factor receptor-like 1 | FGFRL1 | solute carrier family 19 (folate transporter), member 1 | SLC19A1 |
| fibrinogen gamma chain | FGG | solute carrier family 19 (thiamine transporter), member 2 | SLC19A2 |
| fibrinogen-like 2 | FGL2 | solute carrier family 1 (neuronal/epithelial high affinity glutamate transporter, system Xag), member 1 | SLC1A1 |
| FGR proto-oncogene, Src family tyrosine kinase | FGR | solute carrier family 1 (glial high affinity glutamate transporter), member 3 | SLC1A3 |
| fumarate hydratase | FH | solute carrier family 1 (glutamate/neutral amino acid transporter), member 4 | SLC1A4 |
| forkhead-associated (FHA) phosphopeptide binding domain 1 | FHAD1 | solute carrier family 1 (neutral amino acid transporter), member 5 | SLC1A5 |
| four and a half LIM domains 1 | FHL1 | solute carrier family 20 (phosphate transporter), member 1 | SLC20A1 |
| four and a half LIM domains 5 | FHL5 | solute carrier family 20 (phosphate transporter), member 2 | SLC20A2 |
| formin homology 2 domain containing 1 | FHOD1 | solute carrier family 22 (organic anion/urate transporter), member 11 | SLC22A11 |
| fidgetin-like 1 | FIGNL1 | solute carrier family 22 (organic anion/urate transporter), member 12 | SLC22A12 |
| FK506 binding protein 15, 133kDa | FKBP15 | solute carrier family 22 (organic anion/urate transporter), member 13 | SLC22A13 |
| FK506 binding protein 1A, 12kDa | FKBP1A | solute carrier family 22 (organic cation/carnitine transporter), member 16 | SLC22A16 |
| FK506 binding protein 3, 25kDa | FKBP3 | solute carrier family 22 (organic cation transporter), member 2 | SLC22A2 |
| FK506 binding protein 4, 59kDa | FKBP4 | solute carrier family 22 (organic cation/carnitine transporter), member 5 | SLC22A5 |
| FK506 binding protein 5 | FKBP5 | solute carrier family 22 (organic anion transporter), member 6 | SLC22A6 |
| filaggrin family member 2 | FLG2 | solute carrier family 22 (organic anion transporter), member 8 | SLC22A8 |
| flightless I homolog (Drosophila) | FLII | solute carrier family 23 (ascorbic acid transporter), member 1 | SLC23A1 |
| filamin A, alpha | FLNA | solute carrier family 23 (ascorbic acid transporter), member 2 | SLC23A2 |
| filamin B, beta | FLNB | solute carrier family 25 (mitochondrial carrier; citrate transporter), member 1 | SLC25A1 |
| filamin C, gamma | FLNC | solute carrier family 25 (mitochondrial carrier; dicarboxylate transporter), member 10 | SLC25A10 |
| flotillin 1 | FLOT1 | solute carrier family 25 (mitochondrial carrier; oxoglutarate carrier), member 11 | SLC25A11 |
| flotillin 2 | FLOT2 | solute carrier family 25 (aspartate/glutamate carrier), member 13 | SLC25A13 |
| fms-related tyrosine kinase 1 | FLT1 | solute carrier family 25 (mitochondrial carrier; phosphate carrier), member 3 | SLC25A3 |
| feline leukemia virus subgroup C cellular receptor 1 | FLVCR1 | solute carrier family 25 (mitochondrial carrier; adenine nucleotide translocator), member 31 | SLC25A31 |
| formin 1 | FMN1 | solute carrier family 25 (mitochondrial carrier; adenine nucleotide translocator), member 4 | SLC25A4 |
| formin-like 1 | FMNL1 | solute carrier family 25 (mitochondrial carrier; adenine nucleotide translocator), member 5 | SLC25A5 |
| formin-like 2 | FMNL2 | solute carrier family 25 (mitochondrial carrier; adenine nucleotide translocator), member 6 | SLC25A6 |
| formin-like 3 | FMNL3 | solute carrier family 26 (anion exchanger), member 11 | SLC26A11 |
| fibromodulin | FMOD | solute carrier family 26 (anion exchanger), member 2 | SLC26A2 |
| fibronectin 1 | FN1 | solute carrier family 26 (anion exchanger), member 4 | SLC26A4 |
| fructosamine 3 kinase | FN3K | solute carrier family 26 (anion exchanger), member 6 | SLC26A6 |
| fructosamine 3 kinase related protein | FN3KRP | solute carrier family 26 (anion exchanger), member 9 | SLC26A9 |
| formin binding protein 1-like | FNBP1L | solute carrier family 27 (fatty acid transporter), member 2 | SLC27A2 |
| fibronectin type III domain containing 1 | FNDC1 | solute carrier family 29 (equilibrative nucleoside transporter), member 1 | SLC29A1 |
| farnesyltransferase, CAAX box, alpha | FNTA | solute carrier family 29 (equilibrative nucleoside transporter), member 2 | SLC29A2 |
| folate hydrolase (prostate-specific membrane antigen) 1 | FOLH1 | solute carrier family 2 (facilitated glucose transporter), member 1 | SLC2A1 |
| folate receptor 1 (adult) | FOLR1 | solute carrier family 2 (facilitated glucose transporter), member 12 | SLC2A12 |
| forkhead box F1 | FOXF1 | solute carrier family 2 (facilitated glucose transporter), member 14 | SLC2A14 |
| fucose-1-phosphate guanylyltransferase | FPGT | solute carrier family 2 (facilitated glucose transporter), member 3 | SLC2A3 |
| frequently rearranged in advanced T-cell lymphomas 1 | FRAT1 | solute carrier family 2 (facilitated glucose/fructose transporter), member 5 | SLC2A5 |
| FRAS1 related extracellular matrix protein 2 | FREM2 | solute carrier family 30 (zinc transporter), member 1 | SLC30A1 |
| FRAS1 related extracellular matrix 3 | FREM3 | solute carrier family 34 (type II sodium/phosphate cotransporter), member 1 | SLC34A1 |
| fyn-related Src family tyrosine kinase | FRK | solute carrier family 34 (type II sodium/phosphate cotransporter), member 2 | SLC34A2 |
| FERM domain containing 5 | FRMD5 | solute carrier family 35 (adenosine 3'-phospho 5'-phosphosulfate transporter), member B2 | SLC35B2 |
| FERM domain containing 8 | FRMD8 | solute carrier family 35 (UDP-GlcNAc/UDP-glucose transporter), member D2 | SLC35D2 |
| FERM and PDZ domain containing 3 | FRMPD3 | solute carrier family 35, member D3 | SLC35D3 |
| FRY-like | FRYL | solute carrier family 35, member F6 | SLC35F6 |
| fascin actin-bundling protein 1 | FSCN1 | solute carrier family 36 (proton/amino acid symporter), member 2 | SLC36A2 |
| fibronectin type III and SPRY domain containing 2 | FSD2 | solute carrier family 37 (glucose-6-phosphate transporter), member 2 | SLC37A2 |
| fibrous sheath interacting protein 2 | FSIP2 | solute carrier family 38, member 1 | SLC38A1 |
| follistatin | FST | solute carrier family 38, member 2 | SLC38A2 |
| formimidoyltransferase cyclodeaminase | FTCD | solute carrier family 38, member 3 | SLC38A3 |
| ferritin, heavy polypeptide 1 | FTH1 | solute carrier family 38, member 5 | SLC38A5 |
| ferritin, light polypeptide | FTL | solute carrier family 39 (zinc transporter), member 1 | SLC39A1 |
| ferritin, light polypeptide pseudogene 3 | FTLP3 | solute carrier family 39 (zinc transporter), member 10 | SLC39A10 |
| FtsJ homolog 3 (E. coli) | FTSJ3 | solute carrier family 39 (zinc transporter), member 14 | SLC39A14 |
| far upstream element (FUSE) binding protein 1 | FUBP1 | solute carrier family 39 (zinc transporter), member 4 | SLC39A4 |
| far upstream element (FUSE) binding protein 3 | FUBP3 | solute carrier family 39 (zinc transporter), member 5 | SLC39A5 |
| fucosidase, alpha-L- 1, tissue | FUCA1 | solute carrier family 39 (zinc transporter), member 6 | SLC39A6 |
| fucosidase, alpha-L- 2, plasma | FUCA2 | solute carrier family 39, member 9 | SLC39A9 |
| fucokinase | FUK | solute carrier family 3 (amino acid transporter heavy chain), member 1 | SLC3A1 |
| furin (paired basic amino acid cleaving enzyme) | FURIN | solute carrier family 3 (amino acid transporter heavy chain), member 2 | SLC3A2 |
| FUS RNA binding protein | FUS | solute carrier family 43, member 3 | SLC43A3 |
| fucosyltransferase 2 (secretor status included) | FUT2 | solute carrier family 44 (choline transporter), member 1 | SLC44A1 |
| fucosyltransferase 3 (galactoside 3(4)-L-fucosyltransferase, Lewis blood group) | FUT3 | solute carrier family 44 (choline transporter), member 2 | SLC44A2 |
| fucosyltransferase 6 (alpha (1,3) fucosyltransferase) | FUT6 | solute carrier family 44, member 4 | SLC44A4 |
| fucosyltransferase 8 (alpha (1,6) fucosyltransferase) | FUT8 | solute carrier family 45, member 2 | SLC45A2 |
| fuzzy planar cell polarity protein | FUZ | solute carrier family 46 (folate transporter), member 1 | SLC46A1 |
| fragile X mental retardation, autosomal homolog 1 | FXR1 | solute carrier family 46, member 3 | SLC46A3 |
| fragile X mental retardation, autosomal homolog 2 | FXR2 | solute carrier family 4 (anion exchanger), member 1 (Diego blood group) | SLC4A1 |
| FXYD domain containing ion transport regulator 2 | FXYD2 | solute carrier family 4, sodium borate transporter, member 11 | SLC4A11 |
| FXYD domain containing ion transport regulator 3 | FXYD3 | solute carrier family 4 (anion exchanger), member 2 | SLC4A2 |
| FYN binding protein | FYB | solute carrier family 4 (sodium bicarbonate cotransporter), member 4 | SLC4A4 |
| FYVE and coiled-coil domain containing 1 | FYCO1 | solute carrier family 4, sodium bicarbonate cotransporter, member 7 | SLC4A7 |
| FYN proto-oncogene, Src family tyrosine kinase | FYN | solute carrier family 4, sodium bicarbonate cotransporter, member 8 | SLC4A8 |
| frizzled class receptor 2 | FZD2 | solute carrier family 5 (sodium/glucose cotransporter), member 1 | SLC5A1 |
| frizzled class receptor 6 | FZD6 | solute carrier family 5 (sodium/sugar cotransporter), member 10 | SLC5A10 |
| frizzled class receptor 7 | FZD7 | solute carrier family 5 (sodium/monocarboxylate cotransporter), member 12 | SLC5A12 |
| GTPase activating protein (SH3 domain) binding protein 1 | G3BP1 | solute carrier family 5 (sodium/glucose cotransporter), member 2 | SLC5A2 |
| GTPase activating protein (SH3 domain) binding protein 2 | G3BP2 | solute carrier family 5 (sodium/myo-inositol cotransporter), member 3 | SLC5A3 |
| glucose-6-phosphate dehydrogenase | G6PD | solute carrier family 5 (sodium/iodide cotransporter), member 5 | SLC5A5 |
| glucosidase, alpha; acid | GAA | solute carrier family 5 (sodium/multivitamin and iodide cotransporter), member 6 | SLC5A6 |
| GABA(A) receptor-associated protein-like 2 | GABARAP L2 | solute carrier family 5 (sodium/monocarboxylate cotransporter), member 8 | SLC5A8 |
| GA binding protein transcription factor, alpha subunit 60kDa | GABPA | solute carrier family 5 (sodium/sugar cotransporter), member 9 | SLC5A9 |
| gamma-aminobutyric acid (GABA) A receptor, beta 2 | GABRB2 | solute carrier family 6 (neurotransmitter transporter), member 13 | SLC6A13 |
| cyclin G associated kinase | GAK | solute carrier family 6 (amino acid transporter), member 14 | SLC6A14 |
| galactose-3-O-sulfotransferase 4 | GAL3ST4 | solute carrier family 6 (neutral amino acid transporter), member 15 | SLC6A15 |
| UDP-galactose-4-epimerase | GALE | solute carrier family 6 (neutral amino acid transporter), member 17 | SLC6A17 |
| galactokinase 1 | GALK1 | solute carrier family 6 (neutral amino acid transporter), member 19 | SLC6A19 |
| galactose mutarotase (aldose 1-epimerase) | GALM | solute carrier family 6 (neurotransmitter transporter), member 4 | SLC6A4 |
| polypeptide N-acetylgalactosaminyltransferase 13 | GALNT13 | solute carrier family 6 (neurotransmitter transporter), member 6 | SLC6A6 |
| polypeptide N-acetylgalactosaminyltransferase 2 | GALNT2 | solute carrier family 6 (neurotransmitter transporter), member 8 | SLC6A8 |
| polypeptide N-acetylgalactosaminyltransferase 3 | GALNT3 | solute carrier family 6 (neurotransmitter transporter, glycine), member 9 | SLC6A9 |
| polypeptide N-acetylgalactosaminyltransferase 4 | GALNT4 | solute carrier family 7 (cationic amino acid transporter, y+ system), member 1 | SLC7A1 |
| polypeptide N-acetylgalactosaminyltransferase 5 | GALNT5 | solute carrier family 7 (neutral amino acid transporter light chain, asc system), member 10 | SLC7A10 |
| polypeptide N-acetylgalactosaminyltransferase 7 | GALNT7 | solute carrier family 7 (anionic amino acid transporter light chain, xc- system), member 11 | SLC7A11 |
| glucosidase, alpha; neutral AB | GANAB | solute carrier family 7 (cationic amino acid transporter, y+ system), member 2 | SLC7A2 |
| glyceraldehyde-3-phosphate dehydrogenase | GAPDH | solute carrier family 7 (amino acid transporter light chain, L system), member 5 | SLC7A5 |
| glyceraldehyde-3-phosphate dehydrogenase, spermatogenic | GAPDHS | solute carrier family 7 (amino acid transporter light chain, L system), member 8 | SLC7A8 |
| GAR1 ribonucleoprotein | GAR1 | solute carrier family 8 (sodium/calcium exchanger), member 1 | SLC8A1 |
| glycyl-tRNA synthetase | GARS | solute carrier family 9, subfamily A (NHE1, cation proton antiporter 1), member 1 | SLC9A1 |
| phosphoribosylglycinamide formyltransferase, phosphoribosylglycinamide synthetase, phosphoribosylaminoimidazole synthetase | GART | solute carrier family 9, subfamily A (NHE3, cation proton antiporter 3), member 3 | SLC9A3 |
| growth arrest-specific 6 | GAS6 | solute carrier family 9, subfamily A (NHE3, cation proton antiporter 3), member 3 regulator 1 | SLC9A3R1 |
| GATS protein-like 3 | GATSL3 | solute carrier family 9, subfamily A (NHE3, cation proton antiporter 3), member 3 regulator 2 | SLC9A3R2 |
| glucosidase, beta, acid | GBA | solute carrier family 9, subfamily A (NHE9, cation proton antiporter 9), member 9 | SLC9A9 |
| glucan (1,4-alpha-), branching enzyme 1 | GBE1 | solute carrier family 9, subfamily C (Na+-transporting carboxylic acid decarboxylase), member 1 | SLC9C1 |
| golgi brefeldin A resistant guanine nucleotide exchange factor 1 | GBF1 | solute carrier organic anion transporter family, member 3A1 | SLCO3A1 |
| guanylate binding protein 1, interferon-inducible | GBP1 | solute carrier organic anion transporter family, member 4A1 | SLCO4A1 |
| guanylate binding protein family, member 6 | GBP6 | solute carrier organic anion transporter family, member 4C1 | SLCO4C1 |
| group-specific component (vitamin D binding protein) | GC | schlafen family member 11 | SLFN11 |
| grancalcin, EF-hand calcium binding protein | GCA | schlafen family member 5 | SLFN5 |
| glutamate-cysteine ligase, modifier subunit | GCLM | slit homolog 2 (Drosophila) | SLIT2 |
| GCN1 general control of amino-acid synthesis 1-like 1 (yeast) | GCN1L1 | STE20-like kinase | SLK |
| glucosaminyl (N-acetyl) transferase 2, I-branching enzyme (I blood group) | GCNT2 | secretory leukocyte peptidase inhibitor | SLPI |
| glucosaminyl (N-acetyl) transferase 3, mucin type | GCNT3 | SAFB-like, transcription modulator | SLTM |
| guanine deaminase | GDA | SMAD family member 2 | SMAD2 |
| growth differentiation factor 1 | GDF1 | SMAD family member 5 | SMAD5 |
| growth differentiation factor 11 | GDF11 | SWI/SNF related, matrix associated, actin dependent regulator of chromatin, subfamily a, member 4 | SMARCA4 |
| growth differentiation factor 15 | GDF15 | SWI/SNF related, matrix associated, actin dependent regulator of chromatin, subfamily a, member 5 | SMARCA5 |
| growth differentiation factor 2 | GDF2 | SWI/SNF related, matrix associated, actin dependent regulator of chromatin, subfamily b, member 1 | SMARCB1 |
| growth differentiation factor 3 | GDF3 | SWI/SNF related, matrix associated, actin dependent regulator of chromatin, subfamily c, member 1 | SMARCC1 |
| growth differentiation factor 5 | GDF5 | SWI/SNF related, matrix associated, actin dependent regulator of chromatin, subfamily c, member 2 | SMARCC2 |
| growth differentiation factor 9 | GDF9 | SWI/SNF related, matrix associated, actin dependent regulator of chromatin, subfamily d, member 1 | SMARCD1 |
| GDP dissociation inhibitor 1 | GDI1 | SWI/SNF related, matrix associated, actin dependent regulator of chromatin, subfamily d, member 2 | SMARCD2 |
| GDP dissociation inhibitor 2 | GDI2 | SWI/SNF related, matrix associated, actin dependent regulator of chromatin, subfamily e, member 1 | SMARCE1 |
| glycerophosphodiester phosphodiesterase domain containing 3 | GDPD3 | structural maintenance of chromosomes 1A | SMC1A |
| glycerophosphodiester phosphodiesterase domain containing 5 | GDPD5 | structural maintenance of chromosomes 2 | SMC2 |
| gem (nuclear organelle) associated protein 2 | GEMIN2 | structural maintenance of chromosomes 3 | SMC3 |
| gem (nuclear organelle) associated protein 4 | GEMIN4 | structural maintenance of chromosomes 4 | SMC4 |
| gem (nuclear organelle) associated protein 5 | GEMIN5 | structural maintenance of chromosomes 5 | SMC5 |
| golgi to ER traffic protein 4 homolog (S. cerevisiae) | GET4 | structural maintenance of chromosomes flexible hinge domain containing 1 | SMCHD1 |
| glial fibrillary acidic protein | GFAP | small integral membrane protein 22 | SMIM22 |
| G elongation factor, mitochondrial 1 | GFM1 | small integral membrane protein 24 | SMIM24 |
| G elongation factor, mitochondrial 2 | GFM2 | survival of motor neuron 1, telomeric | SMN1 |
| glutamine--fructose-6-phosphate transaminase 1 | GFPT1 | survival of motor neuron 2, centromeric | SMN2 |
| glutamine-fructose-6-phosphate transaminase 2 | GFPT2 | smoothened, frizzled class receptor | SMO |
| GDNF family receptor alpha 1 | GFRA1 | sphingomyelin phosphodiesterase, acid-like 3B | SMPDL3B |
| GDNF family receptor alpha 3 | GFRA3 | spermine synthase | SMS |
| golgi-associated, gamma adaptin ear containing, ARF binding protein 1 | GGA1 | smoothelin | SMTN |
| golgi-associated, gamma adaptin ear containing, ARF binding protein 3 | GGA3 | smu-1 suppressor of mec-8 and unc-52 homolog (C. elegans) | SMU1 |
| gamma-glutamylamine cyclotransferase | GGACT | SMAD specific E3 ubiquitin protein ligase 1 | SMURF1 |
| gamma-glutamylcyclotransferase | GGCT | synaptosomal-associated protein, 23kDa | SNAP23 |
| gamma-glutamyl hydrolase (conjugase, folylpolygammaglutamyl hydrolase) | GGH | synaptosomal-associated protein, 25kDa | SNAP25 |
| gamma-glutamyltransferase 1 | GGT1 | synaptosomal-associated protein, 29kDa | SNAP29 |
| gamma-glutamyltransferase 2 | GGT2 | synuclein, alpha (non A4 component of amyloid precursor) | SNCA |
| gamma-glutamyltransferase 3 pseudogene | GGT3P | synuclein, beta | SNCB |
| gamma-glutamyltransferase light chain 1 | GGTLC1 | synuclein, gamma (breast cancer-specific protein 1) | SNCG |
| gamma-glutamyltransferase light chain 2 | GGTLC2 | staphylococcal nuclease and tudor domain containing 1 | SND1 |
| growth hormone inducible transmembrane protein | GHITM | SNF8, ESCRT-II complex subunit | SNF8 |
| GID complex subunit 8 | GID8 | small nucleolar RNA, H/ACA box 27 | SNORA27 |
| GTPase, IMAP family member 4 | GIMAP4 | small nuclear ribonucleoprotein 200kDa (U5) | SNRNP200 |
| GTPase, IMAP family member 8 | GIMAP8 | small nuclear ribonucleoprotein 40kDa (U5) | SNRNP40 |
| GIPC PDZ domain containing family, member 1 | GIPC1 | small nuclear ribonucleoprotein 70kDa (U1) | SNRNP70 |
| GIPC PDZ domain containing family, member 2 | GIPC2 | small nuclear ribonucleoprotein polypeptide A | SNRPA |
| gap junction protein, alpha 1, 43kDa | GJA1 | small nuclear ribonucleoprotein polypeptide A' | SNRPA1 |
| gap junction protein, beta 1, 32kDa | GJB1 | small nuclear ribonucleoprotein polypeptides B and B1 | SNRPB |
| gap junction protein, gamma 1, 45kDa | GJC1 | small nuclear ribonucleoprotein polypeptide B | SNRPB2 |
| glycerol kinase 2 | GK2 | small nuclear ribonucleoprotein D1 polypeptide 16kDa | SNRPD1 |
| galactosidase, alpha | GLA | small nuclear ribonucleoprotein D2 polypeptide 16.5kDa | SNRPD2 |
| galactosidase, beta 1 | GLB1 | small nuclear ribonucleoprotein D3 polypeptide 18kDa | SNRPD3 |
| glycine dehydrogenase (decarboxylating) | GLDC | small nuclear ribonucleoprotein polypeptide E | SNRPE |
| golgi glycoprotein 1 | GLG1 | small nuclear ribonucleoprotein polypeptide F | SNRPF |
| GLI pathogenesis-related 2 | GLIPR2 | small nuclear ribonucleoprotein polypeptide G | SNRPG |
| glomulin, FKBP associated protein | GLMN | small nuclear ribonucleoprotein polypeptide N | SNRPN |
| glyoxalase I | GLO1 | syntrophin, alpha 1 | SNTA1 |
| glutaredoxin (thioltransferase) | GLRX | syntrophin, beta 1 (dystrophin-associated protein A1, 59kDa, basic component 1) | SNTB1 |
| glutaredoxin 3 | GLRX3 | syntrophin, beta 2 (dystrophin-associated protein A1, 59kDa, basic component 2) | SNTB2 |
| glutaminase | GLS | sorting nexin 1 | SNX1 |
| glycosyltransferase 8 domain containing 1 | GLT8D1 | sorting nexin 12 | SNX12 |
| glutamate dehydrogenase 1 | GLUD1 | sorting nexin 17 | SNX17 |
| glutamate-ammonia ligase | GLUL | sorting nexin 18 | SNX18 |
| glyoxylate reductase 1 homolog (Arabidopsis) | GLYR1 | sorting nexin 2 | SNX2 |
| GM2 ganglioside activator | GM2A | sorting nexin 25 | SNX25 |
| GDP-mannose 4,6-dehydratase | GMDS | sorting nexin family member 27 | SNX27 |
| glia maturation factor, gamma | GMFG | sorting nexin 3 | SNX3 |
| GDP-mannose pyrophosphorylase A | GMPPA | sorting nexin 33 | SNX33 |
| GDP-mannose pyrophosphorylase B | GMPPB | sorting nexin 4 | SNX4 |
| guanosine monophosphate reductase 2 | GMPR2 | sorting nexin 5 | SNX5 |
| guanine monphosphate synthase | GMPS | sorting nexin 6 | SNX6 |
| guanine nucleotide binding protein (G protein), alpha 11 (Gq class) | GNA11 | sorting nexin 9 | SNX9 |
| guanine nucleotide binding protein (G protein) alpha 12 | GNA12 | superoxide dismutase 1, soluble | SOD1 |
| guanine nucleotide binding protein (G protein), alpha 13 | GNA13 | superoxide dismutase 3, extracellular | SOD3 |
| guanine nucleotide binding protein (G protein), alpha 14 | GNA14 | suppressor of glucose, autophagy associated 1 | SOGA1 |
| guanine nucleotide binding protein (G protein), alpha 15 (Gq class) | GNA15 | SON DNA binding protein | SON |
| guanine nucleotide binding protein (G protein), alpha inhibiting activity polypeptide 1 | GNAI1 | sorbin and SH3 domain containing 1 | SORBS1 |
| guanine nucleotide binding protein (G protein), alpha inhibiting activity polypeptide 2 | GNAI2 | sorbin and SH3 domain containing 3 | SORBS3 |
| guanine nucleotide binding protein (G protein), alpha inhibiting activity polypeptide 3 | GNAI3 | sortilin-related VPS10 domain containing receptor 2 | SORCS2 |
| guanine nucleotide binding protein (G protein), alpha activating activity polypeptide, olfactory type | GNAL | sorbitol dehydrogenase | SORD |
| guanine nucleotide binding protein (G protein), alpha activating activity polypeptide O | GNAO1 | sortilin-related receptor, L(DLR class) A repeats containing | SORL1 |
| guanine nucleotide binding protein (G protein), q polypeptide | GNAQ | sortilin 1 | SORT1 |
| GNAS complex locus | GNAS | SRY (sex determining region Y)-box 1 | SOX1 |
| guanine nucleotide binding protein (G protein), alpha transducing activity polypeptide 1 | GNAT1 | SRY (sex determining region Y)-box 18 | SOX18 |
| guanine nucleotide binding protein (G protein), alpha transducing activity polypeptide 2 | GNAT2 | sperm acrosome associated 1 | SPACA1 |
| guanine nucleotide binding protein, alpha transducing 3 | GNAT3 | sperm associated antigen 1 | SPAG1 |
| guanine nucleotide binding protein (G protein), alpha z polypeptide | GNAZ | sperm associated antigen 5 | SPAG5 |
| guanine nucleotide binding protein (G protein), beta polypeptide 1 | GNB1 | sperm associated antigen 9 | SPAG9 |
| guanine nucleotide binding protein (G protein), beta polypeptide 1-like | GNB1 L | secreted protein, acidic, cysteine-rich (osteonectin) | SPARC |
| guanine nucleotide binding protein (G protein), beta polypeptide 2 | GNB2 | spastin | SPAST |
| guanine nucleotide binding protein (G protein), beta polypeptide 2-like 1 | GNB2L1 | spermatogenesis associated 21 | SPATA21 |
| guanine nucleotide binding protein (G protein), beta polypeptide 3 | GNB3 | spermatogenesis associated 5 | SPATA5 |
| guanine nucleotide binding protein (G protein), beta polypeptide 4 | GNB4 | spermatogenesis associated 5-like 1 | SPATA5L1 |
| guanine nucleotide binding protein (G protein), beta 5 | GNB5 | spermatogenesis associated 7 | SPATA7 |
| glucosamine (UDP-N-acetyl)-2-epimerase/N-acetylmannosamine kinase | GNE | signal peptidase complex subunit 2 homolog (S. cerevisiae) | SPCS2 |
| guanine nucleotide binding protein (G protein), gamma 10 | GNG10 | signal peptidase complex subunit 3 homolog (S. cerevisiae) | SPCS3 |
| guanine nucleotide binding protein (G protein), gamma 11 | GNG11 | sperm antigen with calponin homology and coiled-coil domains 1 | SPECC1 |
| guanine nucleotide binding protein (G protein), gamma 12 | GNG12 | sperm antigen with calponin homology and coiled-coil domains 1-like | SPECC1L |
| guanine nucleotide binding protein (G protein), gamma 2 | GNG2 | sperm flagellar 2 | SPEF2 |
| guanine nucleotide binding protein (G protein), gamma 5 | GNG5 | spen family transcriptional repressor | SPEN |
| guanine nucleotide binding protein (G protein), gamma 7 | GNG7 | spastic paraplegia 11 (autosomal recessive) | SPG11 |
| guanine nucleotide binding protein-like 1 | GNL1 | spastic paraplegia 20 (Troyer syndrome) | SPG20 |
| guanine nucleotide binding protein-like 2 (nucleolar) | GNL2 | spastic paraplegia 21 (autosomal recessive, Mast syndrome) | SPG21 |
| guanine nucleotide binding protein-like 3 (nucleolar) | GNL3 | scaffolding protein involved in DNA repair | SPIDR |
| glucosamine-6-phosphate deaminase 1 | GNPDA1 | serine peptidase inhibitor, Kazal type 1 | SPINK1 |
| glucosamine-6-phosphate deaminase 2 | GNPDA2 | serine peptidase inhibitor, Kazal type 5 | SPINK5 |
| glucosamine-phosphate N-acetyltransferase 1 | GNPNAT1 | serine peptidase inhibitor, Kunitz type 1 | SPINT1 |
| N-acetylglucosamine-1-phosphate transferase, gamma subunit | GNPTG | serine peptidase inhibitor, Kunitz type, 2 | SPINT2 |
| glucosamine (N-acetyl)-6-sulfatase | GNS | spire-type actin nucleation factor 1 | SPIRE1 |
| golgin A2 | GOLGA2 | sialophorin | SPN |
| golgin A3 | GOLGA3 | spinster homolog 1 (Drosophila) | SPNS1 |
| golgin A5 | GOLGA5 | sparc/osteonectin, cwcv and kazal-like domains proteoglycan (testican) 1 | SPOCK1 |
| golgin A7 | GOLGA7 | spondin 1, extracellular matrix protein | SPON1 |
| golgin B1 | GOLGB1 | spondin 2, extracellular matrix protein | SPON2 |
| golgi integral membrane protein 4 | GOLIM4 | secreted phosphoprotein 1 | SPP1 |
| golgi membrane protein 1 | GOLM1 | secreted phosphoprotein 2, 24kDa | SPP2 |
| golgi phosphoprotein 3 (coat-protein) | GOLPH3 | signal peptide peptidase like 2A | SPPL2A |
| golgin, RAB6-interacting | GORAB | sepiapterin reductase (7,8-dihydrobiopterin:NADP+ oxidoreductase) | SPR |
| golgi reassembly stacking protein 2, 55kDa | GORASP2 | small proline-rich protein 3 | SPRR3 |
| golgi SNAP receptor complex member 1 | GOSR1 | sprouty homolog 1, antagonist of FGF signaling (Drosophila) | SPRY1 |
| glutamic-oxaloacetic transaminase 1, soluble | GOT1 | sprouty homolog 4 (Drosophila) | SPRY4 |
| glutamic-oxaloacetic transaminase 2, mitochond rial | GOT2 | SPRY domain containing 7 | SPRYD7 |
| glycoprotein Ib (platelet), alpha polypeptide | GP1BA | spectrin, alpha, non-erythrocytic 1 | SPTAN1 |
| glycoprotein Ib (platelet), beta polypeptide | GP1BB | spectrin, beta, erythrocytic | SPTB |
| glycoprotein V (platelet) | GP5 | spectrin, beta, non-erythrocytic 1 | SPTBN1 |
| glycoprotein VI (platelet) | GP6 | spectrin, beta, non-erythrocytic 2 | SPTBN2 |
| glycoprotein IX (platelet) | GP9 | spectrin, beta, non-erythrocytic 4 | SPTBN4 |
| glycoprotein A33 (transmembrane) | GPA33 | spectrin, beta, non-erythrocytic 5 | SPTBN5 |
| glypican 1 | GPC1 | serine palmitoyltransferase, long chain base subunit 1 | SPTLC1 |
| glypican 3 | GPC3 | sequestosome 1 | SQSTM1 |
| glypican 4 | GPC4 | steroid receptor RNA activator 1 | SRA1 |
| glypican 5 | GPC5 | SRC proto-oncogene, non-receptor tyrosine kinase | SRC |
| glypican 6 | GPC6 | splicing regulatory glutamine/lysine-rich protein 1 | SREK1 |
| glycerol-3-phosphate dehydrogenase 1 (soluble) | GPD1 | SLIT-ROBO Rho GTPase activating protein 1 | SRGAP1 |
| glycerol-3-phosphate dehydrogenase 1-like | GPD1 L | SLIT-ROBO Rho GTPase activating protein 2 | SRGAP2 |
| glucose-6-phosphate isomerase | GPI | serglycin | SRGN |
| glycoprotein M6A | GPM6A | sorcin | SRI |
| glycoprotein (transmembrane) nmb | GPNMB | spermidine synthase | SRM |
| G protein-coupled receptor 107 | GPR107 | signal recognition particle 14kDa (homologous Alu RNA binding protein) | SRP14 |
| G protein-coupled receptor 143 | GPR143 | signal recognition particle 54kDa | SRP54 |
| G protein-coupled receptor 155 | GPR155 | signal recognition particle 68kDa | SRP68 |
| G protein-coupled receptor 176 | GPR176 | signal recognition particle 72kDa | SRP72 |
| G protein-coupled receptor 179 | GPR179 | signal recognition particle 9kDa | SRP9 |
| G protein-coupled receptor, class C, group 5, member A | GPRC5A | SRSF protein kinase 1 | SRPK1 |
| G protein-coupled receptor, class C, group 5, member B | GPRC5B | SRSF protein kinase 2 | SRPK2 |
| G protein-coupled receptor, class C, group 5, member C | GPRC5C | signal recognition particle receptor (docking protein) | SRPR |
| G protein pathway suppressor 1 | GPS1 | signal recognition particle receptor, B subunit | SRPRB |
| glutamic-pyruvate transaminase (alanine aminotransferase) | GPT | sushi-repeat containing protein, X-linked | SRPX |
| glutathione peroxidase 1 | GPX1 | sushi-repeat containing protein, X-linked 2 | SRPX2 |
| glutathione peroxidase 2 | GPX2 | serine/arginine repetitive matrix 2 | SRRM2 |
| glutathione peroxidase 3 | GPX3 | serrate, RNA effector molecule | SRRT |
| glutathione peroxidase 4 | GPX4 | serine/arginine-rich splicing factor 1 | SRSF1 |
| GRB2-related adaptor protein 2 | GRAP2 | serine/arginine-rich splicing factor 10 | SRSF10 |
| growth factor receptor-bound protein 2 | GRB2 | serine/arginine-rich splicing factor 2 | SRSF2 |
| gremlin 1, DAN family BMP antagonist | GREM1 | serine/arginine-rich splicing factor 3 | SRSF3 |
| glyoxylate reductase/hydroxypyruvate reductase | GRHPR | serine/arginine-rich splicing factor 4 | SRSF4 |
| glutamate receptor, ionotropic, delta 1 | GRID1 | serine/arginine-rich splicing factor 5 | SRSF5 |
| glutamate receptor, ionotropic, N-methyl D-aspartate 1 | GRIN1 | serine/arginine-rich splicing factor 6 | SRSF6 |
| glutamate receptor interacting protein 2 | GRIP2 | serine/arginine-rich splicing factor 7 | SRSF7 |
| GRIP1 associated protein 1 | GRIPAP1 | serine/arginine-rich splicing factor 9 | SRSF9 |
| G protein-coupled receptor kinase 5 | GRK5 | Sjogren syndrome antigen B (autoantigen La) | SSB |
| G protein-coupled receptor kinase 6 | GRK6 | single-stranded DNA binding protein 1, mitochondrial | SSBP1 |
| glutamate receptor, metabotropic 2 | GRM2 | SCO-spondin | SSPO |
| glutamate receptor, metabotropic 3 | GRM3 | signal sequence receptor, alpha | SSR1 |
| glutamate receptor, metabotropic 7 | GRM7 | signal sequence receptor, gamma (translocon-associated protein gamma) | SSR3 |
| granulin | GRN | signal sequence receptor, delta | SSR4 |
| growth hormone regulated TBC protein 1 | GRTP1 | structure specific recognition protein 1 | SSRP1 |
| glutamate-rich WD repeat containing 1 | GRWD1 | suppression of tumorigenicity 13 (colon carcinoma) (Hsp70 interacting protein) | ST13 |
| gasdermin A | GSDMA | suppression of tumorigenicity 13 (colon carcinoma) (Hsp70 interacting protein) pseudogene 4 | ST13P4 |
| gasdermin D | GSDMD | suppression of tumorigenicity 13 (colon carcinoma) (Hsp70 interacting protein) pseudogene 5 | ST13P5 |
| Gse1 coiled-coil protein | GSE1 | suppression of tumorigenicity 14 (colon carcinoma) | ST14 |
| glycogen synthase kinase 3 alpha | GSK3A | ST3 beta-galactoside alpha-2,3-sialyltransferase 1 | ST3GAL1 |
| glycogen synthase kinase 3 beta | GSK3B | ST3 beta-galactoside alpha-2,3-sialyltransferase 6 | ST3GAL6 |
| gelsolin | GSN | ST6 (alpha-N-acetyl-neuraminyl-2,3-beta-galactosyl-1,3)-N-acetylgalactosaminide alpha-2,6-sialyltransferase 6 | ST6GALNA C6 |
| G1 to S phase transition 1 | GSPT1 | stromal antigen 2 | STAG2 |
| G1 to S phase transition 2 | GSPT2 | stromal antigen 3 | STAG3 |
| glutathione reductase | GSR | signal transducing adaptor molecule (SH3 domain and ITAM motif) 1 | STAM |
| glutathione synthetase | GSS | signal transducing adaptor molecule (SH3 domain and ITAM motif) 2 | STAM2 |
| glutathione S-transferase alpha 1 | GSTA1 | STAM binding protein | STAMBP |
| glutathione S-transferase alpha 2 | GSTA2 | StAR-related lipid transfer (START) domain containing 9 | STARD9 |
| glutathione S-transferase alpha 3 | GSTA3 | signal transducer and activator of transcription 1, 91kDa | STAT1 |
| glutathione S-transferase alpha 5 | GSTA5 | signal transducer and activator of transcription 2, 113kDa | STAT2 |
| glutathione S-transferase, C-terminal domain containing | GSTCD | signal transducer and activator of transcription 3 (acute-phase response factor) | STAT3 |
| glutathione S-transferase kappa 1 | GSTK1 | signal transducer and activator of transcription 5A | STAT5A |
| glutathione S-transferase mu 1 | GSTM1 | signal transducer and activator of transcription 6, interleukin-4 induced | STAT6 |
| glutathione S-transferase mu 2 (muscle) | GSTM2 | staufen double-stranded RNA binding protein 1 | STAU1 |
| glutathione S-transferase mu 3 (brain) | GSTM3 | staufen double-stranded RNA binding protein 2 | STAU2 |
| glutathione S-transferase mu 5 | GSTM5 | stanniocalcin 1 | STC1 |
| glutathione S-transferase omega 1 | GSTO1 | stanniocalcin 2 | STC2 |
| glutathione S-transferase omega 2 | GSTO2 | STEAP family member 3, metalloreductase | STEAP3 |
| glutathione S-transferase pi 1 | GSTP1 | STEAP family member 4 | STEAP4 |
| general transcription factor IIB | GTF2B | stromal interaction molecule 1 | STIM1 |
| general transcription factor IIi | GTF2I | stress-induced phosphoprotein 1 | STIP1 |
| general transcription factor IIIC, polypeptide 5, 63kDa | GTF3C5 | serine/threonine kinase 10 | STK10 |
| GTP binding protein 1 | GTPBP1 | serine/threonine kinase 11 | STK11 |
| GTP binding protein 2 | GTPBP2 | serine/threonine kinase 17b | STK17B |
| G-2 and S-phase expressed 1 | GTSE1 | serine/threonine kinase 24 | STK24 |
| glucuronidase, beta | GUSB | serine/threonine kinase 25 | STK25 |
| glycosyltransferase-like 1B | GYLTL1B | serine/threonine protein kinase 26 | STK26 |
| glycophorin C (Gerbich blood group) | GYPC | serine/threonine kinase 38 | STK38 |
| glycogen synthase 1 (muscle) | GYS1 | serine/threonine kinase 38 like | STK38L |
| glycogen synthase 2 (liver) | GYS2 | serine threonine kinase 39 | STK39 |
| granzyme A (granzyme 1, cytotoxic T-lymphocyte-associated serine esterase 3) | GZMA | serine/threonine kinase 4 | STK4 |
| H1 histone family, member 0 | H1F0 | stathmin 1 | STMN1 |
| H1 histone family, member O, oocyte-specific | H1FOO | stomatin | STOM |
| H1 histone family, member X | H1FX | stomatin (EPB72)-like 2 | STOML2 |
| H2A histone family, member J | H2AFJ | stomatin (EPB72)-like 3 | STOML3 |
| H2A histone family, member V | H2AFV | stonin 2 | STON2 |
| H2A histone family, member X | H2AFX | stimulated by retinoic acid 6 | STRA6 |
| H2A histone family, member Y | H2AFY | serine/threonine kinase receptor associated protein | STRAP |
| H2A histone family, member Y2 | H2AFY2 | spermatid perinuclear RNA binding protein | STRBP |
| H2A histone family, member Z | H2AFZ | striatin interacting protein 1 | STRIP1 |
| H3 histone, family 3A | H3F3A | striatin, calmodulin binding protein 3 | STRN3 |
| H3 histone, family 3B (H3.3B) | H3F3B | STT3A, subunit of the oligosaccharyltransferase complex (catalytic) | STT3A |
| H3 histone, family 3C | H3F3C | STT3B, subunit of the oligosaccharyltransferase complex (catalytic) | STT3B |
| 3-hydroxyacyl-CoA dehydratase 3 | HACD3 | STIP1 homology and U-box containing protein 1, E3 ubiquitin protein ligase | STUB1 |
| hydroxyacyl-CoA dehydrogenase | HADH | syntaxin 10 | STX10 |
| hydroxyacyl-CoA dehydrogenase/3-ketoacyl-CoA thiolase/enoyl-CoA hydratase (trifunctional protein), alpha subunit | HADHA | syntaxin 11 | STX11 |
| hydroxyacyl-CoA dehydrogenase/3-ketoacyl-CoA thiolase/enoyl-CoA hydratase (trifunctional protein), beta subunit | HADHB | syntaxin 12 | STX12 |
| histidine ammonia-lyase | HAL | syntaxin 16 | STX16 |
| hyaluronan and proteoglycan link protein 1 | HAPLN1 | syntaxin 1A (brain) | STX1A |
| hyaluronan and proteoglycan link protein 3 | HAPLN3 | syntaxin 1B | STX1B |
| histidyl-tRNA synthetase | HARS | syntaxin 2 | STX2 |
| histidyl-tRNA synthetase 2, mitochondrial | HARS2 | syntaxin 3 | STX3 |
| hyaluronan synthase 1 | HAS1 | syntaxin 4 | STX4 |
| histone acetyltransferase 1 | HAT1 | syntaxin 6 | STX6 |
| HAUS augmin-like complex, subunit 5 | HAUS5 | syntaxin 7 | STX7 |
| hemoglobin, alpha 1 | HBA1 | syntaxin 8 | STX8 |
| hemoglobin, alpha 2 | HBA2 | syntaxin binding protein 1 | STXBP1 |
| hemoglobin, beta | HBB | syntaxin binding protein 2 | STXBP2 |
| hemoglobin, delta | HBD | syntaxin binding protein 3 | STXBP3 |
| hemoglobin, epsilon 1 | HBE1 | syntaxin binding protein 4 | STXBP4 |
| hemoglobin, gamma A | HBG1 | syntaxin binding protein 5 (tomosyn) | STXBP5 |
| hemoglobin, gamma G | HBG2 | syntaxin binding protein 6 (amisyn) | STXBP6 |
| HBS1-like translational GTPase | HBS1L | SUB1 homolog (S. cerevisiae) | SUB1 |
| host cell factor C1 | HCFC1 | succinate-CoA ligase, ADP-forming, beta subunit | SUCLA2 |
| HCK proto-oncogene, Src family tyrosine kinase | HCK | succinate-CoA ligase, GDP-forming, beta subunit | SUCLG2 |
| hyperpolarization activated cyclic nucleotide gated potassium channel 3 | HCN3 | succinate receptor 1 | SUCNR1 |
| histone deacetylase 1 | HDAC1 | SGT1, suppressor of G2 allele of SKP1 (S. cerevisiae) | SUGT1 |
| histone deacetylase 2 | HDAC2 | sulfatase 2 | SULF2 |
| histone deacetylase 3 | HDAC3 | sulfotransferase family, cytosolic, 1A, phenol-preferring, member 1 | SULT1A1 |
| histone deacetylase 5 | HDAC5 | small ubiquitin-like modifier 2 | SUMO2 |
| histone deacetylase 6 | HDAC6 | small ubiquitin-like modifier 3 | SUMO3 |
| hepatoma-derived growth factor | HDGF | small ubiquitin-like modifier 4 | SUMO4 |
| haloacid dehalogenase-like hydrolase domain containing 2 | HDHD2 | suppressor of Ty 16 homolog (S. cerevisiae) | SUPT16H |
| high density lipoprotein binding protein | HDLBP | suppressor of Ty 20 homolog (S. cerevisiae) | SUPT20H |
| heme binding protein 1 | HEBP1 | suppressor of Ty 5 homolog (S. cerevisiae) | SUPT5H |
| heme binding protein 2 | HEBP2 | surfeit 4 | SURF4 |
| HECT domain containing E3 ubiquitin protein ligase 3 | HECTD3 | sushi domain containing 1 | SUSD1 |
| HECT domain containing E3 ubiquitin protein ligase 4 | HECTD4 | sushi domain containing 2 | SUSD2 |
| heart development protein with EGF-like domains 1 | HEG1 | sushi domain containing 3 | SUSD3 |
| helicase, lymphoid-specific | HELLS | sushi, von Willebrand factor type A, EGF and pentraxin domain containing 1 | SVEP1 |
| hephaestin | HEPH | small VCP/p97-interacting protein | SVIP |
| HECT and RLD domain containing E3 ubiquitin protein ligase 5 | HERC5 | SWAP switching B-cell complex 70kDa subunit | SWAP70 |
| hexosaminidase A (alpha polypeptide) | HEXA | synaptonemal complex protein 2 | SYCP2 |
| hexosaminidase B (beta polypeptide) | HEXB | spleen tyrosine kinase | SYK |
| homogentisate 1,2-dioxygenase | HGD | symplekin | SYMPK |
| hepatocyte growth factor (hepapoietin A; scatter factor) | HGF | synapsin II | SYN2 |
| HGF activator | HGFAC | synaptotagmin binding, cytoplasmic RNA interacting protein | SYNCRIP |
| hepatocyte growth factor-regulated tyrosine kinase substrate | HGS | spectrin repeat containing, nuclear envelope 1 | SYNE1 |
| hypermethylated in cancer 2 | HIC2 | spectrin repeat containing, nuclear envelope 2 | SYNE2 |
| HID1 domain containing | HID1 | synaptogyrin 1 | SYNGR1 |
| histidine triad nucleotide binding protein 1 | HINT1 | synaptogyrin 2 | SYNGR2 |
| histidine triad nucleotide binding protein 3 | HINT3 | synaptojanin 2 | SYNJ2 |
| histone cluster 1, H1 a | HIST1H1A | synergin, gamma | SYNRG |
| histone cluster 1, H1b | HIST1H1B | synaptophysin-like 1 | SYPL1 |
| histone cluster 1, H1 c | HIST1H1C | synaptotagmin I | SYT1 |
| histone cluster 1, H1d | HIST1H1D | synaptotagmin V | SYT5 |
| histone cluster 1, H1 e | HIST1H1E | synaptotagmin IX | SYT9 |
| histone cluster 1, H1t | HIST1H1T | synaptotagmin-like 1 | SYTL1 |
| histone cluster 1, H2aa | HIST1H2AA | synaptotagmin-like 2 | SYTL2 |
| histone cluster 1, H2ab | HIST1H2AB | synaptotagmin-like 4 | SYTL4 |
| histone cluster 1, H2ac | HIST1H2AC | synaptotagmin-like 5 | SYTL5 |
| histone cluster 1, H2ad | HIST1H2AD | seizure threshold 2 homolog (mouse) | SZT2 |
| histone cluster 1, H2ae | HIST1H2AE | trace amine associated receptor 2 | TAAR2 |
| histone cluster 1, H2ag | HIST1H2AG | transforming, acidic coiled-coil containing protein 2 | TACC2 |
| histone cluster 1, H2ah | HIST1H2AH | tumor-associated calcium signal transducer 2 | TACSTD2 |
| histone cluster 1, H2ai | HIST1H2AI | TAF10 RNA polymerase II, TATA box binding protein (TBP)-associated factor, 30kDa | TAF10 |
| histone cluster 1, H2aj | HIST1H2AJ | transgelin | TAGLN |
| histone cluster 1, H2ak | HIST1H2AK | transgelin 2 | TAGLN2 |
| histone cluster 1, H2al | HIST1H2AL | transaldolase 1 | TALDO1 |
| histone cluster 1, H2am | HIST1H2AM | tetratricopeptide repeat, ankyrin repeat and coiled-coil containing 1 | TANC1 |
| histone cluster 1, H2ba | HIST1H2BA | TAO kinase 1 | TAOK1 |
| histone cluster 1, H2bb | HIST1H2BB | TAO kinase 2 | TAOK2 |
| histone cluster 1, H2bc | HIST1H2BC | TAO kinase 3 | TAOK3 |
| histone cluster 1, H2bd | HIST1H2BD | transporter 1, ATP-binding cassette, sub-family B (MDR/TAP) | TAP1 |
| histone cluster 1, H2be | HIST1H2BE | TAP binding protein (tapasin) | TAPBP |
| histone cluster 1, H2bf | HIST1H2BF | TAR (HIV-1) RNA binding protein 1 | TARBP1 |
| histone cluster 1, H2bg | HIST1H2BG | TAR DNA binding protein | TARDBP |
| histone cluster 1, H2bh | HIST1H2BH | threonyl-tRNA synthetase | TARS |
| histone cluster 1, H2bi | HIST1H2BI | threonyl-tRNA synthetase-like 2 | TARSL2 |
| histone cluster 1, H2bj | HIST1H2BJ | taste receptor, type 2, member 60 | TAS2R60 |
| histone cluster 1, H2bk | HIST1H2BK | Tax1 (human T-cell leukemia virus type I) binding protein 1 | TAX1 BP1 |
| histone cluster 1, H2bl | HIST1H2BL | Tax1 (human T-cell leukemia virus type I) binding protein 3 | TAX1BP3 |
| histone cluster 1, H2bm | HIST1H2BM | TBC1 (tre-2/USP6, BUB2, cdc16) domain family, member 1 | TBC1D1 |
| histone cluster 1, H2bn | HIST1H2BN | TBC1 domain family, member 10A | TBC1D10A |
| histone cluster 1, H2bo | HIST1H2BO | TBC1 domain family, member 10B | TBC1D10B |
| histone cluster 1, H3a | HIST1H3A | TBC1 domain family, member 15 | TBC1D15 |
| histone cluster 1, H3b | HIST1H3B | TBC1 domain family, member 2 | TBC1D2 |
| histone cluster 1, H3c | HIST1H3C | TBC1 domain family, member 21 | TBC1D21 |
| histone cluster 1, H3d | HIST1H3D | TBC1 domain family, member 24 | TBC1D24 |
| histone cluster 1, H3e | HIST1H3E | TBC1 domain family, member 32 | TBC1D32 |
| histone cluster 1, H3f | HIST1H3F | tubulin folding cofactor A | TBCA |
| histone cluster 1, H3g | HIST1H3G | tubulin folding cofactor B | TBCB |
| histone cluster 1, H3h | HIST1H3H | tubulin folding cofactor D | TBCD |
| histone cluster 1, H3i | HIST1H3I | tubulin folding cofactor E | TBCE |
| histone cluster 1, H3j | HIST1H3J | TANK-binding kinase 1 | TBK1 |
| histone cluster 1, H4a | HIST1H4A | transducin (beta)-like 1 X-linked receptor 1 | TBL1XR1 |
| histone cluster 1, H4b | HIST1H4B | thromboxane A2 receptor | TBXA2R |
| histone cluster 1, H4c | HIST1H4C | thromboxane A synthase 1 (platelet) | TBXAS1 |
| histone cluster 1, H4d | HIST1H4D | tandem C2 domains, nuclear | TC2N |
| histone cluster 1, H4e | HIST1H4E | TRPM8 channel-associated factor 1 | TCAF1 |
| histone cluster 1, H4f | HIST1H4F | transcription elongation factor A (SII), 1 | TCEA1 |
| histone cluster 1, H4g | HIST1H4G | transcription elongation factor B (SIII), polypeptide 1 (15kDa, elongin C) | TCEB1 |
| histone cluster 1, H4h | HIST1H4H | transcription elongation factor B (SIII), polypeptide 2 (18kDa, elongin B) | TCEB2 |
| histone cluster 1, H4i | HIST1H4I | T-cell, immune regulator 1, ATPase, H+ transporting, lysosomal V0 subunit A3 | TCIRG1 |
| histone cluster 1, H4j | HIST1H4J | transcobalamin I (vitamin B12 binding protein, R binder family) | TCN1 |
| histone cluster 1, H4k | HIST1H4K | Treacher Collins-Franceschetti syndrome 1 | TCOF1 |
| histone cluster 1, H4I | HIST1H4L | t-complex 1 | TCP1 |
| histone cluster 2, H2aa3 | HIST2H2AA3 | tyrosyl-DNA phosphodiesterase 2 | TDP2 |
| histone cluster 2, H2aa4 | HIST2H2AA4 | tudor domain containing 9 | TDRD9 |
| histone cluster 2, H2ab | HIST2H2AB | tec protein tyrosine kinase | TEC |
| histone cluster 2, H2ac | HIST2H2AC | tectorin alpha | TECTA |
| histone cluster 2, H2bc (pseudogene) | HIST2H2BC | tektin 3 | TEKT3 |
| histone cluster 2, H2be | HIST2H2BE | telomere maintenance 2 | TELO2 |
| histone cluster 2, H2bf | HIST2H2BF | teneurin transmembrane protein 3 | TENM3 |
| histone cluster 2, H3a | HIST2H3A | teneurin transmembrane protein 4 | TENM4 |
| histone cluster 2, H3c | HIST2H3C | telomerase-associated protein 1 | TEP1 |
| histone cluster 2, H3d | HIST2H3D | telomeric repeat binding factor (NIMA-interacting) 1 | TERF1 |
| histone cluster 2, H4a | HIST2H4A | telomeric repeat binding factor (NIMA-interacting) 1 pseudogene 2 | TERF1P2 |
| histone cluster 2, H4b | HIST2H4B | telomeric repeat binding factor (NIMA-interacting) 1 pseudogene 3 | TERF1P3 |
| histone cluster 3, H2a | HIST3H2A | telomeric repeat binding factor (NIMA-interacting) 1 pseudogene 5 | TERF1P5 |
| histone cluster 3, H2bb | HIST3H2BB | telomeric repeat binding factor 2, interacting protein | TERF2IP |
| histone cluster 3, H3 | HIST3H3 | testin LIM domain protein | TES |
| histone cluster 4, H4 | HIST4H4 | tescalcin | TESC |
| human immunodeficiency virus type I enhancer binding protein 1 | HIVEP1 | testis-specific kinase 1 | TESK1 |
| human immunodeficiency virus type I enhancer binding protein 3 | HIVEP3 | testis expressed 10 | TEX10 |
| hexokinase 1 | HK1 | transferrin | TF |
| major histocompatibility complex, class I, A | HLA-A | TRK-fused gene | TFG |
| major histocompatibility complex, class I, B | HLA-B | tissue factor pathway inhibitor (lipoprotein-associated coagulation inhibitor) | TFPI |
| major histocompatibility complex, class I, C | HLA-C | transferrin receptor | TFRC |
| major histocompatibility complex, class II, DM beta | HLA-DMB | transforming growth factor, beta 1 | TGFB1 |
| major histocompatibility complex, class II, DP beta 1 | HLA-DPB1 | transforming growth factor, beta 2 | TGFB2 |
| major histocompatibility complex, class II, DQ alpha 1 | HLA-DQA1 | transforming growth factor, beta-induced, 68kDa | TGFBI |
| major histocompatibility complex, class II, DQ beta 1 | HLA-DQB1 | transforming growth factor, beta receptor II (70/80kDa) | TGFBR2 |
| major histocompatibility complex, class II, DR alpha | HLA-DRA | transforming growth factor, beta receptor III | TGFBR3 |
| major histocompatibility complex, class II, DR beta 1 | HLA-DRB1 | transforming growth factor, beta receptor associated protein 1 | TGFBRAP1 |
| major histocompatibility complex, class II, DR beta 3 | HLA-DRB3 | transglutaminase 1 | TGM1 |
| major histocompatibility complex, class II, DR beta 5 | HLA-DRB5 | transglutaminase 2 | TGM2 |
| major histocompatibility complex, class I, E | HLA-E | transglutaminase 3 | TGM3 |
| major histocompatibility complex, class I, G | HLA-G | transglutaminase 4 | TGM4 |
| major histocompatibility complex, class I, H (pseudogene) | HLA-H | trans-golgi network protein 2 | TGOLN2 |
| histocompatibility (minor) 13 | HM13 | thyroid adenoma associated | THADA |
| hydroxymethylbilane synthase | HMBS | THAP domain containing 11 | THAP11 |
| hemicentin 1 | HMCN1 | thrombospondin 1 | THBS1 |
| high mobility group AT-hook 1 | HMGA1 | thrombospondin 2 | THBS2 |
| high mobility group AT-hook 2 | HMGA2 | thrombospondin 4 | THBS4 |
| high mobility group box 1 | HMGB1 | thymocyte selection associated | THEMIS |
| high mobility group box 2 | HMGB2 | thymocyte selection associated family member 2 | THEMIS2 |
| high mobility group box 3 | HMGB3 | threonine synthase-like 1 (S. cerevisiae) | THNSL1 |
| 3-hydroxy-3-methylglutaryl-CoA synthase 1 (soluble) | HMGCS1 | threonine synthase-like 2 (S. cerevisiae) | THNSL2 |
| 3-hydroxy-3-methylglutaryl-CoA synthase 2 (mitochondrial) | HMGCS2 | THO complex 1 | THOC1 |
| histocompatibility (minor) HA-1 | HMHA1 | THO complex 2 | THOC2 |
| heme oxygenase 2 | HMOX2 | THO complex 5 | THOC5 |
| hematological and neurological expressed 1 | HN1 | THO complex 6 | THOC6 |
| hematological and neurological expressed 1-like | HN1L | THO complex 7 | THOC7 |
| histamine N-methyltransferase | HNMT | thimet oligopeptidase 1 | THOP1 |
| heterogeneous nuclear ribonucleoprotein A0 | HNRNPA0 | thyroid hormone receptor associated protein 3 | THRAP3 |
| heterogeneous nuclear ribonucleoprotein A1 | HNRNPA1 | thrombospondin, type I, domain containing 1 | THSD1 |
| heterogeneous nuclear ribonucleoprotein A2/B1 | HNRNPA2B1 | thrombospondin, type I, domain containing 4 | THSD4 |
| heterogeneous nuclear ribonucleoprotein A3 | HNRNPA3 | thrombospondin, type I, domain containing 7A | THSD7A |
| heterogeneous nuclear ribonucleoprotein A/B | HNRNPAB | Thy-1 cell surface antigen | THY1 |
| heterogeneous nuclear ribonucleoprotein C (C1/C2) | HNRNPC | thymocyte nuclear protein 1 | THYN1 |
| heterogeneous nuclear ribonucleoprotein C-like 1 | HNRNPCL1 | TIA1 cytotoxic granule-associated RNA binding protein-like 1 | TIAL1 |
| heterogeneous nuclear ribonucleoprotein C-like 2 | HNRNPCL2 | T-cell lymphoma invasion and metastasis 2 | TIAM2 |
| heterogeneous nuclear ribonucleoprotein C-like 3 | HNRNPCL3 | tigger transposable element derived 4 | TIGD4 |
| heterogeneous nuclear ribonucleoprotein C-like 4 | HNRNPCL4 | translocase of inner mitochondrial membrane 50 homolog (S. cerevisiae) | TIMM50 |
| heterogeneous nuclear ribonucleoprotein D (AU-rich element RNA binding protein 1, 37kDa) | HNRNPD | TIMP metallopeptidase inhibitor 1 | TIMP1 |
| heterogeneous nuclear ribonucleoprotein D-like | HNRNPDL | TIMP metallopeptidase inhibitor 2 | TIMP2 |
| heterogeneous nuclear ribonucleoprotein F | HNRNPF | TIMP metallopeptidase inhibitor 3 | TIMP3 |
| heterogeneous nuclear ribonucleoprotein H1 (H) | HNRNPH1 | tubulointerstitial nephritis antigen-like 1 | TINAGL1 |
| heterogeneous nuclear ribonucleoprotein H2 (H') | HNRNPH2 | TOR signaling pathway regulator | TIPRL |
| heterogeneous nuclear ribonucleoprotein H3 (2H9) | HNRNPH3 | tight junction protein 1 | TJP1 |
| heterogeneous nuclear ribonucleoprotein K | HNRNPK | tight junction protein 2 | TJP2 |
| heterogeneous nuclear ribonucleoprotein L | HNRNPL | triokinase/FMN cyclase | TKFC |
| heterogeneous nuclear ribonucleoprotein M | HNRNPM | transketolase | TKT |
| heterogeneous nuclear ribonucleoprotein R | HNRNPR | talin 1 | TLN1 |
| heterogeneous nuclear ribonucleoprotein U (scaffold attachment factor A) | HNRNPU | talin 2 | TLN2 |
| heterogeneous nuclear ribonucleoprotein U-like 1 | HNRNPUL1 | toll-like receptor 2 | TLR2 |
| heterogeneous nuclear ribonucleoprotein U-like 2 | HNRNPUL2 | transmembrane 7 superfamily member 3 | TM7SF3 |
| homer scaffolding protein 3 | HOMER3 | transmembrane 9 superfamily member 2 | TM9SF2 |
| homeobox B3 | HOXB3 | transmembrane 9 superfamily member 3 | TM9SF3 |
| homeobox B7 | HOXB7 | transmembrane 9 superfamily protein member 4 | TM9SF4 |
| haptoglobin | HP | transmembrane BAX inhibitor motif containing 1 | TMBIM1 |
| heterochromatin protein 1, binding protein 3 | HP1BP3 | transmembrane BAX inhibitor motif containing 6 | TMBIM6 |
| hippocalcin | HPCA | transmembrane channel-like 6 | TMC6 |
| hippocalcin-like 1 | HPCAL1 | transmembrane channel-like 7 | TMC7 |
| 4-hydroxyphenylpyruvate dioxygenase | HPD | transmembrane channel-like 8 | TMC8 |
| 4-hydroxyphenylpyruvate dioxygenase-like | HPDL | transmembrane and coiled-coil domains 1 | TMCO1 |
| hydroxyprostaglandin dehydrogenase 15-(NAD) | HPGD | transmembrane emp24 protein transport domain containing 1 | TMED1 |
| haptoglobin-related protein | HPR | transmembrane emp24-like trafficking protein 10 (yeast) | TMED10 |
| hypoxanthine phosphoribosyltransferase 1 | HPRT1 | transmembrane emp24 domain trafficking protein 2 | TMED2 |
| Hermansky-Pudlak syndrome 6 | HPS6 | transmembrane emp24 protein transport domain containing 4 | TMED4 |
| heparanase | HPSE | transmembrane emp24 protein transport domain containing 5 | TMED5 |
| hemopexin | HPX | transmembrane emp24 protein transport domain containing 7 | TMED7 |
| Harvey rat sarcoma viral oncogene homolog | HRAS | TMED7-TICAM2 readthrough | TMED7-TICAM2 |
| histidine-rich glycoprotein | HRG | transmembrane emp24 protein transport domain containing 8 | TMED8 |
| hornerin | HRNR | transmembrane emp24 protein transport domain containing 9 | TMED9 |
| heat-responsive protein 12 | HRSP12 | transmembrane protein 100 | TMEM100 |
| heparan sulfate 2-O-sulfotransferase 1 | HS2ST1 | transmembrane protein 104 | TMEM104 |
| hydroxysteroid (17-beta) dehydrogenase 10 | HSD17B1 0 | transmembrane protein 109 | TMEM109 |
| hydroxysteroid (17-beta) dehydrogenase 12 | HSD17B1 2 | transmembrane protein 117 | TMEM117 |
| hydroxysteroid (17-beta) dehydrogenase 4 | HSD17B4 | transmembrane protein 165 | TMEM165 |
| hydroxysteroid dehydrogenase like 2 | HSDL2 | TMEM189-UBE2V1 readthrough | TMEM189-UBE2V1 |
| hematopoietic SH2 domain containing | HSH2D | transmembrane protein 192 | TMEM192 |
| heat shock protein 90kDa alpha (cytosolic), class A member 1 | HSP90AA 1 | transmembrane protein 2 | TMEM2 |
| heat shock protein 90kDa alpha (cytosolic), class A member 2, pseudogene | HSP90AA 2P | transmembrane protein 200A | TMEM200A |
| heat shock protein 90kDa alpha (cytosolic), class A member 4, pseudogene | HSP90AA 4P | transmembrane protein 237 | TMEM237 |
| heat shock protein 90kDa alpha (cytosolic), class B member 1 | HSP90AB 1 | transmembrane protein 256 | TMEM256 |
| heat shock protein 90kDa alpha (cytosolic), class B member 2, pseudogene | HSP90AB 2P | transmembrane protein 27 | TMEM27 |
| heat shock protein 90kDa alpha (cytosolic), class B member 3, pseudogene | HSP90AB 3P | transmembrane protein 30A | TMEM30A |
| heat shock protein 90kDa alpha (cytosolic), class B member 6, pseudogene | HSP90AB 6P | transmembrane protein 30B | TMEM30B |
| heat shock protein 90kDa beta (Grp94), member 1 | HSP90B1 | transmembrane protein 33 | TMEM33 |
| heat shock 70kDa protein 12A | HSPA12A | transmembrane protein 38A | TMEM38A |
| heat shock 70kD protein 12B | HSPA12B | transmembrane protein 40 | TMEM40 |
| heat shock protein 70kDa family, member 13 | HSPA13 | transmembrane protein 43 | TMEM43 |
| heat shock 70kDa protein 1A | HSPA1A | transmembrane protein 45B | TMEM45B |
| heat shock 70kDa protein 1B | HSPA1B | transmembrane protein 47 | TMEM47 |
| heat shock 70kDa protein 1-like | HSPA1L | transmembrane protein 50A | TMEM50A |
| heat shock 70kDa protein 2 | HSPA2 | transmembrane protein 51 | TMEM51 |
| heat shock 70kDa protein 4 | HSPA4 | transmembrane protein 52B | TMEM52B |
| heat shock 70kDa protein 4-like | HSPA4L | transmembrane protein 55A | TMEM55A |
| heat shock 70kDa protein 5 (glucose-regulated protein, 78kDa) | HSPA5 | transmembrane protein 55B | TMEM55B |
| heat shock 70kDa protein 6 (HSP70B') | HSPA6 | transmembrane protein 59 | TMEM59 |
| heat shock 70kDa protein 7 (HSP70B) | HSPA7 | transmembrane protein 63A | TMEM63A |
| heat shock 70kDa protein 8 | HSPA8 | transmembrane protein 63B | TMEM63B |
| heat shock 70kDa protein 9 (mortalin) | HSPA9 | transmembrane protein 87A | TMEM87A |
| heat shock 27kDa protein 1 | HSPB1 | transmembrane protein 87B | TMEM87B |
| heat shock 27kDa protein 1 pseudogene 1 | HSPB1P1 | transmembrane protein 9 | TMEM9 |
| heat shock 22kDa protein 8 | HSPB8 | TATA element modulatory factor 1 | TMF1 |
| heat shock 60kDa protein 1 (chaperonin) | HSPD1 | tropomodulin 2 (neuronal) | TMOD2 |
| heat shock 60kDa protein 1 (chaperonin) pseudogene 1 | HSPD1P1 | tropomodulin 3 (ubiquitous) | TMOD3 |
| heat shock 60kDa protein 1 (chaperonin) pseudogene 4 | HSPD1P4 | thymopoietin | TMPO |
| heat shock 60kDa protein 1 (chaperonin) pseudogene 5 | HSPD1P5 | transmembrane protease, serine 11B | TMPRSS11B |
| heat shock 60kDa protein 1 (chaperonin) pseudogene 6 | HSPD1P6 | transmembrane protease, serine 11D | TMPRSS11D |
| heat shock 10kDa protein 1 | HSPE1 | transmembrane protease, serine 2 | TMPRSS2 |
| heparan sulfate proteoglycan 2 | HSPG2 | transmembrane protease, serine 4 | TMPRSS4 |
| heat shock 105kDa/110kDa protein 1 | HSPH1 | thioredoxin-related transmembrane protein 1 | TMX1 |
| HIV-1 Tat interactive protein 2, 30kDa | HTATIP2 | thioredoxin-related transmembrane protein 2 | TMX2 |
| HIV-1 Tat specific factor 1 | HTATSF1 | tenascin C | TNC |
| HtrA serine peptidase 1 | HTRA1 | tumor necrosis factor, alpha-induced protein 2 | TNFAIP2 |
| HtrA serine peptidase 3 | HTRA3 | tumor necrosis factor, alpha-induced protein 3 | TNFAIP3 |
| huntingtin | HTT | tumor necrosis factor, alpha-induced protein 8 | TNFAIP8 |
| HECT, UBA and WWE domain containing 1, E3 ubiquitin protein ligase | HUWE1 | tumor necrosis factor receptor superfamily, member 10a | TNFRSF10A |
| hyaluronoglucosaminidase 2 | HYAL2 | tumor necrosis factor receptor superfamily, member 10b | TNFRSF10B |
| hydroxypyruvate isomerase (putative) | HYI | tumor necrosis factor receptor superfamily, member 10d, decoy with truncated death domain | TNFRSF10D |
| | | tumor necrosis factor receptor superfamily, member 11a, RANK | TNFRSF11A, RANK |
| hypoxia up-regulated 1 | HYOU1 | tumor necrosis factor receptor superfamily, member 11b | TNFRSF11B |
| isoleucyl-tRNA synthetase | IARS | tumor necrosis factor receptor superfamily, member 12A | TNFRSF12A |
| inhibitor of Bruton agammaglobulinemia tyrosine kinase | IBTK | tumor necrosis factor receptor superfamily, member 1A | TNFRSF1A |
| intercellular adhesion molecule 1 | ICAM1 | tumor necrosis factor receptor superfamily, member 21 | TNFRSF21 |
| intercellular adhesion molecule 2 | ICAM2 | tumor necrosis factor receptor superfamily, member 8 | TNFRSF8 |
| intercellular adhesion molecule 3 | ICAM3 | tumor necrosis factor (ligand) superfamily, member 10, tumor necrosis factor-related apoptosis-inducing ligand (TRAIL) | TNFSF10, TRAIL |
| intercellular adhesion molecule 5, telencephalin | ICAM5 | tumor necrosis factor (ligand) superfamily, member 12 | TNFSF12 |
| insulin-degrading enzyme | IDE | tumor necrosis factor (ligand) superfamily, member 13 | TNFSF13 |
| isocitrate dehydrogenase 1 (NADP+), soluble | IDH1 | tumor necrosis factor (ligand) superfamily, member 18 | TNFSF18 |
| isocitrate dehydrogenase 2 (NADP+), mitochondrial | IDH2 | TRAF2 and NCK interacting kinase | TNIK |
| isocitrate dehydrogenase 3 (NAD+) alpha | IDH3A | TNFAIP3 interacting protein 1 | TNIP1 |
| isocitrate dehydrogenase 3 (NAD+) beta | IDH3B | tankyrase 1 binding protein 1, 182kDa | TNKS1 BP1 |
| isopentenyl-diphosphate delta isomerase 1 | IDI1 | troponin I type 2 (skeletal, fast) | TNNI2 |
| idnK, gluconokinase homolog (E. coli) | IDNK | transportin 1 | TNPO1 |
| indolamin-2,3-dioxygenase | IDO | | |
| interferon, gamma-inducible protein 16 | IFI16 | transportin 2 | TNPO2 |
| interferon-induced protein with tetratricopeptide repeats 1 | IFIT1 | transportin 3 | TNPO3 |
| interferon-induced protein with tetratricopeptide repeats 3 | IFIT3 | tensin 3 | TNS3 |
| interferon-induced protein with tetratricopeptide repeats 5 | IFIT5 | tensin 4 | TNS4 |
| interferon induced transmembrane protein 1 | IFITM1 | target of EGR1, member 1 (nuclear) | TOE1 |
| interferon induced transmembrane protein 2 | IFITM2 | toll interacting protein | TOLLIP |
| interferon induced transmembrane protein 3 | IFITM3 | target of myb1 (chicken) | TOM1 |
| interferon, gamma | IFNG | target of myb1 (chicken)-like 1 | TOM1 L1 |
| interferon gamma receptor 1 | IFNGR1 | target of myb1-like 2 (chicken) | TOM1 L2 |
| | | Mitochondrial import receptor subunit TOM20 | TOM20 |
| interferon-related developmental regulator 1 | IFRD1 | translocase of outer mitochondrial membrane 22 homolog (yeast) | TOMM22 |
| intraflagellar transport 140 | IFT140 | translocase of outer mitochondrial membrane 34 | TOMM34 |
| insulin-like growth factor 1 receptor | IGF1R | translocase of outer mitochondrial membrane 40 homolog (yeast) | TOMM40 |
| insulin-like growth factor 2 | IGF2 | translocase of outer mitochondrial membrane 70 homolog A (S. cerevisiae) | TOMM70A |
| insulin-like growth factor 2 mRNA binding protein 2 | IGF2BP2 | topoisomerase (DNA) I | TOP1 |
| insulin-like growth factor 2 mRNA binding protein 3 | IGF2BP3 | topoisomerase (DNA) II alpha 170kDa | TOP2A |
| insulin-like growth factor 2 receptor | IGF2R | topoisomerase (DNA) II beta 180kDa | TOP2B |
| insulin-like growth factor binding protein, acid labile subunit | IGFALS | torsin family 1, member A (torsin A) | TOR1A |
| insulin-like growth factor binding protein 2, 36kDa | IGFBP2 | torsin family 1, member B (torsin B) | TOR1 B |
| insulin-like growth factor binding protein 3 | IGFBP3 | torsin family 3, member A | TOR3A |
| insulin-like growth factor binding protein 4 | IGFBP4 | torsin family 4, member A | TOR4A |
| insulin-like growth factor binding protein 6 | IGFBP6 | TP53 regulating kinase | TP53RK |
| insulin-like growth factor binding protein 7 | IGFBP7 | trophoblast glycoprotein | TPBG |
| IGF-like family member 1 | IGFL1 | trophoblast glycoprotein-like | TPBGL |
| immunoglobulin heavy locus | IGH | two pore segment channel 1 | TPCN1 |
| immunoglobulin heavy constant alpha 1 | IGHA1 | tumor protein D52 | TPD52 |
| immunoglobulin heavy constant alpha 2 (A2m marker) | IGHA2 | tumor protein D52-like 2 | TPD52L2 |
| immunoglobulin heavy constant gamma 1 (G1m marker) | IGHG1 | triosephosphate isomerase 1 | TPI1 |
| immunoglobulin heavy constant gamma 2 (G2m marker) | IGHG2 | triosephosphate isomerase 1 pseudogene 1 | TPI1P1 |
| immunoglobulin heavy constant gamma 3 (G3m marker) | IGHG3 | tropomyosin 1 (alpha) | TPM1 |
| immunoglobulin heavy constant gamma 4 (G4m marker) | IGHG4 | tropomyosin 2 (beta) | TPM2 |
| immunoglobulin heavy constant mu | IGHM | tropomyosin 3 | TPM3 |
| immunoglobulin heavy variable 3-11 (gene/pseudogene) | IGHV3-11 | tropomyosin 4 | TPM4 |
| immunoglobulin heavy variable 3-7 | IGHV3-7 | thiopurine S-methyltransferase | TPMT |
| immunoglobulin heavy variable 4-31 | IGHV4-31 | tripeptidyl peptidase I | TPP1 |
| immunoglobulin kappa locus | IGK | tripeptidyl peptidase II | TPP2 |
| immunoglobulin kappa constant | IGKC | tubulin polymerization promoting protein | TPPP |
| immunoglobulin kappa variable 1-5 | IGKV1-5 | tumor protein p63 regulated 1-like | TPRG1 L |
| immunoglobulin kappa variable 2-24 | IGKV2-24 | taperin | TPRN |
| immunoglobulin kappa variable 3-20 | IGKV3-20 | tyrosylprotein sulfotransferase 2 | TPST2 |
| immunoglobulin kappa variable 3D-15 (gene/pseudogene) | IGKV3D-15 | tumor protein, translationally-controlled 1 | TPT1 |
| immunoglobulin kappa variable 4-1 | IGKV4-1 | transformer 2 alpha homolog (Drosophila) | TRA2A |
| immunoglobulin lambda locus | IGL | transformer 2 beta homolog (Drosophila) | TRA2B |
| immunoglobulin lambda constant 1 (Mcg marker) | IGLC1 | TNFRSF1A-associated via death domain | TRADD |
| immunoglobulin lambda constant 2 (Kern-Oz- marker) | IGLC2 | TNF receptor-associated factor 1 | TRAF1 |
| immunoglobulin lambda constant 3 (Kern-Oz+ marker) | IGLC3 | TNF receptor-associated factor 4 | TRAF4 |
| immunoglobulin lambda constant 6 (Kern+Oz- marker, gene/pseudogene) | IGLC6 | trafficking protein, kinesin binding 1 | TRAK1 |
| immunoglobulin lambda constant 7 | IGLC7 | tetratricopeptide repeat and ankyrin repeat containing 1 | TRANK1 |
| immunoglobulin lambda-like polypeptide 5 | IGLL5 | TNF receptor-associated protein 1 | TRAP1 |
| immunoglobulin lambda variable 1-40 | IGLV1-40 | trafficking protein particle complex 10 | TRAPPC10 |
| immunoglobulin lambda variable 1-44 | IGLV1-44 | trafficking protein particle complex 11 | TRAPPC11 |
| immunoglobulin lambda variable 2-11 | IGLV2-11 | trafficking protein particle complex 12 | TRAPPC12 |
| immunoglobulin lambda variable 3-21 | IGLV3-21 | trafficking protein particle complex 13 | TRAPPC13 |
| immunoglobulin lambda variable 4-3 | IGLV4-3 | trafficking protein particle complex 3 | TRAPPC3 |
| immunoglobulin superfamily, member 3 | IGSF3 | trafficking protein particle complex 4 | TRAPPC4 |
| immunoglobulin superfamily, member 8 | IGSF8 | trafficking protein particle complex 5 | TRAPPC5 |
| inhibitor of kappa light polypeptide gene enhancer in B-cells, kinase complex-associated protein | IKBKAP | trafficking protein particle complex 8 | TRAPPC8 |
| inhibitor of kappa light polypeptide gene enhancer in B-cells, kinase beta | IKBKB | trafficking protein particle complex 9 | TRAPPC9 |
| inhibitor of kappa light polypeptide gene enhancer in B-cells, kinase gamma | IKBKG | T cell receptor associated transmembrane adaptor 1 | TRAT1 |
| IKAROS family zinc finger 5 (Pegasus) | IKZF5 | trehalase (brush-border membrane glycoprotein) | TREH |
| interleukin 10 | IL10 | triggering receptor expressed on myeloid cells 1 | TREM1 |
| interleukin 11 | IL11 | triggering receptor expressed on myeloid cells-like 1 | TREML1 |
| interleukin 13 | IL13 | triggering receptor expressed on myeloid cells-like 2 | TREML2 |
| interleukin 13 receptor, alpha 2 | IL13RA2 | triggering receptor expressed on myeloid cells-like 5, pseudogene | TREML5P |
| interleukin 15 receptor, alpha | IL15RA | thyrotropin-releasing hormone degrading enzyme | TRHDE |
| interleukin 17B | IL17B | tripartite motif containing 16 | TRIM16 |
| interleukin 17 receptor C | IL17RC | tripartite motif containing 21 | TRIM21 |
| interleukin 19 | IL19 | tripartite motif containing 23 | TRIM23 |
| interleukin 1 receptor accessory protein | IL1RAP | tripartite motif containing 25 | TRIM25 |
| interleukin 1 receptor accessory protein-like 1 | IL1RAPL1 | tripartite motif containing 26 | TRIM26 |
| interleukin 1 receptor-like 2 | IL1 RL2 | tripartite motif containing 28 | TRIM28 |
| interleukin 1 receptor antagonist | IL1RN | tripartite motif containing 32 | TRIM32 |
| interleukin 22 receptor, alpha 1 | IL22RA1 | tripartite motif containing 38 | TRIM38 |
| interleukin 23, alpha subunit p19 | IL23A | tripartite motif containing 40 | TRIM40 |
| interleukin 27 receptor, alpha | IL27RA | tripartite motif containing 41 | TRIM41 |
| interleukin 3 | IL3 | tripartite motif containing 56 | TRIM56 |
| interleukin 36, gamma | IL36G | tripartite motif containing 58 | TRIM58 |
| interleukin 4 induced 1 | IL411 | tripartite motif containing 65 | TRIM65 |
| interleukin 5 | IL5 | tripartite motif containing 69 | TRIM69 |
| interleukin 6 signal transducer | IL6ST | trio Rho guanine nucleotide exchange factor | TRIO |
| interleukin 7 | IL7 | TRIO and F-actin binding protein | TRIOBP |
| immunoglobulin-like domain containing receptor 1 | ILDR1 | thyroid hormone receptor interactor 10 | TRIP10 |
| interleukin enhancer binding factor 2 | ILF2 | thyroid hormone receptor interactor 11 | TRIP11 |
| interleukin enhancer binding factor 3, 90kDa | ILF3 | thyroid hormone receptor interactor 12 | TRIP12 |
| integrin-linked kinase | ILK | thyroid hormone receptor interactor 13 | TRIP13 |
| integrin-linked kinase-associated serine/threonine phosphatase | ILKAP | thyroid hormone receptor interactor 6 | TRIP6 |
| inner membrane protein, mitochondrial | IMMT | tRNA methyltransferase 10 homolog A (S. cerevisiae) | TRMT10A |
| IMP3, U3 small nucleolar ribonucleoprotein | IMP3 | tRNA methyltransferase 11-2 homolog (S. cerevisiae) | TRMT112 |
| inositol(myo)-1 (or 4)-monophosphatase 1 | IMPA1 | tRNA methyltransferase 1 homolog (S. cerevisiae)-like | TRMT1 L |
| inositol(myo)-1 (or 4)-monophosphatase 2 | IMPA2 | tRNA methyltransferase 2 homolog A (S. cerevisiae) | TRMT2A |
| inositol monophosphatase domain containing 1 | IMPAD1 | tRNA methyltransferase 61A | TRMT61A |
| IMP (inosine 5'-monophosphate) dehydrogenase 1 | IMPDH1 | TROVE domain family, member 2 | TROVE2 |
| IMP (inosine 5'-monophosphate) dehydrogenase 2 | IMPDH2 | transient receptor potential cation channel, subfamily C, member 6 | TRPC6 |
| internexin neuronal intermediate filament protein, alpha | INA | transient receptor potential cation channel, subfamily M, member 4 | TRPM4 |
| InaD-like (Drosophila) | INADL | transient receptor potential cation channel, subfamily V, member 4 | TRPV4 |
| inverted formin, FH2 and WH2 domain containing | INF2 | transformation/transcription domain-associated protein | TRRAP |
| inhibin, beta A | INHBA | TruB pseudouridine (psi) synthase family member 1 | TRUB1 |
| inhibin, beta B | INHBB | tuberous sclerosis 2 | TSC2 |
| INO80 complex subunit B | INO80B | tumor susceptibility 101 | TSG101 |
| inositol polyphosphate-1-phosphatase | INPP1 | thyroid stimulating hormone receptor | TSHR |
| inositol polyphosphate-4-phosphatase, type I, 107kDa | INPP4A | teashirt zinc finger homeobox 1 | TSHZ1 |
| inositol polyphosphate-5-phosphatase, 40kDa | INPP5A | translin | TSN |
| inositol polyphosphate-5-phosphatase, 75kDa | INPP5B | translin-associated factor X | TSNAX |
| inositol polyphosphate-5-phosphatase, 145kDa | INPP5D | translin-associated factor X interacting protein 1 | TSNAXIP1 |
| inositol polyphosphate phosphatase-like 1 | INPPL1 | tetraspanin 1 | TSPAN1 |
| insulin | INS | tetraspanin 10 | TSPAN10 |
| insulinoma-associated 2 | INSM2 | tetraspanin 14 | TSPAN14 |
| insulin receptor | INSR | tetraspanin 15 | TSPAN15 |
| insulin receptor-related receptor | INSRR | tetraspanin 3 | TSPAN3 |
| integrator complex subunit 1 | INTS1 | tetraspanin 33 | TSPAN33 |
| integrator complex subunit 3 | INTS3 | tetraspanin 4 | TSPAN4 |
| importin 11 | IPO11 | tetraspanin 6 | TSPAN6 |
| importin 4 | IPO4 | tetraspanin 8 | TSPAN8 |
| importin 5 | IPO5 | tetraspanin 9 | TSPAN9 |
| importin 7 | IPO7 | translocator protein (18kDa) | TSPO |
| importin 8 | IPO8 | TSPY-like 4 | TSPYL4 |
| importin 9 | IPO9 | TSR1, 20S rRNA accumulation, homolog (S. cerevisiae) | TSR1 |
| IQ motif containing G | IQCG | tumor suppressing subtransferable candidate 1 | TSSC1 |
| IQ motif containing GTPase activating protein 1 | IQGAP1 | thiosulfate sulfurtransferase (rhodanese) | TST |
| IQ motif containing GTPase activating protein 2 | IQGAP2 | tissue specific transplantation antigen P35B | TSTA3 |
| IQ motif containing GTPase activating protein 3 | IQGAP3 | tau tubulin kinase 2 | TTBK2 |
| interleukin-1 receptor-associated kinase 1 binding protein 1 | IRAK1 BP1 | tetratricopeptide repeat domain 17 | TTC17 |
| interferon regulatory factor 6 | IRF6 | tetratricopeptide repeat domain 21B | TTC21B |
| insulin receptor substrate 2 | IRS2 | tetratricopeptide repeat domain 27 | TTC27 |
| insulin receptor substrate 4 | IRS4 | tetratricopeptide repeat domain 37 | TTC37 |
| iron-sulfur cluster assembly enzyme | ISCU | tetratricopeptide repeat domain 38 | TTC38 |
| ISG15 ubiquitin-like modifier | ISG15 | tetratricopeptide repeat domain 39A | TTC39A |
| interferon stimulated exonuclease gene 20kDa-like 2 | ISG20L2 | tetratricopeptide repeat domain 7A | TTC7A |
| increased sodium tolerance 1 homolog (yeast) | IST1 | tetratricopeptide repeat domain 7B | TTC7B |
| ISY1 splicing factor homolog (S. cerevisiae) | ISY1 | TELO2 interacting protein 1 | TTI1 |
| ISY1-RAB43 readthrough | ISY1-RAB43 | TELO2 interacting protein 2 | TTI2 |
| inositol-3-phosphate synthase 1 | ISYNA1 | tubulin tyrosine ligase-like family member 12 | TTLL12 |
| itchy E3 ubiquitin protein ligase | ITCH | tubulin tyrosine ligase-like family member 3 | TTLL3 |
| integrin alpha FG-GAP repeat containing 3 | ITFG3 | titin | TTN |
| integrin, alpha 1 | ITGA1 | transthyretin | TTR |
| integrin, alpha 11 | ITGA11 | tweety family member 2 | TTYH2 |
| integrin, alpha 2 (CD49B, alpha 2 subunit of VLA-2 receptor) | ITGA2 | tweety family member 3 | TTYH3 |
| integrin, alpha 2b (platelet glycoprotein IIb of IIb/IIIa complex, antigen CD41) | ITGA2B | tubulin, alpha 1a | TUBA1A |
| integrin, alpha 3 (antigen CD49C, alpha 3 subunit of VLA-3 receptor) | ITGA3 | tubulin, alpha 1b | TUBA1 B |
| integrin, alpha 4 (antigen CD49D, alpha 4 subunit of VLA-4 receptor) | ITGA4 | tubulin, alpha 1c | TUBA1C |
| integrin, alpha 5 (fibronectin receptor, alpha polypeptide) | ITGA5 | tubulin, alpha 3c | TUBA3C |
| integrin, alpha 6 | ITGA6 | tubulin, alpha 3d | TUBA3D |
| integrin, alpha 9 | ITGA9 | tubulin, alpha 3e | TUBA3E |
| integrin, alpha L (antigen CD11A (p180), lymphocyte function-associated antigen 1; alpha polypeptide) | ITGAL | tubulin, alpha 4a | TUBA4A |
| integrin, alpha M (complement component 3 receptor 3 subunit) | ITGAM | tubulin, alpha 4b | TUBA4B |
| integrin, alpha V | ITGAV | tubulin, alpha 8 | TUBA8 |
| integrin, alpha X (complement component 3 receptor 4 subunit) | ITGAX | tubulin, alpha-like 3 | TUBAL3 |
| integrin, beta 1 (fibronectin receptor, beta polypeptide, antigen CD29 includes MDF2, MSK12) | ITGB1 | tubulin, alpha pseudogene 2 | TUBAP2 |
| integrin, beta 2 (complement component 3 receptor 3 and 4 subunit) | ITGB2 | tubulin, beta class I | TUBB |
| integrin, beta 3 (platelet glycoprotein IIIa, antigen CD61) | ITGB3 | tubulin, beta 1 class VI | TUBB1 |
| integrin, beta 4 | ITGB4 | tubulin, beta 2A class IIa | TUBB2A |
| integrin, beta 5 | ITGB5 | tubulin, beta 2B class IIb | TUBB2B |
| integrin, beta 6 | ITGB6 | tubulin, beta 3 class III | TUBB3 |
| integrin, beta 7 | ITGB7 | tubulin, beta 4A class IVa | TUBB4A |
| integrin, beta 8 | ITGB8 | tubulin, beta 4B class IVb | TUBB4B |
| inter-alpha-trypsin inhibitor heavy chain 1 | ITIH1 | tubulin, beta 6 class V | TUBB6 |
| inter-alpha-trypsin inhibitor heavy chain 2 | ITIH2 | tubulin, beta 7, pseudogene | TUBB7P |
| inter-alpha-trypsin inhibitor heavy chain 3 | ITIH3 | tubulin, beta 8 class VIII | TUBB8 |
| inter-alpha-trypsin inhibitor heavy chain family, member 4 | ITIH4 | tubulin, beta pseudogene 1 | TUBBP1 |
| IL2-inducible T-cell kinase | ITK | tubulin, beta pseudogene 2 | TUBBP2 |
| intelectin 1 (galactofuranose binding) | ITLN1 | tubulin, beta class I pseudogene 6 | TUBBP6 |
| integral membrane protein 2B | ITM2B | tubulin, gamma 1 | TUBG1 |
| integral membrane protein 2C | ITM2C | tubulin, gamma complex associated protein 2 | TUBGCP2 |
| inosine triphosphatase (nucleoside triphosphate pyrophosphatase) | ITPA | tubulin, gamma complex associated protein 3 | TUBGCP3 |
| inositol 1,4,5-trisphosphate receptor, type 2 | ITPR2 | tubulin, gamma complex associated protein 4 | TUBGCP4 |
| inositol 1,4,5-trisphosphate receptor, type 3 | ITPR3 | Tu translation elongation factor, mitochondrial | TUFM |
| inositol 1,4,5-trisphosphate receptor interacting protein-like 2 | ITPRIPL2 | twinfilin actin binding protein 1 | TWF1 |
| intersectin 1 (SH3 domain protein) | ITSN1 | twinfilin actin binding protein 2 | TWF2 |
| intersectin 2 | ITSN2 | taxilin alpha | TXLNA |
| involucrin | IVL | thioredoxin | TXN |
| jade family PHD finger 2 | JADE2 | thioredoxin domain containing 16 | TXNDC16 |
| jagged 1 | JAG1 | thioredoxin domain containing 17 | TXNDC17 |
| Janus kinase 1 | JAK1 | thioredoxin domain containing 5 (endoplasmic reticulum) | TXNDC5 |
| Janus kinase 2 | JAK2 | thioredoxin domain containing 8 (spermatozoa) | TXNDC8 |
| Janus kinase 3 | JAK3 | thioredoxin-like 1 | TXNL1 |
| janus kinase and microtubule interacting protein 1 | JAKMIP1 | thioredoxin reductase 1 | TXNRD1 |
| junctional adhesion molecule 3 | JAM3 | tyrosine kinase 2 | TYK2 |
| joining chain of multimeric IgA and IgM | JCHAIN | thymidine phosphorylase | TYMP |
| jumonji domain containing 1C | JMJD1C | tyrosinase-related protein 1 | TYRP1 |
| junctophilin 3 | JPH3 | tRNA-yW synthesizing protein 5 | TYW5 |
| junction plakoglobin | JUP | U2 small nuclear RNA auxiliary factor 1 | U2AF1 |
| kalirin, RhoGEF kinase | KALRN | U2 small nuclear RNA auxiliary factor 1-like 4 | U2AF1 L4 |
| lysyl-tRNA synthetase | KARS | U2 small nuclear RNA auxiliary factor 2 | U2AF2 |
| katanin p60 subunit A-like 2 | KATNAL2 | U2 snRNP-associated SURP domain containing | U2SURP |
| kelch repeat and BTB (POZ) domain containing 11 | KBTBD11 | uveal autoantigen with coiled-coil domains and ankyrin repeats | UACA |
| potassium channel, voltage gated subfamily A regulatory beta subunit 2 | KCNAB2 | UDP-N-acetylglucosamine pyrophosphorylase 1 | UAP1 |
| potassium channel, voltage gated modifier subfamily G, member 2 | KCNG2 | UDP-N-acetylglucosamine pyrophosphorylase 1 like 1 | UAP1L1 |
| potassium channel, calcium activated intermediate/small conductance subfamily N alpha, member 4 | KCNN4 | ubiquitin-like modifier activating enzyme 1 | UBA1 |
| potassium voltage-gated channel, modifier subfamily S, member 3 | KCNS3 | ubiquitin-like modifier activating enzyme 2 | UBA2 |
| potassium channel, sodium activated subfamily T, member 1 | KCNT1 | ubiquitin-like modifier activating enzyme 5 | UBA5 |
| potassium channel tetramerization domain containing 12 | KCTD12 | ubiquitin A-52 residue ribosomal protein fusion product 1 | UBA52 |
| potassium channel tetramerization domain containing 14 | KCTD14 | ubiquitin-like modifier activating enzyme 6 | UBA6 |
| potassium channel tetramerization domain containing 21 | KCTD21 | ubiquitin-like modifier activating enzyme 7 | UBA7 |
| KDEL (Lys-Asp-Glu-Leu) endoplasmic reticulum protein retention receptor 2 | KDELR2 | UBA domain containing 1 | UBAC1 |
| lysine (K)-specific demethylase 1A | KDM1A | UBA domain containing 2 | UBAC2 |
| lysine (K)-specific demethylase 6B | KDM6B | ubiquitin associated protein 1 | UBAP1 |
| KH domain containing, RNA binding, signal transduction associated 1 | KHDRBS1 | ubiquitin associated protein 2-like | UBAP2L |
| ketohexokinase (fructokinase) | KHK | ubiquitin associated and SH3 domain containing B | UBASH3B |
| KH-type splicing regulatory protein | KHSRP | ubiquitin B | UBB |
| KIAA0020 | KIAA0020 | ubiquitin C | UBC |
| KIAA0195 | KIAA0195 | ubiquitin-conjugating enzyme E2D 2 | UBE2D2 |
| KIAA0196 | KIAA0196 | ubiquitin-conjugating enzyme E2D 3 | UBE2D3 |
| KIAA0319-like | KIAA0319 L | ubiquitin-conjugating enzyme E2G 1 | UBE2G1 |
| KIAA0368 | KIAA0368 | ubiquitin-conjugating enzyme E2K | UBE2K |
| KIAA0513 | KIAA0513 | ubiquitin-conjugating enzyme E2L 3 | UBE2L3 |
| KIAA1033 | KIAA1033 | ubiquitin-conjugating enzyme E2M | UBE2M |
| KIAA1161 | KIAA1161 | ubiquitin-conjugating enzyme E2N | UBE2N |
| KIAA1217 | KIAA1217 | ubiquitin-conjugating enzyme E2N-like (gene/pseudogene) | UBE2NL |
| KIAA1324 | KIAA1324 | ubiquitin-conjugating enzyme E2O | UBE2O |
| KIAA1468 | KIAA1468 | ubiquitin-conjugating enzyme E2 variant 1 | UBE2V1 |
| KIAA1522 | KIAA1522 | ubiquitin-conjugating enzyme E2 variant 2 | UBE2V2 |
| KIAA1524 | KIAA1524 | ubiquitin-conjugating enzyme E2Z | UBE2Z |
| KIAA1598 | KIAA1598 | ubiquitin protein ligase E3A | UBE3A |
| KIAA2013 | KIAA2013 | ubiquitin protein ligase E3B | UBE3B |
| kinase D-interacting substrate, 220kDa | KIDINS22 0 | ubiquitin protein ligase E3C | UBE3C |
| kinesin family member 12 | KIF12 | ubiquitination factor E4A | UBE4A |
| kinesin family member 13A | KIF13A | ubiquitination factor E4B | UBE4B |
| kinesin family member 13B | KIF13B | ubiquitin-like 3 | UBL3 |
| kinesin family member 15 | KIF15 | ubiquilin 1 | UBQLN1 |
| kinesin family member 17 | KIF17 | ubiquilin 2 | UBQLN2 |
| kinesin family member 18B | KIF18B | ubiquilin 4 | UBQLN4 |
| kinesin family member 1B | KIF1B | ubiquitin protein ligase E3 component n-recognin 2 | UBR2 |
| KIF1 binding protein | KIF1BP | ubiquitin protein ligase E3 component n-recognin 4 | UBR4 |
| kinesin family member 21A | KIF21A | ubiquitin domain containing 1 | UBTD1 |
| kinesin family member 23 | KIF23 | ubiquitin domain containing 2 | UBTD2 |
| kinesin family member 26A | KIF26A | UBX domain protein 1 | UBXN1 |
| kinesin heavy chain member 2A | KIF2A | UBX domain protein 6 | UBXN6 |
| kinesin family member 3A | KIF3A | ubiquitin carboxyl-terminal esterase L1 (ubiquitin thiolesterase) | UCHL1 |
| kinesin family member 3B | KIF3B | ubiquitin carboxyl-terminal esterase L3 (ubiquitin thiolesterase) | UCHL3 |
| kinesin family member 3C | KIF3C | ubiquitin carboxyl-terminal hydrolase L5 | UCHL5 |
| kinesin family member 4A | KIF4A | uridine-cytidine kinase 2 | UCK2 |
| kinesin family member 5A | KIF5A | UEV and lactate/malate dehyrogenase domains | UEVLD |
| kinesin family member 5B | KIF5B | ubiquitin-fold modifier conjugating enzyme 1 | UFC1 |
| kinesin family member 5C | KIF5C | ubiquitin fusion degradation 1 like (yeast) | UFD1L |
| kinesin family member 6 | KIF6 | UFM1-specific ligase 1 | UFL1 |
| kinesin family member 9 | KIF9 | UDP-glucose ceramide glucosyltransferase | UGCG |
| kinesin family member C1 | KIFC1 | UDP-glucose 6-dehydrogenase | UGDH |
| kinesin family member C3 | KIFC3 | UDP-glucose glycoprotein glucosyltransferase 1 | UGGT1 |
| kin of IRRE like (Drosophila) | KIRREL | UDP-glucose pyrophosphorylase 2 | UGP2 |
| v-kit Hardy-Zuckerman 4 feline sarcoma viral oncogene homolog | KIT | ubiquitin-like with PHD and ring finger domains 1 | UHRF1 |
| klotho | KL | unc-51 like kinase 3 | ULK3 |
| kinesin light chain 1 | KLC1 | uromodulin | UMOD |
| kinesin light chain 2 | KLC2 | uridine monophosphate synthetase | UMPS |
| kinesin light chain 4 | KLC4 | unc-119 homolog B (C. elegans) | UNC119B |
| KLF3 antisense RNA 1 | KLF3-AS1 | unc-13 homolog B (C. elegans) | UNC13B |
| kelch domain containing 10 | KLHDC10 | unc-13 homolog C (C. elegans) | UNC13C |
| kelch-like family member 14 | KLHL14 | unc-13 homolog D (C. elegans) | UNC13D |
| kelch-like family member 22 | KLHL22 | unc-45 homolog A (C. elegans) | UNC45A |
| kallikrein 1 | KLK1 | unc-5 homolog D (C. elegans) | UNC5D |
| kallikrein-related peptidase 10 | KLK10 | unc-80 homolog (C. elegans) | UNC80 |
| kallikrein-related peptidase 11 | KLK11 | ureidopropionase, beta | UPB1 |
| kallikrein-related peptidase 5 | KLK5 | UPF1 regulator of nonsense transcripts homolog (yeast) | UPF1 |
| kallikrein-related peptidase 6 | KLK6 | uroplakin 1A | UPK1A |
| kallikrein-related peptidase 7 | KLK7 | uroplakin 1B | UPK1B |
| kallikrein-related peptidase 8 | KLK8 | uroplakin 2 | UPK2 |
| kallikrein B, plasma (Fletcher factor) 1 | KLKB1 | uroplakin 3A | UPK3A |
| killer cell lectin-like receptor subfamily G, member 2 | KLRG2 | uroplakin 3B-like | UPK3BL |
| lysine (K)-specific methyltransferase 2C | KMT2C | uridine phosphorylase 1 | UPP1 |
| lysine (K)-specific methyltransferase 2D | KMT2D | ubiquinol-cytochrome c reductase core protein I | UQCRC1 |
| kininogen 1 | KNG1 | ubiquinol-cytochrome c reductase core protein II | UQCRC2 |
| karyopherin alpha 1 (importin alpha 5) | KPNA1 | URB1 ribosome biogenesis 1 homolog (S. cerevisiae) | URB1 |
| karyopherin alpha 2 (RAG cohort 1, importin alpha 1) | KPNA2 | uroporphyrinogen decarboxylase | UROD |
| karyopherin alpha 4 (importin alpha 3) | KPNA4 | USO1 vesicle transport factor | USO1 |
| karyopherin alpha 6 (importin alpha 7) | KPNA6 | ubiquitin specific peptidase 1 | USP1 |
| karyopherin (importin) beta 1 | KPNB1 | ubiquitin specific peptidase 11 | USP11 |
| keratinocyte proline-rich protein | KPRP | ubiquitin specific peptidase 14 (tRNA-guanine transglycosylase) | USP14 |
| Kirsten rat sarcoma viral oncogene homolog | KRAS | ubiquitin specific peptidase 15 | USP15 |
| KRIT1, ankyrin repeat containing | KRIT1 | ubiquitin specific peptidase 24 | USP24 |
| KRR1, small subunit (SSU) processome component, homolog (yeast) | KRR1 | ubiquitin specific peptidase 39 | USP39 |
| keratin 1, type II | KRT1 | ubiquitin specific peptidase 4 (proto-oncogene) | USP4 |
| keratin 10, type I | KRT10 | ubiquitin specific peptidase 46 | USP46 |
| keratin 12, type I | KRT12 | ubiquitin specific peptidase 49 | USP49 |
| keratin 13, type I | KRT13 | ubiquitin specific peptidase 5 (isopeptidase T) | USP5 |
| keratin 14, type I | KRT14 | USP6 N-terminal like | USP6NL |
| keratin 15, type I | KRT15 | ubiquitin specific peptidase 7 (herpes virus-associated) | USP7 |
| keratin 16, type I | KRT16 | ubiquitin specific peptidase 9, X-linked | USP9X |
| keratin 16 pseudogene 2 | KRT16P2 | ubiquitin specific peptidase 9, Y-linked | USP9Y |
| keratin 17, type I | KRT17 | UTP14, U3 small nucleolar ribonucleoprotein, homolog A (yeast) | UTP14A |
| keratin 17 pseudogene 3 | KRT17P3 | UTP20, small subunit (SSU) processome component, homolog (yeast) | UTP20 |
| keratin 18, type I | KRT18 | utrophin | UTRN |
| keratin 18 pseudogene 19 | KRT18P19 | urotensin 2 | UTS2 |
| keratin 19, type I | KRT19 | UDP-glucuronate decarboxylase 1 | UXS1 |
| keratin 2, type II | KRT2 | Vac14 homolog (S. cerevisiae) | VAC14 |
| keratin 20, type I | KRT20 | vesicle-associated membrane protein 1 (synaptobrevin 1) | VAMP1 |
| keratin 24, type I | KRT24 | vesicle-associated membrane protein 2 (synaptobrevin 2) | VAMP2 |
| keratin 25, type I | KRT25 | vesicle-associated membrane protein 3 | VAMP3 |
| keratin 27, type I | KRT27 | vesicle-associated membrane protein 5 | VAMP5 |
| keratin 28, type I | KRT28 | vesicle-associated membrane protein 7 | VAMP7 |
| keratin 3, type II | KRT3 | vesicle-associated membrane protein 8 | VAMP8 |
| keratin 31, type I | KRT31 | VANGL planar cell polarity protein 1 | VANGL 1 |
| keratin 33B, type I | KRT33B | VAMP (vesicle-associated membrane protein)-associated protein A, 33kDa | VAPA |
| keratin 36, type I | KRT36 | VAMP (vesicle-associated membrane protein)-associated protein B and C | VAPB |
| keratin 37, type I | KRT37 | valyl-tRNA synthetase | VARS |
| keratin 38, type I | KRT38 | vasorin | VASN |
| keratin 4, type II | KRT4 | vasodilator-stimulated phosphoprotein | VASP |
| keratin 5, type II | KRT5 | vesicle amine transport 1 | VAT1 |
| keratin 6A, type II | KRT6A | vesicle amine transport 1-like | VAT1L |
| keratin 6B, type II | KRT6B | vav 1 guanine nucleotide exchange factor | VAV1 |
| keratin 6C, type II | KRT6C | von Hippel-Lindau binding protein 1 | VBP1 |
| keratin 7, type II | KRT7 | versican | VCAN |
| keratin 71, type II | KRT71 | vinculin | VCL |
| keratin 72, type II | KRT72 | valosin containing protein | VCP |
| keratin 73, type II | KRT73 | valosin containing protein (p97)/p47 complex interacting protein 1 | VCPIP1 |
| keratin 74, type II | KRT74 | voltage-dependent anion channel 1 | VDAC1 |
| keratin 75, type II | KRT75 | voltage-dependent anion channel 2 | VDAC2 |
| keratin 76, type II | KRT76 | voltage-dependent anion channel 3 | VDAC3 |
| keratin 77, type II | KRT77 | vascular endothelial growth factor C | VEGFC |
| keratin 78, type II | KRT78 | villin 1 | VIL1 |
| keratin 79, type II | KRT79 | vimentin | VIM |
| keratin 8, type II | KRT8 | vitelline membrane outer layer 1 homolog (chicken) | VMO1 |
| keratin 80, type II | KRT80 | vanin 1 | VNN1 |
| keratin 84, type II | KRT84 | vacuolar protein sorting 11 homolog (S. cerevisiae) | VPS11 |
| keratin 8 pseudogene 45 | KRT8P45 | vacuolar protein sorting 13 homolog C (S. cerevisiae) | VPS13C |
| keratin 8 pseudogene 9 | KRT8P9 | vacuolar protein sorting 13 homolog D (S. cerevisiae) | VPS13D |
| keratin 9, type I | KRT9 | vacuolar protein sorting 16 homolog (S. cerevisiae) | VPS16 |
| keratinocyte differentiation-associated protein | KRTDAP | vacuolar protein sorting 18 homolog (S. cerevisiae) | VPS18 |
| kinectin 1 (kinesin receptor) | KTN1 | vacuolar protein sorting 25 homolog (S. cerevisiae) | VPS25 |
| L1 cell adhesion molecule | L1CAM | vacuolar protein sorting 26 homolog A (S. pombe) | VPS26A |
| lacritin | LACRT | vacuolar protein sorting 26 homolog B (S. pombe) | VPS26B |
| ladinin 1 | LAD1 | vacuolar protein sorting 28 homolog (S. cerevisiae) | VPS28 |
| laminin, alpha 1 | LAMA1 | vacuolar protein sorting 29 homolog (S. cerevisiae) | VPS29 |
| laminin, alpha 3 | LAMA3 | vacuolar protein sorting 33 homolog A (S. cerevisiae) | VPS33A |
| laminin, alpha 4 | LAMA4 | vacuolar protein sorting 33 homolog B (yeast) | VPS33B |
| laminin, alpha 5 | LAMA5 | vacuolar protein sorting 35 homolog (S. cerevisiae) | VPS35 |
| laminin, beta 1 | LAMB1 | vacuolar protein sorting 36 homolog (S. cerevisiae) | VPS36 |
| laminin, beta 2 (laminin S) | LAMB2 | vacuolar protein sorting 37 homolog B (S. cerevisiae) | VPS37B |
| laminin, beta 3 | LAMB3 | vacuolar protein sorting 37 homolog C (S. cerevisiae) | VPS37C |
| laminin, gamma 1 (formerly LAMB2) | LAMC1 | vacuolar protein sorting 37 homolog D (S. cerevisiae) | VPS37D |
| laminin, gamma 2 | LAMC2 | vacuolar protein sorting 39 homolog (S. cerevisiae) | VPS39 |
| lysosomal-associated membrane protein 1 | LAMP1 | vacuolar protein sorting 41 homolog (S. cerevisiae) | VPS41 |
| lysosomal-associated membrane protein 2 | LAMP2 | vacuolar protein sorting 45 homolog (S. cerevisiae) | VPS45 |
| late endosomal/lysosomal adaptor, MAPK and MTOR activator 1 | LAMTOR1 | vacuolar protein sorting 4 homolog A (S. cerevisiae) | VPS4A |
| late endosomal/lysosomal adaptor, MAPK and MTOR activator 3 | LAMTOR3 | vacuolar protein sorting 4 homolog B (S. cerevisiae) | VPS4B |
| late endosomal/lysosomal adaptor, MAPK and MTOR activator 4 | LAMTOR4 | vacuolar protein sorting 51 homolog (S. cerevisiae) | VPS51 |
| LanC lantibiotic synthetase component C-like 1 (bacterial) | LANCL1 | vacuolar protein sorting 52 homolog (S. cerevisiae) | VPS52 |
| LanC lantibiotic synthetase component C-like 2 (bacterial) | LANCL2 | vacuolar protein sorting 53 homolog (S. cerevisiae) | VPS53 |
| leucine aminopeptidase 3 | LAP3 | vacuolar protein sorting 8 homolog (S. cerevisiae) | VPS8 |
| lysosomal protein transmembrane 5 | LAPTM5 | vaccinia related kinase 1 | VRK1 |
| La ribonucleoprotein domain family, member 7 | LARP7 | vaccinia related kinase 3 | VRK3 |
| leucyl-tRNA synthetase | LARS | visual system homeobox 2 | VSX2 |
| LAS1-like (S. cerevisiae) | LAS1 L | vesicle (multivesicular body) trafficking 1 | VTA1 |
| LIM and SH3 protein 1 | LASP1 | vesicle transport through interaction with t-SNAREs 1A | VTI1A |
| linker for activation of T cells | LAT | vesicle transport through interaction with t-SNAREs 1B | VTI1B |
| linker for activation of T cells family, member 2 | LAT2 | vitronectin | VTN |
| lipopolysaccharide binding protein | LBP | von Willebrand factor A domain containing 1 | VWA1 |
| lamin B receptor | LBR | von Willebrand factor A domain containing 2 | VWA2 |
| LCK proto-oncogene, Src family tyrosine kinase | LCK | von Willebrand factor A domain containing 3B | VWA3B |
| leucine carboxyl methyltransferase 1 | LCMT1 | von Willebrand factor | VWF |
| lipocalin 1 | LCN1 | tryptophanyl-tRNA synthetase | WARS |
| lipocalin 1 pseudogene 1 | LCN1P1 | Wiskott-Aldrich syndrome | WAS |
| lipocalin 2 | LCN2 | WAS protein family, member 1 | WASF1 |
| lymphocyte cytosolic protein 1 (L-plastin) | LCP1 | WAS protein family, member 2 | WASF2 |
| lymphocyte cytosolic protein 2 (SH2 domain containing leukocyte protein of 76kDa) | LCP2 | WAS protein family, member 3 | WASF3 |
| lactate dehydrogenase A | LDHA | Wiskott-Aldrich syndrome-like | WASL |
| lactate dehydrogenase A-like 6B | LDHAL6B | WW domain binding protein 2 | WBP2 |
| lactate dehydrogenase B | LDHB | WD repeat and FYVE domain containing 1 | WDFY1 |
| low density lipoprotein receptor | LDLR | WDFY family member 4 | WDFY4 |
| leptin receptor | LEPR | WD repeat domain 1 | WDR1 |
| leptin receptor overlapping transcript-like 1 | LEPROTL 1 | WD repeat domain 11 | WDR11 |
| leucine zipper-EF-hand containing transmembrane protein 1 | LETM1 | WD repeat domain 18 | WDR18 |
| LFNG O-fucosylpeptide 3-beta-N-acetylglucosaminyltransferase | LFNG | WD repeat domain 26 | WDR26 |
| lectin, galactoside-binding, soluble, 1 | LGALS1 | WD repeat domain 33 | WDR33 |
| lectin, galactoside-binding, soluble, 3 | LGALS3 | WD repeat domain 37 | WDR37 |
| lectin, galactoside-binding, soluble, 3 binding protein | LGALS3BP | WD repeat domain 4 | WDR4 |
| lectin, galactoside-binding, soluble, 4 | LGALS4 | WD repeat domain 44 | WDR44 |
| lectin, galactoside-binding, soluble, 7 | LGALS7 | WD repeat domain 48 | WDR48 |
| lectin, galactoside-binding, soluble, 7B | LGALS7B | WD repeat domain 49 | WDR49 |
| lectin, galactoside-binding, soluble, 8 | LGALS8 | WD repeat domain 5 | WDR5 |
| lectin, galactoside-binding, soluble, 9B | LGALS9B | WD repeat domain 61 | WDR61 |
| lectin, galactoside-binding-like | LGALSL | WD repeat domain 63 | WDR63 |
| leucine-rich repeat containing G protein-coupled receptor 4 | LGR4 | WD repeat domain 7 | WDR7 |
| leucine-rich repeat containing G protein-coupled receptor 6 | LGR6 | WD repeat domain 70 | WDR70 |
| lipoma HMGIC fusion partner-like 2 | LHFPL2 | WD repeat domain 77 | WDR77 |
| leukemia inhibitory factor | LIF | WD repeat domain 82 | WDR82 |
| ligase I, DNA, ATP-dependent | LIG1 | WD repeat domain 92 | WDR92 |
| ligase III, DNA, ATP-dependent | LIG3 | WD and tetratricopeptide repeats 1 | WDTC1 |
| LIM domain and actin binding 1 | LIMA1 | WEE1 G2 checkpoint kinase | WEE1 |
| Lck interacting transmembrane adaptor 1 | LIME1 | Wolf-Hirschhorn syndrome candidate 1 | WHSC1 |
| LIM and senescent cell antigen-like domains 1 | LIMS1 | WAS/WASL interacting protein family, member 1 | WIPF1 |
| LIM and senescent cell antigen-like domains 2 | LIMS2 | widely interspaced zinc finger motifs | WIZ |
| LIM and senescent cell antigen-like domains 3-like | LIMS3L | wingless-type MMTV integration site family, member 10B | WNT10B |
| lin-7 homolog A (C. elegans) | LIN7A | wingless-type MMTV integration site family, member 5A | WNT5A |
| lin-7 homolog C (C. elegans) | LIN7C | wingless-type MMTV integration site family, member 5B | WNT5B |
| long intergenic non-protein coding RNA 488 | LINC00488 | Werner helicase interacting protein 1 | WRNIP1 |
| leucine rich repeat and Ig domain containing 1 | LINGO1 | RAB6C-like | WTH3DI |
| lethal giant larvae homolog 1 (Drosophila) | LLGL1 | WW domain containing E3 ubiquitin protein ligase 1 | WWP1 |
| lethal giant larvae homolog 2 (Drosophila) | LLGL2 | WW domain containing E3 ubiquitin protein ligase 2 | WWP2 |
| lectin, mannose-binding, 1 | LMAN1 | XPA binding protein 2 | XAB2 |
| lectin, mannose-binding 2 | LMAN2 | xanthine dehydrogenase | XDH |
| LMBR1 domain containing 1 | LMBRD1 | X-prolyl aminopeptidase (aminopeptidase P) 1, soluble | XPNPEP1 |
| LIM and cysteine-rich domains 1 | LMCD1 | X-prolyl aminopeptidase (aminopeptidase P) 2, membrane-bound | XPNPEP2 |
| lamin A/C | LMNA | X-prolyl aminopeptidase 3, mitochondrial | XPNPEP3 |
| lamin B1 | LMNB1 | exportin 1 | XPO1 |
| lamin B2 | LMNB2 | exportin 4 | XPO4 |
| leucyl/cystinyl aminopeptidase | LNPEP | exportin 5 | XPO5 |
| small nuclear ribonucleoprotein Sm D1 pseudogene | LOC100129492 | exportin 6 | XPO6 |
| Ig kappa chain V-I region Walker-like | LOC100130100 | exportin 7 | XPO7 |
| related RAS viral (r-ras) oncogene homolog 2 pseudogene | LOC100133211 | exportin, tRNA | XPOT |
| LOC100499484-C9orf174 readthrough | LOC100499484-C9ORF174 | xenotropic and polytropic retrovirus receptor 1 | XPR1 |
| uncharacterized LOC100996720 | LOC100996720 | X-ray repair complementing defective repair in Chinese hamster cells 5 (double-strand-break rejoining) | XRCC5 |
| uncharacterized LOC100996740 | LOC100996740 | X-ray repair complementing defective repair in Chinese hamster cells 6 | XRCC6 |
| uncharacterized LOC101060400 | LOC101060400 | 5'-3' exoribonuclease 2 | XRN2 |
| DNA-directed RNA polymerase III subunit RPC5 | LOC101060521 | xylulokinase homolog (H. influenzae) | XYLB |
| 40S ribosomal protein S26 | LOC101929876 | tyrosyl-tRNA synthetase | YARS |
| uroplakin-3b-like protein-like | LOC102724187 | Y box binding protein 1 | YBX1 |
| histone H2B type F-S-like | LOC102724334 | Y box binding protein 2 | YBX2 |
| cystathionine beta-synthase | LOC102724560 | Y box binding protein 3 | YBX3 |
| splicing factor U2AF 35 kDa subunit | LOC102724594 | YES proto-oncogene 1, Src family tyrosine kinase | YES1 |
| alpha-crystallin A chain | LOC102724652 | Yip1 interacting factor homolog B (S. cerevisiae) | YIF1B |
| 40S ribosomal protein S8 pseudogene | LOC102724737 | Yip1 domain family, member 2 | YIPF2 |
| immunoglobulin superfamily member 3-like | LOC102724844 | Yip1 domain family, member 6 | YIPF6 |
| pyridoxal-dependent decarboxylase domain-containing protein 1 | LOC102724985 | YKT6 v-SNARE homolog (S. cerevisiae) | YKT6 |
| peptidylprolyl isomerase A (cyclophilin A) pseudogene | LOC128192 | YTH domain containing 2 | YTHDC2 |
| heat shock 70kDa protein 5 (glucose-regulated protein, 78kDa) pseudogene | LOC400750 | tyrosine 3-monooxygenase/tryptophan 5-monooxygenase activation protein, beta | YWHAB |
| uncharacterized LOC440786 | LOC440786 | tyrosine 3-monooxygenase/tryptophan 5-monooxygenase activation protein, epsilon | YWHAE |
| vitamin K epoxide reductase complex, subunit 1-like 1 pseudogene | LOC441241 | tyrosine 3-monooxygenase/tryptophan 5-monooxygenase activation protein, epsilon pseudogene 5 | YWHAEP5 |
| uncharacterized LOC442497 | LOC442497 | tyrosine 3-monooxygenase/tryptophan 5-monooxygenase activation protein, gamma | YWHAG |
| uncharacterized LOC642441 | LOC642441 | tyrosine 3-monooxygenase/tryptophan 5-monooxygenase activation protein, eta | YWHAH |
| telomeric repeat-binding factor 1 pseudogene | LOC646127 | tyrosine 3-monooxygenase/tryptophan 5-monooxygenase activation protein, theta | YWHAQ |
| IST1 homolog | LOC728533 | tyrosine 3-monooxygenase/tryptophan 5-monooxygenase activation protein, zeta | YWHAZ |
| exonuclease NEF-sp | LOC81691 | zeta-chain (TCR) associated protein kinase 70kDa | ZAP70 |
| Ion peptidase 1, mitochondrial | LONP1 | zinc finger and BTB domain containing 10 | ZBTB10 |
| lysyl oxidase-like 2 | LOXL2 | zinc finger and BTB domain containing 4 | ZBTB4 |
| lysyl oxidase-like 4 | LOXL4 | zinc finger and BTB domain containing 49 | ZBTB49 |
| lipoprotein, Lp(a) | LPA | zinc finger CCCH-type, antiviral 1 | ZC3HAV1 |
| lysophosphatidylcholine acyltransferase 3 | LPCAT3 | zinc finger CCCH-type, antiviral 1-like | ZC3HAV1 L |
| lysophosphatidylglycerol acyltransferase 1 | LPGAT1 | zinc finger, C4H2 domain containing | ZC4H2 |
| lipin 3 | LPIN3 | zinc finger, CCHC domain containing 11 | ZCCHC11 |
| lipoprotein lipase | LPL | zinc finger, DHHC-type containing 1 | ZDHHC1 |
| lactoperoxidase | LPO | zinc finger, DHHC-type containing 13 | ZDHHC13 |
| LIM domain containing preferred translocation partner in lipoma | LPP | zinc finger, DHHC-type containing 18 | ZDHHC18 |
| LPS-responsive vesicle trafficking, beach and anchor containing | LRBA | zinc finger, DHHC-type containing 20 | ZDHHC20 |
| leucine-rich alpha-2-glycoprotein 1 | LRG1 | zinc finger, DHHC-type containing 5 | ZDHHC5 |
| low density lipoprotein receptor-related protein 1 | LRP1 | zinc finger, AN1-type domain 5 | ZFAND5 |
| low density lipoprotein receptor-related protein 10 | LRP10 | zinc finger, C3H1-type containing | ZFC3H1 |
| low density lipoprotein receptor-related protein 1B | LRP1B | zinc finger protein, FOG family member 1 | ZFPM1 |
| low density lipoprotein receptor-related protein 2 | LRP2 | zinc finger, FYVE domain containing 26 | ZFYVE26 |
| low density lipoprotein receptor-related protein 4 | LRP4 | zymogen granule protein 16B | ZG16B |
| low density lipoprotein receptor-related protein 5 | LRP5 | zinc finger, GRF-type containing 1 | ZGRF1 |
| low density lipoprotein receptor-related protein 6 | LRP6 | zinc finger with KRAB and SCAN domains 5 | ZKSCAN5 |
| leucine-rich pentatricopeptide repeat containing | LRPPRC | zinc metallopeptidase STE24 | ZMPSTE24 |
| leucine rich repeat containing 1 | LRRC1 | zinc finger, MYM-type 1 | ZMYM1 |
| leucine rich repeat containing 15 | LRRC15 | zinc finger protein 114 | ZNF114 |
| leucine rich repeat containing 16A | LRRC16A | zinc finger protein 134 | ZNF134 |
| leucine rich repeat containing 26 | LRRC26 | zinc finger protein 169 | ZNF169 |
| leucine rich repeat containing 32 | LRRC32 | zinc finger protein 185 (LIM domain) | ZNF185 |
| leucine rich repeat containing 47 | LRRC47 | zinc finger protein 205 | ZNF205 |
| leucine rich repeat containing 57 | LRRC57 | zinc finger protein 217 | ZNF217 |
| leucine rich repeat containing 59 | LRRC59 | zinc finger protein 254 | ZNF254 |
| leucine rich repeat containing 75A | LRRC75A | zinc finger protein 28 | ZNF28 |
| leucine rich repeat containing 8 family, member A | LRRC8A | zinc finger protein 326 | ZNF326 |
| leucine rich repeat containing 8 family, member C | LRRC8C | zinc finger protein 33A | ZNF33A |
| leucine rich repeat containing 8 family, member D | LRRC8D | zinc finger protein 486 | ZNF486 |
| leucine rich repeat containing 8 family, member E | LRRC8E | zinc finger protein 503 | ZNF503 |
| leucine rich repeat (in FLII) interacting protein 1 | LRRFIP1 | zinc finger protein 518A | ZNF518A |
| leucine rich repeat (in FLII) interacting protein 2 | LRRFIP2 | zinc finger protein 541 | ZNF541 |
| leucine-rich repeat kinase 2 | LRRK2 | zinc finger protein 571 | ZNF571 |
| leucine rich repeat and sterile alpha motif containing 1 | LRSAM1 | zinc finger protein 614 | ZNF614 |
| LSM12 homolog (S. cerevisiae) | LSM12 | zinc finger protein 624 | ZNF624 |
| LSM2 homolog, U6 small nuclear RNA associated (S. cerevisiae) | LSM2 | zinc finger protein 638 | ZNF638 |
| LSM3 homolog, U6 small nuclear RNA associated (S. cerevisiae) | LSM3 | zinc finger protein 764 | ZNF764 |
| LSM6 homolog, U6 small nuclear RNA associated (S. cerevisiae) | LSM6 | zinc finger protein 792 | ZNF792 |
| lymphocyte-specific protein 1 | LSP1 | zinc finger protein 862 | ZNF862 |
| lipolysis stimulated lipoprotein receptor | LSR | zinc finger, HIT-type containing 6 | ZNHIT6 |
| lanosterol synthase (2,3-oxidosqualene-lanosterol cyclase) | LSS | zona pellucida glycoprotein 3 (sperm receptor) | ZP3 |
| leukocyte specific transcript 1 | LST1 | zw10 kinetochore protein | ZW10 |
| leukotriene A4 hydrolase | LTA4H | zyxin | ZYX |
| | | zinc finger, ZZ-type with EF-hand domain 1 | ZZEF1 |

## Claims

1. A method for producing synthetic extracellular vesicles comprising:
a) providing a water phase comprising at least two lipids, optionally one or more extracellular vesicle associated proteins or fragments thereof, and optionally one or more nucleic acid molecules, wherein the at least two lipids are a negative charged lipid, and a lipid coupled to a functional ligand for conjugation to an extracellular vesicle associated protein;
b) providing an amphiphilic copolymer dissolved in an oil phase;
c) combining said water phase and said oil phase;
d) producing polymer shell-stabilized synthetic extracellular vesicles by emulsifying the combined phases of step c) using a mechanic or electronic emulsifier;
wherein the amphiphilic copolymer forms a polymer shell stabilizing the synthetic extracellular vesicle, and
wherein the synthetic extracellular vesicles have a diameter between 70 nm and 5000 nm.

2. The method according to claim 1, further comprising the steps d') and e) after step d):
d') removing the polymer shell from the polymer shell-stabilized synthetic extracellular vesicles obtained in step d);
e) purifying the synthetic extracellular vesicles by centrifugation.

3. The method according to claim 1 or 2, wherein the water phase of step a) comprises at least one lipid coupled to a functional ligand selected from biotin, N-hydroxysuccinimide ester, N-Hydroxysulfosuccinimide, nitrilotriacetic acid - nickel, amine, pyridyl disulfide , pyridyldithiopropionate, carboxylic acid, maleimide, aromatic maleimid, dithiopyridinyl, N-benzylguanine, cyanuric chloride, thiol and
wherein the method optionally comprises after step e) the following step:
f) coupling the synthetic extracellular vesicles with at least one macromolecule comprising at least one moiety reacting with one of said functional ligands, wherein the macromolecule is selected from the group comprising an extracellular vesicle associated protein or a fragment thereof, a carbohydrate, a nucleic acid, a polypeptide, a cell receptor, an imaging probe.

4. The method according to any one of the claims 1 - 3, wherein the extracellular vesicle associated protein, or a fragment thereof, is selected from the group comprising:
a transmembrane protein selected from the group comprising tetraspanin proteins CD9, CD37, CD47, CD53, CD63, CD81, CD82, CD151, Tspan8, heterotrimeric G protein subunit alpha, integrin α - chains and β-chains, transferrin receptor 1, transferrin receptor 2, lysosome associated membrane proteins, heparan sulfate proteoglycans, syndecans, extracellular matrix metalloproteinase inducer, A Disintegrin And Metalloproteinase Domain 10, CD3, CD11c, CD14, CD29, CD31, CD41, CD42a, CD44, CD45, CD50 or intercellular adhesion molecule 1, CD55, CD59, CD73, CD80, CD86, CD90, sonic hedgehog, major histocompatibility complex I, major histocompatibility complex II, epidermal growth factor receptor 2, epithelial cell adhesion molecule, glycophorin A, Acetylcholinesterase S and E, amyloid beta precursor protein, multidrug resistance-associated protein 1, stem cells antigen-1, or a fragment thereof;
a cytosolic protein selected from the group comprising the protein complexes endosomal sorting complexes required for transport I, II and III, tumour susceptibility gene 101, charged multivesicular body protein, Apoptosis-Linked Gene 2-Interacting Protein X, vacuolar protein sorting 4 homolog A 4A and 4B, arrestin domain-containing protein, flotillin-1, flotillin-2; caveolins, EH-domain containing 1 - 4, ras homolog family member A, annexins, heat shock proteins, ADP-ribosylation factor 6, syntenin, microtubule-associated protein Tau, or a fragment thereof;
a functional protein selected from the group comprising cytokines, growth factors, interleukins, milk fat globule-EGF factor 8 protein , adhesion proteins, extracellular matrix proteins, nicotinamide phosphoribosyltransferase, signal transduction proteins, Wnta and Wntb, Fas, Fas Ligand, RANK, RANK Ligand, indolamin-2,3-dioxygenase, cytotoxic T-lymphocyte-associated protein 4 - immunoglobulin fusion protein, tumor necrosis factor-related apoptosis-inducing ligand, or a fragment thereof;
a protein associated to intracellular compartments selected from the group comprising histone proteins, lamin A/C, inner membrane mitochondrial protein, cytochrome C-1, mitochondrial import receptor subunit TOM20, calnexin, heat shock protein 90kDa beta, member 1, heat shock 70kDa protein 5, Golgin A2, Autophagy Related 9A, actinin1, actinin4, cytokeratin 18, or a fragment thereof.

5. The method according to any one of the claims 1 - 4, wherein the water phase of step a) comprises one or more nucleic acid molecules selected from the group comprising miRNA molecules miR-17, miR-18a, miR-19a, miR-19b-1, miR-20a, miR-92a; miR-21, miR-30d-5p, miR-33b, miR-124, miR-125, miR-126, miR-130, miR-132, miR-133b, miR-140-5p, miR-191, miR-222, miR-451, miR-494, miR-575, miR-630, miR-638, miR-1202, miR-1207-5p, miR-1225-5p, miR-1268, miR-6087, miR-92a-3p-e, miR-K12-3, let-7a.

6. The method according to any one of claims 1 - 5, wherein the water phase of step a) comprises at least two lipids selected from the group comprising:
a neutral lipid selected from the group comprising ceramide, sphingomyelin, cephalin, cholesterol, cerebrosides, diacylglycerols, phosphatidylcholines, lysophosphatidylcholines, phosphatidylethanolamines, lysophosphatidylethanolamine, lysoethanolamines, inverted headgroup lipids, sphingosins, sterol-modified phospholipids, ether ester lipids, diether lipids, vinyl ether, plasmalogen;
an anionic lipid selected from the group comprising phosphatidic acids, lysophosphatidic acid derivatives, phosphatidylglycerols, lysophosphatidylglycerols, phosphatidylserines, lysophosphatidylserines, phosphatidylinositols, phosphatidylinositolphosphates, cardiolipins, Bis(Monoacylglycero)Phosphate derivatives;
a cationic lipid selected from the group comprising dioleyl-N,N-dimethylammonium chloride; N-(2,3-dioleyloxy)propyl)-N,N,N-trimethylammonium chloride; N,N-distearyl-N,N-dimethylammonium bromide; N-(2,3- dioleyloxy)propyl)-N,N,N-trimethylammonium chloride; 3β-(N-(N',N'-dimethylaminoethane)-carbamoyl)cholesterol; 1,2-dimyristyloxypropyl-3-dimethyl-hydroxy ethyl ammonium bromide; 2,3-dioleyloxy-N-[2(sperminecarboxamido) ethyl]-N,N-dimethyl-1-propanaminium trifluoroacetate; dioctadecylamidoglycyl carboxyspermine; N-(2,3-dioleyloxy)propyl)-N,N-dimethylammonium chloride and 1,2-Dioleoyl-3-dimethylammonium-propane;
a pH- sensitive lipid selected from the group comprising lipid N-(4-carboxybenzyl)-N,N-dimethyl-2,3-bis(oleoyloxy)propan-1-aminium, 1,2-distearoyl-3-dimethylammonium-propane, 1,2-dipalmitoyl-sn-glycero-3-succinate, 1,2-dioleoyl-sn-glycero-3-succinate, N-palmitoyl homocysteine ;
a photoswitchable lipid;
acylglycine derivatives, prenol derivatives, prostaglandine derivatives, glycosylated diacyl glycerols, eicosanoid derivatives, (palmitoyloxy)octadecanoic acid derivatives, diacetylene derivatives, diphytanoyl derivatives, fluorinated lipids, brominated lipids, lipopolysaccharides;
one of the aforementioned lipids coupled to a functional ligand selected from the group comprising biotin, N-hydroxysuccinimide ester, nitrilotriacetic acid-nickel, amine, carboxylic acid, maleimides, dithiopyridinyl, pyridyl disulfide , pyridyldithiopropionate, N-benzylguanine, carboxyacyl, cyanur, folate, square, galloyl, glycan, thiol, arginylglycylaspartic acid, a fluorescent dye molecule, a magnetic resonance imaging reagent, a chelator;
one of the aforementioned lipids coupled to polyethyleneglycol with a molecular weight comprised between 350 and 50,000 g/mole.

7. The method according to any of the claims 1 - 6, wherein step d) comprises producing polymer shell stabilized synthetic extracellular vesicles by emulsifying the combined phases at step c) using a mechanic or electronic emulsifier for at least 5 seconds at speed higher than 1,000 rpm.

8. A synthetic extracellular vesicle having a diameter comprised between 70 and 5000 nm, comprising:
a lipid bilayer comprising at least two lipids selected from the group comprising:
a neutral lipid selected from the group comprising ceramide, sphingomyelin, cephalin, cholesterol, cerebrosides, diacylglycerols, phosphatidylcholines, lysophosphatidylcholines, phosphatidylethanolamines, lysophosphatidylethanolamine, lysoethanolamines, inverted headgroup lipids, sphingosins, sterol-modified phospholipids, ether ester lipids, diether lipids, vinyl ether (plasmalogen);
an anionic lipid selected from the group comprising phosphatidic acids, lysophosphatidic acid derivatives, phosphatidylglycerols, lysophosphatidylglycerols, phosphatidylserines, lysophosphatidylserines, phosphatidylinositols, phosphatidylinositolphosphates, cardiolipins, Bis(Monoacylglycero)Phosphate derivatives;
a cationic lipid selected from the group comprising dioleyl-N,N-dimethylammonium chloride; N-(2,3-dioleyloxy)propyl)-N,N,N-trimethylammonium chloride; N,N-distearyl-N,N-dimethylammonium bromide; N-(2,3- dioleyloxy)propyl)-N,N,N-trimethylammonium chloride; 3β-(N-(N',N'-dimethylaminoethane)-carbamoyl)cholesterol; 1,2-dimyristyloxypropyl-3-dimethyl-hydroxy ethyl ammonium bromide; 2,3-dioleyloxy-N-[2(sperminecarboxamido) ethyl]-N,N-dimethyl-1-propanaminium trifluoroacetate; dioctadecylamidoglycyl carboxyspermine; N-(2,3-dioleyloxy)propyl)-N,N-dimethylammonium chloride and 1,2-Dioleoyl-3-dimethylammonium-propane;
a pH- sensitive selected from the group comprising lipid N-(4-carboxybenzyl)-N,N-dimethyl-2,3-bis(oleoyloxy)propan-1-aminium, 1,2-distearoyl-3-dimethylammonium-propane, 1,2-dipalmitoyl-sn-glycero-3-succinate, 1,2-dioleoyl-sn-glycero-3-succinate, N-palmitoyl homocysteine;
a photoswitchable lipid;
acylglycine derivatives, prenol derivatives, prostaglandine derivatives, glycosylated diacyl glycerols, eicosanoid derivatives, (palmitoyloxy)octadecanoic acid derivatives, diacetylene derivatives, diphytanoyl derivatives, fluorinated lipids, brominated lipids, lipopolysaccharides;
one of the aforementioned lipids coupled to a functional ligand selected from the group comprising biotin, N-hydroxysuccinimide ester, nitrilotriacetic acid - nickel, amine, carboxylic acid, maleimides, dithiopyridinyl, pyridyl disulfide , pyridyldithiopropionate, N-benzylguanine, carboxyacyl, cyanur, folate, square, galloyl, glycan, thiol, arginylglycylaspartic acid, a fluorescent dye molecule, a magnetic resonance imaging reagent, a chelator;
one of the aforementioned lipids coupled to polyethyleneglycol with a molecular weight comprised between 350 and 50,000 g/mole.
optionally one or more nucleic acids selected from the group comprising DNA, cDNA, mRNA, siRNA, antisense nucleotides, shRNA, piRNA, snRNA, IncRNA, PNA, left handed DNA, Clustered Regularly Interspaced Short Palindromic Repeats guide RNA;
optionally one or more nucleic acids selected from the group comprising miRNA molecules miR-17, miR-18a, miR-19a, miR-19b-1, miR-20a, miR-92a; miR-21, miR-30d-5p, miR-33b, miR-124, miR-125, miR-126, miR-130, miR-132, miR-133b, miR-140-5p, miR-191, miR-222, miR-451, miR-494, miR-575, miR-630, miR-638, miR-1202, miR-1207-5p, miR-1225-5p, miR-1268, miR-6087, miR-92a-3p-e, miR-K12-3, let-7a.
one or more transmembrane proteins selected from the group comprising tetraspanin proteins CD9, CD37, CD47, CD53, CD63, CD81, CD82, CD151, Tspan8, heterotrimeric G protein subunit alpha, integrin α - chains and β-chains, transferrin receptor 1, transferrin receptor 2, lysosome associated membrane proteins, heparan sulfate proteoglycans, syndecans, extracellular matrix metalloproteinase inducer, A Disintegrin And Metalloproteinase Domain 10, CD3, CD11c, CD14, CD29, CD31, CD41, CD42a, CD44, CD45, CD50 or intercellular adhesion molecule 1, CD55, CD59, CD73, CD80, CD86, CD90, sonic hedgehog, major histocompatibility complex I, major histocompatibility complex II, epidermal growth factor receptor 2, epithelial cell adhesion molecule, Glycophorin A, Acetylcholinesterase S and E, amyloid beta precursor protein, multidrug resistance-associated protein 1, stem cells antigen-1, or a fragment thereof;
optionally one or more cytosolic proteins selected from the group comprising the protein complexes endosomal sorting complexes required for transport I, II and III, tumour susceptibility gene 101, charged multivesicular body protein, Apoptosis-Linked Gene 2-Interacting Protein X, vacuolar protein sorting 4 homolog A 4A and 4B, arrestin domain-containing protein, flotillin-1, flotillin-2; caveolins, EH-domain containing 1 - 4, ras homolog family member A, annexins, heat shock proteins, ADP-ribosylation factor 6, syntenin, microtubule-associated protein Tau, or a fragment thereof;
optionally one or more functional proteins selected from the group comprising cytokines, growth factors, interleukins, milk fat globule-EGF factor 8 protein,
adhesion proteins, extracellular matrix proteins, nicotinamide phosphoribosyltransferase, signal transduction proteins, Wnta and Wntb, Fas, Fas Ligand, RANK, RANK Ligand, indolamin-2,3-dioxygenase, cytotoxic T-lymphocyte-associated protein 4 - immunoglobulin fusion protein, tumor necrosis factor-related apoptosis-inducing ligand, or a fragment thereof;
optionally one or more proteins associated to intracellular compartments selected from the group comprising histone proteins, lamin A/C, inner membrane mitochondrial protein, cytochrome C-1, mitochondrial import receptor subunit TOM20, calnexin, heat shock protein 90kDa beta, member 1, heat shock 70kDa protein 5, Golgin A2, Autophagy Related 9A, actinin1, actinin4, cytokeratin 18, or a fragment thereof.

9. The synthetic extracellular vesicle according to claim 8, comprising:
a lipid bilayer comprising cholesterol, N-stearoyl-D-erythro-sphingosylphosphorylcholine, 1,2-dioleoyl-sn-glycero-3-phosphocholine, 1,2-dioleoyl-sn-glycero-3-phospho-L-serine, 1,2-dioleoyl-sn-glycero-3-phospho-ethanolamine, 1,2-dioleoyl-sn-glycero-3-phospho-rac-(1-glycerol), 1,2-dioleoyl-sn-glycero-3-phosphate (sodium salt), diacylglycerol, phosphatidylinositol, 1,2-dioleoyl-sn-glycero-3-phosphoethanolamine-N-(lissamine rhodamine B sulfonyl), 1,2-dioleoyl-sn-glycero-3-[(N-(5-amino-1-carboxypentyl)iminodiacetic acid) succinyl] (nickel salt),
one or more nucleic acid molecules selected from the group comprising miRNA miR-21, miR-124, miR-125, miR-126, miR-130 and miR-132, and
one or more transmembrane proteins selected from the group comprising tetraspanin proteins CD9, CD63 and CD81, or a fragment thereof.

10. The synthetic extracellular vesicle according to claim 8 or 9, comprising:
one or more functional protein nicotinamide phosphoribosyltransferase, or a fragment thereof;
one or more transmembrane proteins selected from the group comprising tetraspanin proteins CD9, CD63 and CD81, or a fragment thereof;
one or more cytosolic proteins selected from the group comprising Apoptosis-Linked Gene 2-Interacting Protein X, tumour susceptibility gene 101 protein, or a fragment thereof;
wherein the synthetic extracellular vesicle does not comprise transferrin and albumin.

11. The synthetic extracellular vesicle according to any one of the claims 8 - 10, comprising:
one or more transmembrane proteins selected from the group comprising MHCII, CD80, and CD86;
optionally one or more transmembrane proteins selected from the group comprising CD11c, MHC I, integrin α and β-chains, intercellular adhesion molecule-1, and CD71, or a fragment thereof;
one or more functional proteins selected from the group comprising cytokines, interleukins, interleukin 4, milk fat globule-EGF factor 8 protein, growth factors, Fas, Fas Ligand, indolamin-2,3-dioxygenase, cytotoxic T-lymphocyte-associated protein 4 - immunoglobulin fusion protein, tumor necrosis factor-related apoptosis-inducing ligand, or a fragment thereof.

12. The synthetic extracellular vesicle according to any one of the claims 8 - 11, comprising:
a lipid bilayer comprising 1,2-dioleoyl-sn-glycero-3-phosphocholine, 1,2-dioleoyl-sn-glycero-3-phospho-rac-(1-glycerol), 1,2-dioleoyl-sn-glycero-3-phosphoethanolamine-N-(lissamine rhodamine B sulfonyl), 1,2-dioleoyl-sn-glycero-3-[(N-(5-amino-1-carboxypentyl)iminodiacetic acid) succinyl] (nickel salt);
functional protein Fas Ligand, or a fragment thereof;
optionally functional protein intercellular adhesion protein - 1, or a fragment thereof.

13. The synthetic extracellular vesicle according to any one of the claims 8 - 12, comprising:
one or more transmembrane proteins selected from the group comprising CD29, CD44, CD90, CD73, Sca-1, or a fragment thereof;
one or more transmembrane proteins selected from the group comprising tetraspanin proteins CD9, CD63, and CD81, or a fragment thereof;
one or more functional proteins selected from the group comprising Wnta and Wntb, or a fragment thereof;
at least one nucleic acid molecule selected from the group comprising miR-140-5p, miR-92a-3p-e;
one or more nucleic acid molecules selected from the group comprising miR-17, miR-18a, miR-19a, miR-19b-1, miR-20a, miR-92a, let-7a, miR-21, miR124, miR126, miR-133b, miR-191, miR-222, miR-494, miR-6087, miR-30d-5p;
optionally one or more nucleic acid molecules selected from the group comprising miR-33b, miR-451, miR-575, miR-630, miR-638, miR-1202, miR-1207-5p, miR-1225-5p, miR-1268, miR-K12-3.

14. The synthetic extracellular vesicle according to any one of the claims 8 - 13, comprising:
a lipid bilayer comprising 1,2-dioleoyl-sn-glycero-3-phosphocholine, 1,2-dioleoyl-sn-glycero-3-phospho-rac-(1-glycerol), 1,2-dioleoyl-sn-glycero-3-phosphoethanolamine-N-(lissamine rhodamine B sulfonyl), 1,2-dioleoyl-sn-glycero-3-[(N-(5-amino-1-carboxypentyl)iminodiacetic acid) succinyl] (nickel salt),
functional protein RANK, or a fragment thereof.

15. The synthetic extracellular vesicle according to any one of the claims 8 - 14 for use in the treatment of a disorder selected from the group comprising inflammation, cancer, rheumatic disorder, osteoarthritis, cardiovascular disorder, epithelial diseases, neurodegenerative disorders, autoimmune disorders, bone and cartilage disorders, osteoporosis, renal osteodystrophy, paget's disease of bone, osteopetrosis, rickets, neurological disorders, intoxication, neuroendocrinology disorders, endocrinology disorders, genetic disorders, infectious diseases, dental disorders, cosmetic procedures, coagulation disorders, dermatoses, diabetes, age-associated disorders.
